# EUROPEAN PATENT APPLICATION

(11) **EP 4 674 852 A1**
(43) Date of publication of application: **07.01.2026**
(21) Application number: 24763173.2
(22) Date of filing: 28.02.2024
(51) Int. Cl.: C07D 495/04, C07D 519/00, A61K 31/519, A61P 11/00, A61P 11/04, A61P 1/00, A61P 35/00, A61P 37/06

(54) **PREPARATION METHOD FOR AND USE OF DIHYDROTHIENOPYRIMIDINE DERIVATIVE**

(30) Priority: 28.02.2023 CN 202310183376; 25.08.2023 CN 202311082792
(71) Applicant: Innovstone Therapeutics Limited, Shanghai 201203 (CN)
(72) Inventor: SONG, Yunlong, Shanghai 201203 (CN); WANG, Feng, Shanghai 201203 (CN); WANG, Pengpeng, Shanghai 201203 (CN); MU, Yongzhao, Shanghai 201203 (CN); WANG, Disha, Shanghai 201203 (CN); KOU, Hongyan, Shanghai 201203 (CN); YU, Ning, Shanghai 201203 (CN); LU, Kai, Shanghai 201203 (CN); LEI, Xiang, Shanghai 201203 (CN); XU, Dengyun, Shanghai 201203 (CN); CHEN, Jian, Shanghai 201203 (CN); LI, Wei, Shanghai 201203 (CN); ZHANG, Kun, Shanghai 201203 (CN); XIE, Xingxing, Shanghai 201203 (CN)
(74) Representative: Barrow, Nicholas Martin
(86) International application number: PCT/CN2024/078912
(87) International publication number: WO 2024/179493

(57) **Abstract**

The present invention provides a compound having a new structure as a PDE4B inhibitor, a preparation method for the compound, and a use of the compound in treating PDE4B-mediated diseases. Experiments confirm that the compound has a strong inhibitory effect on PDE4B enzyme and can be used as a promising compound for treating PDE4B-mediated diseases.

## Description

### Technical Field

The present invention relates to the field of medical technology, specifically to compounds as PDE4B inhibitors, in particular to preparation methods and uses of substituted dihydrothieno azaaryl derivatives and prodrugs thereof.

### Background of Art

Phosphodiesterases (PDEs) have the function of hydrolyzing intracellular second messengers (cAMP, cyclic adenosine monophosphate or cGMP, cyclic guanosine monophosphate), degrading intracellular cAMP or cGMP, thereby terminating the biochemical effects transmitted by these second messengers. cAMP and cGMP play important regulatory roles in cellular activities, and the regulation of their concentrations is primarily determined by the balance between synthesis by nucleotide cyclases and hydrolysis by phosphodiesterases (PDEs). PDEs are widely distributed in the human body, and their physiological actions involve multiple research areas. Among them, human PDE4 isoenzymes are divided into four subtypes: PDE4A, 4B, 4C, and 4D. PDE4 is associated with cAMP hydrolysis in various inflammatory cells; therefore, inhibiting PDE4 can suppress immune and inflammatory cells. Additionally, according to literature reports, PDE4 plays a role in controlling the calcium-induced calcium release process, and preferentially inhibiting PDE4B can maintain therapeutic efficacy for pulmonary fibrosis. Therefore, selective inhibitors of PDE4B are expected to become effective drugs for treating idiopathic pulmonary fibrosis and progressive fibrosing interstitial lung diseases through dual inhibition of inflammation and fibrosis.

### Summary of the Invention

An object of the present invention is to provide a novel PDE4B inhibitor compound, a preparation method for said compound, and its use in treating PDE4B-mediated diseases. The novel compound is biochemically effective and physiologically active, and possesses good pharmacokinetic properties and low toxicological characteristics. The first aspect of the present invention provides a compound represented by the following formula (I), a stereoisomer or tautomer of said compound, a mixture thereof, or a pharmaceutically acceptable salt of said compound: 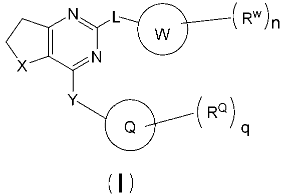
wherein, X is selected from S, SO, and SO₂;
Y is NR^{Y};
R^{Y} is hydrogen or deuterium;
ring Q is selected from C₃₋₈cycloalkyl, C₄₋₈cycloalkenyl, 3-10 membered heterocyclyl, 5-6 membered heteroaryl, and C₆₋₁₂ aryl;
each R^{Q} is selected from halogen, oxo, formyl, acetyl, methylsulfonyl, ethylsulfonyl, methylsulfonamido, ethylsulfonamido, phosphate, carboxylate, -CN, -OH, -SH, -NO₂, -NH₂, -CONH₂, 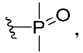 -L^{Y}-OH, -L^{Y}-SH, - L^{Y}-C(O)OH, -L^{Y}-NH-C(O)H, -L^{Y}-C(O)NH₂, -L^{Y}-NHC(O)OH, -L^{Y}-C(O)H, 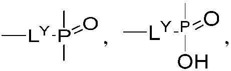 C₁₋₆alkyl, C₂₋₆alkenyl, C₂₋₆alkynyl, C₁₋₆alkoxyl, C₁₋₆alkylthio, C₃₋₆cycloalkyl, C₄₋₆cycloalkenyl, 3-6 membered heterocyclyl, haloC₁₋₃alkyl, -L^{Y}-OC(O)R^{d}, and -L^{Y}-OC(O)OR^{d}; preferably, each R^{Q} is selected from halogen, oxo, formyl, acetyl, methylsulfonyl, ethylsulfonyl, methylsulfonamido, ethylsulfonamido, phosphate, carboxylate, -CN, -OH, -SH, - NO₂, -NH₂, -CONH₂, 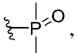 -L^{Y}-OH, -L^{Y}-SH, -L^{Y}-C(O)OH, -L^{Y}-NH-C(O)H, -L^{Y}-C(O)NH₂, -L^{Y}-NHC(O)OH, -L^{Y}-C(O)H, 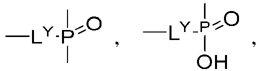 C₁₋₆alkyl, C₂₋₆alkenyl, C₂₋₆alkynyl, C₁₋₆alkoxyl, C₁₋₆alkylthio, C₃₋₆cycloalkyl, C₄₋₆cycloalkenyl, 3-6 membered heterocyclyl, haloC₁₋₃alkyl, and -L^{Y}-OC(O)R^{d}; more preferably, each R^{Q} is selected from halogen, oxo, formyl, acetyl, methylsulfonyl, ethylsulfonyl, methylsulfonamido, ethylsulfonamido, phosphate, carboxylate, -CN, -OH, -SH, -NO₂, -NH₂, -CONH₂, 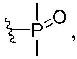 -L^{Y}-OH, -L^{Y}-SH, -L^{Y}-C(O)OH, -L^{Y}-NH-C(O)H, -L^{Y}-C(O)NH₂, -L^{Y}-NHC(O)OH, -L^{Y}-C(O)H, 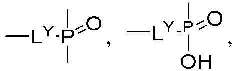 C₁₋₆alkyl, C₂₋₆alkenyl, C₂₋₆alkynyl, C₁₋₆alkoxyl, C₁₋₆alkylthio, C₃₋₆cycloalkyl, C₄₋₆cycloalkenyl, 3-6 membered heterocyclyl, and haloC₁₋₃alkyl;
L^{Y} is optionally substituted and selected from C₁₋₄alkylene, C₂₋₆alkenylene, C₂₋₆alkynylene, and C₁₋₄alkyleneoxy, wherein "optionally substituted" refers to being optionally substituted by one or more substituents selected from halogen, oxo, -CN, -OH, -SH, -NO₂, -NH₂, C₁₋₃alkyl, and haloC₁₋₃alkyl;
L is a bond or an optionally substituted group selected from C₁₋₄alkylene, C₂₋₄alkenylene, C₂₋₄alkynylene, and C₁₋₄alkyleneoxy, wherein "optionally substituted" refers to being optionally substituted by one or more substituents selected from deuterium, halogen, oxo, -CN, -OH, -SH, -NO₂, -NH₂, C₁₋₆alkyl, C₂₋₆alkenyl, C₂₋₆alkynyl, C₁₋₆alkoxyl, C₁₋₆alkylthio, C₃₋₈cycloalkyl, and haloC₁₋₆alkyl;
ring W is C₄₋₁₆cycloalkyl, C₄₋₁₆cycloalkenyl, saturated or partially unsaturated 4-18 membered heterocyclyl, 5-16 membered heteroaryl, or C₆₋₁₈ aryl;
each R^{W} is independently selected from halogen, oxo, formyl, acetyl, methylsulfonyl, ethylsulfonyl, methylsulfonamido, ethylsulfonamido, acetylamino, -CN, -OH, -SH, -NO₂, -NH₂, -CONH₂, -Z-(R^{Z})ₘ, -NR^{b}R^{c}, - C(O)OR^{d}, and an optionally substituted group selected from C₁₋₆alkyl, C₂₋₆alkenyl, C₂₋₆alkynyl, C₁₋₆alkoxyl, and C₁₋₆alkylthio, wherein "optionally substituted" refers to being optionally substituted by one or more substituents selected from deuterium, halogen, oxo, oxime, -CN, -OH, -SH, -NO₂, -NH₂, -C(O)OR^{d}, C₁₋₆alkyl, C₂₋₆alkenyl, C₂₋₆alkynyl, C₁₋₆alkoxyl, C₁₋₆alkylthio, C₃₋₈cycloalkyl, C₃₋₈cycloalkenyl, 3-8 membered heterocyclyl, 5-6 membered heteroaryl, C₆₋₁₂ aryl, haloC₁₋₆alkyl, haloC₁₋₆alkoxyl, halophenyl, and -O-C₃₋₆cycloalkyl; preferably, each R^{W} is independently selected from halogen, oxo, formyl, acetyl, methylsulfonyl, ethylsulfonyl, methylsulfonamido, ethylsulfonamido, -CN, -OH, -SH, -NO₂, -NH₂, -CONH₂, -Z-(R^{Z})ₘ, and an optionally substituted group selected from C₁₋₆alkyl, C₂₋₆alkenyl, C₂₋₆alkynyl, C₁₋₆alkoxyl, and C₁₋₆alkylthio, wherein "optionally substituted" refers to being optionally substituted by one or more substituents selected from halogen, oxo, oxime, -CN, -OH, -SH, -NO₂, -NH₂, C₁₋₆alkyl, C₂₋₆alkenyl, C₂₋₆alkynyl, C₁₋₆alkoxyl, C₁₋₆alkylthio, C₃₋₈cycloalkyl, C₃₋₈cycloalkenyl, 3-8 membered heterocyclyl, 5-6 membered heteroaryl, C₆₋₁₂ aryl, haloC₁₋₆alkyl, haloC₁₋₆alkoxyl, halophenyl, and -O-C₃₋₆cycloalkyl;
R^{b} and R^{c} are each independently selected from H, C₁₋₆alkyl, C₂₋₆alkenyl, C₁₋₆hydroxyalkyl, and haloC₁₋₆alkyl; R^{d} is selected from H, C₁₋₆alkyl, C₁₋₆hydroxyalkyl, haloC₁₋₆alkyl, and C₁₋₆alkylamino; preferably, R^{d} is selected from H, C₁₋₆alkyl, C₁₋₆hydroxyalkyl, and haloC₁₋₆alkyl;
Z is C₄₋₁₆cycloalkyl, C₄₋₁₆cycloalkenyl, saturated or partially unsaturated 4-18 membered heterocyclyl, 5-16 membered heteroaryl, or C₆₋₁₂ aryl;
R^{Z} is each independently selected from halogen, -CN, -OH, -SH, -NO₂, -NH₂, oxo, C₁₋₆alkyl, C₂₋₆alkenyl, C₂₋₆alkynyl, C₁₋₆alkoxyl, C₁₋₆hydroxyalkyl, haloC₁₋₆alkyl, haloC₁₋₆alkoxyl, saturated or partially unsaturated 4-6 membered heterocyclyl, -OR^{Z1}, and -NR^{Z2}R^{Z3}; R^{Z1} is selected from C₁₋₆alkyl and haloC₁₋₆alkyl; R^{Z2} and R^{Z3} are each independently selected from H and C₁₋₆alkyl; preferably, R^{Z} is each independently selected from halogen, - CN, -OH, -SH, -NO₂, -NH₂, oxo, C₁₋₆alkyl, C₂₋₆alkenyl, C₂₋₆alkynyl, C₁₋₆alkoxyl, C₁₋₆hydroxyalkyl, haloC₁₋₆alkyl, and haloC₁₋₆alkoxyl; more preferably, R^{Z} is each independently selected from halogen, -CN, -OH, -SH, -NO₂, - NH₂, C₁₋₆alkyl, C₂₋₆alkenyl, C₂₋₆alkynyl, C₁₋₆alkoxyl, C₁₋₆hydroxyalkyl, haloC₁₋₆alkyl, and haloC₁₋₆alkoxyl;
n is 0, 1, 2, 3, 4 or 5;
q is 0, 1, 2, 3, 4 or 5;
m is 0, 1, 2, 3, 4 or 5;
provided that:
   (1) when L is a bond, 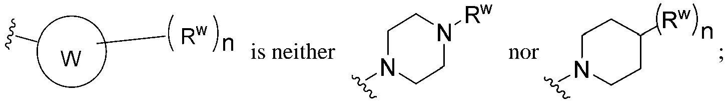
   (2) when L is a bond, and 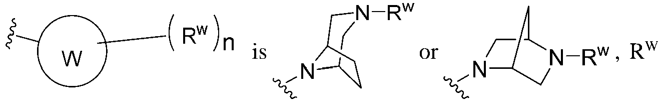 is neither a substituted phenyl nor 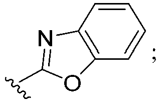
   (3) when X is SO, and L is a bond, 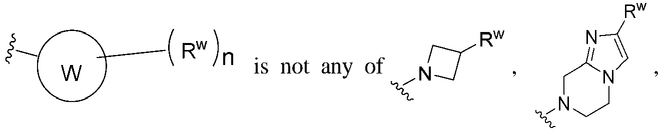 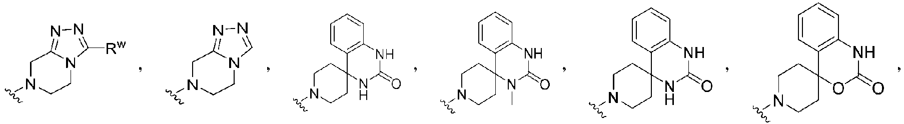 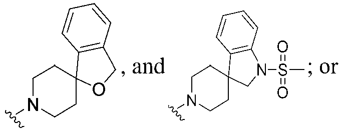
   (3') when X is SO, and L is a bond, 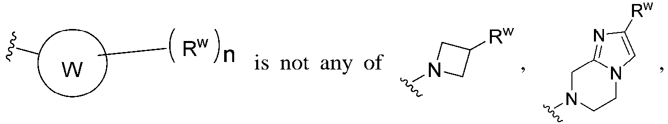 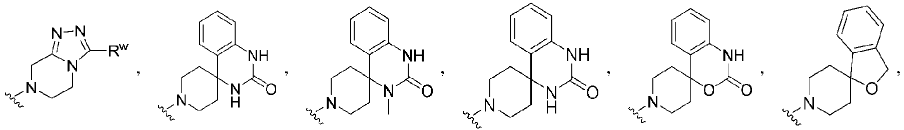 and 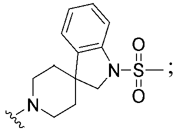
   (4) when X is S or SO₂, and L is a bond, ring Q and ring W are not a substituted phenyl at the same time; or when X is S, L is a bond, and ring Q is phenyl, ring W is not 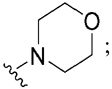 and
   (5) when X is S or SO₂, and L is a bond, ring Q is not

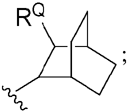

The heteroatoms in the heterocyclyl and heteroaryl are independently selected from O, N and S, and the number of heteroatoms is preferably 1, 2 or 3.

In a preferable embodiment of the present invention, R^{Y} is deuterium;
In a preferable embodiment of the present invention, R^{Y} is hydrogen;
In a preferable embodiment of the present invention, Y is NH;
In a preferable embodiment of the present invention, X is S;
In a preferable embodiment of the present invention, X is SO;
In a preferable embodiment of the present invention, X is SO₂;
In a preferable embodiment of the present invention, ring Q is selected from C₄₋₆cycloalkyl, C₄₋₆cycloalkenyl, 3-8 membered heterocyclyl, 5-6 membered heteroaryl, and phenyl;
In a preferable embodiment of the present invention, ring Q is selected from C₄₋₆cycloalkyl, 3-6 membered heterocyclyl, and phenyl;
In a preferable embodiment of the present invention, ring Q is selected from cyclobutyl, cyclohexenyl, tetrahydrofuranyl, tetrahydropyrrolyl, piperidinyl, piperazinyl, phenyl, and tetrahydropyranyl;
In a preferable embodiment of the present invention, each R^{Q} is independently selected from halogen, oxo, formyl, acetyl, methylsulfonyl, ethylsulfonyl, methylsulfonamido, ethylsulfonamido, phosphate, carboxylate, -CN, -OH, - SH, -NO₂, -NH₂, -CONH₂, 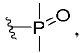 -L^{Y}-OH, -L^{Y}-C(O)OH, -L^{Y}-C(O)H, 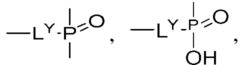 C₁₋₃alkyl, C₂₋₄alkenyl, C₂₋₄alkynyl, C₁₋₃alkoxyl, C₁₋₃alkylthio, C₃₋₆cycloalkyl, C₃₋₆cycloalkenyl, 3-6 membered heterocyclyl, haloC₁₋₃alkyl, -L^{Y}-OC(O)R^{d}, and -L^{Y}-OC(O)OR^{d};
In a preferable embodiment of the present invention, each R^{Q} is independently selected from halogen, oxo, acetyl, 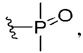 -L^{Y}-OH, -L^{Y}-C(O)OH, C₁₋₃alkyl, C₃₋₆cycloalkyl, 3-6 membered heterocyclyl, -L^{Y}-OC(O)R^{d}, and -L^{Y}-OC(O)OR^{d};
In a preferable embodiment of the present invention, each R^{Q} is independently selected from halogen, oxo, acetyl, 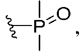 -L^{Y}-OH, -L^{Y}-C(O)OH, C₁₋₃alkyl, C₃₋₆cycloalkyl, -L^{Y}-OC(O)R^{d}, and -L^{Y}-OC(O)OR^{d};
In a preferable embodiment of the present invention, each R^{Q} is independently selected from hydroxymethyl, carboxymethyl, acetyl, 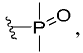 hydroxyisopropyl, Cl, oxo, cyclopropyl, 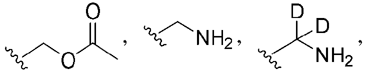 and 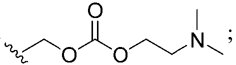
In a preferable embodiment of the present invention, L^{Y} is optionally substituted and selected from C₁₋₄alkylene, wherein "optionally substituted" refers to being optionally substituted by one or more substituents selected from halogen, oxo, -CN, -OH, -SH, -NO₂, -NH₂, C₁₋₃alkyl, and haloC₁₋₃alkyl;
In a preferable embodiment of the present invention, L^{Y} is optionally substituted and selected from C₁₋₂alkylene, wherein "optionally substituted" refers to being optionally substituted by one or more substituents selected from halogen, -OH, -SH, -NH₂, C₁₋₃alkyl, and haloC₁₋₃alkyl;
In a preferable embodiment of the present invention, L^{Y} is optionally substituted and selected from methylene and ethylene, wherein "optionally substituted" refers to being optionally substituted by one or more substituents selected from halogen, -OH, -SH, methyl, and ethyl;
In a preferable embodiment of the present invention, 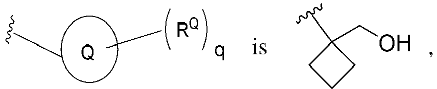 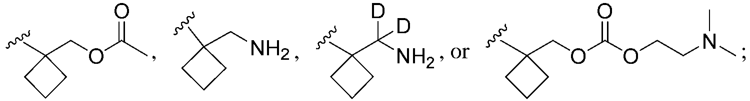
In a preferable embodiment of the present invention, 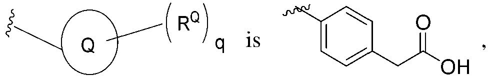 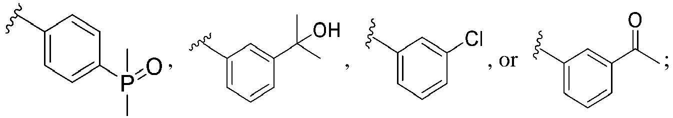
In a preferable embodiment of the present invention, 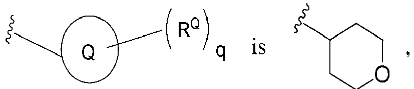 or 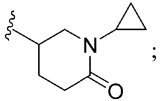
In a preferable embodiment of the present invention, L is a bond or an optionally substituted group selected from: C₁₋₃alkylene, C₂₋₄alkenylene, and C₂₋₄alkynylene, wherein "optionally substituted" refers to being optionally substituted by one or more substituents selected from deuterium, halogen, oxo, -CN, -OH, -SH, -NH₂, C₁₋₃alkyl, C₂₋₄alkenyl, C₂₋₄alkynyl, C₁₋₃alkoxyl, C₁₋₃alkylthio, C₃₋₈cycloalkyl, and haloC₁₋₃alkyl;
In a preferable embodiment of the present invention, L is a bond or an optionally substituted group selected from: C₂₋₄alkenylene and C₂₋₄alkynylene, wherein "optionally substituted" refers to being optionally substituted by one or more substituents selected from deuterium, -CN, -OH, -SH, -NH₂, and C₁₋₃alkyl;
In a preferable embodiment of the present invention, L is a bond or an optionally substituted group selected from: ethenyl, propenyl, ethynyl, and propynyl, wherein "optionally substituted" refers to being optionally substituted by one or more substituents selected from deuterium, -CN, -OH, -SH, -NH₂, and C₁₋₃alkyl;
In a preferable embodiment of the present invention, L is a bond;
In a preferable embodiment of the present invention, L is ethynyl;
In a preferable embodiment of the present invention, L is an optionally substituted propynyl, wherein "optionally substituted" refers to being optionally substituted by one or more substituents selected from deuterium, -CN, -OH, -SH, -NH₂, and C₁₋₃alkyl;
In a preferable embodiment of the present invention, ring W is C₄₋₁₂cycloalkyl, C₄₋₁₂cycloalkenyl, saturated or partially unsaturated 4-12 membered heterocyclyl, 5-16 membered heteroaryl, or C₆₋₁₂ aryl;
In a preferable embodiment of the present invention, ring W is C₄₋₁₀monocyclic cycloalkyl, C₅₋₁₂bicyclic cycloalkyl, C₉₋₁₂tricyclic cycloalkyl, C₄₋₁₀monocyclic cycloalkenyl, C₇₋₁₂bicyclic cycloalkenyl, C₉₋₁₂tricyclic cycloalkenyl, saturated or partially unsaturated 4-12 membered monocyclic heterocyclyl, saturated or partially unsaturated 7-12 membered bicyclic heterocyclyl, saturated or partially unsaturated 9-12 membered tricyclic heterocyclyl, 5-8 membered monocyclic heteroaryl, 9-16 membered bicyclic heteroaryl, 9-16 membered tricyclic heteroaryl, phenyl, or naphthyl;
In a preferable embodiment of the present invention, ring W is C₄₋₅monocyclic cycloalkyl, C₅₋₁₀bicyclic cycloalkyl, C₄₋₈ monocyclic cycloalkenyl, C₇₋₁₀bicyclic cycloalkenyl, saturated or partially unsaturated 4-8 membered monocyclic heterocyclyl, saturated or partially unsaturated 7-12 membered bicyclic heterocyclyl, 5-6 membered monocyclic heteroaryl, 9-16 membered bicyclic heteroaryl, phenyl, or naphthyl;
In a preferable embodiment of the present invention, ring W is 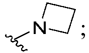
In a preferable embodiment of the present invention, ring W is 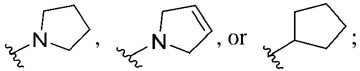
In a preferable embodiment of the present invention, ring W is 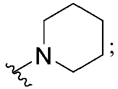
In a preferable embodiment of the present invention, ring W is 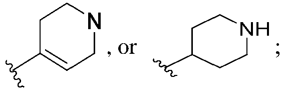
In a preferable embodiment of the present invention, ring W is 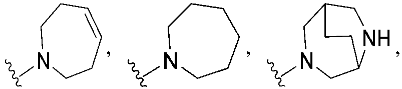 or 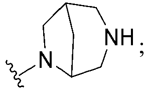
In a preferable embodiment of the present invention, ring W is 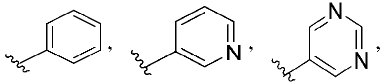 or 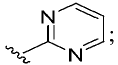
In a preferable embodiment of the present invention, ring W is 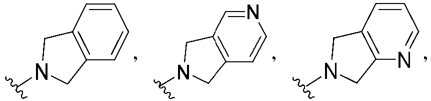 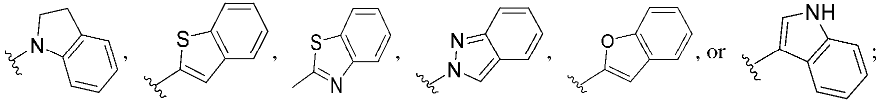
In a preferable embodiment of the present invention, ring W is 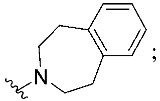
In a preferable embodiment of the present invention, ring W is 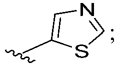
In a preferable embodiment of the present invention, ring W is 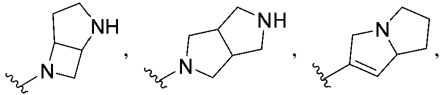 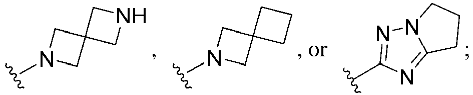
In a preferable embodiment of the present invention, 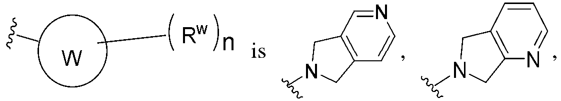 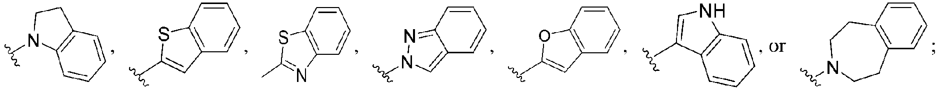
In a preferable embodiment of the present invention, 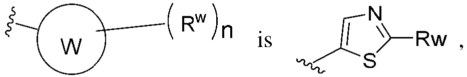 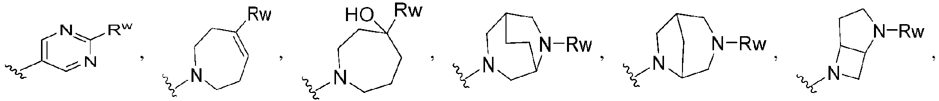 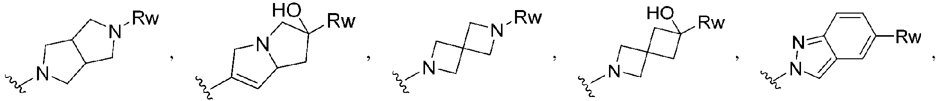 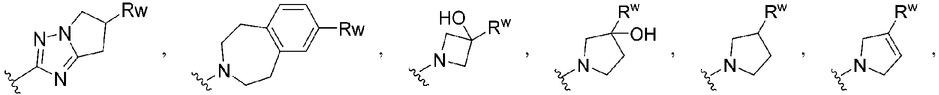 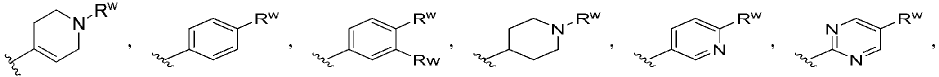 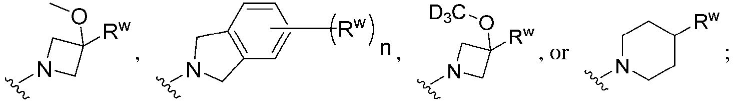
In a preferable embodiment of the present invention, 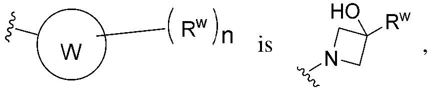 or 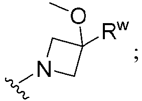
In a preferable embodiment of the present invention, each R^{W} is independently selected from halogen, oxo, formyl, acetyl, methylsulfonyl, ethylsulfonyl, methylsulfonamido, ethylsulfonamido, acetylamino, -CN, -OH, -SH, -NO₂, -CONH₂, -Z-(R^{Z})ₘ, -NR^{b}R^{c}, -C(O)OR^{d}, an optionally substituted C₁₋₆alkyl, and an optionally substituted C₁₋₆alkoxyl, wherein "optionally substituted" refers to being optionally substituted by one or more substituents selected from deuterium, halogen, oxo, oxime, -CN, -OH, -SH, -NO₂, -NH₂, C₁₋₆alkyl, C₂₋₆alkenyl, C₂₋₆alkynyl, C₁₋₆alkoxyl, C₁₋₆alkylthio, C₃₋₈cycloalkyl, C₃₋₈cycloalkenyl, 3-8 membered heterocyclyl, 5-6 membered heteroaryl, C₆₋₁₂ aryl, haloC₁₋₆alkyl, haloC₁₋₆alkoxyl, halophenyl, and -O-C₃₋₆cycloalkyl;
R^{b} and R^{c} are each independently selected from H, C₁₋₃alkyl, C₁₋₃hydroxyalkyl, and haloC₁₋₃alkyl;
R^{d} is selected from H, C₁₋₃alkyl, C₁₋₃hydroxyalkyl, and haloC₁₋₃alkyl;
In a preferable embodiment of the present invention, each R^{W} is independently selected from halogen, oxo, formyl, acetyl, methylsulfonyl, ethylsulfonyl, methylsulfonamido, ethylsulfonamido, -CN, -OH, -SH, -NO₂, -NH₂, - CONH₂, -Z-(R^{Z})ₘ, an optionally substituted C₁₋₆alkyl, and an optionally substituted C₁₋₆alkoxyl, wherein "optionally substituted" refers to being optionally substituted by one or more substituents selected from halogen, oxo, oxime, -CN, -OH, -SH, -NO₂, -NH₂, C₁₋₆alkyl, C₂₋₆alkenyl, C₂₋₆alkynyl, C₁₋₆alkoxyl, C₁₋₆alkylthio, C₃₋₈cycloalkyl, C₃₋₈cycloalkenyl, 3-8 membered heterocyclyl, 5-6 membered heteroaryl, C₆₋₁₂ aryl, haloC₁₋₆alkyl, haloC₁₋₆alkoxyl, halophenyl, and -O-C₃₋₆cycloalkyl;
Z is C₄₋₁₀cycloalkyl, C₄₋₁₀cycloalkenyl, saturated or partially unsaturated 4-12 membered heterocyclyl, 5-12 membered heteroaryl, or C₆₋₁₂ aryl;
R^{Z} is each independently selected from halogen, -CN, -OH, -SH, -NO₂, -NH₂, C₁₋₃alkyl, C₂₋₆alkenyl, C₂₋₆alkynyl, C₁₋₃alkoxyl, C₁₋₃hydroxyalkyl, haloC₁₋₃alkyl, haloC₁₋₃alkoxyl, saturated or partially unsaturated 4-6 membered heterocyclyl, -OR^{Z1}, and -NR^{Z2}R^{Z3};
R^{Z1} is C₁₋₃alkyl or haloC₁₋₃alkyl;
R^{Z2} and R^{Z3} are each independently selected from H, and C₁₋₃alkyl;
In a preferable embodiment of the present invention, each R^{W} is independently selected from halogen, oxo, formyl, acetyl, methylsulfonyl, ethylsulfonyl, methylsulfonamido, ethylsulfonamido, -CN, -OH, -SH, -NO₂, -NH₂, - CONH₂, -Z-(R^{Z})ₘ, an optionally substituted C₁₋₆alkyl, and an optionally substituted C₁₋₆alkoxyl, wherein "optionally substituted" refers to being optionally substituted by one or more substituents selected from halogen, -CN, -OH, -SH, -NO₂, -NH₂, C₁₋₆alkyl, C₂₋₆alkenyl, C₂₋₆alkynyl, C₁₋₆alkoxyl, C₁₋₆alkylthio, C₃₋₈cycloalkyl, C₃₋₈cycloalkenyl, 3-8 membered heterocyclyl, 5-6 membered heteroaryl, C₆₋₁₂ aryl, haloC₁₋₆alkyl, haloC₁₋₆alkoxyl, halophenyl, and -O-C₃₋₆cycloalkyl;
Z is saturated or partially unsaturated 4-12 membered heterocyclyl, 5-12 membered heteroaryl, or C₆₋₁₂ membered aryl;
R^{Z} is each independently selected from halogen, -CN, -OH, -SH, -NO₂, -NH₂, C₁₋₃alkyl, C₂₋₆alkenyl, C₂₋₆alkynyl, C₁₋₃alkoxyl, C₁₋₃hydroxyalkyl, haloC₁₋₃alkyl, haloC₁₋₃alkoxyl, saturated or partially unsaturated 4-6 membered heterocyclyl, -OR^{Z1}, and -NR^{Z2}R^{Z3};
R^{Z1} is C₁₋₃alkyl or haloC₁₋₃alkyl;
R^{Z2} and R^{Z3} are each independently selected from H, and C₁₋₃alkyl;
In a preferable embodiment of the present invention, each R^{W} is independently selected from halogen, formyl, acetyl, methylsulfonyl, ethylsulfonyl, methylsulfonamido, ethylsulfonamido, -CN, -OH, -SH, -NH₂, -Z-(R^{Z})ₘ, an optionally substituted C₁₋₄alkyl, and an optionally substituted C₁₋₄alkoxyl, wherein "optionally substituted" refers to being optionally substituted by one or more substituents selected from halogen, -CN, -OH, -SH, -NO₂, -NH₂, - CONH₂, C₁₋₃alkyl, C₁₋₃alkoxyl, C₃₋₈cycloalkyl, 3-8 membered heterocyclyl, 5-6 membered heteroaryl, C₆₋₁₂ aryl, haloC₁₋₃alkyl, haloC₁₋₃alkoxyl, and halophenyl;
Z is 5-6 membered heteroaryl or phenyl;
R^{Z} is each independently selected from halogen, -CN, -OH, -SH, -NO₂, -NH₂, C₁₋₃alkyl, C₁₋₃alkoxyl, C₁₋₃hydroxyalkyl, haloC₁₋₃alkyl, haloC₁₋₃alkoxyl, saturated or partially unsaturated 4-6 membered heterocyclyl, -OR^{Z1}, and -NR^{Z2}R^{Z3};
R^{Z1} is C₁₋₃alkyl or haloC₁₋₃alkyl;
R^{Z2} and R^{Z3} are each independently selected from H, and C₁₋₃alkyl;
In a preferable embodiment of the present invention, each R^{W} is independently selected from halogen, formyl, acetyl, methylsulfonyl, ethylsulfonyl, methylsulfonamido, ethylsulfonamido, -CN, -OH, -SH, -NH₂, -Z-(R^{Z})ₘ, an optionally substituted C₁₋₄alkyl, and an optionally substituted C₁₋₄alkoxyl, wherein "optionally substituted" refers to being optionally substituted by one or more substituents selected from halogen, -CN, -OH, -SH, -NO₂, -NH₂, C₁₋₃alkyl, C₁₋₃alkoxyl, C₃₋₆cycloalkyl, 3-8 membered heterocyclyl, 5-6 membered heteroaryl, C₆₋₁₂ aryl, haloC₁₋₃alkyl, haloC₁₋₃alkoxyl, and halophenyl;
Z is pyridinyl, pyridazinyl, pyrimidinyl, pyrazinyl, 1, 3, 5-triazinyl, or phenyl;
R^{Z} is each independently selected from halogen, -CN, -OH, -SH, -NO₂, -NH₂, C₁₋₃alkyl, C₁₋₃alkoxyl, C₁₋₃hydroxyalkyl, haloC₁₋₃alkyl, haloC₁₋₃alkoxyl, saturated or partially unsaturated 4-6 membered heterocyclyl, -OR^{Z1}, and -NR^{Z2}R^{Z3};
R^{Z1} is C₁₋₃alkyl or haloC₁₋₃alkyl;
R^{Z2} and R^{Z3} are each independently selected from H, and C₁₋₃alkyl;
In a preferable embodiment of the present invention, each R^{W} is independently selected from methylsulfonamido, ethylsulfonamido, F, Cl, -CN, -OH, -SH, -NH₂, -Z-(R^{Z})ₘ, and an optionally substituted group selected from: methyl, ethyl, iso-propyl, methoxy, ethoxy, methylthio, and ethylthio, wherein "optionally substituted" refers to being optionally substituted by one or more substituents selected from halogen, -CN, -OH, -NH₂, C₁₋₃alkyl, C₁₋₃alkoxyl, C₁₋₃haloalkyl, and C₁₋₃haloalkoxyl;
In a preferable embodiment of the present invention, -Z-(R^{Z})ₘ is selected from 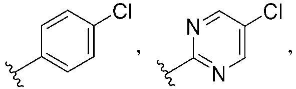 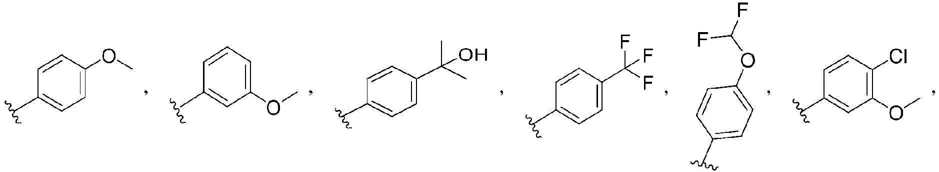 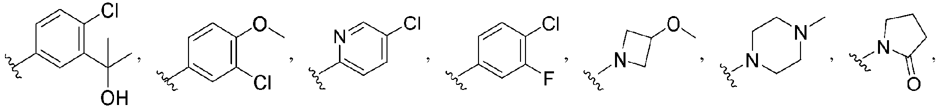 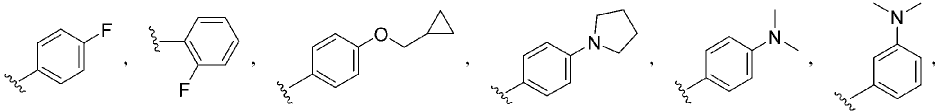 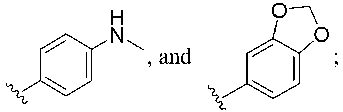
In a preferable embodiment of the present invention, each R^{W} is independently selected from methylsulfonyl, methylsulfonamido, -F, -Cl, -CN, -OH, -SH, -NH₂, -CF₃, and -CH₃;
In a preferable embodiment of the present invention, each R^{W} is independently selected from 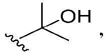 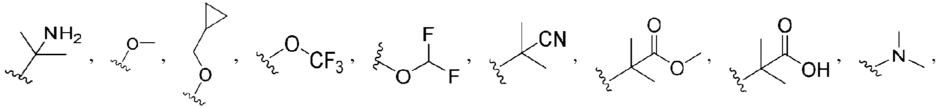 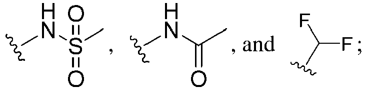
In a preferable embodiment of the present invention, each R^{W} is independently selected from 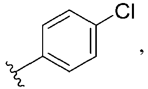 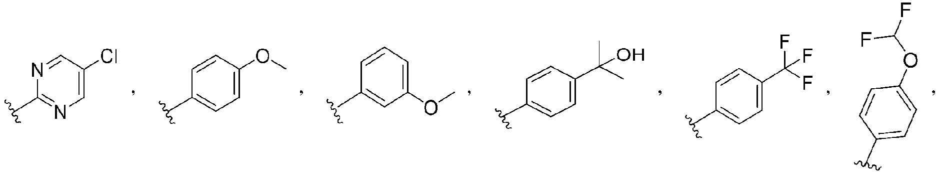 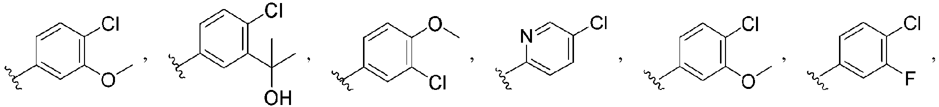 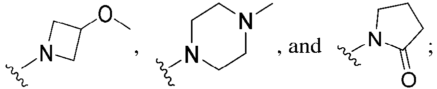
In a preferable embodiment of the present invention, m is 1, 2 or 3;
In a preferable embodiment of the present invention, m is 1 or 2;
In a preferable embodiment of the present invention, n is 0, 1, 2 or 3;
In a preferable embodiment of the present invention, n is 0, 1 or 2;
In a preferable embodiment of the present invention, n is 1 or 2;
In a preferable embodiment of the present invention, q is 0, 1, 2 or 3;
In a preferable embodiment of the present invention, q is 0, 1 or 2;
In a preferable embodiment of the present invention, q is 1 or 2;
The present invention further provides a compound represented by any of formulae (I-A), (I-B) and (I-C), a stereoisomer or tautomer of said compound, a mixture thereof, or a pharmaceutically acceptable salt of said compound: 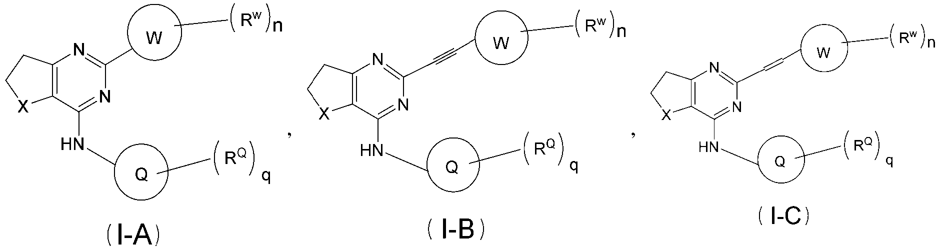
wherein, X, q, R^{Q}, ring Q, n, R^{W}, and ring W are as defined for the compound of formula (I);
The present invention further provides a compound represented by any of formulae (II-A) to (II-E), a stereoisomer or tautomer of said compound, a mixture thereof, or a pharmaceutically acceptable salt of said compound: 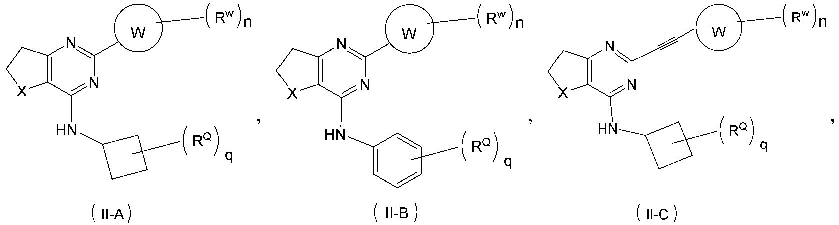 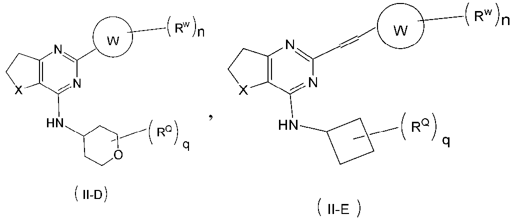
wherein X, n, R^{W}, ring W, R^{Q}, and q are as defined for the compound of formula (I);
The present invention further provides a compound represented by any of formulae (III-A) to (III-H), a stereoisomer or tautomer of said compound, a mixture thereof, or a pharmaceutically acceptable salt of said compound: 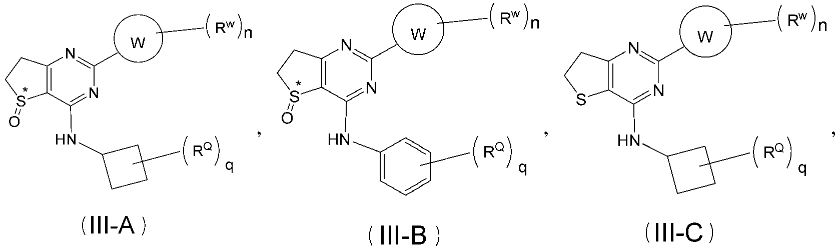 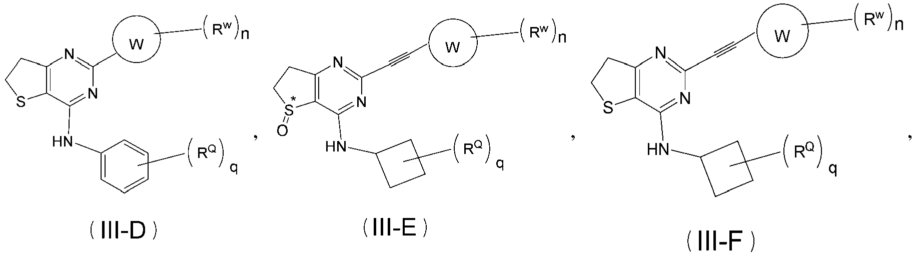 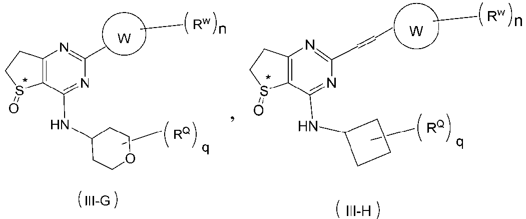
wherein, n, R^{W}, ring W, R^{Q}, and q are as defined for the compound of formulae (I) and (II-A) to (II-E);
S* is a chiral sulfur atom;
In a preferable embodiment of the present invention, S* is a chiral sulfur atom having R/S-enantiomers;
In a preferable embodiment of the present invention, S* is a chiral sulfur atom having R-enantiomer;
In a preferable embodiment of the present invention, S* is a chiral sulfur atom having S-enantiomer;
The present invention further provides a compound represented by any of formulae (IV-A) to (IV-E), a stereoisomer or tautomer of said compound, a mixture thereof, or a pharmaceutically acceptable salt of said compound: 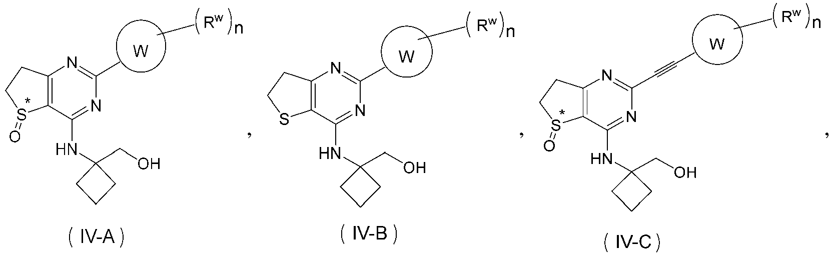 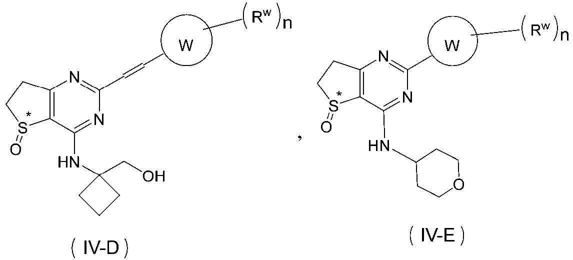
wherein, n, R^{W}, and ring W are as defined for the compound of formulae (I), (II-A) to (II-E), and (III-A) to (III-H);
In a preferable embodiment of the present invention, in the above formula (IV-A) or formula (IV-E), 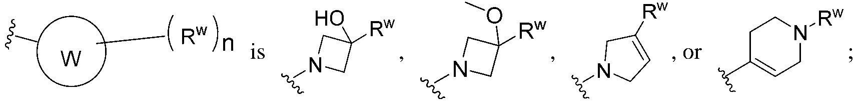
In a preferable embodiment of the present invention, in the above formula (IV-A) or formula (IV-E), 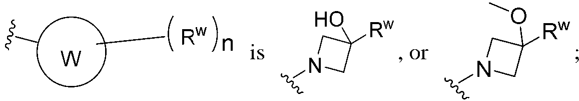 further preferably R^{W} is Z-(R^{Z})ₘ;
In a preferable embodiment of the present invention, in the above formula (IV-A) or formula (IV-E),

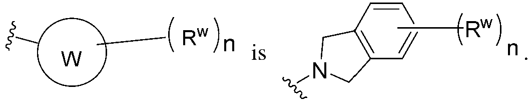

On the basis of conforming to common knowledge in the art, the above preferred embodiments can be combined arbitrarily to obtain more preferred embodiments of the present invention.

Preferably, the present invention provides a compound, or a stereoisomer or tautomer of said compound, a mixture thereof, or a pharmaceutically acceptable salt of said compound, wherein said compound has any of the following structures:

| 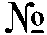 | Structure | 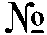 | Structure | 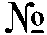 | Structure | 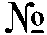 | Structure |
|---|---|---|---|---|---|---|---|
| 1 | 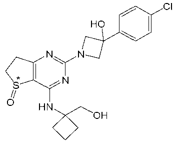 | 2 | 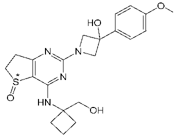 | 3 | 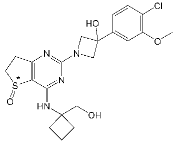 | 4 | 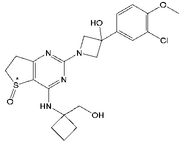 |
| 5 | 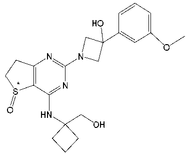 | 6 | 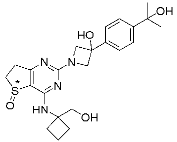 | 7 | 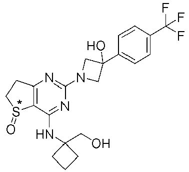 | 8 | 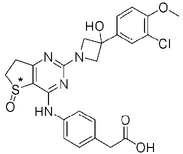 |
| 9 | 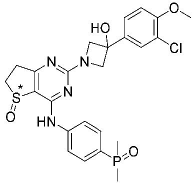 | 10 | 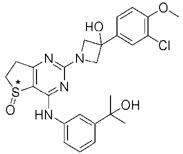 | 11 | 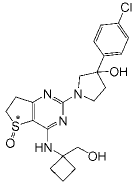 | 12 | 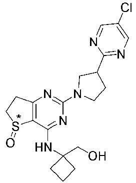 |
| 13 | 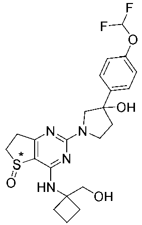 | 14 | 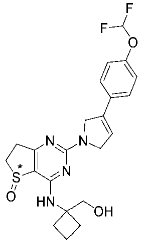 | 15 | 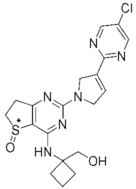 | 16 | 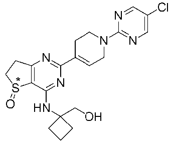 |
| 17 | 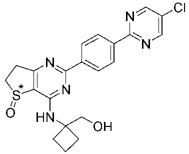 | 18 | 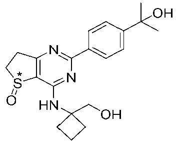 | 19 | 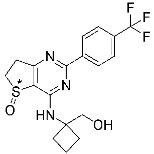 | 20 | 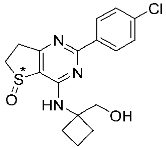 |
| 21 | 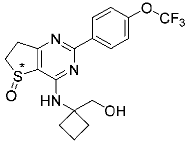 | 22 | 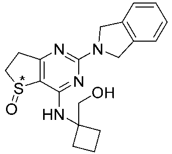 | 23 | 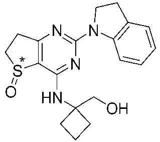 | 24 | 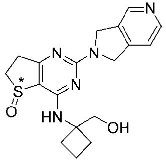 |
| 25 | 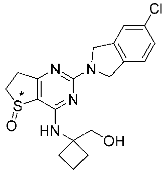 | 26 | 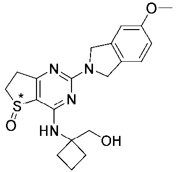 | 27 | 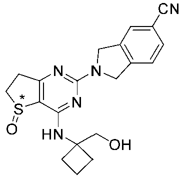 | 28 | 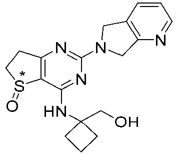 |
| 29 | 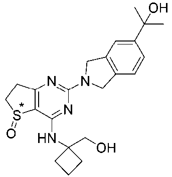 | 30 | 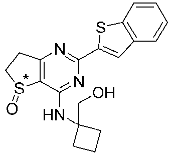 | 31 | 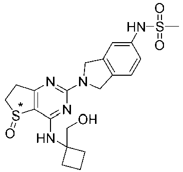 | 32 | 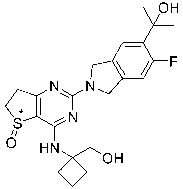 |
| 33 | 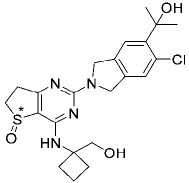 | 34 | 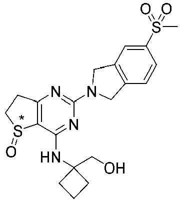 | 35 | 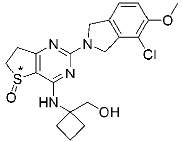 | 36 | 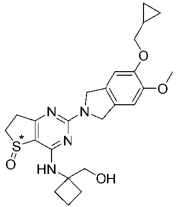 |
| 37 | 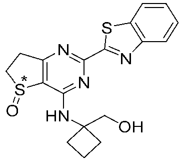 | 38 | 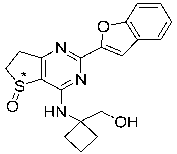 | 39 | 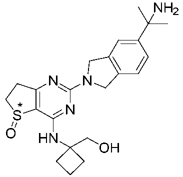 | 40 | 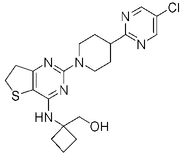 |
| 41 | 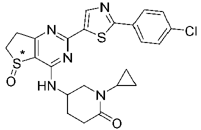 | 42 | 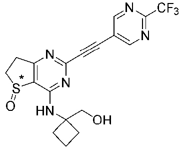 | 43 | 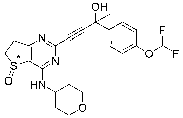 | 44 | 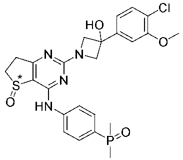 |
| 45 | 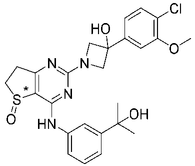 | 46 | 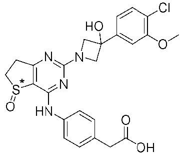 | 47 | 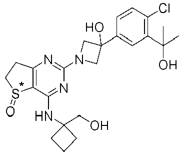 | 48 | 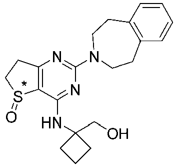 |
| 49 | 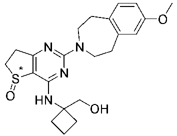 | 50 | 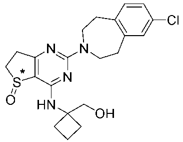 | 51 | 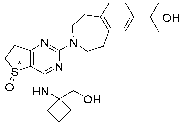 | 52 | 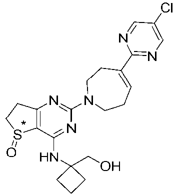 |
| 53 | 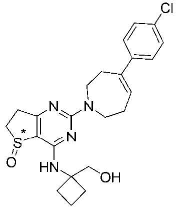 | 54 | 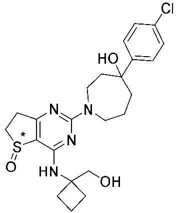 | 55 | 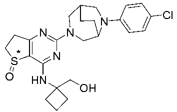 | 56 | 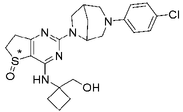 |
| 57 | 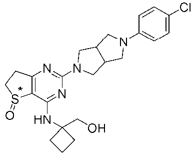 | 58 | 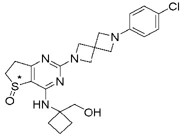 | 59 | 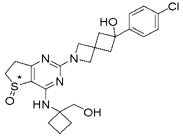 | 60 | 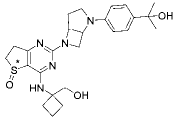 |
| 61 | 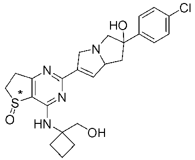 | 62 | 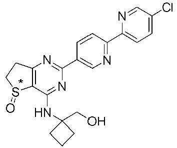 | 63 | 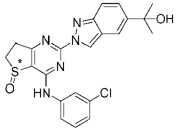 | 64 | 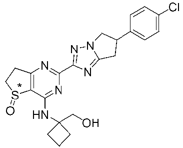 |
| 65 | 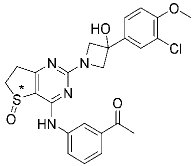 | 66 | 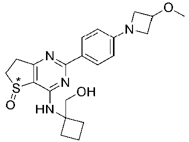 | 67 | 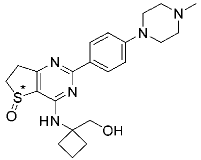 | 68 | 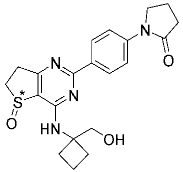 |
| 69 | 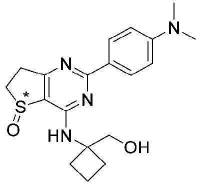 | 70 | 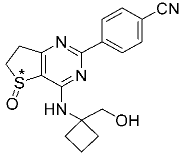 | 71 | 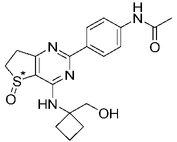 | 72 | 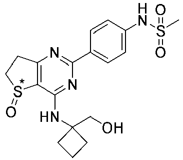 |
| 73 | 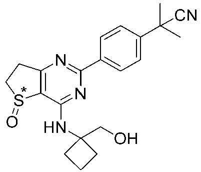 | 74 | 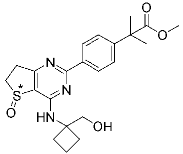 | 75 | 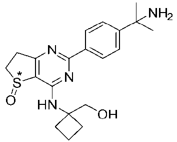 | 76 | 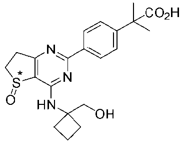 |
| 77 | 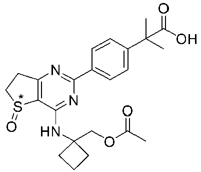 | 78 | 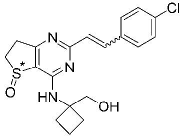 | 79 | 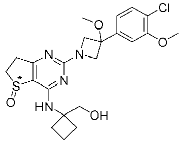 | 80 | 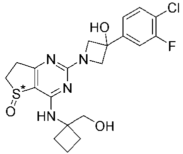 |
| 81 | 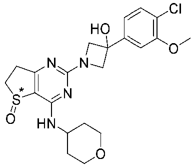 | 82 | 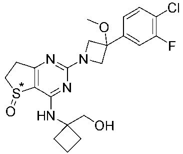 | 83 | 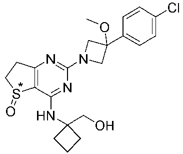 | 84 | 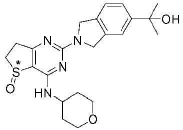 |
| 85 | 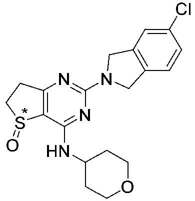 | 86 | 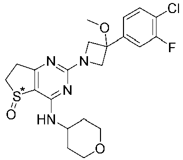 | 87 | 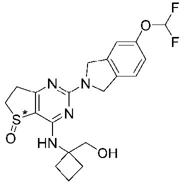 | 88 | 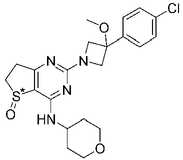 |
| 89 | 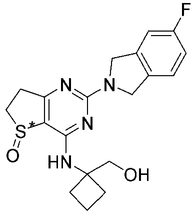 | 90 | 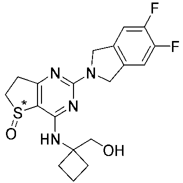 | 91 | 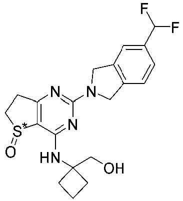 | 92 | 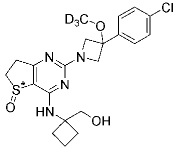 |
| 93 | 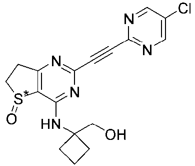 | 94 | 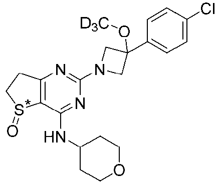 | 95 | 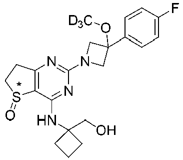 | 96 | 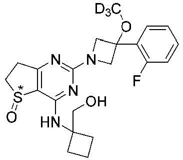 |
| 97 | 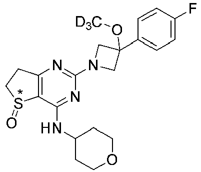 | 98 | 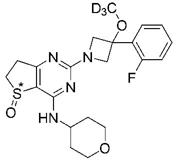 | 99 | 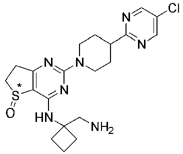 | 100 | 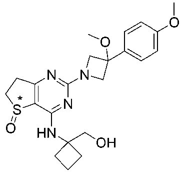 |
| 101 | 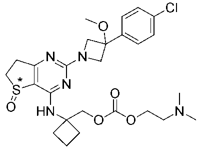 | 102 | 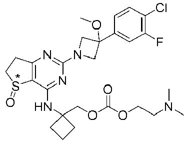 | 103 | 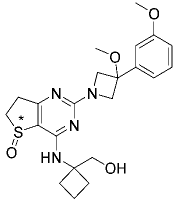 | 104 | 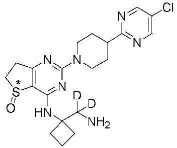 |
| 105 | 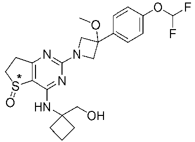 | 106 | 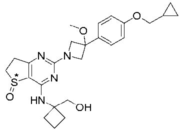 | 107 | 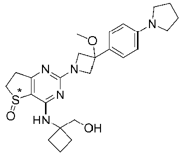 | 108 | 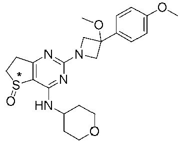 |
| 109 | 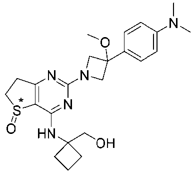 | 110 | 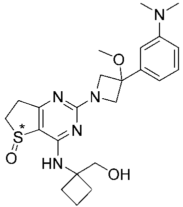 | 111 | 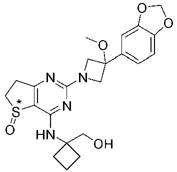 | 112 | 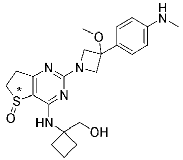 |

The object of the present invention also includes providing a method for preparing a compound represented by the above general formula, a stereoisomer or tautomer of said compound, a mixture thereof, or a pharmaceutically acceptable salt of said compound.

The compound of said general formula can be prepared by various methods, including but not limited to the following methods: 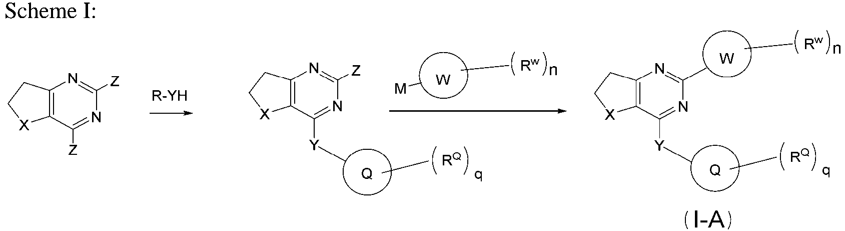 wherein, M is a ligand-containing boron or metal
Z is a leaving group such as halogen, a sulfonate ester group, alkylthio, alkylsulfonyl, and alkylsulfinyl. 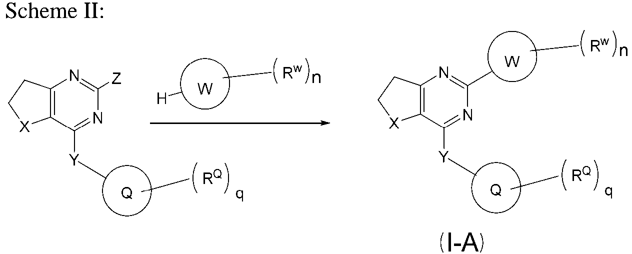 wherein Z is a leaving group such as halogen, a sulfonate ester group, alkylthio, alkylsulfonyl, and alkylsulfinyl.

The present invention also provides a pharmaceutical composition comprising a compound of the present invention, a stereoisomer or tautomer of said compound, a mixture thereof, or a pharmaceutically acceptable salt of said compound.

The present invention also provides a pharmaceutical composition comprising a compound of the present invention, a stereoisomer or tautomer of said compound, a mixture thereof, or a pharmaceutically acceptable salt of said compound, and a pharmaceutically acceptable excipient.

The object of the present invention also includes providing the use of a compound of the present invention, a stereoisomer or tautomer of said compound, a mixture thereof, or a pharmaceutically acceptable salt of said compound in manufacture of a medicament for treating or preventing a PDE4B-mediated disease.

In some embodiments, said PDE4B-mediated disease is a fibrosis-related disease or an Immune-mediated inflammatory disease.

In some embodiments, said PDE4B-mediated disease includes respiratory disease, gastrointestinal disease, inflammatory disease, allergic disease, autoimmune disease, and cancer.

Preferably, said respiratory disease is selected from respiratory or pulmonary diseases accompanied by increased mucus production, airway inflammation and/or obstructive diseases; further preferably, said respiratory disease is selected from idiopathic pulmonary fibrosis, progressive pulmonary fibrosis, interstitial pneumonia, chronic obstructive pulmonary disease (COPD), alpha-1 antitrypsin deficiency, chronic rhinosinusitis, asthma or chronic bronchitis;
Preferably, said gastrointestinal disease is selected from regional ileitis, ulcerative colitis or Crohn's disease;
Preferably, said inflammatory disease is selected from dry eye syndrome or glaucoma;
Preferably, said autoimmune disease is selected from atopic dermatitis, seborrheic dermatitis, psoriasis, urticaria, multiple sclerosis and diffuse connective tissue diseases such as systemic lupus erythematosus, rheumatoid arthritis, dermatomyositis, polymyositis, vasculitis, and Sjogren's syndrome.

Further preferably, in some embodiments, said PDE4B-mediated disease is chronic obstructive pulmonary disease (COPD), idiopathic pulmonary fibrosis, asthma or interstitial lung disease.

The object of the present invention also includes providing a method for preventing and/or treating a PDE4B-mediated disease, which method comprises administering to a patient a therapeutically effective amount of a compound of the present invention, a stereoisomer or tautomer of said compound, a mixture thereof, or a pharmaceutically acceptable salt of said compound, or a pharmaceutical composition of the present invention. The compound of the present invention, a stereoisomer or tautomer of said compound, a mixture thereof, or a pharmaceutically acceptable salt of said compound can be administered in combination with another agent for treating or preventing a PDE4B-mediated disease, such as a fibrosis or immune inflammation inhibitor.

When the compound of the present invention, a stereoisomer or tautomer of said compound, a mixture thereof, or a pharmaceutically acceptable salt of said compound is administered in combination with another agent for treating or preventing a PDE4B-mediated disease, such as a fibrosis or immune inflammation inhibitor, the compound of the present invention or a pharmaceutically acceptable salt thereof can provide an enhanced therapeutic effect on the PDE4B-mediated disease, such as an antifibrotic effect or an anti-immune-inflammatory effect.

### Definition

Unless otherwise specified, the term "alkyl" refers to a monovalent saturated aliphatic hydrocarbon group, including a linear or branched group containing 1-20 carbon atoms, preferably containing 1-10 carbon atoms (namely C₁₋₁₀ alkyl), further preferably containing 1-8 carbon atoms (C₁₋₈ alkyl), more preferably containing 1-6 carbon atoms (namely C₁₋₆ alkyl). For example "C₁₋₆ alkyl" means that the group is an alkyl group, and the number of carbon atoms in the carbon chain is between 1 and 6 (specifically 1, 2, 3, 4, 5 or 6). Its example includes but is not limited to methyl, ethyl, n-propyl, iso-propyl, n-butyl, iso-butyl, tert-butyl, sec-butyl, n-pentyl, neo-pentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl, and the like.

Unless otherwise specified, the term "alkenyl" refers to a linear or branched unsaturated aliphatic hydrocarbon group consisting of carbon atoms and hydrogen atoms and having at least one double bond. The alkenyl can contain 2-20 carbon atoms, preferably contain 2-10 carbon atoms (namely C₂₋₁₀alkenyl), further preferably contain 2-8 carbon atoms (C₂₋₈alkenyl), more preferably contain 2-6 carbon atoms (namely C₂₋₆alkenyl), 2-5 carbon atoms (namely C₂₋₅alkenyl), 2-4 carbon atoms (namely C₂₋₄alkenyl), 2-3 carbon atoms (namely C₂₋₃alkenyl), 2 carbon atoms (namely C₂alkenyl). For example "C₂₋₆alkenyl" means that the group is an alkenyl group, and the number of carbon atoms in the carbon chain is between 2 and 6 (specifically 2, 3, 4, 5 or 6). The non-limiting examples of alkenyl include but are not limited to ethenyl, 1-propenyl, 2-propenyl, 1-butenyl, iso-butenyl, 1,3-butadienyl, and the like.

Unless otherwise specified, the term "alkynyl" refers to a linear or branched unsaturated aliphatic hydrocarbon group consisting of carbon atoms and hydrogen atoms and having at least one triple bond. The alkynyl can contain 2-20 carbon atoms, preferably contain 2-10 carbon atoms (namely C₂₋₁₀alkynyl), further preferably contain 2-8 carbon atoms (C₂₋₈alkynyl), more preferably contain 2-6 carbon atoms (namely C₂₋₆alkynyl), 2-5 carbon atoms (namely C₂₋₅alkynyl), 2-4 carbon atoms (namely C₂₋₄alkynyl), 2-3 carbon atoms (namely C₂₋₃alkynyl), 2 carbon atoms (namely C₂alkynyl). For example "C₂₋₆ alkynyl" means that the group is an alkynyl group, and the number of carbon atoms in the carbon chain is between 2 and 6 (specifically 2, 3, 4, 5 or 6). The non-limiting examples of alkynyl include but are not limited to ethynyl, 1-propynyl, 2-propynyl, 1-butynyl, and the like.

Unless otherwise specified, the term "cycloalkyl" refers to a hydrocarbon group selected from saturated cyclic hydrocarbon groups. Said saturated cyclic hydrocarbon group comprises monocyclic and polycyclic (e.g., bicyclic and tricyclic) groups, including fused cycloalkyl groups, bridged cycloalkyl groups or spirocycloalkyl groups. For example, the cycloalkyl can include 3-16 (e.g., 3-10, 3-8, 3-6, 3-5 or 3-4) carbon atoms. Further for example, the cycloalkyl can be selected from monocyclic groups containing 3-12 (e.g. 3-10, 3-8, or 3-6) carbon atoms. Examples of monocyclic cycloalkyl include cyclopropyl, cyclobutyl, cyclopentyl, cyclohexyl, cyclohexadienyl, cycloheptyl, cyclooctyl, cyclononyl, cyclodecyl, cycloundecyl, and cyclododecyl. Specifically, the example of saturated monocyclic cycloalkyl (e.g., C₃₋₈cycloalkyl) includes but is not limited to cyclopropyl, cyclobutyl, cyclopentyl, cyclohexyl, cycloheptyl and cyclooctyl. In a preferable embodiment, the cycloalkyl is a monocyclic group containing 3-6 carbon atoms (abbreviated as C₃₋₆cycloalkyl), and it includes but is not limited to cyclopropyl, cyclobutyl, cyclopentyl, and cyclohexyl. Examples of bicyclic cycloalkyl include those having 5-12, 7-12 or 5-10 ring atoms, which are arranged as fused bicyclic ring selected from [4,4], [4,5], [5,5], [5,6] or [6,6] ring systems, or arranged as bridged bicyclic ring selected from bicyclo[2.2.1]heptane, bicyclo[2.2.2]octane and bicyclo[3.2.2]nonane. Other examples of bicyclic cycloalkyl include cyclic groups arranged as bicyclic ring selected from [5,6] and [6,6] ring systems. The rings may be saturated or have at least one double bond (i.e., partially unsaturated), but are not fully conjugated and are not aromatic, since the aromatic groups are defined herein.

The term "spirocycloalkyl" refers to a cyclic structure containing carbon atoms and formed by at least two rings sharing one atom. For example, the term "7-12 membered spirocycloalkyl" refers to a cyclic structure containing 7-12 carbon atoms and formed by at least two rings sharing one atom.

The term "fused cycloalkyl" refers to a fused ring containing carbon atoms and formed by two or more rings that share two adjacent atoms. For example, the term "5-10 membered fused cycloalkyl" refers to a fused ring containing 5-10 ring carbon atoms and formed by two or more rings that share two adjacent atoms. Its example includes but is not limited to bicyclo[1.1.0]butyl, bicyclo[2.1.0]pentyl, bicyclo[3.1.0]hexyl, bicyclo[4.1.0]heptyl, bicyclo[3.3.0]octyl, bicyclo[4.2.0]octyl, decahydronaphthalyl, benzene-fused (benzo) 3-8 membered cycloalkyl, benzene-fused C₄₋₆cycloalkenyl, 2,3-dihydro-1H-indenyl, 1H-indenyl, 1,2,3,4-tetrazolyl, 1,4-dihydronaphthyl and the like. Preferred are 8-9 membered fused rings, which refers to a cyclic structure containing 8 to 9 ring atoms in the above examples.

The term "bridged cycloalkyl" refers to a cyclic structure containing carbon atoms and formed by two rings sharing two atoms that are not adjacent to each other. The term "7-12 membered bridged cycloalkyl" refers to a cyclic structure containing 7-12 carbon atoms and formed by two rings sharing two atoms that are not adjacent to each other.

The term "cycloalkenyl" refers to a non-aromatic cyclic hydrocarbyl of 4-16, e.g. 4-12 carbon atoms, which has a monocyclic or polycyclic structure and has at least one double bond and preferably 1-2 double bonds. In one embodiment, the cycloalkenyl group is cyclopentenyl (1-cyclopent-1-enyl, 1-cyclopent-2-enyl, 1-cyclopent-3-enyl) or cyclohexenyl (1-cyclohex-1-enyl, 1-cyclohex-2-enyl, 1-cyclohex-3-enyl), preferably cyclohexenyl.

Unless otherwise specified, the term "oxaalkyl" refers to an alkyl residue in which one or more carbons (and their associated hydrogens) have been replaced by oxygen, such as "alkoxy", and "alkoxyalkyl". Examples include methoxy, ethoxy, propoxy, methoxypropyl, and the like. The term oxaalkyl is intended as it is understood in the art [see Naming and Indexing of Chemical Substances for Chemical Abstracts, published by the American Chemical Society, 196, but without the restriction of 127(a)], i.e., it refers to compounds in which the oxygen is bonded via a single bond to its adjacent atoms (forming ether bonds); it does not refer to doubly bonded oxygen, as would be found in carbonyl groups.

"Alkoxy" refers to -O-alkyl, and the alkyl is defined as above, i.e., contains 1-20 carbon atoms, preferably, contains 1-10 carbon atoms, more preferably 1-8 carbon atoms, and further preferably 1-6 carbon atoms (specifically 1, 2, 3, 4, 5 or 6). Representative examples include, but are not limited to, methoxy, ethoxy, propoxy, isopropoxy, butoxy, 1-methylpropoxy, 2-methylpropoxy, tert-butoxy, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, and the like.

Unless otherwise specified, the terms "thiaalkyl" and "azaalkyl" refer to groups in which the oxygen in the above "oxaalkyl" is replaced by sulfur or nitrogen.

Unless otherwise specified, the term "halogen" or "halo" refers to F, Cl, Br, or I. The term "haloalkyl" refers to an alkyl group as defined above in which one, two or more hydrogen atoms or all hydrogen atoms are replaced by halogen. Representative examples of haloalkyl include CCl₃, CF₃, CHCl₂, CH₂Cl, CH₂Br, CH₂I, CH₂CF₃, CF2CF₃, and the like.

Unless otherwise specified, the term "heterocyclyl" or "heterocycle" refers to a non-aromatic heterocyclic group containing one or more heteroatoms selected from nitrogen, oxygen or optionally oxidized sulfur as ring member with the remaining ring member being carbon, including monocyclic rings, fused rings, bridged rings and spiro rings, that is, including monocyclic heterocyclic groups, bridged heterocyclic groups, spiro heterocyclic groups, and fused heterocyclic groups.

The term "monocyclic heterocyclyl" refers to a monocyclic group wherein at least one ring member is a heteroatom selected from nitrogen, oxygen or optionally oxidized sulfur. The heterocycle may be saturated or partially saturated. Exemplary monocyclic 4-18 membered heterocyclyl includes but is not limited to (as numbered from the attachment position designated as priority 1) pyrrolidin-1-yl, pyrrolidin-2-yl, pyrrolidin-3-yl, imidazolidin-2-yl, imidazolidin-4-yl, pyrazolidin-2-yl, pyrazolidin-3-yl, piperidin-1-yl, piperidin-2-yl, piperidin-3-yl, piperidin-4-yl, 2,5-piperazinyl, pyranyl, morpholinyl, morpholino, morpholin-2-yl, morpholin-3-yl, oxiranyl, aziridin-1-yl, aziridin-2-yl, azocan-1-yl, azocan-2-yl, azocan-3-yl, azocan-4-yl, azocan-5-yl, thiiranyl, azetidin-1-yl, azetidin-2-yl, azetidin-3-yl, oxetanyl, thietanyl, 1,2-dithietanyl, 1,3-dithietanyl, dihydropyridinyl, tetrahydropyridinyl, thiomorpholinyl, oxathianyl, piperazinyl, homopiperazinyl, homopiperidinyl, azepan-1-yl, azepan-2-yl, azepan-3-yl, azepan-4-yl, oxepanyl, thiepanyl, 1,4-oxathianyl, 1,4-dioxepanyl, 1,4-oxathiepanyl, 1,4-oxazepanyl, 1,4-dithiepanyl, 1,4-thiazepanyl, 1,4-diazepanyl, 1,4-dithianyl, 1,4-thiazinanyl, oxazepinyl, diazepinyl, thiazepinyl, dihydrothienyl, dihydropyranyl, dihydrofuranyl, tetrahydrofuranyl, tetrahydrothienyl, tetrahydropyranyl, tetrahydrothiopyranyl, 1-pyrrolinyl, 2-pyrrolinyl, 3-pyrrolinyl, indolinyl, 2H-pyranyl, 4H-pyranyl, 1,4-dioxanyl, 1,3-dioxolanyl, pyrazolinyl, pyrazolidinyl, dithianyl, dithiolanyl, pyrazolidinyl, imidazolinyl, pyrimidinonyl, or 1,1-dioxo-thiomorpholinyl.

The term "spiro heterocyclyl" refers to a 5-18 membered polycyclic heterocyclyl having rings connected via a single shared carbon atom (called the spiro atom), which contains one or more heteroatoms selected from nitrogen, oxygen or optionally oxidized sulfur as ring members together with the remaining ring members being carbon. One or more rings of the spiro heterocyclyl can contain one or more double bonds, but no ring has a fully conjugated π-electron system. Preferably, the spiro heterocyclyl is 6-14 membered, and more preferably 7-12 membered. According to the number of the shared spiro atoms, the spiro heterocyclyl is divided into monospiro heterocyclyl, dispiro heterocyclyl, or polyspiro heterocyclyl, and preferably refers to monospiro heterocyclyl or dispiro heterocyclyl, and more preferably 4-membered/4-membered, 3-membered/5-membered, 4-membered/5-membered, 4-membered/6-membered, 5-membered/5-membered, or 5-membered/6-membered monospiro heterocyclyl. Representative examples of spiro heterocyclyl include but are not limited to the following groups: 2,3-dihydrospiro[indene-1,2'-pyrrolidine] (e.g., 2,3-dihydrospiro[indene-1,2'-pyrrolidine]-1'-yl), 1,3-dihydrospiro[indene-2,2'-pyrrolidine] (e.g., 1,3-dihydrospiro[indene-2,2'-pyrrolidine]-1'-yl), azaspiro[2.4]heptane (e.g., 5-azaspiro[2.4]heptane-5-yl), azaspiro[3.4]octane (e.g., 6-azaspiro[3.4]octane-6-yl), 2-oxa-6-azaspiro[3.4]octane (e.g., 2-oxa-6-azaspiro[3.4]octane-6-yl), azaspiro[3.4]octane (e.g., 6-azaspiro[3.4]oct-6-yl), azaspiro[3.4]octane (e.g., 6-azaspiro[3.4]oct-6-yl), 1,7-dioxaspiro[4.5]decane, 2-oxa-7-azaspiro[4.4]nonane (e.g., 2-oxa-7-aza-spiro[4.4]non-7-yl), 7-oxa-spiro[3.5]nonyl and 5-oxa-spiro[2.4]heptyl.

The term "fused heterocyclyl" refers to a 5-18 membered polycyclic heterocyclyl in which each ring in the system shares a pair of adjacent atoms (carbon and carbon atoms or carbon and nitrogen atoms) with another ring, which contains one or more heteroatoms selected from nitrogen, oxygen or optionally oxidized sulfur as ring members together with the remaining ring members being carbon. One or more rings of the fused heterocyclyl can contain one or more double bonds, but no ring has a fully conjugated π-electron system. Preferably, the fused heterocyclyl is 6-14 membered, preferably 7-12 membered and more preferably 7-10 membered. According to the number of the member rings, the fused heterocyclyl is divided into bicyclic, tricyclic, tetracyclic, or polycyclic fused heterocyclyl, preferably refers to bicyclic or tricyclic fused heterocyclyl, and more preferably 5-membered/5-membered, or 5-membered/6-membered, or 6-membered/7-membered bicyclic fused heterocyclyl. Representative examples of fused heterocyclyl include but are not limited to the following groups: octahydrocyclopenta[c]pyrrole(e.g., octahydrocyclopenta[c]pyrrole-2-yl), octahydropyrrolo[3,4-c]pyrrolyl, octahydroisoindolyl, isoindolinyl (e.g., isoindolin-2-yl or isoindolin-5-yl), octahydro-benzo[b][1,4]dioxine, dihydropyridooxazinyl (e.g., 2,3-dihydro-1H-pyrido[2,3-b][1,4]oxazinyl) or dihydrobenzooxazepinyl (e.g., 5-oxo-3,4-dihydrobenzo[f][1,4]oxazepinyl), benzoazepinyl (e.g., 2,3,4,5-tetrahydro-1-oxo-2-benzoazepin-6-yl), benzooxazepinyl (e.g., 5-oxo-2,3,4,5-tetrahydro-1,4-benzooxaazepin-8-yl), dihydroisoquinolinyl (e.g., 1-oxo-2-methyl-3,4-dihydroisoquinolin-6-yl), tetrahydroisoquinolinyl (e.g., 2-methyl-1-oxo-1,2,3,4-tetrahydroisoquinoline-6-yl), dihydrobenzoxazine (e.g., 3,4-dihydro-2H-1,4-benzoxazin-6-yl).

The term "bridged heterocyclyl" refers to a 5-18 membered polycyclic heterocyclyl, in which every two rings in the system share two non-connected atoms, and which contains one or more heteroatoms selected from nitrogen, oxygen or optionally oxidized sulfur as ring members together with the remaining ring atoms being carbon. One or more rings of the bridged heterocyclyl can contain one or more double bonds, but no ring has a fully conjugated π-electron system. Preferably, the bridged heterocyclyl is 6-14 membered, and more preferably 7-10 membered. According to the number of member rings, bridged heterocyclyl is divided into bicyclic, tricyclic, tetracyclic or polycyclic bridged heterocyclyl, and preferably refers to bicyclic, tricyclic or tetracyclic bridged heterocyclyl, and more preferably bicyclic or tricyclic bridged heterocyclyl. Representative examples of bridged heterocyclyl include but are not limited to the following groups: 2-azabicyclo[2.2.1]heptyl, azabicyclo[3.1.0]hexyl, 2-azabicyclo[2.2.2]octyl and 2-azabicyclo[3.3.2]decyl.

Unless otherwise specified, the term "heterocycloalkyl" refers to a monocyclic, saturated "heterocyclyl" or "heterocycle" as defined above, with ring atoms defined as above, i.e., containing 3-20 ring atoms ("3-20 membered heterocycloalkyl"), with the number of heteroatoms being 1, 2, 3 or 4 (1-4), preferably 1, 2 or 3 (1-3), wherein the heteroatoms are each independently selected from N, O or S; preferably containing 3-12 ring atoms ("3-12 membered heterocycloalkyl"), further preferably containing 3-10 ring atoms ("3-10 membered heterocycloalkyl"), even more preferably containing 3-8 ring atoms ("3-8 membered heterocycloalkyl"), or even more preferably containing 4-7 ring atoms ("4-7 membered heterocycloalkyl"), or even more preferably containing 5-10 ring atoms ("5-10 membered heterocycloalkyl"), or even more preferably containing 5-6 ring atoms ("5-6 membered heterocycloalkyl"). In some embodiments, each example of heterocycloalkyl is independently optionally substituted, for example, unsubstituted (an "unsubstituted heterocycloalkyl") or substituted by one or more substituents (a "substituted heterocycloalkyl"). Some examples of "heterocycloalkyl" have been given in the "heterocyclyl" or "heterocycle" section above, and also include, but are not limited to, oxacyclohexyl (oxanyl), thiomorpholinyl, oxathiacyclohexyl (oxathianyl), oxazolidinyl, thiazolidinyl, pyrazolidinyl, imidazolidinyl, etc.

Unless otherwise specified, the term "heterocycloalkenyl" refers to a monocyclic, unsaturated "heterocyclyl" or "heterocycle" as defined above, with ring atoms defined as above, i.e., containing 4-12 ring atoms ("4-12 membered heterocycloalkenyl"), with the number of heteroatoms being 1, 2, 3 or 4 (1-4), preferably 1, 2 or 3 (1-3), wherein the heteroatoms are each independently selected from N, O or S; preferably containing 4-10 ring atoms ("4-10 membered heterocycloalkenyl"), further preferably containing 4-8 ring atoms ("4-8 membered heterocycloalkenyl"), even more preferably containing 4-6 ring atoms ("4-6 membered heterocycloalkenyl"), or even more preferably containing 5-6 ring atoms ("5-6 membered heterocycloalkenyl"). In some embodiments, each example of heterocycloalkenyl is independently optionally substituted, for example, unsubstituted (an "unsubstituted heterocycloalkenyl") or substituted by one or more substituents (a "substituted heterocycloalkenyl"). Unless otherwise specified, the term "aryl" refers to a monocyclic, bicyclic or tricyclic aromatic carbocyclic system containing 6-16 carbon atoms, or 6-14 carbon atoms, or 6-12 carbon atoms, or 6-10 carbon atoms, preferably 6-10 carbon atoms. The term "aryl" can be used interchangeably with the term "aromatic ring". Examples of the aryl can include but are not limited to phenyl, naphthyl, anthryl, phenanthryl or pyrenyl, etc.

Unless otherwise specified, the term "heteroaryl" refers to an aromatic monocyclic, bicyclic or polycyclic ring system containing 5-16 membered structure, or 5-14 membered structure, 5-12 membered structure, 5-10 membered structure, 5-8 membered structure, or 5-6 membered structure, wherein 1, 2, 3 or more ring atoms are heteroatoms and the remaining atoms are carbon, the heteroatoms are independently selected from O, N or S, and the number of heteroatoms is preferably 1, 2 or 3. Examples of the heteroaryl can include but are not limited to furanyl, thienyl, oxazolyl, thiazolyl, isoxazolyl, oxadiazolyl, thiadiazolyl, pyrrolyl, pyrazolyl, imidazolyl, triazolyl, tetrazolyl, pyridinyl, pyrimidinyl, pyrazinyl, pyridazinyl, thiodiazolyl, triazinyl, phthalazinyl, quinolinyl, isoquinolinyl, pteridinyl, purinyl, indolyl, isoindolyl, indazolyl, benzofuranyl, benzothienyl, benzopyridinyl, benzopyrimidinyl, benzopyrazinyl, benzimidazolyl, benzophthalazinyl, pyrrolo[2,3-b]pyridinyl, imidazo[1,2-a]pyridinyl, pyrazolo[1,5-a]pyridinyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridinyl, etc.

The term "alkylene" refers to a divalent alkyl group as defined above. The term "alkenylene" refers to a divalent alkenyl group as defined above. The term "alkynylene" refers to a divalent alkynyl group as defined above. The term "cycloalkylene" refers to a divalent cycloalkyl group as defined above. The term "heterocyclylene" refers to a divalent heterocyclyl group as defined above. The term "arylene" refers to a divalent aryl group as defined above. The term "heteroarylene" refers to a divalent heteroaryl group as defined above.

Unless specified otherwise, the term "pharmaceutically acceptable salt" refers to a salt that is suitable for use in contact with the tissues of mammals, especially humans, without undue toxicity, irritation, allergic response or the like, and commensurates with a reasonable benefit/risk ratio within the range of reasonable medical judgment. For example, the pharmaceutically acceptable salts of amines, carboxylic acids and other types of compounds are well known in the art. The salts can be prepared in situ during the final isolation and purification of the compounds of the present invention, or separately by reacting the free base or acid with a suitable reagent.

The compound of the present invention also includes its "isotopic derivative". Unless otherwise specified, the term "isotope derivative" means that compounds of the present invention can exist in isotope labeled or enriched form containing one or more atoms having an atomic mass or mass number different from the atomic mass or mass number most abundantly found in nature. Isotopes can be radioactive or non-radioactive isotopes. Isotopes commonly used as isotopic labels are: hydrogen isotopes: ²H and ³H; carbon isotopes: ¹³C and ¹⁴C; chlorine isotopes: ³⁵Cl and ³⁷Cl; fluorine isotope: ¹⁸F; iodine isotopes: ¹²³I and ¹²⁵I; nitrogen isotopes: ¹³N and ¹⁵N; oxygen isotopes: ¹⁵O, ¹⁷O and ¹⁸O; and sulfur isotope: ³⁵S. These isotope labeled compounds can be used to study the distribution of pharmaceutical molecules in tissues. In particular, ³H and ¹³C are more widely used due to their ease of labeling and ease of detection. The substitution with certain heavy isotopes, such as heavy hydrogen (²H), can enhance the stability of metabolism, and prolong the half-life, so as to achieve the purpose of reducing dosage and providing therapeutic advantages. Isotope labeled compounds are generally synthesized starting from labeled starting materials in the same way as non-isotope labeled compounds using known synthetic techniques.

The compound of the present invention also includes its "solvate". Unless specified otherwise, the term "solvate" refers to a physical association of the compound of the present invention with one or more solvent molecules, regardless of organic or inorganic. This physical association includes hydrogen bond. In some cases, for example, when one or more solvent molecules are incorporated into the crystal lattice of crystalline solid, the solvates can be isolated. Solvent molecules in the solvates may exist in regular and/or disordered arrangements. The solvates may contain stoichiometric or non-stoichiometric amounts of solvent molecules. The "solvate" encompasses both solution phase and isolatable solvate. Solvation methods are well known in the art.

Unless otherwise specified, the term "stereoisomers" refers to compounds that have identical chemical constitution, but differ with regard to the arrangement of the atoms or groups in space. Stereoisomers include enantiomers, diastereomers, conformers (rotamers), geometric (cis/trans) isomer, atropisomers, etc. A mixture of any resulting stereoisomers can be separated into the pure or substantially pure geometric isomers, enantiomers, diastereomers, according to physicochemical differences of constituents, for example, by chromatography and/or fractional crystallization.

Unless specified otherwise, the term "tautomer" refers to structural isomers of different energies which are interconvertible via a low energy barrier. If tautomerism is possible (for example, in solution), chemical equilibrium of tautomers can be reached. For example, proton tautomers (also known as prototropic tautomers) include interconversions via migration of a proton, such as keto-enol isomerization and imine-enamine isomerization. Valence tautomer includes interconversion through recombination of some bonding electrons.

Unless otherwise specified, the term "optionally substituted" means that the hydrogen at the substitutable position of said group is unsubstituted, or substituted by one or more substituents, preferably substituted by 1, 2 or 3 substituents, said substituents are preferably selected from: halogen, hydroxy, mercapto, cyano, nitro, amino, azido, oxo, carboxy, C₂₋₆alkenyl, C₂₋₆alkynyl, C₁₋₆alkyl, C₁₋₆alkoxyl, C₃₋₁₀cycloalkyl, C₃₋₁₀cycloalkylsulfonyl, 3-10 membered heterocycloalkyl, C₆₋₁₄ aryl or 5-10 membered heteroaryl, wherein said C₂₋₆alkenyl, C₂₋₆alkynyl, C₁₋₆alkyl, C₁₋₆alkoxyl, C₃₋₁₀cycloalkyl, C₃₋₁₀cycloalkylsulfonyl, 3-10 membered heterocycloalkyl, C₆₋₁₄ aryl or 5-10 membered heteroaryl may be optionally substituted by one or more substituents selected from halogen, hydroxy, amino, cyano, C₁₋₆alkyl and C₁₋₆alkoxyl, said "oxo" group means that two H's on the same substitution site are replaced by the same O to form a double bond.

The term "therapeutically effective amount" refers to a sufficient amount of a drug or agent that is non-toxic but can achieve the expected effect. In embodiments of the present invention, when treating a patient according to the present invention, the amount of a given drug depends on many factors, such as the specific dosage regimen, the type and severity of the disease or condition, the uniqueness (e.g., body weight) of the subject or host in need of treatment, however, depending on the particular circumstances, including for example the adopted specific drug, the administration route, the condition being treated, and the subject or host being treated, the administration dose can be routinely determined by methods known in the art. For the pharmaceutical composition of the present invention, it comprises a therapeutically effective amount of the compound of the present invention and a pharmaceutically acceptable excipient, wherein the "therapeutically effective amount" means that based on the weight of said pharmaceutical composition, said compound of the present invention comprises 1-99wt%, e.g. 20-80wt%, 30-70wt% or 45-55wt%, while the pharmaceutically acceptable excipient comprises 99-1wt%, e.g. 80-20wt%, 70-30wt% or 55-45wt%. Pharmaceutically acceptable excipients are those well known in the art and will not be described in detail herein.

### Beneficial effects of the present invention:

The present invention designs a class of compounds with novel structures, providing a new direction for the development of PDE4B inhibitor drugs. In vitro biochemical enzyme activity studies show that these compounds have strong inhibitory activity against the PDE4B enzyme with good selectivity. Mouse in vivo pharmacokinetic assay also shows good bioavailability and exposure with excellent metabolic characteristics, and therefore they can be promising compounds for treating PDE4B-mediated diseases.

### Detailed description

The present invention is further described below in connection with specific examples. It should be understood that these examples are intended to illustrate the present invention only and are not intended to limit the scope of the present invention. Experimental methods for which no specific conditions are indicated in the following examples are generally in accordance with conventional conditions or in accordance with the conditions recommended by the manufacturers. Unless otherwise defined, all professional and scientific terms used herein have the same meaning as those familiar to those skilled in the art. In addition, any methods and materials similar or equivalent to those described herein may be used in the methods of the present invention. The preferred embodiments and materials shown herein are illustrative only.

The structures of the compounds according to the present invention are determined by nuclear magnetic resonance (NMR) or/and liquid chromatograph mass spectrometry (LC-MS) or/and liquid chromatography (HPLC). The instrument used for NMR is Bruker 400 MHz and/or Varian 400 MHz; the instrument used for LC-MS is Agilent, 1260 Infinity II-6120/6125MSD; and the instrument used for HPLC is Waters UPCC(CA-352).

The starting materials in the examples of the present invention are known and commercially available, or can be synthesized using those methods known in the art or according to those methods known in the art.

This invention provides processes for preparing the compounds. The compounds can be prepared through the following steps.

### Preparation Example 1

Preparation of (R/S)-2-chloro-4-((1-(hydroxymethyl)cyclobutyl)amino)-6,7-dihydrothieno[3,2-d]pyrimidine 5-oxide:

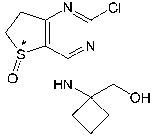

### Step 1: Preparation of (1-((2-chloro-6,7-dihydrothieno[3,2-d]pyrimidin-4-yl)amino)cyclobutyl)methanol:

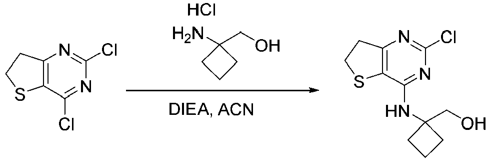

2,4-Dichloro-6,7-dihydrothieno[3,2-d]pyrimidine (6.3g), (1-aminocyclobutyl)methanol hydrochloride (4.6g) and N,N-diisopropylethylamine (DIEA)(15.6g) were dissolved in acetonitrile (ACN)(30mL), and then reacted at 85°C overnight. Then water (100mL) was added. The mixture was extracted with ethyl acetate (100mL×2), and washed with water (100mL×3). Then the organic phase was dried with anhydrous sodium sulfate, filtered and dried by rotary evaporation to produce a crude product. The crude product was separated and purified by flash chromatography (100-200 mesh silica gel, petroleum ether:ethyl acetate=0-50%) to produce a white solid. Then acetonitrile (20mL) and water (40mL) were added. The mixture was stirred at room temperature overnight. The solid was filtered by suction, and then washed with acetonitrile/water (1:2, 20mL). The filter cake was dried by rotary evaporation to produce the target compound (4.2g). LCMS(ESI)[M+H]⁺=272.10

### Step 2: Preparation of (R/S)-2-chloro-4-((1-(hydroxymethyl)cyclobutyl)amino)-6,7-dihydrothieno[3,2-d]pyrimidine 5-oxide:

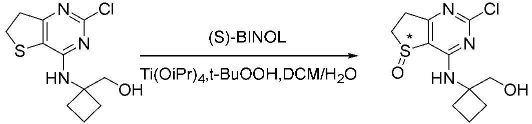

(1-((2-Chloro-6,7-dihydrothieno[3,2-d]pyrimidin-4-yl)amino)cyclobutyl)methanol (1g), Ti(OⁱPr)₄ (52.4mg) and S-binaphthol ((S)-BINOL)(105.6mg) were dissolved in dichloromethane (DCM)(5mL) and water (0.5mL), and then stirred under the protection of nitrogen gas for 0.5 hours. Then tert-butyl peroxide (t-BuOOH)(522mg, 70% aqueous solution) was added under an ice bath. The mixture was stirred at room temperature for 1 hour. LCMS detection showed the product appeared as the main peak. Then the solution was dried by rotary evaporation, and isopropyl acetate (15mL) was added. The mixture was ultrasonicated for 1 minute, then filtered, washed with isopropyl acetate (2mL), and dried to produce the target compound (1g). LCMS(ESI)[M+H]⁺=288.08

### Preparation Example 2

### Step 1: Preparation of tert-butyl 3-(4-chlorophenyl)-3-(methoxy-d3)azetidine-1-carboxylate:

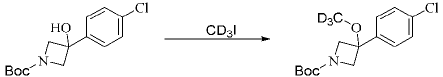

Tert-butyl 3-(4-chlorophenyl)-3-hydroxyazetidine-1-carboxylate (0.50g) was dissolved in DMF(5mL). The atmosphere was replaced with nitrogen gas three times. 60% NaH (141mg) was added under an ice bath. The mixture was stirred under the ice bath for 10 minutes. Iodomethane-d3 (0.51g) was added, and then the stirring continued under the ice bath for 30 minutes. A saturated NH₄Cl aqueous solution was added to quench the reaction, and water was added to the reaction. The mixture was extracted with methyl tert-butyl ether. The combined organic phases were washed with water and a saturated saline solution, dried over anhydrous sodium sulfate, filtered and dried by rotary evaporation to produce the target compound (700mg, crude).

### Step 2: Preparation of 3-(4-chlorophenyl)-3-(methoxy-d3)azetidine hydrochloride:

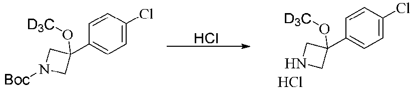

Tert-butyl 3-(4-chlorophenyl)-3-(methoxy-d3)azetidine-1-carboxylate (650mg) was added to a 4M solution of hydrogen chloride in ethyl acetate (5mL). The mixture was stirred at room temperature for 0.5 hours. The reaction solution was concentrated under a reduced pressure to produce a crude product. The crude product was slurrized with petroleum ether to produce the target compound (500mg). LCMS(ESI)[M+H]⁺=201.1.

### Preparation Example 3

### Step 1: Preparation of tert-butyl 3-(4-(difluoromethoxy)phenyl)-3-hydroxyazetidine-1-carboxylate:

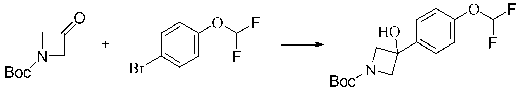

Under the protection of nitrogen gas, 1-bromo-4-(difluoromethoxy)benzene (5.08g) was dissolved in anhydrous tetrahydrofuran (40mL). At -78°C, a 2.5M n-butyl lithium/hexane solution (9.8mL) was added dropwise, and the mixture was reacted at -78°C for 0.5 hours to produce an off-white turbid liquid. Tert-butyl 3-oxoazetidine-1-carboxylate (3.00g) was dissolved in anhydrous tetrahydrofuran (10mL), and slowly added to the obtained liquid at -78°C. The mixture was stirred and reacted for 1 hour. The reaction was warmed up to 0°C, and a saturated ammonium chloride solution (20mL) was added to quench the reaction. Water (200mL) was added and the mixture was extracted with methyl tert-butyl ether. The combined organic phases were washed with a saturated NaCl solution, dried over anhydrous sodium sulfate, filtered and concentrated under a reduced pressure to produce a crude product. The crude product was purified by silica gel column chromatography (PE:EA=3:1) to produce the target compound (4.10g). LCMS(ESI)[M+H-56]⁺=260.2.

### Step 2: Preparation of tert-butyl 3-(4-(difluoromethoxy)phenyl)-3-methoxyazetidine-1-carboxylate:

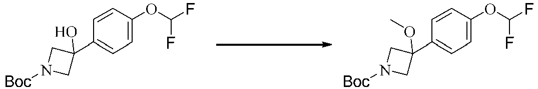

Tert-butyl 3-(4-(difluoromethoxy)phenyl)-3-hydroxyazetidine-1-carboxylate (4.00g) was dissolved in DMF(40mL). The atmosphere was replaced with nitrogen gas three times. 60% NaH (0.61g) was added under an ice bath. The mixture was stirred under the ice bath for 10 minutes. Iodomethane (2.70g) was added, and then the stirring continued under the ice bath for 1 hour. A saturated NH₄Cl aqueous solution was added to quench the reaction, and water (200mL) was added to the reaction. The mixture was extracted with methyl tert-butyl ether. The combined organic phases were washed with water and a saturated saline solution, dried over anhydrous sodium sulfate, filtered and concentrated under a reduced pressure to produce the target compound (4.46g).

### Step 3: Preparation of 3-(4-(difluoromethoxy)phenyl)-3-methoxyazetidine hydrochloride:

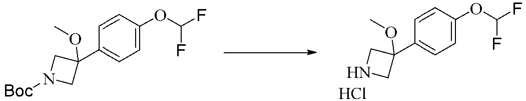

Tert-butyl 3-(4-(difluoromethoxy)phenyl)-3-methoxyazetidine-1-carboxylate (4.00g) was added to a 4M solution of hydrogen chloride in ethyl acetate (20mL). The mixture was stirred at room temperature for 0.5 hours. LCMS detection showed that the starting material was completely consumed. The reaction mixture was concentrated under a reduced pressure to produce the target compound (3.23g, crude).LCMS(ESI)[M+H]⁺=230.1.

### Preparation Example 4

The same method as that used in Preparation Example 3 was used except that 1-bromo-4-(difluoromethoxy)benzene was replaced with 1-bromo-4-(cyclopropylmethoxy)benzene to produce 3-(4-(cyclopropylmethoxy)phenyl)-3-methoxyazetidine hydrochloride. LCMS(ESI)[M+H]⁺=234.2.

### Preparation Example 5

The same method as that used in Preparation Example 3 was used except that 1-bromo-4-(difluoromethoxy)benzene was replaced with 1-(4-bromophenyl)pyrrolidine to produce 1-(4-(3-methoxyazetidin-3-yl)phenyl)pyrrolidine hydrochloride. LCMS(ESI)[M+H-MeO]⁺=201.3.

### Preparation Example 6

The same method as that used in Preparation Example 3 was used except that 1-bromo-4-(difluoromethoxy)benzene was replaced with 4-bromo-N,N-dimethylaniline to produce 4-(3-methoxyazetidin-3-yl)-N,N-dimethylaniline hydrochloride.

### Preparation Example 7

The same method as that used in Preparation Example 3 was used except that 1-bromo-4-(difluoromethoxy)benzene was replaced with 3-bromo-N,N-dimethylaniline to produce 3-(3-methoxyazetidin-3-yl)-N,N-dimethylaniline hydrochloride.

### Preparation Example 8

The same method as that used in Preparation Example 3 was used except that 1-bromo-4-(difluoromethoxy)benzene was replaced with 5-bromobenzo[d][1,3]dioxole to produce 3-(benzo[d][1,3]dioxol-5-yl)-3-methoxyazetidine hydrochloride. LCMS(ESI)[M+H]⁺=208.1.

### Preparation Example 9

The same method as that used in Preparation Example 3 was used except that 1-bromo-4-(difluoromethoxy)benzene was replaced with tert-butyl (4-bromophenyl)(methyl)carbamate to produce 4-(3-methoxyazetidin-3-yl)-N-methylaniline dihydrochloride. LCMS(ESI)[M+H]⁺=193.1.

### Example 1 (Compound 1)

### Synthesis of (R/S)-2-(3-(4-chlorophenyl)-3-hydroxyazetidin-1-yl)-4-((1-(hydroxymethyl)cyclobutyl)amino)-6,7-dihydrothieno[3,2-d]pyrimidine 5-oxide:

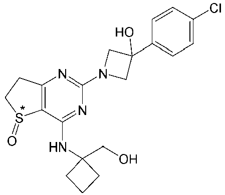

### Step 1: Preparation of tert-butyl 3-(4-chlorophenyl)-3-hydroxyazetidine-1-carboxylate:

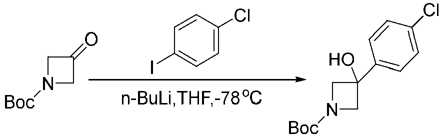

1-Chloro-4-iodobenzene (2g) was dissolved in anhydrous tetrahydrofuran (THF)(20mL). n-Butyl lithium (n-BuLi)(5.25mL, 1.6M solution in n-hexane) was added under the protection of nitrogen gas at -78°C. The system was stirred at -78°C for 10 minutes. Then a solution of tert-butyl 3-oxoazetidine-1-carboxylate (0.95g) in tetrahydrofuran (5mL) was added dropwise to the system. The mixture was reacted under stirring at -78°C for 1 hour. LCMS detection showed the product appeared as the main peak. Then an aqueous saturated ammonium chloride solution (20mL) was added to quench the reaction. The mixture was extracted with ethyl acetate (30mL×2). The organic phases were combined, dried over anhydrous sodium sulfate, and filtered. The filtrate was concentrated to produce a crude product. The crude product was separated and purified by flash chromatography (silica gel, petroleum ether:ethyl acetate=5:1-2:1) to produce the target compound (1.2g). LCMS(ESI)[M-55]⁺=228.14.

### Step 2: Preparation of 3-(4-chlorophenyl)azetidin-3-ol trifluoroacetate:

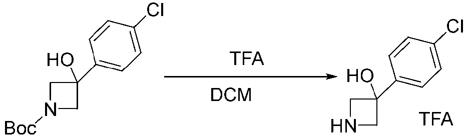

Tert-butyl 3-(4-chlorophenyl)-3-hydroxyazetidine-1-carboxylate (200mg) was dissolved in dichloromethane (2mL), and added to trifluoroacetic acid (TFA)(1mL). The mixture was reacted under stirring at room temperature for 1 hour. LCMS detection showed the reaction was completed. Then the solution was dried by rotary evaporation to produce the target compound (crude). LCMS(ESI)[M+H]⁺=184.14.

### Step 3: Preparation of (R/S)-2-(3-(4-chlorophenyl)-3-hydroxyazetidin-1-yl)-4-((1-(hydroxymethyl)cyclobutyl)amino)-6,7-dihydrothieno[3,2-d]pyrimidine 5-oxide:

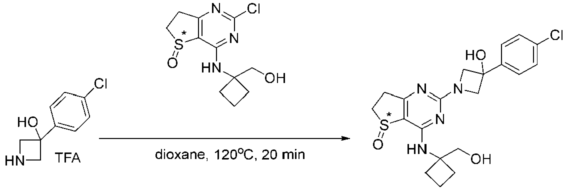

3-(4-chlorophenyl)azetidin-3-ol trifluoroacetate (200mg, crude, obtained from the previous step) was dissolved in dioxane (4mL). N,N-diisopropylethylamine (1mL) was added. The system was stirred evenly and then (R/S)-2-chloro-4-((1-(hydroxymethyl)cyclobutyl)amino)-6,7-dihydrothieno[3,2-d]pyrimidine 5-oxide (70mg) was added. The system was reacted under microwave at 120°C for 20 minutes. LCMS detection showed the reaction was completed. Water (20mL) was added. The mixture was extracted with ethyl acetate (30mL×2). The organic phases were combined, dried over anhydrous sodium sulfate, and filtered. The filtrate was concentrated to produce the target compound (crude). The crude target compound was added to ethyl acetate (15mL), and the mixture was stirred at 85°C overnight. The system was cooled and filtered to produce the target compound (63.3mg). LCMS(ESI)[M+H]⁺=435.28; Chiral HPLC: ee%=100%; ¹H NMR(400 MHz, DMSO-*d*₆)δ 7.54(d, J=8.4Hz, 2H), 7.44(d, J=8.8Hz, 3H), 6.48(s, 1H), 4.85(t, J=5.5Hz, 1H), 4.19(s, 4H), 3.70(d, J=5.5Hz, 2H), 3.51-3.36(m, 1H), 3.26-3.15(m, 1H), 2.92(ddd, J=20.7, 15.4, 7.4Hz, 2H), 2.41-2.25(m, 2H), 2.12(t, J=9.4Hz, 2H), 1.73(dt, J=19.7, 10.2Hz, 2H).

### Example 2 (Compound 2)

### Synthesis of (R/S)-2-(3-hydroxy-3-(4-methoxyphenyl)azetidin-1-yl)-4-((1-(hydroxymethyl)cyclobutyl)amino)-6,7-dihydrothieno[3,2-d]pyrimidine 5-oxide:

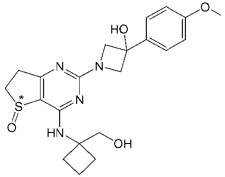

### Step 1: tert-butyl 3-hydroxy-3-(4-methoxyphenyl)azetidine-1-carboxylate:

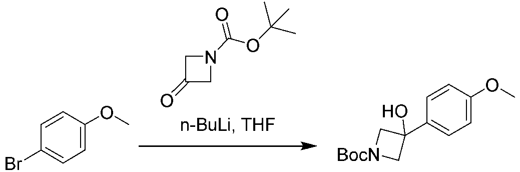

1-bromo-4-methoxybenzene (2g) was dissolved in tetrahydrofuran (40mL). n-Butyl lithium (4.3mL, 2.5M solution in n-hexane) was added dropwise under the protection of nitrogen gas at -78°C. The mixture was stirred for 0.5 hours while keeping the same temperature. Tert-butyl 3-oxoazetidine-1-carboxylate (2.75g) was dissolved in tetrahydrofuran (10mL). This solution was added dropwise to the above lithium reagent solution at -78°C. The mixture was stirred for 0.5 hours while keeping the same temperature. LCMS detection showed the formation of a new product. The reaction mixture was poured into water (100mL), and extracted with ethyl acetate (30mL×3). The organic phases were dried over sodium sulfate, and dried by rotary evaporation to produce a crude product. The crude product was purified by silica gel column chromatography (petroleum ether/ethyl acetate=3/1) to produce the target compound (1.9g). LCMS:(ESI)[M-Boc-OH+H]⁺=162.1.

### Step 2: Preparation of 3-(4-methoxyphenyl)azetidin-3-ol trifluoroacetate:

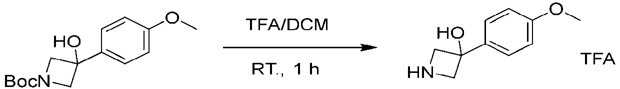

Tert-butyl 3-hydroxy-3-(4-methoxyphenyl)azetidine-1-carboxylate (145mg) was dissolved in dichloromethane (2mL), and added to trifluoroacetic acid (1mL) at room temperature. The mixture was stirred at room temperature for 1 hour. LCMS detection showed the raw material disappeared. The reaction mixture was dried by rotary evaporation to produce the target compound (150mg).

### Step 3: Preparation of (R/S)-2-(3-hydroxy-3-(4-methoxyphenyl)azetidin-1-yl)-4-((1-(hydroxymethyl)cyclobutyl)amino)-6,7-dihydrothieno[3,2-d]pyrimidine 5-oxide:

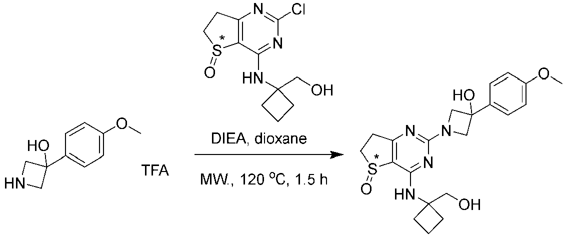

(R/S)-2-chloro-4-((1-(hydroxymethyl)cyclobutyl)amino)-6,7-dihydrothieno[3,2-d]pyrimidine 5-oxide (100mg), 3-(4-methoxyphenyl)azetidin-3-ol trifluoroacetate (153.95mg), and N,N-diisopropylethylamine (226.17mg) were dissolved in dioxane (2mL). The mixture was reacted at 120°C under microwave for 1.5 hours. LCMS detection showed the reaction was completed. The reaction mixture was directly separated and purified by Prep-HPLC (C18, 10 mmol/L, aqueous ammonium bicarbonate solution, acetonitrile) to produce the target compound (40.3mg). LCMS:(ESI)[M+H]⁺=431.33; ¹H NMR(400 MHz, DMSO-*d*₆+D₂O)δ 7.38(d, J=8.4Hz, 2H), 6.92(d, J=8.5Hz, 2H), 4.30-4.05(m, 4H), 3.75-3.63(m, 5H), 3.47-3.35(m, 1H), 3.30-3.19(m, 1H), 3.01-2.85(m, 2H), 2.34-2.19(m, 2H), 2.17-2.00(m, 2H), 1.84-1.64(m, 2H).

### Example 3 (Compound 3)

### Synthesis of (R/S)-2-(3-(4-chloro-3-methoxyphenyl)-3-hydroxyazetidin-1-yl)-4-((1-(hydroxymethyl)cyclobutyl)amino)-6,7-dihydrothieno[3,2-d]pyrimidine 5-oxide:

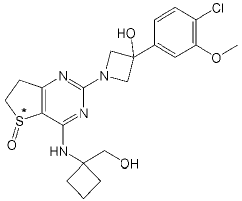

### Step 1: tert-butyl 3-hydroxy-3-(4-chloro-3-methoxyphenyl)azetidine-1-carboxylate:

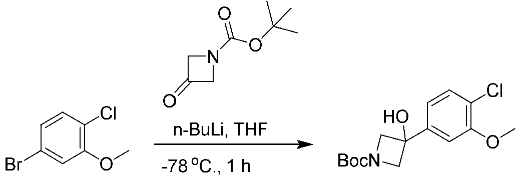

4-bromo-1-chloro-2-methoxybenzene (2g) was dissolved in tetrahydrofuran (40mL). n-Butyl lithium (3.6mL, 2.5M solution in n-hexane) was added dropwise at -78°C under the protection of nitrogen gas. The mixture was stirred for 0.5 hours. Tert-butyl 3-oxoazetidine-1-carboxylate (2.32g) was dissolved in tetrahydrofuran (15mL). This solution was added dropwise to the above lithium reagent solution at -78°C. The mixture was stirred for 0.5 hours. LCMS detection showed the formation of the product. Water (10mL) was added dropwise at -78°C to quench the reaction. The reaction mixture was poured into water (300mL), and extracted with ethyl acetate (100mL×3). The organic phases were washed with water (200mL×3), dried over anhydrous sodium sulfate, and dried by rotary evaporation to produce a crude product. The crude product was purified by silica gel column chromatography (petroleum ether/ethyl acetate=3/1) to produce the target compound (1.3g). LCMS:(ESI)[M-OH+H]⁺=196.17.

### Step 2: Preparation of 3-(4-chloro-3-methoxyphenyl)azetidin-3-ol trifluoroacetate:

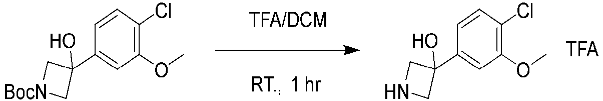

Tert-butyl 3-hydroxy-3-(4-chloro-3-methoxyphenyl)azetidine-1-carboxylate (145mg) was dissolved in dichloromethane (2mL), and added to trifluoroacetic acid (1mL) at room temperature. The mixture was stirred at room temperature for 1 hour. LCMS detection showed the raw material disappeared. The reaction mixture was dried by rotary evaporation to produce the target compound (160mg).

### Step 3: Preparation of (R/S)-2-(3-(4-chloro-3-methoxyphenyl)-3-hydroxyazetidin-1-yl)-4-((1-(hydroxymethyl)cyclobutyl)amino)-6,7-dihydrothieno[3,2-d]pyrimidine 5-oxide:

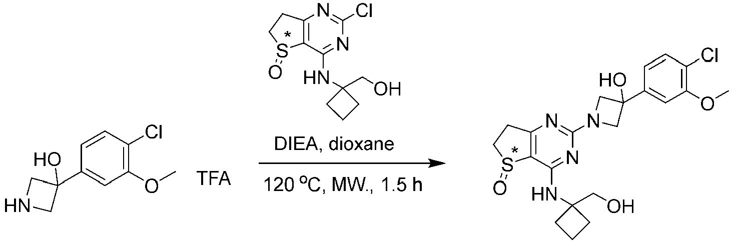

(R/S)-2-chloro-4-((1-(hydroxymethyl)cyclobutyl)amino)-6,7-dihydrothieno[3,2-d]pyrimidine 5-oxide (100mg), 3-(4-chloro-3-methoxyphenyl)azetidin-3-ol trifluoroacetate (153.95mg), and N,N-diisopropylethylamine (DIEA)(226.17mg) were dissolved in dioxane (2mL). The mixture was reacted at 120°C under microwave for 1.5 hours. LCMS detection showed the raw material disappeared. The reaction mixture was separated and purified to produce the target product (31.9mg). LCMS:(ESI)[M+H]⁺=465.2; ¹H NMR(400 MHz, DMSO-*d*₆)δ 7.52-7.37(m, 2H), 7.25(d, *J*=1.9Hz, 1H), 7.08(d, *J*=8.3Hz, 1H), 6.50(s, 1H), 4.85(t, *J*=5.6Hz, 1H), 4.40-4.15(m, 4H), 3.86(s, 3H), 3.70(d, *J*=5.8Hz, 2H), 3.48-3.37(m, 1H), 3.25-3.15(m, 1H), 3.10-2.85(m, 2H), 2.43-2.26(m, 2H), 2.20-2.15(m, 2H), 1.85-1.65(m, 2H).

### Example 4 (Compound 4)

### Synthesis of (R/S)-2-(3-(3-chloro-4-methoxyphenyl)-3-hydroxyazetidin-1-yl)-4-((1-(hydroxymethyl)cyclobutyl)amino)-6,7-dihydrothieno[3,2-d]pyrimidine 5-oxide:

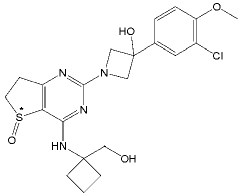

### Step 1: Preparation of tert-butyl 3-(3-chloro-4-methoxyphenyl)-3-hydroxyazetidine-1-carboxylate:

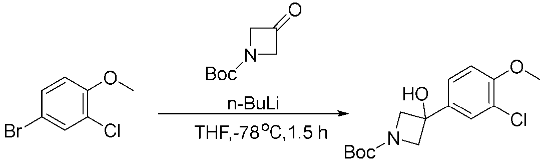

4-bromo-2-chloro-1-methoxybenzene (2.5g) was dissolved in anhydrous tetrahydrofuran (20mL). The system was cooled to -78°C under the protection of nitrogen gas. Then n-butyl lithium (7mL, 1.6M solution in n-hexane) was added. The system was reacted at -78°C for 0.5 hours. Then a solution of tert-butyl 3-oxoazetidine-1-carboxylate (1.28g) in tetrahydrofuran (2mL) was slowly added dropwise to the reaction solution. Then the mixture was reacted under stirring at -78°C for 1 hour. LCMS detection showed the product appeared as the main peak. Then an aqueous ammonium chloride solution (10mL) was added to quench the reaction. The aqueous phase was extracted with ethyl acetate (30mL×2). The combined organic phases were dried over anhydrous sodium sulfate, and dried by rotary evaporation to produce a crude product. The crude product was separated and purified by flash chromatography (Silica gel, petroleum ether:ethyl acetate=3:1-2:1) to produce the target compound (1.57g). LCMS(ESI)[M+H]⁺=258.12.

### Step 2: Preparation of 3-(3-chloro-4-methoxyphenyl)azetidin-3-ol trifluoroacetate:

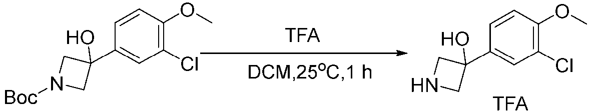

Tert-butyl 3-(3-chloro-4-methoxyphenyl)-3-hydroxyazetidine-1-carboxylate (120mg) was dissolved in dichloromethane (2mL), and added to trifluoroacetic acid (1mL). The system was stirred at room temperature for 1 hour. LCMS detection showed the product appeared as the main peak. Then the solvent was removed by rotary evaporation to produce the target compound (crude), which was not further purified but directly used in the next step. LCMS(ESI)[M+H]⁺=214.17.

### Step 3: Preparation of (R/S)-2-(3-(3-chloro-4-methoxyphenyl)-3-hydroxyazetidin-1-yl)-4-((1-(hydroxymethyl)cyclobutyl)amino)-6,7-dihydrothieno[3,2-d]pyrimidine 5-oxide:

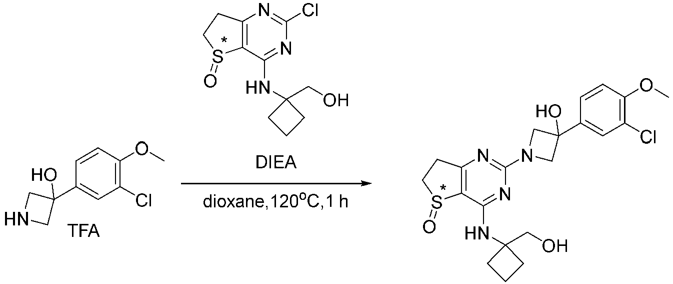

(R/S)-2-chloro-4-((1-(hydroxymethyl)cyclobutyl)amino)-6,7-dihydrothieno[3,2-d]pyrimidine 5-oxide (60mg), 3-(3-chloro-4-methoxyphenyl)azetidin-3-ol trifluoroacetate (80mg, crude) and N,N-diisopropylethylamine (0.5mL) were dissolved in dioxane (3mL). The system was reacted at 120°C for 1 hour. LCMS detection showed the product appeared as the main peak. Then the solvent was removed by rotary evaporation to produce a crude product. The crude product was separated and purified by Prep-HPLC (C18, 10mmol/L NH₄HCO₃/water, MeCN) to produce the target compound (62.66mg). LCMS(ESI)[M+H]⁺=465.34; ¹H NMR(400 MHz, DMSO-*d*₆)δ 7.52(d, J=2.0Hz, 1H), 7.43(dd, J=7.5, 3.2Hz, 2H), 7.16(d, J=8.7Hz, 1H), 6.42(s, 1H), 4.85(t, J=5.7Hz, 1H), 4.16(s, 4H), 3.85(s, 3H), 3.70(d, J=5.8Hz, 2H), 3.49-3.36(m, 1H), 3.26-3.16(m, 1H), 3.04-2.80(m, 2H), 2.34(td, J=21.4, 9.7Hz, 2H), 2.12(t, J=9.4Hz, 2H), 1.85-1.62(m, 2H).

### Example 5 (Compound 5)

### Synthesis of (R/S)-2-(3-hydroxy-3-(3-methoxyphenyl)azetidin-1-yl)-4-((1-(hydroxymethyl)cyclobutyl)amino)-6,7-dihydrothieno[3,2-d]pyrimidine 5-oxide:

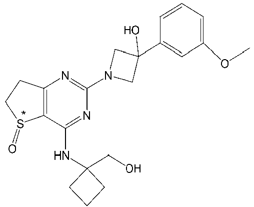

### Step 1: Preparation of tert-butyl 3-hydroxy-3-(3-methoxyphenyl)azetidine-1-carboxylate:

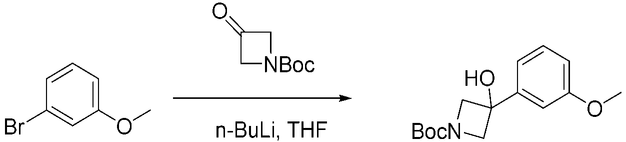

Under the protection of nitrogen gas, 1-bromo-3-methoxybenzene (1.87g) was dissolved into anhydrous tetrahydrofuran (30mL), and under cooling with an external dry ice ethanol bath to keep the internal temperature lower than -65°C, n-butyl lithium (1.6M solution in n-hexane, 6.6mL) was added dropwise under stirring. After the completion of the dropwise addition, the mixture was reacted for 30 minutes while keeping the same temperature. Tert-butyl 3-oxoazetidine-1-carboxylate (1.88g) was dissolved in tetrahydrofuran (8mL), and added to the reaction mixture while keeping the internal temperature lower than -60°C. After reacting for 30 minutes, LCMS detection showed the raw material was completely consumed, and the cooling bath was removed. Water (50mL) was added. The reaction mixture was naturally warmed up to room temperature, and extracted with ethyl acetate (100mL×3). The organic phases were combined. The combined organic phases were washed with a saturated saline solution (100mL), dried over anhydrous sodium sulfate, and filtered. The filtrate was concentrated under a reduced pressure to produce a crude product. The crude product was purified by flash silica gel column chromatography (silica gel, petroleum ether:tetrahydrofuran=100:0 to 50:50 gradient dilution) to produce the target compound (1.20g). LCMS(ESI)[2M+H]⁺=559.4;

### Step 2: Preparation of 3-(3-methoxyphenyl)azetidin-3-ol hydrochloride:

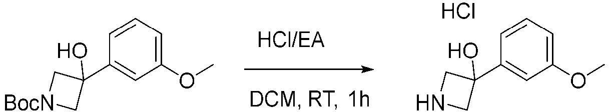

Under the protection of nitrogen gas, tert-butyl 3-hydroxy-3-(3-methoxyphenyl)azetidine-1-carboxylate (1.20g) was dissolved in dichloromethane (20mL), and the mixture was stirred under an ice-water bath for 10 minutes. At 0°C, a 4M solution of hydrogen chloride in ethyl acetate (EA)(3.23mL) was added to the reaction mixture. The ice-water bath was removed, and the mixture was warmed up to room temperature and reacted for 1 hour. LCMS detection showed that the raw material was completely consumed and converted to the product. The reaction mixture was filtered. The filter cake was washed with dichloromethane (5mL), and concentrated to dryness to produce the target compound (0.83g). LCMS(ESI)[M+H]⁺=216.1.

### Step 3: Preparation of (R/S)-2-(3-hydroxy-3-(3-methoxyphenyl)azetidin-1-yl)-4-((1-(hydroxymethyl)cyclobutyl)amino)-6,7-dihydrothieno[3,2-d]pyrimidine 5-oxide:

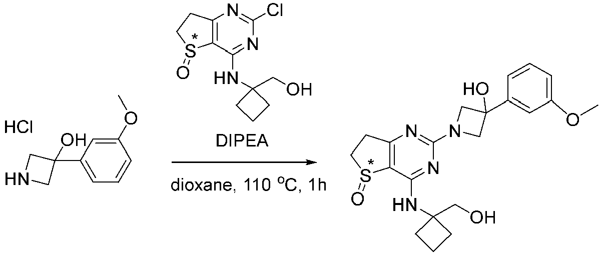

Under the protection of nitrogen gas, (R/S)-2-chloro-4-((1-(hydroxymethyl)cyclobutyl)amino)-6,7-dihydrothieno[3,2-d]pyrimidine 5-oxide (100mg) and 3-(3-methoxyphenyl)azetidin-3-ol hydrochloride (90mg) were added to dioxane (5mL). Then N,N-diisopropylethylamine (114mg) was added. After the atmosphere was replaced with nitrogen gas three times, the reaction mixture was warmed up to 110°C and reacted for 1 hour. LCMS detection showed that the raw material was completely consumed and converted to the product. The heating was removed, and the reaction mixture was concentrated to dryness. The residue was separated and purified by Prep-HPLC (C18, 10mmol/L NH₄HCO₃/water, MeCN) to produce the target compound (118.5mg). LCMS(ESI)[M+H]⁺=431; ¹H NMR(400 MHz, DMSO-*d*₆)δ 7.43(s, 1H), 7.31(t, J=7.9Hz, 1H), 7.07(t, J=5.1Hz, 2H), 6.89-6.82(m, 1H), 6.37(s, 1H), 4.86(t, J=5.7Hz, 1H), 4.17(s, 4H), 3.76(s, 3H), 3.70(d, J=5.7Hz, 2H), 3.48-3.36(m, 1H), 3.23(dt, J=13.5, 8.3Hz, 1H), 3.02-2.83(m, 2H), 2.34(td, J=21.4, 9.7Hz, 2H), 2.12(t, J=9.3Hz, 2H), 1.87-1.63(m, 2H).

### Example 6 (Compound 11)

### Synthesis of (R/S)-2-(3-(4-chlorophenyl)-3-hydroxypyrrolidin-1-yl)-4-((1-(hydroxymethyl)cyclobutyl)amino)-6,7-dihydrothieno[3,2-d]pyrimidine 5-oxide:

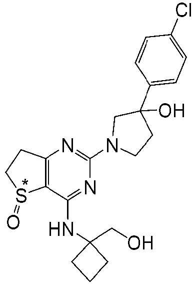

### Step 1: Preparation of tert-butyl 3-(4-chlorophenyl)-3-hydroxypyrrolidine-1-carboxylate

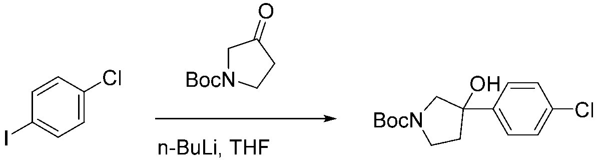

Under the protection of nitrogen gas, 1-chloro-4-iodobenzene (2.38g) was dissolved into anhydrous tetrahydrofuran (30mL). Under cooling with an external dry ice ethanol bath to keep the internal temperature lower than -65°C, n-butyl lithium (1.6M solution in n-hexane, 6.6mL) was added dropwise under stirring. After the completion of the dropwise addition, the mixture was reacted for 30 minutes while keeping the same temperature. Tert-butyl 3-oxopyrrolidine-1-carboxylate (2.03g) was dissolved in tetrahydrofuran (8mL), and added to the reaction mixture while keeping the internal temperature lower than -60°C. After reacting for 30 minutes, LCMS detection showed the raw material was completely consumed, and the cooling bath was removed. Water (50mL) was added. The reaction mixture was naturally warmed up to room temperature, and extracted with ethyl acetate (100mL×3). The phases were separated. The organic phases were combined, washed with a saturated saline solution (100mL), dried over anhydrous sodium sulfate, and concentrated under a reduced pressure to produce a crude product. The crude product was purified by flash silica gel column chromatography (silica gel, petroleum ether:tetrahydrofuran=100:0 to 50:50 gradient dilution) to produce the target compound (1.34g). LCMS(ESI)[2M+Na⁺]⁺=617.3;

### Step 2: Preparation of 3-(4-chlorophenyl)pyrrolidin-3-ol hydrochloride:

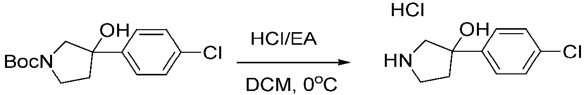

Under the protection of nitrogen gas, tert-butyl 3-(4-chlorophenyl)-3-hydroxypyrrolidine-1-carboxylate (1.34g) was dissolved in dichloromethane (20mL), and the mixture was stirred under an ice-water bath for 10 minutes. At 0°C, a 4M solution of hydrogen chloride in ethyl acetate (3.38mL) was added to the reaction mixture. The ice-water bath was removed, and the reaction mixture was warmed up to 20°C and reacted for 1 hour. LCMS detection showed the raw material was completely consumed, and the cooling bath was removed. A white solid was separated from the reaction. The reaction mixture was filtered, and the filter cake was washed with dichloromethane (5mL), and concentrated to dryness to produce the target product (0.90g). LCMS(ESI)[M+H]⁺=198.1;

### Step 3: Preparation of (5R/S)-2-(3-(4-chlorophenyl)-3-hydroxypyrrolidin-1-yl)-4-((1-(hydroxymethyl)cyclobutyl)amino)-6,7-dihydrothieno[3,2-d]pyrimidine 5-oxide:

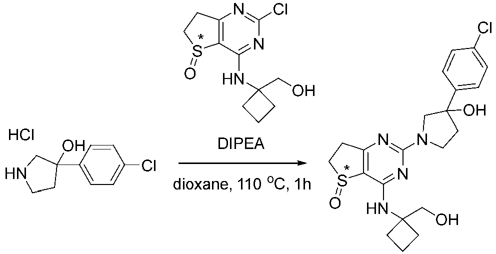

Under the protection of nitrogen gas, (R/S)-2-chloro-4-((1-(hydroxymethyl)cyclobutyl)amino)-6,7-dihydrothieno[3,2-d]pyrimidine 5-oxide (100mg) and 3-(4-chlorophenyl)pyrrolidin-3-ol hydrochloride (98mg) were added to dioxane (5mL). Then N,N-diisopropylethylamine (114mg) was added. After the atmosphere was replaced with nitrogen gas three times, the reaction mixture was warmed up to 110°C and reacted for 1 hour. LCMS detection showed that the raw material was completely consumed and converted to the product. The heating was removed, and the reaction mixture was concentrated to dryness. The residue was separated and purified by Prep-HPLC (C18, 10mmol/L NH₄HCO₃/water, MeCN) to produce the target compound (83mg). LCMS(ESI)[M+H]⁺=449/451; ¹H NMR(400 MHz, DMSO-*d*₆)δ 7.56(d, J=8.5Hz, 2H), 7.46-7.37(m, 2H), 7.32-7.20(m, 1H), 5.55(dd, J=19.9, 11.6Hz, 1H), 4.86(dt, J=18.9, 5.6Hz, 1H), 3.91-3.70(m, 3H), 3.70-3.55(m, 3H), 3.49-3.35(m, 1H), 3.28-3.15(m, 1H), 3.01-2.80(m, 2H), 2.47-2.25(m, 3H), 2.23-2.02(m, 3H), 1.87-1.61(m, 2H).

### Example 7 (Compound 12)

### Synthesis of (5R/S)-2-(3-(5-chloropyrimidin-2-yl)pyrrolidin-1-yl)-4-((1-(hydroxymethyl)cyclobutyl)amino)-6,7-dihydrothieno[3,2-d]pyrimidine 5-oxide:

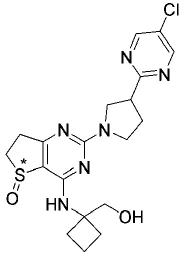

### Step 1: Preparation of pyrrolidine-3-carbonitrile hydrochloride:

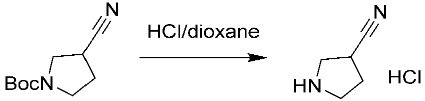

Tert-butyl 3-cyanopyrrolidine-1-carboxylate (5g) was dissolved in dioxane (20mL). A 4M solution of HCl/dioxane (50mL) was added at room temperature. The mixture was stirred at room temperature for 2 hours. The reaction mixture was dried by rotary evaporation to produce the target compound (3.38g).

### Step 2: Preparation of pyrrolidine-3-carboximidamide hydrochloride:

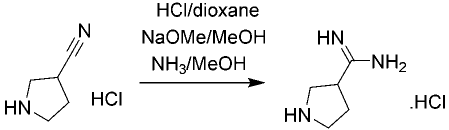

Pyrrolidine-3-carbonitrile hydrochloride (2.4g) was dissolved in HCl/dioxane (10mL). Methanol (MeOH)(1.74g) was added under an ice-water bath, while keeping the temperature lower than 10°C. The above mixture was stirred at room temperature for 1 hour. Sodium methoxide (NaOMe)(1.96g) was dissolved in anhydrous methanol (8mL) to formulate into a sodium methoxide solution. The sodium methoxide solution was slowly added to the above mixture, while keeping the temperature lower than 15°C. The resulting mixture was stirred for 1 hour. Finally, a 7M solution of ammonia/methanol (8mL) was added to the above mixture. The resulting mixture was stirred at room temperature overnight, and concentrated under a reduced pressure to produce the target compound (crude), which was not further purified but directly used in the next step.

### Step 3: Preparation of 5-chloro-2-(pyrrolidin-3-yl)pyrimidine 4-methylbenzenesulfonate:

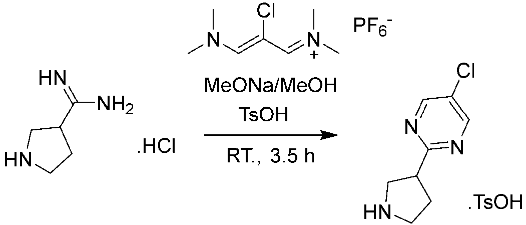

To the solution of pyrrolidine-3-carboximidamide hydrochloride (obtained from the previous step) in dioxane was added a solution of sodium methoxide (2.39g) in anhydrous methanol (40mL). The mixture was stirred at room temperature for 30 minutes. (Z)-N-(2-chloro-3-(dimethylamino)allylidene)-N-methylmethanaminium hexafluorophosphate (4.34g) was added. The mixture was continuously stirred at room temperature for 3.5 hours. The mixture was concentrated under a reduced pressure to about 60 mL. 2-methyltetrahydrofuran (80mL) was added, and then the mixture was concentrated to about 100 mL. Then 2-methyltetrahydrofuran (80mL) was added. The mixture was cooled to 20°C. Water (150mL) was added. The mixture was stirred for 5 minutes. The mixture was allowed to stand for phase separation and the organic layer was collected. The organic layer was washed with a 30% aqueous NaOH solution (120mL) and collected. The organic layer was concentrated to 50 mL. n-Propanol (20mL) was added. A solution of 4-methylbenzenesulfonic acid (TsOH)(2.43g) in n-butanol (20mL) was slowly added dropwise over 10 minutes at 65°C. The reaction mixture was concentrated under a reduced pressure to 50mL, cooled to room temperature, and filtered to produce the target compound (6.6g, crude). LCMS:(ESI)[M+H]⁺=184.17.

### Step 4: Preparation of (R/S)-2-(3-(5-chloropyrimidin-2-yl)pyrrolidin-1-yl)-4-((1-(hydroxymethyl)cyclobutyl)amino)-6,7-dihydrothieno[3,2-d]pyrimidine 5-oxide:

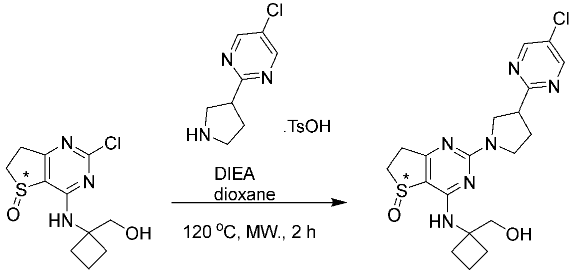

(R/S)-2-chloro-4-((1-(hydroxymethyl)cyclobutyl)amino)-6,7-dihydrothieno[3,2-d]pyrimidine 5-oxide (50mg), 5-chloro-2-(pyrrolidin-3-yl)pyrimidine 4-methylbenzenesulfonate (74.19mg), and N,N-diisopropylethylamine (65.91mg) were dissolved in dioxane (2mL). The mixture was heated at 120°C under microwave (MW) for 2 hours. LCMS detection showed the raw material disappeared. The reaction mixture was poured into water (100mL), and extracted with ethyl acetate (30mL×3). The organic phases were dried over sodium sulfate, and dried by rotary evaporation to produce a crude product. The crude product was separated and purified by Prep-HPLC (C18, 10 mmol/L, NH₄HCO₃/water, MeCN) to produce the target compound (4.15mg). LCMS:(ESI)[M+H]⁺=435.29; ¹HNMR(400 MHz, DMSO-*d*₆)δ 8.90(s, 2H), 7.23(s, 1H), 4.84(s, 1H), 4.00-3.55(m, 7H), 3.46-3.35(m, 1H), 3.25-3.15(m, 1H), 3.00-2.80(m, 2H), 2.42-2.10(m, 6H), 1.88-1.64(m, 2H).

### Example 8 (Compound 13 and compound 14)

### Synthesis of (R/S)-2-(3-(4-(difluoromethoxy)phenyl)-3-hydroxypyrrolidin-1-yl)-4-((1-(hydroxymethyl)cyclobutyl)amino)-6,7-dihydrothieno[3,2-d]pyrimidine 5-oxide (Compound 13) and (R/S)-2-(3-(4-(difluoromethoxy)phenyl)-2,5-dihydro-1H-pyrrol-1-yl)-4-((1-(hydroxymethyl)cyclobutyl)amino)-6,7-dihydrothieno[3,2-d]pyrimidine 5-oxide (Compound 14):

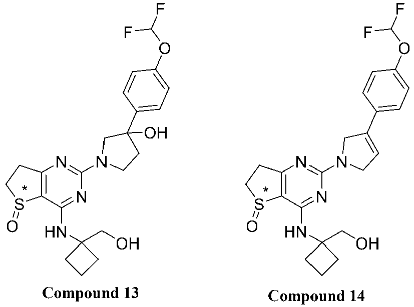

### Step 1: Preparation of tert-butyl 3-(4-(difluoromethoxy)phenyl)-3-hydroxypyrrolidine-1-carboxylate:

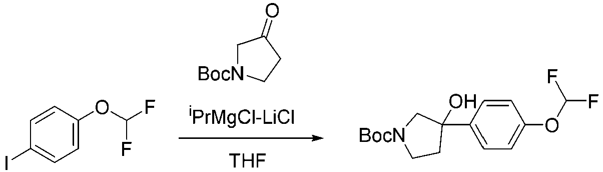

Under the protection of nitrogen gas, 1-(difluoromethoxy)-4-iodobenzene (2.7g) was dissolved into anhydrous tetrahydrofuran (30mL). Under cooling with an external dry ice ethanol bath to keep the internal temperature lower than -65°C, isopropyl magnesium chloride-lithium chloride (1.3M, 8mL) was added dropwise under stirring. After the completion of the dropwise addition, the mixture was kept at about -65°C and reacted for 30 minutes. Tert-butyl 3-oxopyrrolidine-1-carboxylate (2.03g) was dissolved in tetrahydrofuran (8mL), and added to the reaction mixture while keeping the internal temperature lower than -60°C. After reacting for 30 minutes, LCMS detection showed the raw material was completely consumed, and the cooling bath was removed. Water (100mL) was added. The reaction mixture was naturally warmed up to room temperature, and extracted with ethyl acetate (100mL×3). The phases were separated. The organic phases were combined, washed with a saturated saline solution (100mL), dried over anhydrous sodium sulfate, concentrated under a reduced pressure to produce a crude product. The crude product was purified by flash silica gel column chromatography (silica gel, petroleum ether:tetrahydrofuran=100:0 to 50:50 gradient dilution) to produce the target compound (0.8g). LCMS(ESI)[2M+Na]⁺=681.5;

### Step 2: Preparation of 3-(4-(difluoromethoxy)phenyl)pyrrolidin-3-ol hydrochloride and 3-(4-(difluoromethoxy)phenyl)-2,5-dihydro-1H-pyrrole hydrochloride:

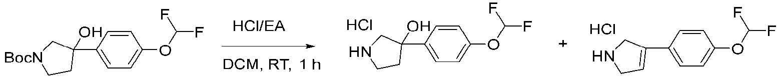

Under the protection of nitrogen gas, tert-butyl 3-(4-(difluoromethoxy)phenyl)-3-hydroxypyrrolidine-1-carboxylate (0.8g) was dissolved in dichloromethane (20mL). The mixture was stirred under an ice-water bath for 10 minutes. A 4M solution of hydrogen chloride in ethyl acetate (3mL) was added to the reaction mixture at 0°C. The ice-water bath was removed, and the mixture was warmed up to room temperature and reacted for 1 hour. LCMS detection showed that the raw material was completely consumed and converted into the product and the dehydration by-product. The reaction mixture was concentrated to dryness to produce the target compound (400mg, crude). LCMS(ESI)[M+H]⁺=230;

### Step 3: Preparation of (R/S)-2-(3-(4-(difluoromethoxy)phenyl)-3-hydroxypyrrolidin-1-yl)-4-((1-(hydroxymethyl)cyclobutyl)amino)-6,7-dihydrothieno[3,2-d]pyrimidine 5-oxide (Compound 13) and (R/S)-2-(3-(4-(difluoromethoxy)phenyl)-2,5-dihydro-1H-pyrrol-1-yl)-4-((1-(hydroxymethyl)cyclobutyl)amino)-6,7-dihydrothieno[3,2-d]pyrimidine 5-oxide (Compound 14):

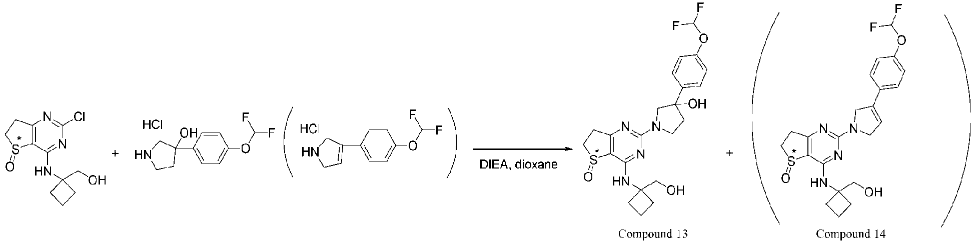

Under the protection of nitrogen gas, (R/S)-2-chloro-4-((1-(hydroxymethyl)cyclobutyl)amino)-6,7-dihydrothieno[3,2-d]pyrimidine 5-oxide (100mg) and 3-(4-(difluoromethoxy)phenyl)pyrrolidin-3-ol hydrochloride and 3-(4-(difluoromethoxy)phenyl)-2,5-dihydro-1H-pyrrole hydrochloride (148mg, a crude mixture product, obtained from the previous step) were added to dioxane (5mL). Then N,N-diisopropylethylamine (137mg) was added. After the atmosphere was replaced with nitrogen gas three times, the reaction mixture was warmed up to 110°C and reacted for 1 hour. LCMS detection showed that the raw material was completely consumed and converted into the target product. The reaction mixture was concentrated to dryness, and the residue was separated and purified by Prep-HPLC (C18, 10mmol/L NH₄HCO₃/water, MeCN) to produce the target compound (5R/S)-2-(3-(4-(difluoromethoxy)phenyl)-3-hydroxypyrrolidin-1-yl)-4-((1-(hydroxymethyl)cyclobutyl)amino)-6,7-dihydrothieno[3,2-d]pyrimidine 5-oxide (Compound 13)(47mg) LCMS(ESI)[M+H]⁺=481; ¹H NMR(400 MHz, DMSO-*d*₆)67.63-7.55(m, 2H), 7.41 -7.04(td, J=72Hz, 1H), 7.26-7.24(m, 1H), 7.19-7.15(m, 2H), 7.04(d, J=2.2Hz, 1H), 5.51(dd, J=21.0, 11.8Hz, 1H), 4.86(dt, J=18.8, 5.5Hz, 1H), 3.94-3.56(m, 6H), 3.48-3.36(m, 1H), 3.22(dd, J=8.4, 5.2Hz, 1H), 3.01-2.80(m, 2H), 2.35(tt, J=20.3, 10.0Hz, 3H), 2.23-2.02(m, 3H), 1.75(pd, J=20.0, 10.6Hz, 2H); and the target compound (R/S)-2-(3-(4-(difluoromethoxy)phenyl)-2,5-dihydro-1H-pyrrol-1-yl)-4-((1-(hydroxymethyl)cyclobutyl)amino)-6,7-dihydrothieno[3,2-d]pyrimidine 5-oxide (Compound 14)(45mg). LCMS(ESI)[M+H]⁺=463; ¹H NMR(400 MHz, DMSO-*d*₆)δ 7.61(d, J=8.7Hz, 1H), 7.56(d, J=8.6Hz, 1H), 7.46-7.06(td, J=72Hz, 1H), 7.35(d, J=5.9Hz, 1H), 7.21(dd, J=15.7, 8.6Hz, 2H), 7.09(d, J=4.0Hz, 1H), 6.49(s, 1H), 4.88(dt, J=11.5, 5.6Hz, 1H), 4.61(d, J=11.0Hz, 2H), 4.42(s, 2H), 3.77(dd, J=9.0, 5.8Hz, 2H), 3.45(dq, J=14.2, 7.2Hz, 1H), 3.29-3.18(m, 1H), 2.98(dt, J=16.4, 8.2Hz, 1H), 2.88(dd, J=13.4, 7.5Hz, 1H), 2.47-2.31(m, 2H), 2.20(dd, J=23.1, 10.3Hz, 2H), 1.89-1.69(m, 2H).

### Example 9 (Compound 15)

### Synthesis of (R/S)-2-(3-(5-chloropyrimidin-2-yl)-2,5-dihydro-1H-pyrrol-1-yl)-4-((1-(hydroxymethyl)cyclobutyl)amino)-6,7-dihydrothieno[3,2-d]pyrimidine 5-oxide:

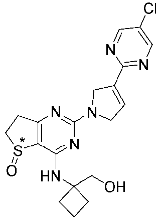

### Step 1: Preparation of tert-butyl 3-(5-chloropyrimidin-2-yl)-2,5-dihydro-1H-pyrrole-1-carboxylate:

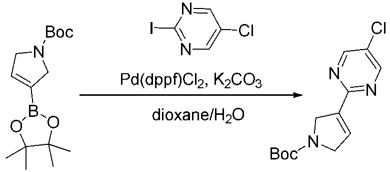

Tert-butyl 3-(4,4,5,5-tetramethyl-1,3,2-dioxaborolan-2-yl)-2,5-dihydro-1H-pyrrole-1-carboxylate (500mg), 5-chloro-2-iodopyrimidine (447mg), [1,1'-bis(diphenylphosphino)ferrocene]dichloropalladium (Pd(dppf)Cl₂)(247mg) and potassium carbonate (700mg) were dissolved in dioxane (5mL) and water (0.5mL). The system was reacted at 110°C under the protection of nitrogen gas for 16 hours. LCMS detection showed the product appeared as the main peak. Then the system was cooled to room temperature. The solid was separated by filtering, and the filtrate was dried by rotary evaporation, separated and purified by flash chromatography (silica gel, petroleum ether:ethyl acetate=3:1-2:1) to produce the target compound (550mg, crude). LCMS(ESI)[M-55]⁺=226.12.

### Step 2: Preparation of 5-chloro-2-(2,5-dihydro-1H-pyrrol-3-yl)pyrimidine:

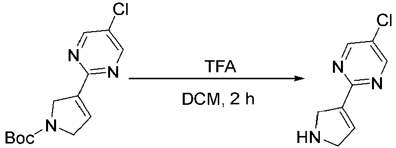

Tert-butyl 3-(5-chloropyrimidin-2-yl)-2,5-dihydro-1H-pyrrole-1-carboxylate (200mg) was dissolved in dichloromethane (2mL), and added to trifluoroacetic acid (1mL). The mixture was reacted under stirring at room temperature for 2 hours. TLC detection showed that the raw material was completely reacted. Then the reaction mixture was dried by rotary evaporation to produce the target compound (crude), which was directly used in the next step. LCMS(ESI)[M+H]⁺=182.13.

### Step 3: Preparation of (R/S)-2-(3-(5-chloropyrimidin-2-yl)-2,5-dihydro-1H-pyrrol-1-yl)-4-((1-(hydroxymethyl)cyclobutyl)amino)-6,7-dihydrothieno[3,2-d]pyrimidine 5-oxide:

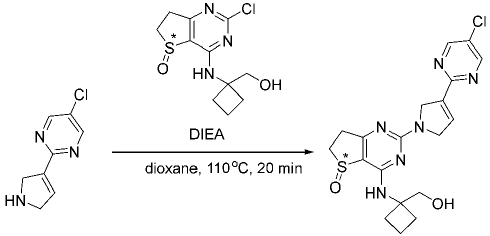

5-chloro-2-(2,5-dihydro-1H-pyrrol-3-yl)pyrimidine (200mg, crude, obtained from the previous step) was dissolved in dioxane (4mL). Then N,N-diisopropylethylamine (2mL) was added. The mixture was evenly stirred and then (R/S)-2-chloro-4-((1-(hydroxymethyl)cyclobutyl)amino)-6,7-dihydrothieno[3,2-d]pyrimidine 5-oxide (70mg) was added. Then the system was reacted at 110°C under microwave for 20 minutes. LCMS detection showed the reaction was completed. The system was cooled to room temperature, stirred overnight, and filtered. The filter cake was rinsed with ethyl acetate. Then the filter cake was concentrated to dryness to produce the target compound (50.63mg). LCMS(ESI)[M+H]⁺=433.27; ¹H NMR(400 MHz, DMSO-*d*₆)δ 8.96(d, *J*=8.4Hz, 2H), 7.36(s, 1H), 7.06(s, 1H), 4.87(dt, *J*=11.4, 5.6Hz, 1H), 4.65(s, 2H), 4.51(s, 2H), 3.77(t, *J*=5.6Hz, 2H), 3.52-3.39(m, 1H), 3.23(dd, *J=14.7,* 7.0Hz, 1H), 2.93(ddd, *J*=20.5, 14.3, 6.8Hz, 2H), 2.38(dd, *J*=33.3, 12.6Hz, 2H), 2.19(t, *J*=9.3Hz, 2H), 1.88-1.71(m, 2H).

### Example 10 (Compound 16)

### Synthesis of (R/S)-2-(1-(5-chloropyrimidin-2-yl)-1,2,3,6-tetrahydropyridin-4-yl)-4-((1-(hydroxymethyl)cyclobutyl)amino)-6,7-dihydrothieno[3,2-d]pyrimidine 5-oxide:

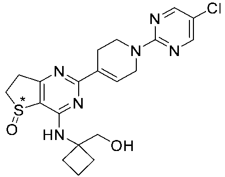

### Step 1 : Preparation of tert-butyl (R/S)-4-(4-((1-(hydroxymethyl)cyclobutyl)amino)-5-oxido-6,7-dihydrothieno[3,2-d]pyrimidin-2-yl)-3,6-dihydropyridine-1(2H)-carboxylate:

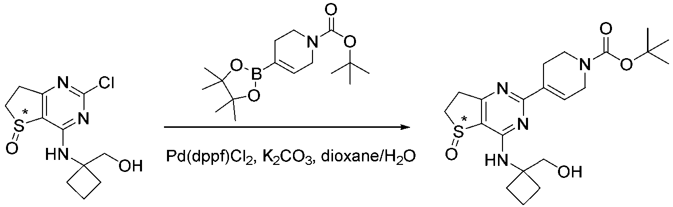

Under the protection of nitrogen gas, (R/S)-2-chloro-4-((1-(hydroxymethyl)cyclobutyl)amino)-6,7-dihydrothieno[3,2-d]pyrimidine 5-oxide (100mg) and tert-butyl 4-(4,4,5,5-tetramethyl-1,3,2-dioxaborolan-2-yl)-3,6-dihydropyridine-1(2H)-carboxylate (130mg) were added to dioxane (5mL). Water (2mL) was added to the reaction mixture, and then [1,1'-bis(diphenylphosphino)ferrocene]dichloropalladium (25mg) and potassium carbonate (121mg) were added under stirring. After the atmosphere was replaced with nitrogen gas three times, the reaction mixture was warmed up to 90°C and reacted for 1 hour. LCMS detection showed that the raw material was completely consumed and converted to the product. The reaction mixture was cooled to room temperature. Then water and ethyl acetate (each 20mL) were added to the reaction mixture. The mixture was separated by extraction to produce an organic phase. The organic phase was concentrated to dryness. The residue was separated by column chromatography (petroleum ether/ethyl acetate=from 5:1 to 3:1) to produce the target compound (90mg). LCMS:(ESI)[M+H]⁺=435.2.

### Step 2: Preparation of (R/S)-4-((1-(hydroxymethyl)cyclobutyl)amino)-2-(1,2,3,6-tetrahydropyridin-4-yl)-6,7-dihydrothieno[3,2-d]pyrimidine 5-oxide hydrochloride:

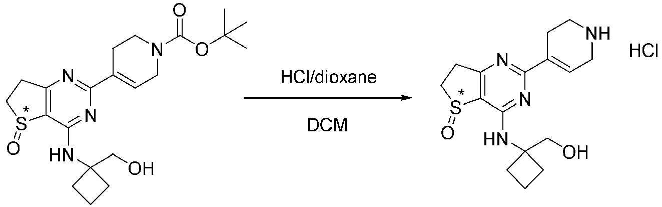

Under the protection of nitrogen gas, tert-butyl (R/S)-4-(4-((1-(hydroxymethyl)cyclobutyl)amino)-5-oxido-6,7-dihydrothieno[3,2-d]pyrimidin-2-yl)-3,6-dihydropyridine-1(2H)-carboxylate (90mg) was dissolved in dichloromethane (4mL). The mixture was stirred under an ice-water bath for about 10 minutes. A 4M solution of hydrogen chloride in dioxane (1mL) was added to the reaction mixture at 0°C. The ice-water bath was removed, and the reaction mixture was warmed up to room temperature and stirred for 1 hour. LCMS detection showed the raw material was completely consumed and converted to the product. The reaction mixture was concentrated to produce the target compound (97mg, crude). LCMS:(ESI)[M+H]⁺=335.2.

### Step 3: Preparation of (R/S)-2-(1-(5-chloropyrimidin-2-yl)-1,2,3,6-tetrahydropyridin-4-yl)-4-((1-(hydroxymethyl)cyclobutyl)amino)-6,7-dihydrothieno[3,2-d]pyrimidine 5-oxide:

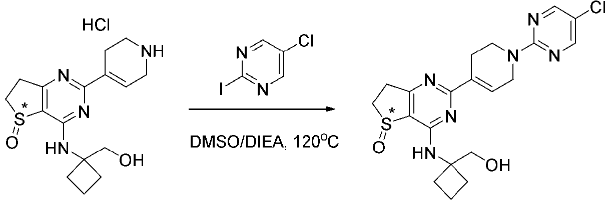

Under the protection of nitrogen gas, (R/S)-4-((1-(hydroxymethyl)cyclobutyl)amino)-2-(1,2,3,6-tetrahydropyridin-4-yl)-6,7-dihydrothieno[3,2-d]pyrimidine 5-oxide hydrochloride (37mg, crude, obtained from the previous step) was dissolved in dimethyl sulfoxide (2mL). 5-Chloro-2-iodopyrimidine (36mg) and N,N-diisopropylethylamine (39mg) were added. The mixture was warmed up to 120°C, and stirred for 1 hour. LCMS detection showed the raw material was completely consumed and converted to the product. The reaction mixture was directly purified by high performance liquid chromatography (0.5% ammonium bicarbonate//water/acetonitrile) to produce the target compound (9mg). LCMS:(ESI)[M+H]⁺=447.1; ¹H NMR(400 MHz, DMSO-*d*₆)δ 8.46(s, 2H), 7.88(s, 1H), 7.23(s, 1H), 4.86(t, *J*=5.7Hz, 1H), 4.39(d, *J*=2.8Hz, 2H), 3.95(t, *J*=5.8Hz, 2H), 3.83-3.68(m, 2H), 3.62-3.49(m, 1H), 3.29(d, *J*=8.1Hz, 1H), 3.18-3.07(m, 1H), 3.05-2.94(m, 1H), 2.61(s, 2H), 2.41-2.19(m, 4H), 1.78(dt, *J*=19.9, 9.8Hz, 2H).

### Example 11 (Compound 17)

### Synthesis of (R/S)-2-(4-(5-chloropyrimidin-2-yl)phenyl)-4-((1-(hydroxymethyl)cyclobutyl)amino)-6,7-dihydrothieno[3,2-d]pyrimidine 5-oxide:

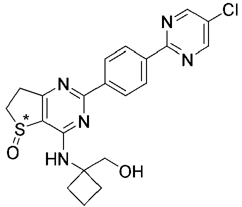

### Step 1: Synthesis of 4-(5-chloropyrimidin-2-yl)phenol:

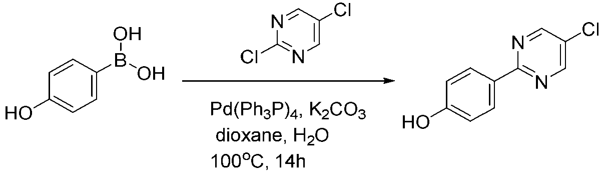

Under the protection of nitrogen gas, tetrakis(triphenylphosphine)palladium (Pd(Ph₃P)₄)(2.52g) was added to a mixture of (4-hydroxyphenyl)boronic acid (3.00g), 2,5-dichloropyrimidine (3.24g) and potassium carbonate (9.02g) in dioxane (90mL) and water (15mL). The resulting mixture was heated to 100°C and reacted for 14 hours. LCMS detection showed the reaction was completed. Then water (100mL) was added to the reaction mixture. The mixture was extracted with ethyl acetate (300mL), and separated into phases. The organic phase was dried over anhydrous sodium sulfate, and filtered. The filtrate was concentrated under a reduced pressure, and then separated and purified by flash chromatography (silica gel, ethyl acetate:petroleum ether=1:5) to produce the target compound (2.50g). LCMS(ESI)[M+H]⁺=207.1.

### Step 2: Synthesis of 4-(5-chloropyrimidin-2-yl)phenyl trifluoromethanesulfonate:

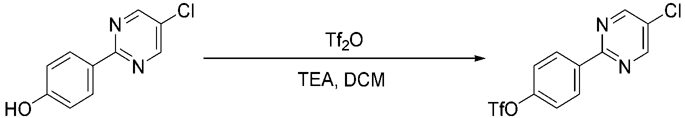

Under an ice-water bath, trifluoromethanesulfonic anhydride (Tf₂O)(2.05g) was added to a solution of 4-(5-chloropyrimidin-2-yl)phenol (1.00g) in dichloromethane (20mL). Then triethylamine (TEA)(1.47g) was added dropwise. After the completion of the dropwise addition, the reaction mixture was stirred for 2 hours under the ice-water bath, and then reacted for 12 hours at room temperature. TLC detection showed the reaction was completed. Then a saturated sodium bicarbonate solution (20mL) was added. The mixture was extracted with dichloromethane (50mL×3), and separated into phases. The organic phase was dried over anhydrous sodium sulfate, and filtered. The filtrate was concentrated under a reduced pressure, and then separated and purified by flash chromatography (silica gel, ethyl acetate:petroleum ether=1:10) to produce the target compound (1.00g). LCMS(ESI)[M+H]⁺=339.0.

### Step 3: Synthesis of 5-chloro-2-(4-(4,4,5,5-tetramethyl-1,3,2-dioxaborolan-2-yl)phenyl)pyrimidine:

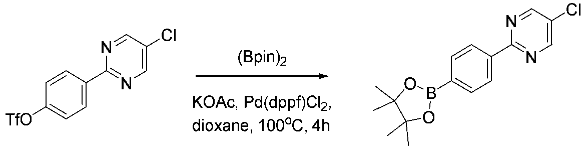

Under the protection of nitrogen gas, [1,1'-bis(diphenylphosphino)ferrocene]dichloropalladium (108mg) was added to a solution of 4-(5-chloropyrimidin-2-yl)phenyl trifluoromethanesulfonate (500mg), bis(pinacolato)diboron ((Bpin)₂)(562mg) and potassium acetate (435mg) in dioxane (3mL). The mixture was heated to 100°C, and reacted for 4 hours. TLC detection showed the reaction was completed. The reaction mixture was diluted with water (100mL) and ethyl acetate (300mL), and separated into phases. The organic phases were washed with a saturated saline solution (100mL×3), dried over anhydrous sodium sulfate, and filtered. The filtrate was concentrated under a reduced pressure, and separated and purified by flash chromatography (silica gel, ethyl acetate:petroleum ether=1:20) to produce the target compound (300mg). LCMS(ESI)[M+H]⁺=317.1.

### Step 4: Synthesis of (R/S)-2-(4-(5-chloropyrimidin-2-yl)phenyl)-4-((1-(hydroxymethyl)cyclobutyl)amino)-6,7-dihydrothieno[3,2-d]pyrimidine 5-oxide:

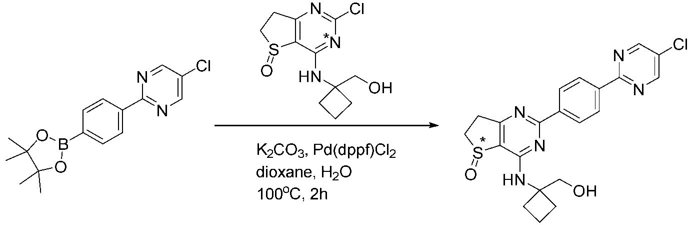

Under the protection of nitrogen gas, [1,1'-bis(diphenylphosphino)ferrocene]dichloropalladium (13mg) was added to a mixed solution of (R/S)-2-chloro-4-((1-(hydroxymethyl)cyclobutyl)amino)-6,7-dihydrothieno[3,2-d]pyrimidine 5-oxide (50mg), 5-chloro-2-(4-(4,4,5,5-tetramethyl-1,3,2-dioxaborolan-2-yl)phenyl)pyrimidine (55mg) and potassium carbonate (60mg) in dioxane (2mL) and water (0.5mL). The mixture was heated to 100°C, and reacted for 2 hours. LCMS detection showed the reaction was completed. The reaction mixture was diluted with water (10mL) and ethyl acetate (50mL), and separated into phases. The organic phase was washed with a saturated saline solution (30mL×3), dried over anhydrous sodium sulfate, and filtered. The filtrate was concentrated, and then separated and purified by Prep-HPLC (C18, 10mmol/L HCOOH/water, MeCN) to produce the target compound (20mg). LCMS(ESI)[M+H]⁺=442.2; ¹H NMR(400 MHz, DMSO-*d*₆)δ 9.07(s, 2H), 8.48(s, 4H), 8.11(s, 1H), 4.94(t, *J*=5.7Hz, 1H), 3.85(d, *J*=4.0Hz, 2H), 3.74-3.59(m, 1H), 3.40(dd, *J*=14.8, 6.6Hz, 1H), 3.27-3.21(m, 1H), 3.05(dd, *J*=13.5, 6.2Hz, 1H), 2.47-2.39(m, 2H), 2.36(d, *J*=9.7Hz, 2H), 1.94-1.78(m, 2H).

### Example 12 (Compound 22)

### Synthesis of (R/S)-4-((1-(hydroxymethyl)cyclobutyl)amino)-2-(isoindolin-2-yl)-6,7-dihydrothieno[3,2-d]pyrimidine 5-oxide:

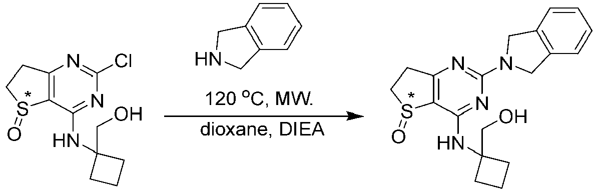

(R/S)-2-chloro-4-((1-(hydroxymethyl)cyclobutyl)amino)-6,7-dihydrothieno[3,2-d]pyrimidine 5-oxide (100mg), isoindoline (45.55mg), N,N-diisopropylethylamine (134.73mg) were added to dioxane (2mL). The mixture was reacted at 120°C under microwave for 20 minutes. LCMS detection showed the raw material disappeared. The reaction mixture was added to water (15mL), and stirred for 30 minutes. A large amount of insoluble substance appeared, and the mixture was filtered. The insoluble substance was added to ethyl acetate (20mL). The mixture was stirred at 65°C for 1 hour, cooled to room temperature, and filtered to produce the target compound (56.79mg). LCMS:(ESI)[M+H]⁺=371.1; ¹H NMR(400 MHz, DMSO-*d*₆)δ 7.49-7.35(m, 3H), 7.34-7.27(m, 2H), 4.90-4.72(m, 5H), 3.85-3.75(m, 2H), 3.51-3.40(m, 1H), 3.30-3.18(m, 1H), 3.04-2.94(m, 1H), 2.93-2.84(m, 1H), 2.45-2.32(m, 2H), 2.28-2.15(m, 2H), 1.85-1.60(m, 2H).

### Example 13 (Compound 23)

### Synthesis of (R/S)-4-((1-(hydroxymethyl)cyclobutyl)amino)-2-(indolin-1-yl)-6,7-dihydrothieno[3,2-d]pyrimidine 5-oxide:

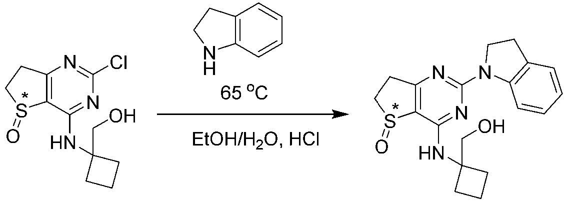

(R/S)-2-chloro-4-((1-(hydroxymethyl)cyclobutyl)amino)-6,7-dihydrothieno[3,2-d]pyrimidine 5-oxide (100mg), and indoline(83.3mg) were added to ethanol (5mL). Water (2mL) was added. 1M dilute hydrochloric acid (0.3mL) was added under stirring. The mixture was warmed up to 65°C and stirred for 1 hour. LCMS detection showed the raw material substantially disappeared and a product formed. A saturated sodium bicarbonate solution (3mL) was added to the reaction mixture, and the pH was adjusted to 8. Water (20mL) and ethyl acetate (20mL) were added to the reaction mixture. The mixture was separated into phases. The organic phase was washed with water and then concentrated. The residue was purified by preparative liquid phase chromatography (acetonitrile/water with 0.5% ammonium bicarbonate system) and freeze-dried to produce the target compound (32mg). LCMS:(ESI)[M+H]⁺=371.2; ee%=100%. ¹H NMR(400 MHz, DMSO-*d*₆)δ 8.35(d, *J*=8.0Hz, 1H), 7.56(s, 1H), 7.27-7.11(m, 2H), 6.91(td, *J*=7.4, 0.9Hz, 1H), 4.87(t, *J*=5.7Hz, 1H), 4.14(t, *J*=8.7Hz, 2H), 3.79(d, *J*=5.7Hz, 2H), 3.60-3.47(m, 1H), 3.28(dd, *J*=10.9, 5.8Hz, 1H), 3.17-3.10(m, 2H), 3.10-3.04(m, 1H), 2.93(ddd, *J*=13.4, 7.3, 1.7Hz, 1H), 2.39(td, *J*=22.0, 9.7Hz, 2H), 2.25(s, 2H), 1.89-1.70(m, 2H).

### Example 14 (Compound 24)

### Synthesis of (R/S)-2-(1,3-dihydro-2H-pyrrolo[3,4-c]pyridin-2-yl)-4-((1-(hydroxymethyl)cyclobutyl)amino)-6,7-dihydrothieno[3,2-d]pyrimidine 5-oxide:

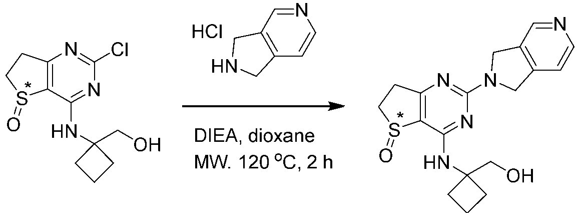

(R/S)-2-chloro-4-((1-(hydroxymethyl)cyclobutyl)amino)-6,7-dihydrothieno[3,2-d]pyrimidine 5-oxide (50mg), 2,3-dihydro-1H-pyrrolo[3,4-c]pyridine hydrochloride (49.23mg), and N,N-diisopropylethylamine (87.88mg) were added to dioxane (1.5mL). The mixture was heated at 120°C under microwave for 2 hours. LCMS detection showed the raw material disappeared. The reaction mixture was directly separated and purified by Prep-HPLC (C18, 10 mmol/L, NH₄HCO₃/water, MeCN) to produce the target compound (16.88mg). LCMS(ESI)[M+H]⁺=372.2; ¹H NMR(400 MHz, DMSO-*d*₆)δ 8.64(d, *J*=16.4Hz, 1H), 8.49(d, *J*=5.0Hz, 1H), 7.54-7.37(m, 2H), 4.95-4.75(m, 5H), 3.79(d, *J*=5.2Hz, 2H), 3.53-3.40(m, 1H), 3.28-3.18(m, 1H), 3.05-2.96(m, 1H), 2.93-2.82(m, 1H), 2.45-2.31(m, 2H), 2.30-2.18(m, 2H), 1.95-1.71(m, 2H).

### Example 15 (Compound 25)

### Synthesis of (R/S)-2-(5-chloroisoindolin-2-yl)-4-((1-(hydroxymethyl)cyclobutyl)amino)-6,7-dihydrothieno[3,2-d]pyrimidine 5-oxide:

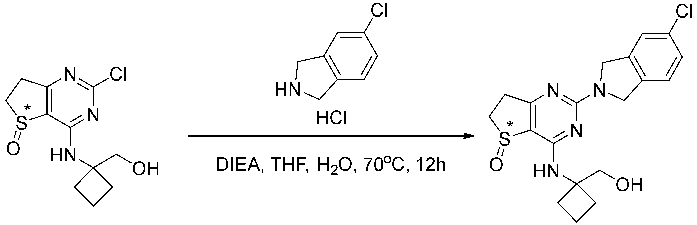

5-chloro-2,3-dihydro-1H-isoindoline hydrochloride (66mg) was added to a mixed solution of (R/S)-2-chloro-4-((1-(hydroxymethyl)cyclobutyl)amino)-6,7-dihydrothieno[3,2-d]pyrimidine 5-oxide (50mg) and N,N-diisopropylethylamine (67mg) in tetrahydrofuran (5mL) and water (1mL). The mixture was heated to 70°C and stirred for 12 hours. LCMS detection showed the reaction was completed. Then the reaction mixture was diluted with water (10mL), exacted with ethyl acetate (30mL), and separated into phases. The organic phases were combined, concentrated under a reduced pressure, and then separated and purified by Prep-HPLC (C18, 10mmol/L HCOOH/water, MeCN) to produce the target compound (35mg). LCMS(ESI)[M+H]⁺=405.1; ¹H NMR(400 MHz, DMSO-*d*₆)δ 7.50(d, J=18.1Hz, 1H), 7.43(dd, J=16.8, 8.5Hz, 2H), 7.36(dd, J=8.2, 1.8Hz, 1H), 4.86(t, J=5.6Hz, 1H), 4.77(t, J=8.7Hz, 4H), 3.83-3.74(m, 2H), 3.52-3.40(m, 1H), 3.24(dt, J=13.4, 8.3Hz, 1H), 3.04-2.94(m, 1H), 2.93-2.84(m, 1H), 2.38(dt, J=21.1, 10.6Hz, 2H), 2.23(dd, J=11.1, 7.4Hz, 2H), 1.90-1.70(m, 2H).

### Example 16 (Compound 26)

### Synthesis of (R/S)-4-((1-(hydroxymethyl)cyclobutyl)amino)-2-(5-methoxyisoindolin-2-yl)-6,7-dihydrothieno[3,2-d]pyrimidine 5-oxide:

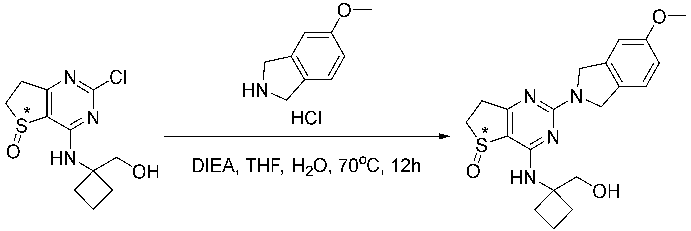

5-methoxy-2,3-dihydro-1H-isoindoline hydrochloride (65mg) was added to a mixed solution of (R/S)-2-chloro-4-((1-(hydroxymethyl)cyclobutyl)amino)-6,7-dihydrothieno[3,2-d]pyrimidine 5-oxide (50mg) and N,N-diisopropylethylamine (16mg) in tetrahydrofuran (5mL) and water (1mL). The mixture was heated to 70°C, and stirred for 12 hours. LCMS detection showed the reaction was completed. Then the reaction mixture was diluted with water (10mL), extracted with ethyl acetate (50mL), and separated into phases. The organic phase was dried over anhydrous sodium sulfate, and filtered. The filtrate was concentrated, and then separated and purified by Prep-HPLC (C18, 10mmol/L HCOOH/water, MeCN) to produce the target compound (30mg). LCMS(ESI)[M+H]⁺=401.2; ¹H NMR(400 MHz, DMSO-*d*₆)δ 7.39(s, 1H), 7.29(dd, J=17.7, 8.4Hz, 1H), 6.99(d, J=23.6Hz, 1H), 6.87(d, J=8.3Hz, 1H), 4.87(s, 1H), 4.73(dd, J=17.4, 7.7Hz, 4H), 3.79(s, 2H), 3.76(s, 3H), 3.51-3.41(m, 1H), 3.24(dt, J=13.4, 8.4Hz, 1H), 2.98(dd, J=17.0, 8.1Hz, 1H), 2.88(dd, J=14.0, 6.4Hz, 1H), 2.47-2.31(m, 2H), 2.22(d, J=8.9Hz, 2H), 1.92-1.70(m, 2H).

### Example 17 (Compound 27)

Synthesis of (R/S)-2-(4-((1-(hydroxymethyl)cyclobutyl)amino)-5-oxido-6,7-dihydrothieno[3,2-d]pyrimidin-2-yl)isoindoline-5-carbonitrile:

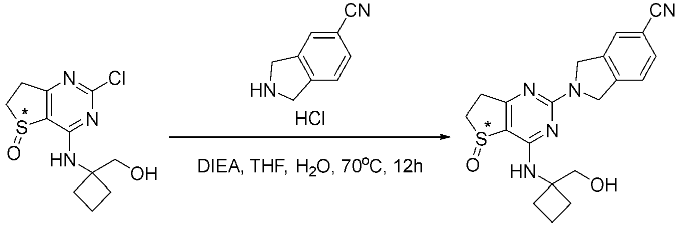

2,3-dihydro-1H-isoindoline-5-carbonitrile hydrochloride (63mg) was added to a mixed solution of (R/S)-2-chloro-4-((1-(hydroxymethyl)cyclobutyl)amino)-6,7-dihydrothieno[3,2-d]pyrimidine 5-oxide (50mg) and N,N-diisopropylethylamine (67mg) in tetrahydrofuran (5mL) and water (1mL). The mixture was heated to 70°C, and stirred for 12 hours. LCMS detection showed the reaction was completed. Then the reaction mixture was diluted with water (10mL), extracted with ethyl acetate (30mL), and separated into phases. The organic phase was dried over anhydrous sodium sulfate, concentrated, and then separated and purified by Prep-HPLC (C18, 10mmol/L HCOOH/water, MeCN) to produce the target compound (35mg). LCMS(ESI)[M+H]⁺=396.2; ¹H NMR(400 MHz, DMSO-*d*₆)δ 7.89(d, J=16.4Hz, 1H), 7.77(d, J=7.9Hz, 1H), 7.62(dd, J=19.2, 7.9Hz, 1H), 7.45(s, 1H), 4.94-4.72(m, 5H), 3.86-3.75(m, 2H), 3.53-3.40(m, 1H), 3.24(dt, J=13.5, 8.3Hz, 1H), 2.98(dd, J=17.0, 8.1Hz, 1H), 2.93-2.85(m, 1H), 2.39(td, J=20.4, 10.0Hz, 2H), 2.23(t, J=9.3Hz, 2H), 1.80(dt, J=28.2, 9.5Hz, 2H).

### Example 18 (Compound 28)

### Synthesis of (R/S)-2-(5,7-dihydro-6H-pyrrolo[3,4-b]pyridin-6-yl)-4-((1-(hydroxymethyl)cyclobutyl)amino)-6,7-dihydrothieno[3,2-d]pyrimidine 5-oxide:

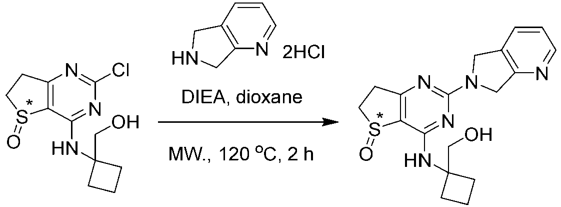

(R/S)-2-chloro-4-((1-(hydroxymethyl)cyclobutyl)amino)-6,7-dihydrothieno[3,2-d]pyrimidine 5-oxide (100mg), 6,7-dihydro-5H-pyrrolo[3,4-b]pyridine dihydrochloride (100.64mg) and N,N-diisopropylethylamine (179.64mg) were added to dioxane (2mL). The mixture was stirred 120°C under microwave for 2 hours. LCMS detection showed the raw material disappeared. The reaction mixture was directly separated and purified by Prep-HPLC (C18, 10 mmol/L, NH₄HCO₃/water, MeCN) to produce the target compound (53.14mg). LCMS:(ESI)[M+H]⁺=372.30; ¹H NMR(400 MHz, DMSO-*d*₆)δ 8.55-8.42(m, 1H), 7.83(dd, *J*=19.4, 7.5Hz, 1H), 7.44(s, 1H), 7.33(dd, *J*=7.7, 4.9Hz, 1H), 4.94-4.72(m, 5H), 3.79(d, *J*=5.5Hz, 2H), 3.52-3.40(m, 1H), 3.30-3.19(m, 1H), 3.02-2.88(m, 1H), 2.94-2.85(m, 1H), 2.49-2.33(m, 2H), 2.25-2.21(m, 2H), 1.90-1.70(m, 2H).

### Example 19 (Compound 29)

### Synthesis of (R/S)-4-((1-(hydroxymethyl)cyclobutyl)amino)-2-(5-(2-hydroxypropan-2-yl)isoindolin-2-yl)-6,7-dihydrothieno[3,2-d]pyrimidine 5-oxide:

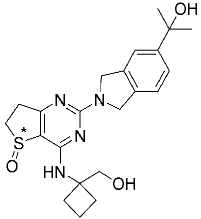

### Step 1: Preparation of methyl (R/S)-2-(4-((1-(hydroxymethyl)cyclobutyl)amino)-5-oxido-6,7-dihydrothieno[3,2-d]pyrimidin-2-yl)isoindoline-5-carboxylate:

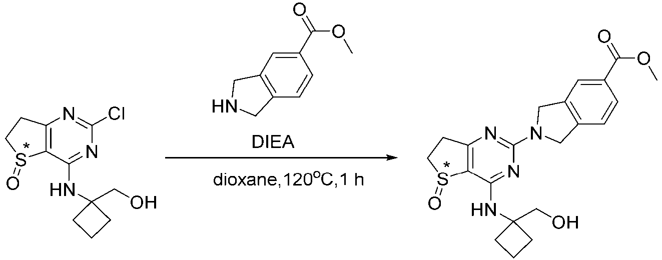

(R/S)-2-chloro-4-((1-(hydroxymethyl)cyclobutyl)amino)-6,7-dihydrothieno[3,2-d]pyrimidine 5-oxide (120mg), methyl isoindoline-5-carboxylate hydrochloride (106.8mg) and N,N-diisopropylethylamine (1mL) were dissolved in dioxane (3mL). Then the system was reacted at 120°C for 1 hour. LCMS detection showed the product appeared as the main peak. Then the solvent was removed by rotary evaporation. The crude product was added to ethyl acetate (5mL), and ultrasonicated for 1 minute. Then the solid was filtered by suction. The filter cake was rinsed with ethyl acetate, and then concentrated to dryness to produce the target compound (200mg). LCMS(ESI)[M+H]⁺=429.31.

### Step 2: Preparation of (R/S)-4-((1-(hydroxymethyl)cyclobutyl)amino)-2-(5-(2-hydroxypropan-2-yl)isoindolin-2-yl)-6,7-dihydrothieno[3,2-d]pyrimidine 5-oxide:

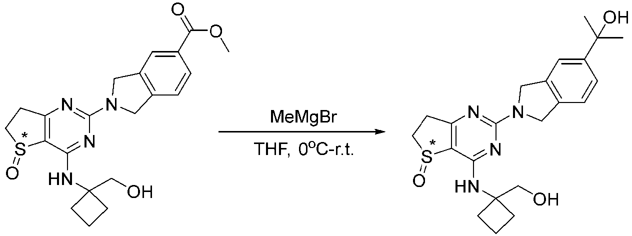

Methyl (R/S)-2-(4-((1-(hydroxymethyl)cyclobutyl)amino)-5-oxido-6,7-dihydrothieno[3,2-d]pyrimidin-2-yl)isoindoline-5-carboxylate (150mg) was dissolved in anhydrous tetrahydrofuran (10mL). Methyl magnesium bromide (2mL, 3M, in 2-methyltetrahydrofuran ) was added under an ice bath. Then the system was stirred at room temperature for 2 hours. LCMS detection showed the product appeared as the main peak. Then a saturated aqueous ammonium chloride solution (5mL) was added to quench the reaction. The aqueous phase was extracted with ethyl acetate (30mL×2). The combined organic phases were dried over anhydrous sodium sulfate, and concentrated under a reduced pressure. The residue was separated and purified by Prep-HPLC (C18, 10mmol/L NH₄HCO₃/water, MeCN) to produce the target compound (41.24mg). LCMS(ESI)[M+H]⁺=429.32; ¹H NMR(400 MHz, DMSO-*d*₆)δ 7.50(s, 1H), 7.45-7.36(m, 2H), 7.31(dd, J=13.9, 8.0Hz, 1H), 5.02(d, J=8.7Hz, 1H), 4.87(t, J=5.5Hz, 1H), 4.76(d, J=10.1Hz, 4H), 3.80(d, J=5.5Hz, 2H), 3.51-3.41(m, 1H), 3.24(dt, J=13.3, 8.3Hz, 1H), 3.03-2.85(m, 2H), 2.40(dd, J=21.4, 9.7Hz, 2H), 2.23(s, 2H), 1.90-1.72(m, 2H), 1.43(s, 6H).

### Example 20 (Compound 30)

### Synthesis of (R/S)-2-(benzo[b]thiophen-2-yl)-4-((1-(hydroxymethyl)cyclobutyl)amino)-6,7-dihydrothieno[3,2-d]pyrimidine 5-oxide:

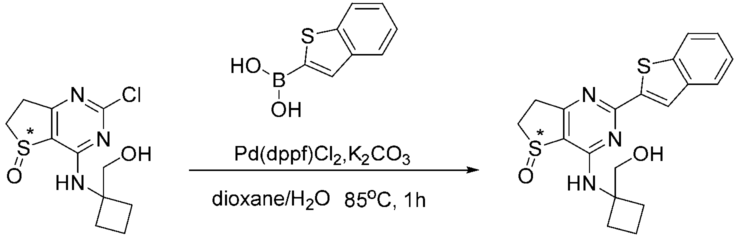

Under the protection of nitrogen gas, (R/S)-2-chloro-4-((1-(hydroxymethyl)cyclobutyl)amino)-6,7-dihydrothieno[3,2-d]pyrimidine 5-oxide (100mg) and benzo[b]thiophen-2-ylboronic acid (75mg) were added to dioxane (8mL). Water (2mL) was added to the reaction mixture. [1,1'-bis(diphenylphosphino)ferrocene]dichloropalladium (25mg) and potassium carbonate (121mg) were added under stirring. After the atmosphere was replaced with nitrogen gas three times, the reaction mixture was warmed up to 85°C and reacted for 1 hour. The raw material was completely consumed and converted to the product. The heating was removed, and the reaction mixture was cooled to room temperature. Then water (30mL) was added to the reaction mixture. The mixture was extracted with ethyl acetate (50mL×3), and separated to obtain organic phases. The organic phases were combined, dried over anhydrous sodium sulfate, filtered, and concentrated under a reduced pressure. The residue was separated and purified by Prep-HPLC (C18, 10mmol/L NH₄HCO₃/water, MeCN) to produce the target compound (22mg). LCMS(ESI)[M+H]⁺=386; ¹H NMR(400 MHz, DMSO-*d*₆)δ 8.24(s, 1H), 8.20(s, 1H), 8.05-7.94(m, 2H), 7.47-7.39(m, 2H), 4.91(t, J=5.6Hz, 1H), 3.92-3.81(m, 2H), 3.72-3.59(m, 1H), 3.42-3.34(m, 1H), 3.22(dd, J=17.1, 7.9Hz, 1H), 3.04(dd, J=13.7, 6.2Hz, 1H), 2.39(dt, J=16.2, 9.7Hz, 4H), 1.84(dd, J=18.1, 9.2Hz, 2H).

### Example 21 (Compound 31)

### Synthesis of (R/S)-N-(2-(4-((1-(hydroxymethyl)cyclobutyl)amino)-5-oxido-6,7-dihydrothieno[3,2-d]pyrimidin-2-yl)isoindolin-5-yl)methanesulfonamide:

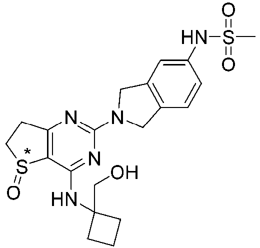

### Step 1: Preparation of tert-butyl 5-(methylsulfonamido)isoindoline-2-carboxylate :

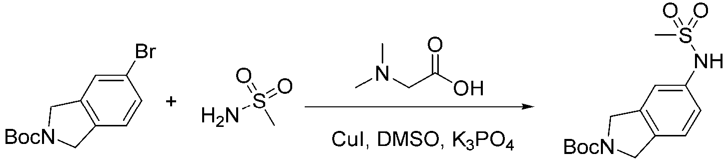

Under the protection of nitrogen gas, tert-butyl 5-bromoisoindoline-2-carboxylate (1.2g), N,N-dimethylglycine (41.3mg), methylsulfonamide (457mg), cuprous iodide (76mg) and anhydrous potassium phosphate (1.7g) were added to anhydrous dimethyl sulfoxide (DMSO)(30mL). The mixture was heated to 150°C and reacted for 48 hours. LCMS detection showed that the raw material was partially converted to the product. The reaction mixture was cooled to room temperature, then diluted with water (100mL), extracted with ethyl acetate (100mL×3). The organic phases were combined, washed with a saturated saline solution (100mL), dried over anhydrous sodium sulfate, and filtered. The filtrate was concentrated under a reduced pressure to produce a crude product. The crude product was purified by flash silica gel column chromatography (silica gel, petroleum ether:tetrahydrofuran=100:0 to 50:50 gradient dilution) to produce the target compound (400mg). LCMS(ESI)[2M+Na]⁺=647.3;

### Step 2: Preparation of N-(isoindolin-5-yl)methanesulfonamide hydrochloride:

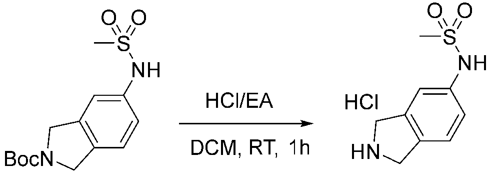

Under the protection of nitrogen gas, tert-butyl 5-(methylsulfonamido)isoindoline-2-carboxylate (400mg) was dissolved in dichloromethane (20mL), and the mixture was stirred under an ice-water bath for 10 minutes. At 0°C, a 4M solution of hydrogen chloride in ethyl acetate (0.96mL) was added to the reaction mixture. The ice-water bath was removed, and the mixture was warmed up to room temperature and reacted for 1 hour. The raw material was completely consumed and converted to the product. The reaction mixture was filtered. The filter cake was washed with dichloromethane (5mL) and concentrated to dryness to produce the target compound (230mg). LCMS(ESI)[M+H]⁺=213;

### Step 3: Preparation of (R/S)-N-(2-(4-((1-(hydroxymethyl)cyclobutyl)amino)-5-oxido-6,7-dihydrothieno[3,2-d]pyrimidin-2-yl)isoindolin-5-yl)methanesulfonamide:

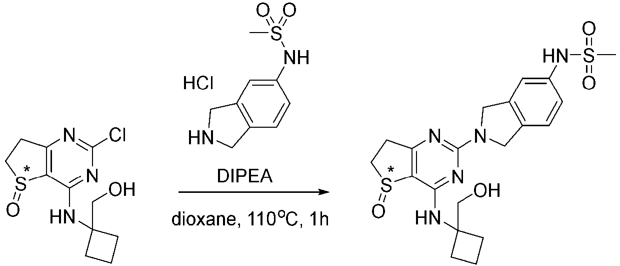

Under the protection of nitrogen gas, (R/S)-2-chloro-4-((1-(hydroxymethyl)cyclobutyl)amino)-6,7-dihydrothieno[3,2-d]pyrimidine 5-oxide (100mg) and N-(isoindolin-5-yl)methanesulfonamide hydrochloride (104mg) were added to dioxane (5mL). Then N,N-diisopropylethylamine (114mg) was added. After the atmosphere was replaced with nitrogen gas three times, the reaction mixture was warmed up to 110°C and reacted for 1 hour. LCMS detection showed that the raw material was completely consumed and converted to the product. The heating was removed. The reaction mixture was concentrated to dryness, and the residue was separated and purified by Prep-HPLC (C18, 10mmol/L NH₄HCO₃/water, MeCN) to produce the target compound (25.5mg). LCMS(ESI)[M+H]⁺=464.1; ¹H NMR(400 MHz, DMSO-*d*₆)δ 9.72(s, 1H), 7.40(s, 1H), 7.35(dd, J=18.7, 8.3Hz, 1H), 7.24(d, J=13.0Hz, 1H), 7.14(dd, J=13.8, 5.7Hz, 1H), 4.86(t, J=5.7Hz, 1H), 4.76(t, J=10.5Hz, 4H), 3.79(d, J=5.5Hz, 2H), 3.46(dt, J=16.3, 8.0Hz, 1H), 3.23(dt, J=13.4, 8.3Hz, 1H), 3.04-2.93(m, 4H), 2.92-2.84(m, 1H), 2.47-2.30(m, 2H), 2.23(d, J=7.8Hz, 2H), 1.90-1.70(m, 2H).

### Example 22 (Compound 40)

### Synthesis of (1-((2-(4-(5-chloropyrimidin-2-yl)piperidin-1-yl)-6,7-dihydrothieno[3,2-d]pyrimidin-4-yl)amino)cyclobutyl)methanol:

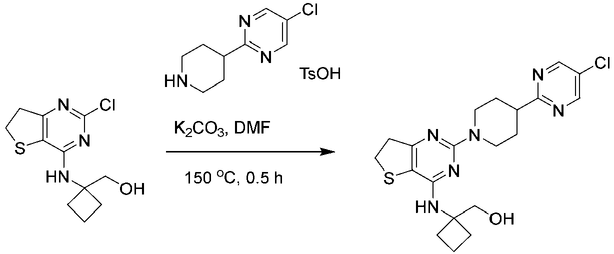

(1-((2-chloro-6H,7H-thieno[3,2-d]pyrimidin-4-yl)amino)cyclobutyl)methanol (50mg), 5-chloro-2-(piperidin-4-yl)pyrimidine p-toluenesulfonate (102.07mg) and potassium carbonate (74.63mg) were added to N,N-dimethylformamide (DMF)(1mL). The mixture was reacted at 150°C under microwave for 0.5 hours. LCMS detection showed that the raw material was almost completely consumed, and a new product was formed. The reaction mixture was filtered. The filtrate was directly separated and purified by Prep-HPLC (C18, 10 mmol/L, NH₄HCO₃/water, MeCN) to produce the target compound (22.49mg). LCMS(ESI)[M+H]⁺=433.30; ¹H NMR(400 MHz, DMSO-*d*₆)δ 8.85(s, 2H), 5.97(s, 1H), 4.80(t, J=5.6Hz, 1H), 4.65-4.50(m, 2H), 3.68(d, J=5.6Hz, 1H), 3.21(t, J=8.1Hz, 2H), 3.17-3.07(m, 1H), 3.00-2.85(m, 4H), 2.36-2.25(m, 2H), 2.17-2.05(m, 2H), 1.85-1.85(m, 2H), 1.81-1.57(m, 4H).

### Example 23 (Compound 7)

### Synthesis of (R/S)-2-(3-hydroxy-3-(4-(trifluoromethyl)phenyl)azetidin-1-yl)-4-((1-(hydroxymethyl)cyclobutyl)amino)-6,7-dihydrothieno[3,2-d]pyrimidine 5-oxide:

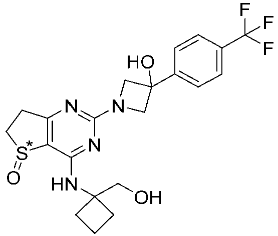

### Step 1: Preparation of tert-butyl 3-hydroxy-3-(4-(trifluoromethyl)phenyl)azetidine-1-carboxylate:

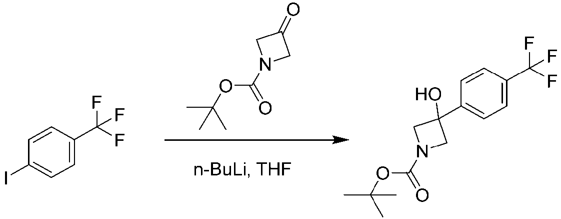

1-Iodo-4-(trifluoromethyl)benzene (2.00g, 1.0eq) was dissolved in anhydrous tetrahydrofuran (40mL). n-Butyl lithium (2.5M, in hexane, 1.0eq) was added dropwise at -78°C under the protection of nitrogen gas. The mixture was stirred for 0.5 hours while keeping the same temperature. Tert-butyl 3-oxoazetidine-1-carboxylate (1.5eq) was dissolved in anhydrous tetrahydrofuran (15mL). This solution was added dropwise to the above lithium reagent solution at -78°C. The mixture was stirred for 0.5 hours while keeping the same temperature. LCMS detection showed the raw material disappeared and the product appeared as the main peak. Water (10mL) was added dropwise at -78°C to quench the reaction. The reaction mixture was poured into water (300mL). The mixture was extracted with ethyl acetate (100mL×3). The organic phases were washed with water (200mL×3), dried over sodium sulfate, concentrated under a reduced pressure to produce a crude product. The crude product was purified by silica gel column chromatography (petroleum ether/ethyl acetate=3/1) to produce the target compound (1.30g). LCMS:(ESI)[M-56]⁺=262.

### Step 2: Preparation of 3-(4-(trifluoromethyl)phenyl)azetidin-3-ol 2,2,2-trifluoroacetate:

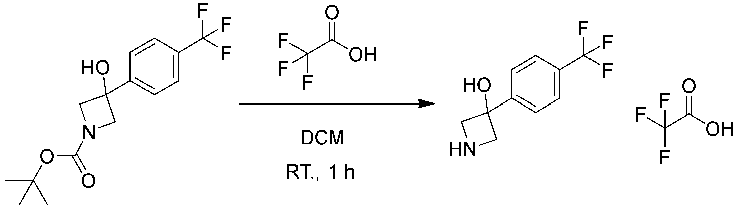

Tert-butyl 3-hydroxy-3-(4-(trifluoromethyl)phenyl)azetidine-1-carboxylate (165mg, 1eq) was dissolved in dichloromethane (2mL), and added to trifluoroacetic acid (1mL) at room temperature. The mixture was stirred at room temperature for 1 hour. LCMS detection showed the raw material disappeared and the product appeared as the main peak. The reaction mixture was dried by rotary evaporation to produce the target compound (170mg).

### Step 3: Preparation of (R/S)-2-(3-hydroxy-3-(4-(trifluoromethyl)phenyl)azetidin-1-yl)-4-((1-(hydroxymethyl)cyclobutyl)amino)-6,7-dihydrothieno[3,2-d]pyrimidine 5-oxide:

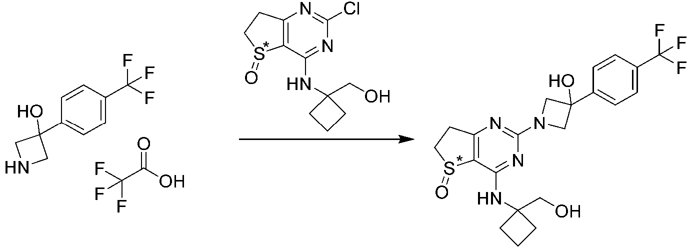

(R/S)-2-chloro-4-((1-(hydroxymethyl)cyclobutyl)amino)-6,7-dihydrothieno[3,2-d]pyrimidine 5-oxide (100mg, 1eq), 3-(4-(trifluoromethyl)phenyl)azetidin-3-ol 2,2,2-trifluoroacetate (1.5eq), and DIEA(5eq) were dissolved in dioxane (2mL). The mixture was reacted at 120°C under microwave for 1 hour. LCMS detection showed the raw material disappeared and the product appeared as the main peak. The reaction mixture was directly separated and purified by Prep-HPLC (C18, 10 mmol/L, aqueous ammonium bicarbonate solution, acetonitrile) to produce the target compound (42mg). LCMS:(ESI)[M+H]⁺=469.2; ¹H NMR(400 MHz, DMSO-*d*₆ + D₂O)δ 7.80-7.68(m, 4H), 4.35-4.10(m, 4H), 3.75-3.71(m, 1H), 3.69-3.64(m, 1H), 3.48-3.34(m, 1H), 3.31-3.20(m, 1H), 3.02-2.84(m, 2H), 2.38-2.20(m, 2H), 2.15-2.05(m, 2H), 1.85-1.62(m, 2H).

### Example 24 (Compound 8)

### Synthesis of (R/S)-2-(4-((2-(3-(3-chloro-4-methoxyphenyl)-3-hydroxyazetidin-1-yl)-5-oxido-6,7-dihydrothieno[3,2-d]pyrimidin-4-yl)amino)phenyl)acetic acid:

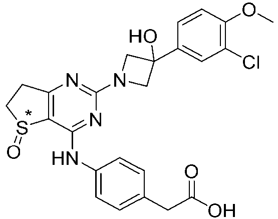

### Step 1: Preparation of ethyl 2-(4-((2-chloro-6,7-dihydrothieno[3,2-d]pyrimidin-4-yl)amino)phenyl)acetate:

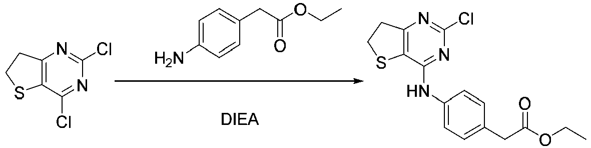

2,4-dichloro-6,7-dihydrothieno[3,2-d]pyrimidine (1.5g, 1.0eq), ethyl 2-(4-aminophenyl)acetate (1.2eq) and N,N-diisopropylethylamine (3.0eq) were dissolved in dimethyl sulfoxide (20mL). Then the system was reacted at 145°C for 16 hours. LCMS detection showed the product appeared as the main peak. The system was cooled to room temperature. Water (50mL) was added. The aqueous phase was extracted with ethyl acetate (100mL×2). The organic phases were combined, washed with water (100mL×2), dried over anhydrous sodium sulfate, and concentrated under a reduced pressure to produce a crude product. The crude product was separated and purified by flash chromatography (silica gel, petroleum ether:ethyl acetate=3:1) to produce the target compound (1.98g). LCMS(ESI)[M+H]⁺=350.1.

### Step 2: Preparation of ethyl (R/S)-2-(4-((2-chloro-5-oxido-6,7-dihydrothieno[3,2-d]pyrimidin-4-yl)amino)phenyl)acetate:

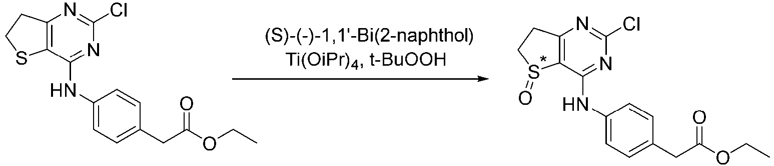

Ethyl 2-(4-((2-chloro-6,7-dihydrothieno[3,2-d]pyrimidin-4-yl)amino)phenyl)acetate (600mg, 1.0eq) and S-(-)binaphthol (0.1eq) were dissolved in dichloromethane (5mL). The mixture was stirred under the protection of nitrogen gas for 5 minutes. Then tetraisopropyl titanate (Ti(OⁱPr)₄)(0.1eq) and water (1.0eq) were added. The mixture was reacted under stirring at room temperature for 1 hour. Tert-butyl peroxide (70%/water, 1.5eq) was added under an ice bath, and the mixture was stirred at room temperature for 16 hours. LCMS detection showed the product was dominant. The solution was concentrated under a reduced pressure to produce a crude product. Then the crude product was separated and purified by flash chromatography (silica gel, dichloromethane:methanol=20:1) to produce the target compound (230mg). LCMS(ESI)[M+H]⁺=366.2.

### Step 3: Preparation of ethyl (R/S)-2-(4-((2-(3-(3-chloro-4-methoxyphenyl)-3-hydroxyazetidin-1-yl)-5-oxido-6,7-dihydrothieno[3,2-d]pyrimidin-4-yl)amino)phenyl)acetate:

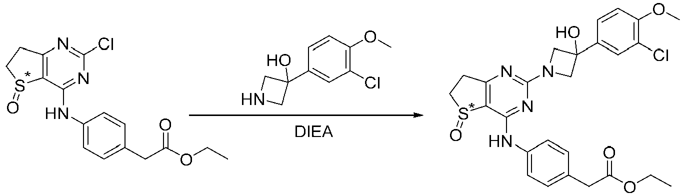

Ethyl (R/S)-2-(4-((2-chloro-5-oxido-6,7-dihydrothieno[3,2-d]pyrimidin-4-yl)amino)phenyl)acetate (230mg, 1.0eq), 3-(3-chloro-4-methoxyphenyl)azetidin-3-ol trifluoroacetate (1.3eq) and N,N-diisopropylethylamine (3.0eq) were dissolved in 1,4-dioxane (4mL). Then the mixture was reacted at 120°C for 2 hours. LCMS detection showed the product appeared as the main peak. The system was cooled to room temperature. Water (30mL) was added. The mixture was stirred for 0.5 hours, and filtered. The filter cake was washed with water twice to produce the target compound (200mg). LCMS(ESI)[M+H]⁺=543.4.

### Step 4: Preparation of (R/S)-2-(4-((2-(3-(3-chloro-4-methoxyphenyl)-3-hydroxyazetidin-1-yl)-5-oxido-6,7-dihydrothieno[3,2-d]pyrimidin-4-yl)amino)phenyl)acetic acid:

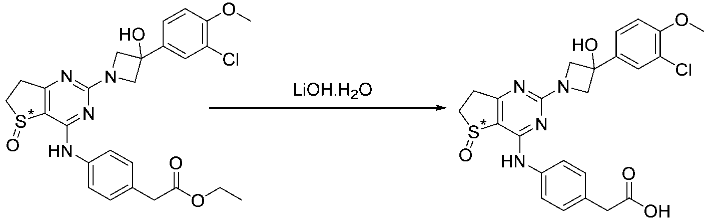

Ethyl (R/S)-2-(4-((2-(3-(3-chloro-4-methoxyphenyl)-3-hydroxyazetidin-1-yl)-5-oxido-6,7-dihydrothieno[3,2-d]pyrimidin-4-yl)amino)phenyl)acetate (200mg, 1.0eq) and lithium hydroxide hydrate (10eq) were dissolved in tetrahydrofuran (3mL), MeOH(3mL) and water (1.5mL). Then the system was stirred at 25°C for 2 hours. LCMS detection showed the product appeared as the main peak. Then an aqueous citric acid solution was added to adjust the pH to 5-6. The aqueous phase was extracted with ethyl acetate (30mL×2). The organic phases were dried over anhydrous sodium sulfate, and concentrated under a reduced pressure to produce a crude product. The crude product was separated and purified by Prep-HPLC (C18, 10mmol/L aqueous formic acid solution, acetonitrile) to produce the target compound (42mg). LCMS(ESI)[M+H]⁺=515.2; ¹H NMR(400 MHz, DMSO-*d*₆)δ 9.55(s, 1H), 7.73(d, J=8.6Hz, 2H), 7.55(s, 1H), 7.46(d, J=8.3Hz, 1H), 7.17(dd, J=13.7, 8.6Hz, 3H), 6.48(s, 1H), 4.26(t, J=8.9Hz, 4H), 3.85(s, 3H), 3.57-3.51(m, 1H), 3.50(s, 1H), 3.31-3.23(m, 3H), 3.07(dd, J=17.1, 8.0Hz, 1H), 2.98(dd, J=14.1, 6.6Hz, 1H).

### Example 25 (Compound 10)

### Synthesis of (R/S)-2-(3-(3-chloro-4-methoxyphenyl)-3-hydroxyazetidin-1-yl)-4-((3-(2-hydroxypropan-2-yl)phenyl)amino)-6,7-dihydrothieno[3,2-d]pyrimidine 5-oxide:

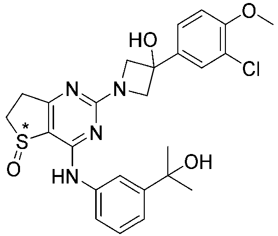

### Step 1: Preparation of methyl 3-((2-chloro-6,7-dihydrothieno[3,2-d]pyrimidin-4-yl)amino)benzoate:

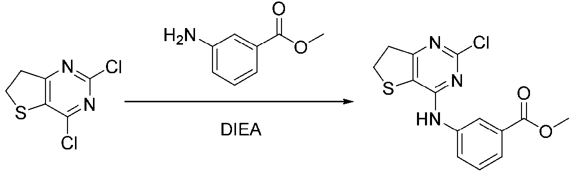

2,4-Dichloro-6,7-dihydrothieno[3,2-d]pyrimidine (1.2g, 1.0eq), methyl 3-aminobenzoate (1.2eq) and N,N-diisopropylethylamine (3.0eq) were dissolved in dimethyl sulfoxide (20mL). Then the system was reacted at 120°C for 16 hours. LCMS detection showed the product appeared as the main peak. Then the system was cooled, and water was added. The aqueous phase was extracted with ethyl acetate (100mL×2). The organic phase was washed with water (100mL×2). The combined organic phases were dried over anhydrous sodium sulfate, and concentrated under a reduced pressure to produce a crude product. The crude product was separated and purified by flash chromatography (silica gel, petroleum ether:ethyl acetate=3:1) to produce the target compound (550mg). LCMS(ESI)[M+H]⁺=322.1.

### Step 2: Preparation of methyl (R/S)-3-((2-chloro-5-oxido-6,7-dihydrothieno[3,2-d]pyrimidin-4-yl)amino)benzoate:

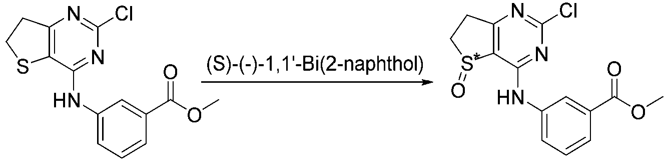

Methyl 3-((2-chloro-6,7-dihydrothieno[3,2-d]pyrimidin-4-yl)amino)benzoate (340mg, 1.0eq) and S-(-)-binaphthol (0.1eq) were dissolved in dichloromethane (3mL). Water (1.0eq) and tetraisopropyl titanate (1.0eq) were added under the protection of nitrogen gas. The mixture was stirred at room temperature for 1 hour. Then tert-butyl peroxide (1.5eq) was added, and the mixture was stirred at room temperature overnight. LCMS detection showed the product appeared as the main peak. The reaction mixture was concentrated under a reduced pressure to produce a crude product. The crude product was separated and purified by flash chromatography (silica gel, dichloromethane:methanol=20:1) to produce the target compound (120mg). LCMS(ESI)[M+H]⁺=338.1.

### Step 3: Preparation of methyl (R/S)-3-((2-(3-(3-chloro-4-methoxyphenyl)-3-hydroxyazetidin-1-yl)-5-oxido-6,7-dihydrothieno[3,2-d]pyrimidin-4-yl)amino)benzoate:

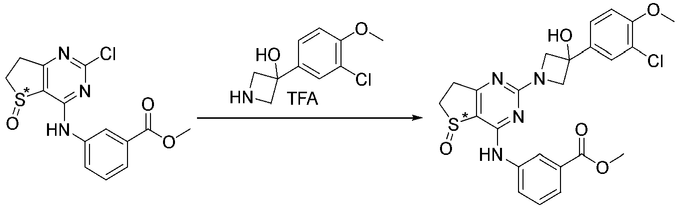

Methyl (R/S)-3-((2-chloro-5-oxido-6,7-dihydrothieno[3,2-d]pyrimidin-4-yl)amino)benzoate (120mg, 1.0eq), 3-(3-chloro-4-methoxyphenyl)azetidin-3-ol trifluoroacetate (2.0eq) and N,N-diisopropylethylamine (10eq) were dissolved in 1,4-dioxane (4mL). The system was reacted at 120°C for 2 hours. LCMS detection showed the reaction was completed. Then the system was cooled to room temperature. Water (30mL) was added. The mixture was stirred overnight and filtered. The filter cake was successively washed with water (10mL×2) and acetone (5mL) to produce the target compound (164mg). LCMS(ESI)[M+H]⁺=515.3.

### Step 4: Preparation of (R/S)-2-(3-(3-chloro-4-methoxyphenyl)-3-hydroxyazetidin-1-yl)-4-((3-(2-hydroxypropan-2-yl)phenyl)amino)-6,7-dihydrothieno[3,2-d]pyrimidine 5-oxide:

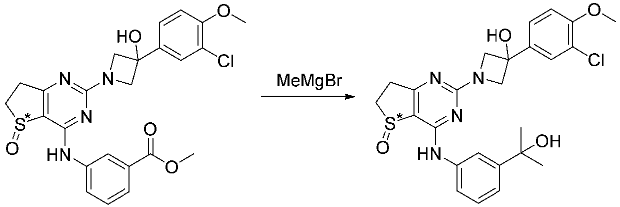

Methyl (R/S)-3-((2-(3-(3-chloro-4-methoxyphenyl)-3-hydroxyazetidin-1-yl)-5-oxido-6,7-dihydrothieno[3,2-d]pyrimidin-4-yl)amino)benzoate (100mg, 1.0eq) was dissolved in anhydrous tetrahydrofuran (5mL). Then methyl magnesium bromide (3M diethylether solution, 10.0eq) was added under an ice bath. The system was stirred at room temperature for 16 hours. LCMS detection showed the reaction was completed. Then a saturated aqueous ammonium chloride solution (10mL) was added to quench the reaction. The aqueous solution was extracted with ethyl acetate (30mL×2). The organic phases were dried over anhydrous sodium sulfate, and concentrated under a reduced pressure to produce a crude product. The crude product was separated and purified by Prep-HPLC (C18, 10mmol/L aqueous ammonium bicarbonate solution, acetonitrile) to produce the target compound (4mg). LCMS(ESI)[M+H]⁺=515.2; ¹H NMR(400 MHz, DMSO-*d*₆)δ 9.52(s, 1H), 8.00(s, 1H), 7.62-7.51(m, 2H), 7.45(d, *J*=8.4Hz, 1H), 7.22(t, *J*=7.9Hz, 1H), 7.15(t, *J*=6.2Hz, 2H), 6.48(s, 1H), 4.94(s, 1H), 4.25(d, *J*=8.5Hz, 4H), 3.85(s, 3H), 3.51(dd, *J*=16.8, 8.2Hz, 1H), 3.26(d, *J*=8.4Hz, 1H), 3.07(dd, *J*=17.1, 7.8Hz, 1H), 2.97(dd, *J*=13.7, 6.7Hz, 1H), 1.41(s, 6H).

### Example 26 (Compound 18)

### Synthesis of (R/S)-4-((1-(hydroxymethyl)cyclobutyl)amino)-2-(4-(2-hydroxypropan-2-yl)phenyl)-6,7-dihydrothieno[3,2-d]pyrimidine 5-oxide:

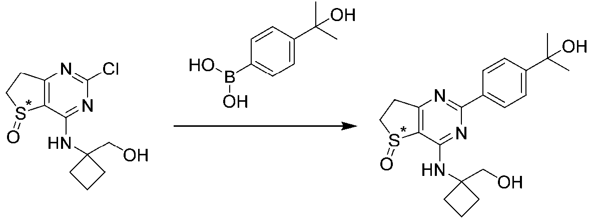

(R/S)-2-chloro-4-((1-(hydroxymethyl)cyclobutyl)amino)-6,7-dihydrothieno[3,2-d]pyrimidine 5-oxide (50mg, 1eq), (4-(2-hydroxypropan-2-yl)phenyl)boronic acid (1.5eq), Pd(dppf)Cl₂(0.1eq) and potassium carbonate (2.5eq) were added to dioxane (3mL) and water (1mL). The atmosphere was replaced with nitrogen gas three times, and the mixture was stirred at 110°C under the protection of nitrogen gas for 3 hours. LCMS detection showed most of the raw material disappeared and a product was formed. The reaction mixture was poured into water (100mL). The mixture was extracted with ethyl acetate (30mL×3). The organic phases were combined, dried over anhydrous sodium sulfate, and concentrated under a reduced pressure to produce a crude product. The crude product was separated and purified by Prep-HPLC (C18, 10mmol/L aqueous ammonium bicarbonate solution, acetonitrile) to produce the target compound (8mg). LCMS:(ESI)[M+1]⁺=388.28; ¹H NMR(400 MHz, DMSO-*d*₆)δ 8.25(d, J=8.4Hz, 2H), 7.97(s, 1H), 7.58(d, J=8.4Hz, 2H), 5.09(s, 1H), 4.92(t, J=5.7Hz, 1H), 3.87-3.77(m, 2H), 3.69-3.57(m, 1H), 3.42-3.33(m, 1H), 3.25-3.15(m, 1H), 3.10-2.95(m, 1H), 2.49-2.10(m, 4H), 1.93-1.76(m, 2H), 1.45(s, 6H).

### Example 27 (Compound 19)

### Synthesis of (R/S)-4-((1-(hydroxymethyl)cyclobutyl)amino)-2-(4-(trifluoromethyl)phenyl)-6,7-dihydrothieno[3,2-d]pyrimidine 5-oxide:

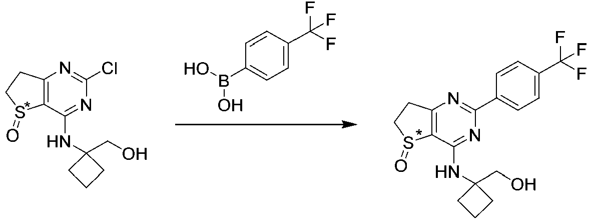

(R/S)-2-chloro-4-((1-(hydroxymethyl)cyclobutyl)amino)-6,7-dihydrothieno[3,2-d]pyrimidine 5-oxide (50mg, 1eq), (4-(trifluoromethyl)phenyl)boronic acid (1.5eq), Pd(dppf)Cl₂(0.1eq) and potassium carbonate (2.5eq) were added to dioxane (3mL) and water (1mL). The atmosphere was replaced with nitrogen gas three times, and the mixture was stirred at 110°C under the protection of nitrogen gas for 3 hours. LCMS detection showed the raw material disappeared and the product appeared as the main peak. The reaction mixture was poured into water (100mL), and extracted with ethyl acetate (30mL×3). The organic phases were combined, dried over anhydrous sodium sulfate, and concentrated under a reduced pressure to produce a crude product. The crude product was separated and purified by Prep-HPLC (C18, 10mmol/L aqueous ammonium bicarbonate solution, acetonitrile) to produce the target compound (1mg). LCMS:(ESI)[M+1]⁺=398.1; ¹H NMR(400 MHz, DMSO-*d*₆)δ 8.50(d, J=8.1Hz, 2H), 8.20(s, 1H), 7.88(d, J=8.3Hz, 2H), 4.93(t, J=5.7Hz, 1H), 3.90-3.76(m, 2H), 3.75 -3.60(m, 1H), 3.44-3.36(m, 1H), 3.30-3.20(m, 1H), 3.10-3.00(m, 1H), 2.44-2.30(m, 4H), 1.93-1.75(m, 2H).

### Example 28 (Compound 20)

### Synthesis of (R/S)-2-(4-chlorophenyl)-4-((1-(hydroxymethyl)cyclobutyl)amino)-6,7-dihydrothieno[3,2-d]pyrimidine 5-oxide:

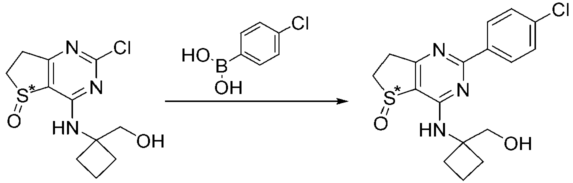

Under the protection of nitrogen gas, [1,1'-bis(diphenylphosphino)ferrocene]dichloropalladium (0.1eq) was added to a solution of (R/S)-2-chloro-4-((1-(hydroxymethyl)cyclobutyl)amino)-6,7-dihydrothieno[3,2-d]pyrimidine 5-oxide (50mg, 1eq), (4-chlorophenyl)boronic acid (1.2eq) and potassium carbonate (2.5eq) in dioxane (3mL) and water (0.6mL). The resulting mixture was warmed up to 110°C under stirring and reacted for 2 hours. LCMS detection showed the reaction was completed. The reaction mixture was diluted with water (20mL), extracted with ethyl acetate (50mL), and separated into phases. The organic phase was dried over anhydrous sodium sulfate, and filtered. The filtrate was concentrated, separated and purified by Prep-HPLC (C18, 10mmol/L aqueous ammonium bicarbonate solution, acetonitrile) to produce the target compound (16mg). LCMS(ESI)[M+H]⁺=364.2; ¹H NMR(400 MHz, DMSO-*d*₆)δ 8.31(d, J=8.7Hz, 2H), 8.11(s, 1H), 7.57(d, J=8.6Hz, 2H), 4.92(t, J=5.7Hz, 1H), 3.90-3.76(m, 2H), 3.70-3.57(m, 1H), 3.41-3.35(m, 1H), 3.21(ddd, J=17.1, 8.1, 1.8Hz, 1H), 3.11-2.97(m, 1H), 2.40(d, J=12.1Hz, 1H), 2.34(dd, J=15.4, 6.3Hz, 3H), 1.82(dd, J=17.9, 9.2Hz, 2H).

### Example 29 (Compound 21)

### Synthesis of (R/S)-4-((1-(hydroxymethyl)cyclobutyl)amino)-2-(4-(trifluoromethoxy)phenyl)-6,7-dihydrothieno[3,2-d]pyrimidine 5-oxide:

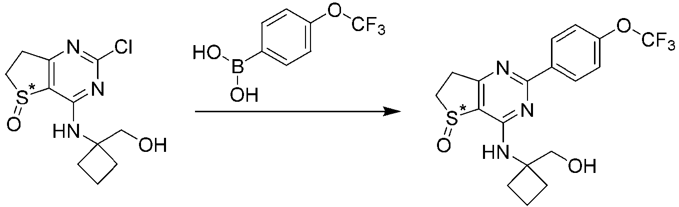

(R/S)-2-chloro-4-((1-(hydroxymethyl)cyclobutyl)amino)-6,7-dihydrothieno[3,2-d]pyrimidine 5-oxide (50mg, 1eq), (4-(trifluoromethoxy)phenyl)boronic acid (1.5eq), Pd(dppf)Cl₂ (0.1eq), and potassium carbonate (2.5eq) were added to dioxane (3mL) and water (0.6mL). After the atmosphere was replaced with nitrogen gas, the mixture was heated to 110°C and stirred for 3 hours. LCMS detection showed the raw material disappeared and the product appeared as the main peak. The reaction mixture was concentrated under a reduced pressure to produce a crude product. The crude product was separated and purified by Prep-HPLC (C18, 10mmol/L aqueous ammonium bicarbonate solution, acetonitrile) to produce the target compound (3mg). LCMS:(ESI)[M+H]⁺=414.2; ¹H NMR(400 MHz, DMSO-*d*₆)δ 8.42(d, J=8.8Hz, 2H), 8.12(s, 1H), 7.49(d, J=8.5Hz, 2H), 4.91(t, J=5.7Hz, 1H), 3.87-3.75(m, 2H), 3.70-3.58(m, 1H), 3.44-3.34(m, 1H), 3.24-3.18(m, 1H), 3.10-2.98(m, 1H), 2.50-2.10(m, 4H), 1.90-1.72(m, 2H).

### Example 30 (Compound 35)

### Synthesis of (R/S)-2-(4-chloro-5-methoxyisoindolin-2-yl)-4-((1-(hydroxymethyl)cyclobutyl)amino)-6,7-dihydrothieno[3,2-d]pyrimidine 5-oxide:

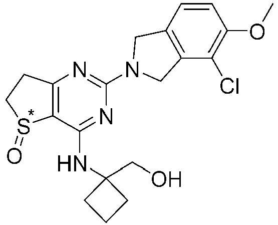

### Step 1: Preparation of tert-butyl 5-methoxyisoindoline-2-carboxylate:

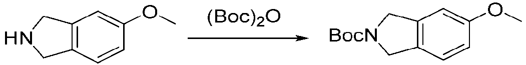

Under the protection of nitrogen gas, 5-methoxyisoindoline (300mg, 1.0eq) was added to dichloromethane (10mL). Triethylamine (2.0eq) and di-tert-butyl dicarbonate (1.5eq) were added to the reaction mixture. The system was reacted at room temperature for 16 hours. LCMS detection showed that the raw material was completely consumed and converted to the product. Water (30mL) was added to the reaction mixture. The mixture was extracted with dichloromethane (20mL×3). The organic phases were combined, dried over anhydrous sodium sulfate, filtered, and concentrated. The residue was purified by silica gel column chromatography (petroleum ether:ethyl acetate=20:1) to produce the target compound (300mg). LCMS(ESI)[M+H]⁺=250.2.

### Step 2: Preparation of tert-butyl 4-chloro-5-methoxyisoindoline-2-carboxylate:

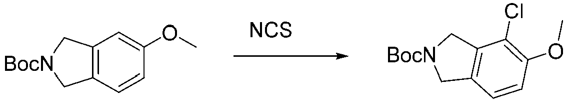

Under the protection of nitrogen gas, tert-butyl 5-methoxyisoindoline-2-carboxylate (300mg, 1.0eq) was added to acetonitrile (5mL) and tetrahydrofuran (5mL). N-chlorosuccinimide (1.5eq) was added to the reaction mixture. The mixture was reacted at room temperature for 16 hours. LCMS detection showed that the raw material was completely consumed and converted to the product. Water (30mL) was added to the reaction mixture. The mixture was extracted with ethyl acetate (40mL×3). The organic phases were combined, dried over anhydrous sodium sulfate, filtered, and concentrated. The residue was separated and purified by Prep-HPLC (C18, 10mmol/L aqueous ammonium bicarbonate solution, acetonitrile) to produce the target compound (60mg). LCMS(ESI)[M+H]⁺=284.2.

### Step 3: Preparation of 4-chloro-5-methoxyisoindoline hydrochloride:

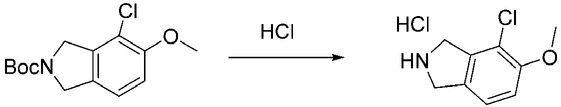

Under the protection of nitrogen gas, tert-butyl 4-chloro-5-methoxyisoindoline-2-carboxylate (60mg, 1.0eq) was added to 1,4-dioxane (5mL), and the mixture was stirred under an external ice-water bath for 20 minutes. A 4M solution of hydrogen chloride/1,4-dioxane (19.0eq) was added to the reaction mixture, and the mixture was stirred under an ice-water bath for 10 minutes. The ice-water bath was removed, and the mixture was reacted at room temperature for 2 hours. LCMS detection showed that the raw material was completely consumed and converted to the product. The reaction mixture was concentrated to produce the target compound (46mg). LCMS(ESI)[M+H]⁺=184.1.

### Step 4: Preparation of (R/S)-2-(4-chloro-5-methoxyisoindolin-2-yl)-4-((1-(hydroxymethyl)cyclobutyl)amino)-6,7-dihydrothieno[3,2-d]pyrimidine 5-oxide:

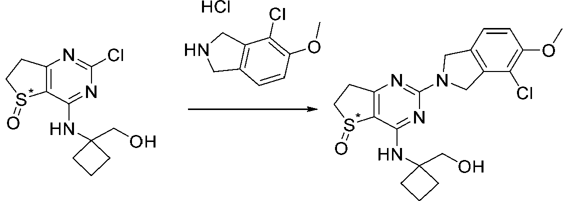

Under the protection of nitrogen gas, (R/S)-2-chloro-4-((1-(hydroxymethyl)cyclobutyl)amino)-6,7-dihydrothieno[3,2-d]pyrimidine 5-oxide (60mg, 1.0eq) and 4-chloro-5-methoxyisoindoline hydrochloride (1.0eq) were added to 1,4-dioxane (5mL). N,N-diisopropylethylamine (3eq) was added to the reaction mixture. The reaction mixture was reacted at 100°C for 6 hours. LCMS detection showed that the raw material was completely consumed and converted to the product. The mixture was cooled to room temperature. Water (30mL) was added to the reaction mixture. The mixture was extracted with ethyl acetate (50mL×3). The organic phases were combined, dried over anhydrous sodium sulfate, filtered, and concentrated. The residue was separated and purified by Prep-HPLC (C18, 10mmol/L aqueous ammonium bicarbonate solution, acetonitrile) to produce the target compound (42mg). LCMS(ESI)[M+H]⁺=435.1; ¹H NMR(400 MHz, DMSO-*d*₆)δ 7.48(d, J=20.9Hz, 2H), 7.23(d, J=32.6Hz, 1H), 4.84(d, J=29.1Hz, 1H), 4.75(s, 2H), 4.71(d, J=7.1Hz, 2H), 3.86(s, 3H), 3.79(s, 2H), 3.53-3.40(m, 1H), 3.23(dt, J=13.5, 8.3Hz, 1H), 3.05-2.93(m, 1H), 2.89(dd, J=13.5, 6.5Hz, 1H), 2.47-2.31(m, 2H), 2.19(d, J=28.1Hz, 2H), 1.91-1.69(m, 2H).

### Example 31 (Compound 65)

### Synthesis of (R/S)-1-(3-((2-(3-(3-chloro-4-methoxyphenyl)-3-hydroxyazetidin-1-yl)-5-oxido-6,7-dihydrothieno[3,2-d]pyrimidin-4-yl)amino)phenyl)ethan-1-one:

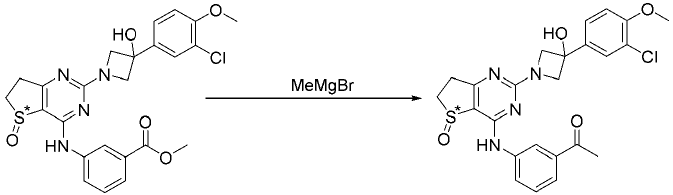

Methyl (R/S)-3-((2-(3-(3-chloro-4-methoxyphenyl)-3-hydroxyazetidin-1-yl)-5-oxido-6,7-dihydrothieno[3,2-d]pyrimidin-4-yl)amino)benzoate (100mg, 1.0eq) was dissolved in anhydrous tetrahydrofuran (5mL). Then methyl magnesium bromide (3M diethylether solution, 10.0eq) was added under an ice bath. The system was stirred at room temperature for 16 hours. LCMS detection showed the reaction was completed. Then a saturated aqueous ammonium chloride solution (10mL) was added to quench the reaction. The aqueous solution was extracted with ethyl acetate (30mL×2). The organic phases were dried over anhydrous sodium sulfate and then dried by rotary evaporation to produce a crude product. The crude product was separated and purified by Prep-HPLC (C18, 10mmol/L aqueous ammonium bicarbonate solution, acetonitrile) to produce the target compound (2mg). LCMS(ESI)[M+H]⁺=499.2; ¹H NMR(400 MHz, DMSO-*d*₆)δ 9.81(s, 1H), 8.63(s, 1H), 8.04(d, *J*=8.5Hz, 1H), 7.65(d, *J*=8.1Hz, 1H), 7.55(s, 1H), 7.47(t, *J*=7.8Hz, 2H), 7.17(s, 1H), 6.52(d, *J*=11.7Hz, 1H), 4.29(d, *J*=14.5Hz, 4H), 3.85(s, 3H), 3.54(d, *J*=8.8Hz, 1H), 3.26(d, *J*=7.2Hz, 1H), 3.14-3.07(m, 1H), 3.05-2.97(m, 1H), 2.56(s, 3H).

### Example 32 (Compound 66)

### Synthesis of (R/S)-4-((1-(hydroxymethyl)cyclobutyl)amino)-2-(4-(3-methoxyazetidin-1-yl)phenyl)-6,7-dihydrothieno[3,2-d]pyrimidine 5-oxide:

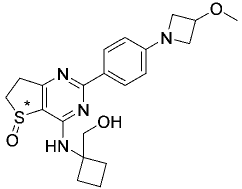

### Step 1: Preparation of 1-(4-bromophenyl)-3-methoxyazetidine:

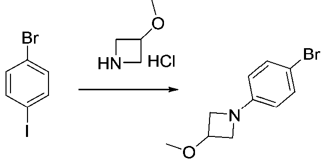

Under the protection of nitrogen gas, 1-bromo-4-iodobenzene (565mg, 1.0eq) and 3-methoxyazetidine hydrochloride (1.0eq) were added to 1,4-dioxane (10mL). Pd₂(dba)₃(0.05eq), 4,5-bis(diphenylphosphino)-9,9-dimethylxanthene (0.05eq) and cesium carbonate (2.5eq) were added to the reaction mixture. The reaction mixture was warmed up to 100°C and reacted for 2 hours. LCMS detection showed that the raw material was completely consumed and converted to the product. The heating was removed, and the reaction mixture was concentrated under a reduced pressure. The residue was purified by silica gel column chromatography (petroleum ether:ethyl acetate=20:1) to produce the target compound (370mg). LCMS(ESI)[M+H]⁺=242.1.

### Step 2: Preparation of 3-methoxy-1-(4-(4,4,5,5-tetramethyl-1,3,2-dioxaborolan-2-yl)phenyl)azetidine:

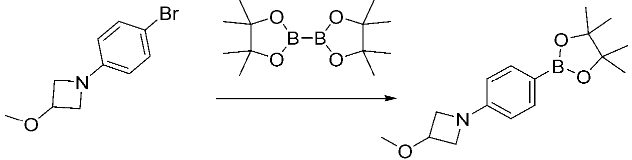

Under the protection of nitrogen gas, 1-(4-bromophenyl)-3-methoxyazetidine (370mg, 1.0eq) and bis(pinacolato)diboron (1.2eq) were added to 1,4-dioxane (15mL). Pd(dppf)Cl₂ (0.1eq) and potassium acetate (2.5eq) were added to the reaction mixture. The reaction mixture was warmed up to 90°C and reacted for 2 hours. LCMS detection showed that the raw material was completely consumed and converted to the product. The reaction mixture was concentrated under a reduced pressure. The residue was purified by silica gel column chromatography (petroleum ether:ethyl acetate=20:1 to 10:1) to produce the target compound (350mg). LCMS(ESI)[M+H]⁺=290.2.

### Step 3: Preparation of (R/S)-4-((1-(hydroxymethyl)cyclobutyl)amino)-2-(4-(3-methoxyazetidin-1-yl)phenyl)-6,7-dihydrothieno[3,2-d]pyrimidine 5-oxide:

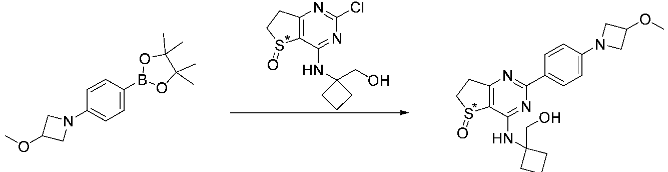

Under the protection of nitrogen gas, (R/S)-2-chloro-4-((1-(hydroxymethyl)cyclobutyl)amino)-6,7-dihydrothieno[3,2-d]pyrimidine 5-oxide (100mg, 1.0eq) and 3-methoxy-1-(4-(4,4,5,5-tetramethyl-1,3,2-dioxaborolan-2-yl)phenyl)azetidine (1.2eq) were added to 1,4-dioxane (8mL). Water (2mL) was added to the reaction mixture. Pd(PPh₃)₄ (0.1eq) and sodium carbonate (2.5eq) were added under stirring. The reaction mixture was warmed up to 80°C and reacted for 2 hours. LCMS detection showed that the raw material was completely consumed and converted to the product. The reaction mixture was cooled to room temperature. Water (30mL) was added. The mixture was extracted with ethyl acetate (50mL×3). The organic phases were combined, dried over anhydrous sodium sulfate, filtered, and concentrated under a reduced pressure. The residue was separated and purified by Prep-HPLC (C18, 10mmol/L aqueous ammonium bicarbonate solution, acetonitrile) to produce the target compound (23mg). LCMS(ESI)[M+H]⁺=415.3; ¹H NMR(400 MHz, DMSO-*d*₆)δ 8.17(d, J=8.7Hz, 2H), 7.80(s, 1H), 6.49(d, J=8.8Hz, 2H), 4.92(t, J=5.3Hz, 1H), 4.39-4.30(m, 1H), 4.18-4.07(m, 2H), 3.80(d, J=4.9Hz, 2H), 3.71(dd, J=8.5, 4.0Hz, 2H), 3.65-3.52(m, 1H), 3.32-3.28(m, 1H), 3.26(s, 3H), 3.14(dd, J=17.0, 8.1Hz, 1H), 2.98(dd, J=13.7, 5.7Hz, 1H), 2.46-2.33(m, 2H), 2.30(dd, J=8.2, 4.7Hz, 2H), 1.82(dt, J=18.0, 9.1Hz, 2H).

### Example 33 (Compound 67)

### Synthesis of (R/S)-4-((1-(hydroxymethyl)cyclobutyl)amino)-2-(4-(4-methylpiperazin-1-yl)phenyl)-6,7-dihydrothieno[3,2-d]pyrimidine 5-oxide:

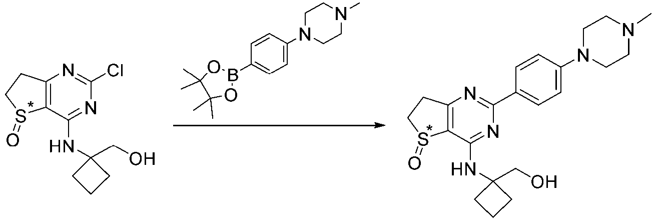

Under the protection of nitrogen gas, [1,1'-bis(diphenylphosphino)ferrocene]dichloropalladium (13mg, 0.1eq) was added to a mixture of (R/S)-2-chloro-4-((1-(hydroxymethyl)cyclobutyl)amino)-6,7-dihydrothieno[3,2-d]pyrimidine 5-oxide (1eq), 1-methyl-4-(4-(4,4,5,5-tetramethyl-1,3,2-dioxaborolan-2-yl)phenyl)piperazine (1eq) and potassium carbonate (2.5eq) in dioxane (1mL) and water (0.2mL). The reaction mixture was warmed up to 110°C and reacted for 2 hours. TLC detection showed the reaction was completed. The reaction mixture was diluted with ethyl acetate (100mL). Activated carbon (0.05g) was added to the diluted mixture, and the resulting mixture was stirred for 4 hours and filtered through diatomite (2.00g). The filtrate was concentrated, separated and purified by Prep-HPLC (C18, 10mmol/L aqueous ammonium bicarbonate solution, acetonitrile) to produce the target compound (30mg). LCMS(ESI)[M+H]⁺=428.4; ¹H NMR(400 MHz, DMSO-*d*₆)δ 8.17(d, J=8.9Hz, 2H), 7.82(s, 1H), 7.00(d, J=9.0Hz, 2H), 4.92(t, J=5.6Hz, 1H), 3.81(d, J=5.4Hz, 2H), 3.65-3.53(m, 1H), 3.38-3.34(m, 1H), 3.30-3.24(m, 4H), 3.15(dd, J=17.1, 8.2Hz, 1H), 2.99(dd, J=13.7, 5.7Hz, 1H), 2.47-2.42(m, 4H), 2.41-2.33(m, 2H), 2.33-2.24(m, 2H), 2.22(s, 3H), 1.84(dd, J=16.6, 8.4Hz, 2H).

### Example 34 (Compound 68)

### Synthesis of (R/S)-1-(4-(4-((1-(hydroxymethyl)cyclobutyl)amino)-5-oxido-6,7-dihydrothieno[3,2-d]pyrimidin-2-yl)phenyl)pyrrolidin-2-one:

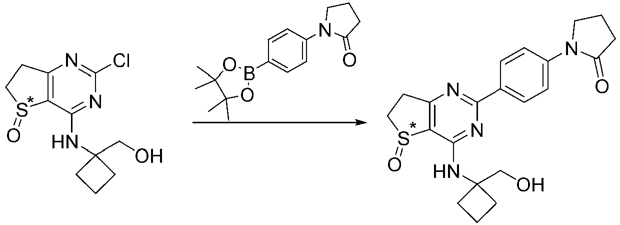

Under the protection of nitrogen gas, [1,1'-bis(diphenylphosphino)ferrocene]dichloropalladium (13mg, 0.1eq) was added to a mixture of (R/S)-2-chloro-4-((1-(hydroxymethyl)cyclobutyl)amino)-6,7-dihydrothieno[3,2-d]pyrimidine 5-oxide (1eq), 1-(4-(4,4,5,5-tetramethyl-1,3,2-dioxaborolan-2-yl)phenyl)pyrrolidin-2-one (1.2eq) and potassium carbonate (2.5eq) in dioxane (2mL) and water (0.5mL). The mixture was warmed up to 110°C and reacted for 2 hours. TLC detection showed the reaction was completed. The reaction mixture was diluted with ethyl acetate (100mL). Activated carbon (0.1g) was added to the diluted mixture, and the resulting mixture was stirred for 4 hours and filtered through diatomite (2g). The filtrate was concentrated, separated and purified by Prep-HPLC (C18, 10mmol/L aqueous ammonium bicarbonate solution, acetonitrile) to produce the target compound (30mg). LCMS(ESI)[M+H]⁺=413.1; ¹H NMR(400 MHz, DMSO-*d*₆)δ 8.32(d, J=8.8Hz, 2H), 8.00(s, 1H), 7.81(d, J=8.9Hz, 2H), 4.93(t, J=5.6Hz, 1H), 3.89(t, J=7.0Hz, 2H), 3.82(d, J=4.7Hz, 2H), 3.70-3.52(m, 1H), 3.37(t, J=6.9Hz, 1H), 3.20(dd, J=17.1, 8.1Hz, 1H), 3.08-2.97(m, 1H), 2.54(t, J=8.1Hz, 2H), 2.46-2.39(m, 1H), 2.38-2.25(m, 3H), 2.15-2.01(m, 2H), 1.93-1.74(m, 2H).

### Example 35 (Compound 69)

### Synthesis of (R/S)-2-(4-(dimethylamino)phenyl)-4-((1-(hydroxymethyl)cyclobutyl)amino)-6,7-dihydrothieno[3,2-d]pyrimidine 5-oxide:

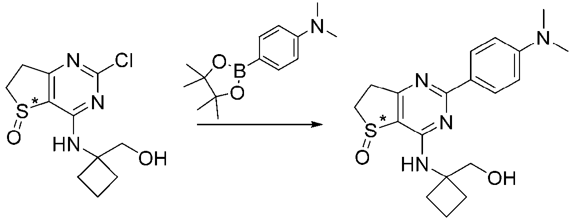

Under the protection of nitrogen gas, [1,1'-bis(diphenylphosphino)ferrocene]dichloropalladium (0.1eq) was added to a mixture of (R/S)-2-chloro-4-((1-(hydroxymethyl)cyclobutyl)amino)-6,7-dihydrothieno[3,2-d]pyrimidine 5-oxide (50mg, 1eq), N,N-dimethyl-4-(4,4,5,5-tetramethyl-1,3,2-dioxaborolan-2-yl)aniline (1.5eq) and potassium carbonate (2.5eq) in dioxane (2mL) and water (0.5mL). The mixture was warmed up to 110°C and reacted for 2 hours. TLC detection showed the reaction was completed. Water (10mL) was added to the reaction mixture. The resulting mixture was diluted with ethyl acetate (30mL) and separated into phases. The organic phase was concentrated, and then separated and purified by Prep-HPLC (C18, 10mmol/L aqueous ammonium bicarbonate solution, acetonitrile) to produce the target compound (10mg). LCMS(ESI)[M+H]⁺=373.1; ¹H NMR(400 MHz, DMSO-*d*₆)δ 8.17(d, J=9.0Hz, 2H), 7.74(s, 1H), 6.77(d, J=9.0Hz, 2H), 4.91(d, J=5.5Hz, 1H), 3.81(d, J=5.1Hz, 2H), 3.63-3.53(m, 1H), 3.31-3.28(m, 1H), 3.14(dd, J=17.5, 6.8Hz, 1H), 3.00(s, 6H), 2.96(s, 1H), 2.39(dd, J=22.3, 12.3Hz, 2H), 2.29(s, 2H), 1.91-1.75(m, 2H).

### Example 36 (Compound 70)

### Synthesis of (R/S)-4-(4-((1-(hydroxymethyl)cyclobutyl)amino)-5-oxido-6,7-dihydrothieno[3,2-d]pyrimidin-2-yl)benzonitrile:

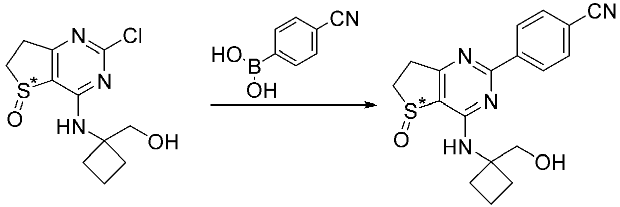

Under the protection of nitrogen gas, [1,1'-bis(diphenylphosphino)ferrocene]dichloropalladium (0.1eq) was added to a mixture of (R/S)-2-chloro-4-((1-(hydroxymethyl)cyclobutyl)amino)-6,7-dihydrothieno[3,2-d]pyrimidine 5-oxide (50mg, 1eq), (4-cyanophenyl)boronic acid (1.5eq) and potassium carbonate (2.5eq) in dioxane (2mL) and water (0.5mL). The mixture was warmed up to 110°C and reacted for 2 hours. LCMS detection showed the reaction was completed. The reaction mixture was diluted with water (10mL), extracted with ethyl acetate (30mL), and separated into phases. The organic phase was dried over anhydrous sodium sulfate, concentrated under a reduced pressure, and separated and purified by Prep-HPLC (C18, 10mmol/L aqueous ammonium bicarbonate solution, acetonitrile) to produce the target compound (50mg). LCMS(ESI)[M+H]⁺=355.2; ¹H NMR(400 MHz, DMSO-*d*₆)δ 8.45(d, J=8.5Hz, 2H), 8.25(s, 1H), 7.98(d, J=8.5Hz, 2H), 4.94(t, J=5.7Hz, 1H), 3.82(dd, J=5.7, 2.7Hz, 2H), 3.72-3.60(m, 1H), 3.43-3.34(m, 1H), 3.24(dd, J=17.3, 8.3Hz, 1H), 3.05(dd, J=13.7, 5.6Hz, 1H), 2.45-2.38(m, 1H), 2.33(t, J=8.3Hz, 3H), 1.85(dd, J=17.1, 8.7Hz, 2H).

### Example 37 (Compound 71)

### Synthesis of (R/S)-N-(4-(4-((1-(hydroxymethyl)cyclobutyl)amino)-5-oxido-6,7-dihydrothieno[3,2-d]pyrimidin-2-yl)phenyl)acetamide:

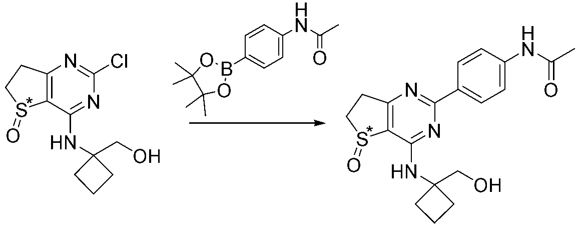

Under the protection of nitrogen gas, [1,1'-bis(diphenylphosphino)ferrocene]dichloropalladium (0.1eq) was added to a mixture of (R/S)-2-chloro-4-((1-(hydroxymethyl)cyclobutyl)amino)-6,7-dihydrothieno[3,2-d]pyrimidine 5-oxide (50mg, 1eq), N-(4-(4,4,5,5-tetramethyl-1,3,2-dioxaborolan-2-yl)phenyl)acetamide(1.5eq) and potassium carbonate (2.5eq) in dioxane (2mL) and water (0.5mL). The mixture was warmed up to 110°C and reacted for 2 hours. LCMS detection showed the reaction was completed. Water (10mL) was added to the reaction mixture. The resulting mixture was diluted with ethyl acetate (30mL) and separated into phases. The organic phase was concentrated under a reduced pressure. The residue was separated and purified by Prep-HPLC (C18, 10mmol/L aqueous ammonium bicarbonate solution, acetonitrile) to produce the target compound (10mg). LCMS(ESI)[M+H]⁺=387.1; ¹H NMR(400 MHz, DMSO-*d*₆)δ 10.18(s, 1H), 8.25(d, J=8.8Hz, 2H), 7.96(s, 1H), 7.70(d, J=8.8Hz, 2H), 4.92(t, J=5.7Hz, 1H), 3.82(d, J=5.7Hz, 2H), 3.62(dt, J=15.8, 7.7Hz, 1H), 3.38(d, J=8.2Hz, 1H), 3.19(dd, J=16.3, 7.4Hz, 1H), 3.01(dd, J=13.6, 5.6Hz, 1H), 2.42(dd, J=11.9, 9.7Hz, 1H), 2.37-2.27(m, 3H), 2.08(s, 3H), 1.88-1.77(m, 2H).

### Example 38 (Compound 72)

### Synthesis of (R/S)-N-(4-(4-((1-(hydroxymethyl)cyclobutyl)amino)-5-oxido-6,7-dihydrothieno[3,2-d]pyrimidin-2-yl)phenyl)methanesulfonamide:

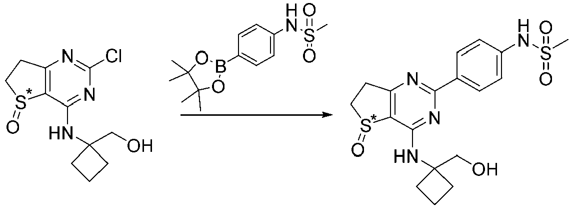

Under the protection of nitrogen gas, [1,1'-bis(diphenylphosphino)ferrocene]dichloropalladium (0.1eq) was added to a mixture of (R/S)-2-chloro-4-((1-(hydroxymethyl)cyclobutyl)amino)-6,7-dihydrothieno[3,2-d]pyrimidine 5-oxide (50mg, 1eq), N-(4-(4,4,5,5-tetramethyl-1,3,2-dioxaborolan-2-yl)phenyl)methanesulfonamide(1.5eq) and potassium carbonate (2.5eq) in dioxane (2mL) and water (0.5mL). The mixture was warmed up to 110°C and reacted for 2 hours. LCMS detection showed the reaction was completed. Water (10mL) was added to the reaction mixture. The resulting mixture was diluted with ethyl acetate (30mL) and separated into phases. The organic phase was concentrated under a reduced pressure. The residue was separated and purified by Prep-HPLC (C18, 10mmol/L aqueous ammonium bicarbonate solution, acetonitrile) to produce the target compound (15mg). LCMS(ESI)[M+H]⁺=423.1; ¹H NMR(400 MHz, DMSO-*d*₆)δ 9.93(s, 1H), 8.27(d, J=8.7Hz, 2H), 8.01(s, 1H), 7.29(d, J=8.7Hz, 2H), 4.93(t, J=5.7Hz, 1H), 3.81(d, J=5.6Hz, 2H), 3.61(dd, J=16.7, 8.5Hz, 1H), 3.36(s, 1H), 3.19(dd, J=16.9, 7.9Hz, 1H), 3.08(s, 3H), 3.01(dd, J=13.8, 5.9Hz, 1H), 2.40(d, J=12.3Hz, 1H), 2.33(s, 3H), 1.89-1.76(m, 2H).

### Example 39 (Compound 73)

### Synthesis of (R/S)-2-(4-(4-((1-(hydroxymethyl)cyclobutyl)amino)-5-oxido-6,7-dihydrothieno[3,2-d]pyrimidin-2-yl)phenyl)-2-methylpropanenitrile:

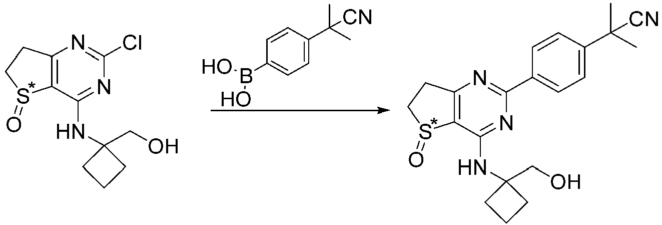

Under the protection of nitrogen gas, [1,1'-bis(diphenylphosphino)ferrocene]dichloropalladium (0.1eq) was added to a mixture of (R/S)-2-chloro-4-((1-(hydroxymethyl)cyclobutyl)amino)-6,7-dihydrothieno[3,2-d]pyrimidine 5-oxide (50mg, 1eq), (4-(2-cyanopropan-2-yl)phenyl)boronic acid (1.5eq) and potassium carbonate (2.5eq) in dioxane (2mL) and water (0.5mL). The mixture was warmed up to 110°C and reacted for 2 hours. LCMS detection showed the reaction was completed. Water (10mL) was added to the reaction mixture. The resulting mixture was diluted with ethyl acetate (30mL) and separated into phases. The organic phase was concentrated under a reduced pressure. The residue was separated and purified by Prep-HPLC (C18, 1 0mmol/L aqueous ammonium bicarbonate solution, acetonitrile) to produce the target compound (25mg). LCMS(ESI)[M+H]⁺=397.1; ¹H NMR(400 MHz, DMSO-*d*₆)δ 8.35(d, J=8.5Hz, 2H), 8.10(s, 1H), 7.66(d, J=8.6Hz, 2H), 4.93(t, J=5.7Hz, 1H), 3.82(d, J=4.1Hz, 2H), 3.71-3.59(m, 1H), 3.41-3.35(m, 1H), 3.22(dd, J=17.2, 8.2Hz, 1H), 3.04(dd, J=13.7, 5.6Hz, 1H), 2.39(dd, J=17.5, 7.9Hz, 1H), 2.36-2.27(m, 3H), 1.89-1.78(m, 2H), 1.73(s, 6H).

### Example 40 (Compound 74)

### Synthesis of methyl (R/S)-2-(4-(4-((1-(hydroxymethyl)cyclobutyl)amino)-5-oxido-6,7-dihydrothieno[3,2-d]pyrimidin-2-yl)phenyl)-2-methylpropanoate:

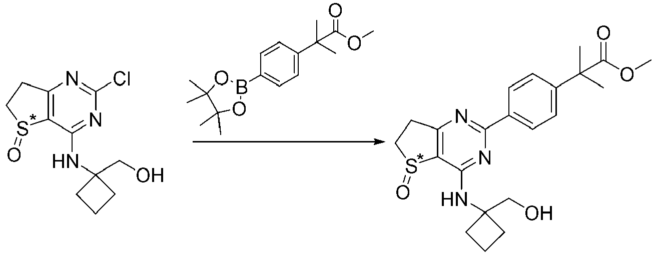

Under the protection of nitrogen gas, [1,1'-bis(diphenylphosphino)ferrocene]dichloropalladium (0.1eq) was added to a mixture of (R/S)-2-chloro-4-((1-(hydroxymethyl)cyclobutyl)amino)-6,7-dihydrothieno[3,2-d]pyrimidine 5-oxide (100mg, 1eq), methyl 2-methyl-2-(4-(4,4,5,5-tetramethyl-1,3,2-dioxaborolan-2-yl)phenyl)propanoate (1.5eq) and potassium carbonate (2.5eq) in dioxane (2mL) and water (0.5mL). The mixture was warmed up to 110°C and reacted for 2 hours. LCMS detection showed the reaction was completed. The reaction mixture was diluted with ethyl acetate (100mL). Activated carbon (0.10g) was added to the diluted mixture, and the resulting mixture was stirred for 4 hours and filtered through diatomite (3.00g). The filtrate was concentrated, separated and purified by Prep-HPLC (C18, 10mmol/L aqueous ammonium bicarbonate solution, acetonitrile) to produce the target compound (100mg). LCMS(ESI)[M+H]⁺=430.3; ¹H NMR(400 MHz, DMSO-*d*₆)δ 8.28(d, J=8.5Hz, 2H), 8.04(s, 1H), 7.44(d, J=8.5Hz, 2H), 4.92(t, J=5.6Hz, 1H), 3.81(d, J=5.0Hz, 2H), 3.65(dd, J=15.7, 6.5Hz, 1H), 3.59(s, 3H), 3.43-3.35(m, 1H), 3.21(dd, J=16.4, 7.5Hz, 1H), 3.03(dd, J=13.8, 5.8Hz, 1H), 2.46-2.39(m, 1H), 2.38-2.25(m, 3H), 1.92-1.77(m, 2H), 1.54(s, 6H).

### Example 41 (Compound 75)

### Synthesis of (R/S)-2-(4-(2-aminopropan-2-yl)phenyl)-4-((1-(hydroxymethyl)cyclobutyl)amino)-6,7-dihydrothieno[3,2-d]pyrimidine 5-oxide:

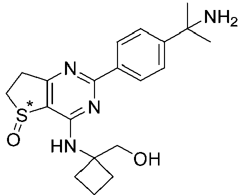

### Step 1: Preparation of tert-butyl (R/S)-(2-(4-(4-((1-(hydroxymethyl)cyclobutyl)amino)-5-oxido-6,7-dihydrothieno[3,2-d]pyrimidin-2-yl)phenyl)propan-2-yl)carbamate:

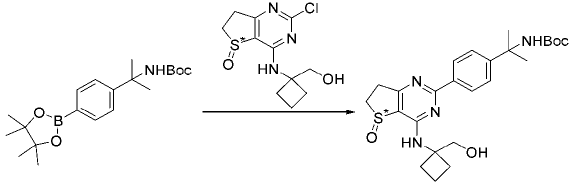

Under the protection of nitrogen gas, [1,1'-bis(diphenylphosphino)ferrocene]dichloropalladium (0.1eq) was added to a solution of tert-butyl (2-(4-(4,4,5,5-tetramethyl-1,3,2-dioxaborolan-2-yl)phenyl)propan-2-yl)carbamate (1.2eq) and (R/S)-2-chloro-4-((1-(hydroxymethyl)cyclobutyl)amino)-6,7-dihydrothieno[3,2-d]pyrimidine 5-oxide (100mg, 1eq) in potassium carbonate (2.5eq). The mixture was warmed up to 110°C and reacted for 2 hours. TLC detection showed the reaction was completed. The reaction mixture was diluted with ethyl acetate (100mL). Activated carbon (0.50g) was added to the diluted mixture, and the resulting mixture was stirred for 6 hours and filtered through diatomite (3.00g). The filtrate was concentrated, and purified by silica gel column chromatography (methanol/dichloromethane=1:20) to produce the target compound (100mg). LCMS(ESI)[M+H]⁺=487.1.

### Step 2: Preparation of (R/S)-2-(4-(2-aminopropan-2-yl)phenyl)-4-((1-(hydroxymethyl)cyclobutyl)amino)-6,7-dihydrothieno[3,2-d]pyrimidine 5-oxide:

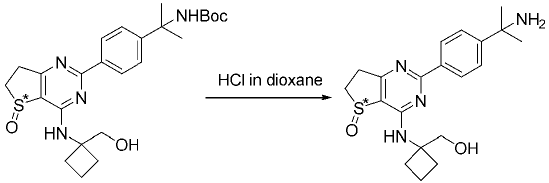

A 4M solution of hydrogen chloride/dioxane (20eq) was added dropwise to a solution of tert-butyl (R/S)-(2-(4-(4-((1-(hydroxymethyl)cyclobutyl)amino)-5-oxido-6,7-dihydrothieno[3,2-d]pyrimidin-2-yl)phenyl)propan-2-yl)carbamate (60mg, 1eq) in dichloromethane (2mL). The mixture was reacted at room temperature for 6 hours. TLC detection showed the reaction was completed. A saturated sodium bicarbonate solution was dropwise added to quench the reaction and the pH was adjusted to 9-10. The reaction mixture was extracted with dichloromethane (50mL) and separated into phases. The organic phase was concentrated under a reduced pressure, and separated and purified by Prep-HPLC (C18, 10mmol/L aqueous sodium bicarbonate solution, acetonitrile) to produce the target compound (30mg). LCMS(ESI)[M+H]⁺=387.1; ¹H NMR(400 MHz, DMSO-*d*₆)δ 8.24(d, J=8.4Hz, 2H), 7.99(s, 1H), 7.65(d, J=8.4Hz, 2H), 4.93(t, J=5.6Hz, 1H), 3.82(d, J=4.7Hz, 2H), 3.69-3.57(m, 1H), 3.41-3.35(m, 1H), 3.21(dd, J=16.4, 7.4Hz, 1H), 3.02(dd, J=13.6, 5.8Hz, 1H), 2.44-2.37(m, 1H), 2.36-2.25(m, 3H), 2.08(s, 2H), 1.89-1.77(m, 2H), 1.40(s, 6H).

### Example 42 (Compound 76)

### Synthesis of (R/S)-2-(4-(4-((1-(hydroxymethyl)cyclobutyl)amino)-5-oxido-6,7-dihydrothieno[3,2-d]pyrimidin-2-yl)phenyl)-2-methylpropanoic acid:

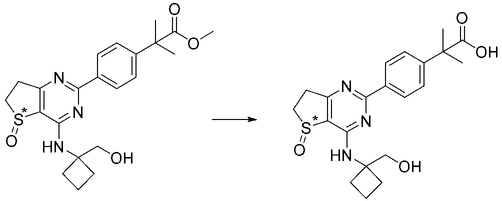

A concentrated hydrochloric acid (0.5mL, 37wt%, 30eq) was added to a solution of methyl (R/S)-2-(4-(4-((1-(hydroxymethyl)cyclobutyl)amino)-5-oxido-6,7-dihydrothieno[3,2-d]pyrimidin-2-yl)phenyl)-2-methylpropanoate (50mg, 1eq) in acetic acid (1mL). The mixture was heated to 75°C and reacted for 6 hours. LCMS detection showed the reaction was completed. The reaction mixture was quenched with concentrated aqueous ammonia, adjusted to the pH of 6-7, and then separated and purified by Prep-HPLC (C18, 10mmol/L aqueous ammonium bicarbonate solution, acetonitrile) to produce the target compound (10mg). LCMS(ESI)[M+H]⁺=416.2; ¹H NMR(400 MHz, DMSO-d₆)δ 12.14(s, 1H), 8.27(d, J=8.4Hz, 2H), 8.03(s, 1H), 7.47(d, J=8.4Hz, 2H), 4.93(s, 1H), 3.81(s, 2H), 3.68-3.58(m, 1H), 3.39(d, J=8.2Hz, 1H), 3.21(dd, J=16.7, 7.5Hz, 1H), 3.02(dd, J=13.6, 5.8Hz, 1H), 2.41(d, J=11.7Hz, 1H), 2.34(dd, J=13.1, 6.2Hz, 3H), 1.90-1.77(m, 2H), 1.51(s, 6H).

### Example 43 (Compound 77)

### Synthesis of (R/S)-2-(4-(4-((1-(acetoxymethyl)cyclobutyl)amino)-5-oxido-6,7-dihydrothieno[3,2-d]pyrimidin-2-yl)phenyl)-2-methylpropanoic acid:

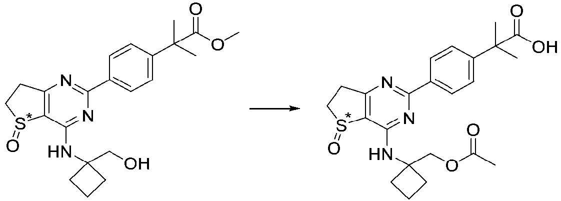

A concentrated hydrochloric acid (0.5mL, 37wt%, 30eq) was added to a solution of methyl (R/S)-2-(4-(4-((1-(hydroxymethyl)cyclobutyl)amino)-5-oxido-6,7-dihydrothieno[3,2-d]pyrimidin-2-yl)phenyl)-2-methylpropanoate (50mg, 1eq) in acetic acid (1mL). The mixture was heated to 75°C and reacted for 6 hours. LCMS detection showed the reaction was completed. The reaction mixture was quenched with concentrated aqueous ammonia, adjusted to the pH of 6-7, and then separated and purified by Prep-HPLC (C18, 10mmol/L aqueous ammonium bicarbonate solution, acetonitrile) to produce the target compound (10mg). LCMS(ESI)[M+H]⁺=458.2; ¹H NMR(400 MHz, DMSO-d₆)δ 12.42(s, 1H), 8.40(s, 1H), 8.28(d, J=8.4Hz, 2H), 7.48(d, J=8.4Hz, 2H), 4.56(q, J=11.1Hz, 2H), 3.71-3.58(m, 1H), 3.42-3.37(m, 1H), 3.23(dd, J=16.8, 7.8Hz, 1H), 3.02(dd, J=13.7, 6.1Hz, 1H), 2.46-2.40(m, 1H), 2.36(t, J=7.6Hz, 3H), 2.00(s, 3H), 1.94-1.82(m, 2H), 1.51(s, 6H).

### Example 44 (Compound 78)

### Synthesis of (R/S,E)-2-(4-chlorostyryl)-4-((1-(hydroxymethyl)cyclobutyl)amino)-6,7-dihydrothieno[3,2-d]pyrimidine 5-oxide:

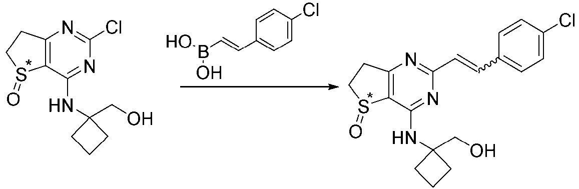

Under the protection of nitrogen gas, [1,1'-bis(diphenylphosphino)ferrocene]dichloropalladium (0.1eq) was added to a mixture of (R/S)-2-chloro-4-((1-(hydroxymethyl)cyclobutyl)amino)-6,7-dihydrothieno[3,2-d]pyrimidine 5-oxide (50mg, 1eq), (E)-(4-chlorostyryl)boronic acid (1.2eq) and potassium carbonate (2.5eq) in dioxane (2mL) and water (0.4mL). The mixture was heated to 110°C and reacted for 2 hours. TLC detection showed the reaction was completed. The reaction mixture was diluted with water (20mL), extracted with ethyl acetate (50mL), and separated into phases. The organic phase was concentrated, and then separated and purified by Prep-HPLC (C18, 10mmol/L aqueous formic acid solution, acetonitrile) to produce the target compound (30mg). LCMS(ESI)[M+H]⁺=390.1; ¹H NMR(400 MHz, DMSO-d₆)δ 7.92(s, 1H), 7.82(d, J=8.7Hz, 1H), 7.78-7.72(m, 2H), 7.47(d, J=8.5Hz, 2H), 7.06(d, J=16.0Hz, 1H), 4.90(t, J=5.7Hz, 1H), 3.79(d, J=5.7Hz, 2H), 3.58(dt, J=15.9, 7.8Hz, 1H), 3.37(d, J=8.3Hz, 1H), 3.15(dd, J=16.3, 7.3Hz, 1H), 3.00(dd, J=13.9, 5.7Hz, 1H), 2.39(d, J=11.1Hz, 1H), 2.36-2.23(m, 3H), 1.90-1.75(m, 2H).

### Example 45 (Compound 79)

### Synthesis of (R/S)-2-(3-(4-chloro-3-methoxyphenyl)-3-methoxyazetidin-1-yl)-4-((1-(hydroxymethyl)cyclobutyl)amino)-6,7-dihydrothieno[3,2-d]pyrimidine 5-oxide:

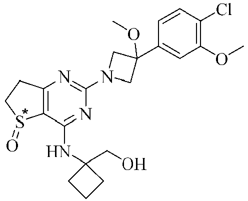

### Step 1: Preparation of tert-butyl 3-(4-chloro-3-methoxyphenyl)-3-methoxyazetidine-1-carboxylate:

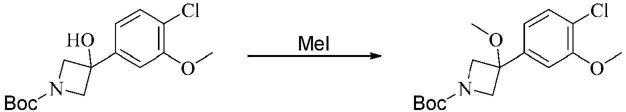

Tert-butyl 3-(4-chloro-3-methoxyphenyl)-3-hydroxyazetidine-1-carboxylate (500mg, 1eq) was dissolved in N,N-dimethylformamide (5mL). The atmosphere was replaced with nitrogen gas three times. 60%NaH (2.05eq) was added under an ice-water bath, and the mixture was stirred under an ice bath for 10 minutes. Iodomethane (5eq) was added, and the mixture was continuously stirred under the ice bath for 30 minutes. LCMS detection showed that the starting material was completely consumed. A saturated NH₄Cl solution was added to the reaction mixture to quench the reaction. The mixture was added to water (30mL). The resulting mixture was extracted with ethyl acetate (20mL×2). The organic phases were combined, then washed with a saturated saline solution (10mL), dried over anhydrous sodium sulfate, and then concentrated under a reduced pressure to produce a crude product. The crude product was purified by silica gel column chromatography (petroleum ether:ethyl acetate=10:1) to produce the target compound (417mg).

### Step 2: Preparation of 3-(4-chloro-3-methoxyphenyl)-3-methoxyazetidine trifluoroacetate:

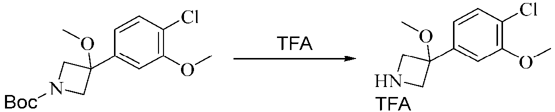

Tert-butyl 3-(4-chloro-3-methoxyphenyl)-3-methoxyazetidine-1-carboxylate (1eq) was dissolved in dichloromethane (10mL). Trifluoroacetic acid (2mL) was added under an ice bath. The mixture was stirred at room temperature for 0.5 hours. LCMS detection showed that the starting material was completely consumed. The reaction mixture was concentrated under a reduced pressure to produce the target compound (800mg, crude, directly used in the next step). LCMS(ESI)[M+H]⁺=228.1.

### Step 3: Preparation of (R/S)-2-(3-(4-chloro-3-methoxyphenyl)-3-methoxyazetidin-1-yl)-4-((1-(hydroxymethyl)cyclobutyl)amino)-6,7-dihydrothieno[3,2-d]pyrimidine 5-oxide:

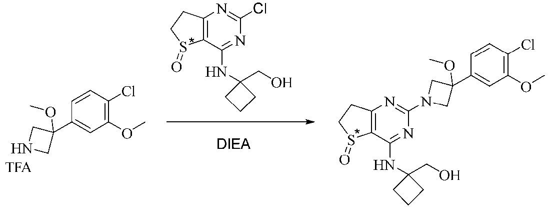

(R/S)-2-chloro-4-((1-(hydroxymethyl)cyclobutyl)amino)-6,7-dihydrothieno[3,2-d]pyrimidine 5-oxide (150mg, crude, obtained from the previous step), 3-(4-chloro-3-methoxyphenyl)-3-methoxyazetidine trifluoroacetate (231mg), and N,N-diisopropylethylamine (538mg) were dissolved in dioxane (5mL). The mixture was heated to 100°C and stirred for 1 hour. LCMS detection showed that the starting material was completely consumed. The reaction mixture was added to water (50mL). The resulting mixture was filtered. The filtrate was separated and purified by Prep-HPLC (C18, 10mmol/L aqueous ammonium bicarbonate solution, acetonitrile) to produce the target compound (110mg). LCMS(ESI)[M+H]⁺=479.3; ¹H NMR(400 MHz, DMSO-*d*₆)δ 7.53(s, 1H), 7.47(d, *J*=8.2Hz, 1H), 7.14(d, *J*=2.0Hz, 1H), 7.05(dd, *J*=8.1, 1.9Hz, 1H), 4.85(t, *J*=5.6Hz, 1H), 4.21(s, 4H), 3.90(s, 3H), 3.72(dd, *J*=5.8, 2.4Hz, 2H), 3.42(dt, *J*=16.9, 8.3Hz, 4H), 3.21(dt, *J*=13.6, 8.4Hz, 1H), 3.00-2.83(m, 2H), 2.37-2.22(m, 2H), 2.20-2.10(m, 2H), 1.75(dq, *J*=19.4, 9.9Hz, 2H).

### Example 46 (Compound 80)

### Synthesis of (R/S)-2-(3-(4-chloro-3-fluorophenyl)-3-hydroxyazetidin-1-yl)-4-((1-(hydroxymethyl)cyclobutyl)amino)-6,7-dihydrothieno[3,2-d]pyrimidine 5-oxide:

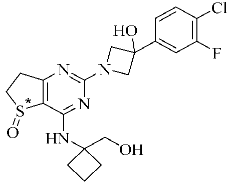

### Step 1: Preparation of 4-chloro-3-fluorophenylmagnesium bromide:

Under the protection of nitrogen gas, magnesium chips whose oxide layer had been removed (696mg, 1.2eq), elemental iodine (0.01eq), and 4-bromo-1-chloro-2-fluorobenzene (0.6eq) were added to anhydrous THF (3mL). The mixture was heated until the brown color disappeared, and then a solution of 4-bromo-1-chloro-2-fluorobenzene (0.4eq) in anhydrous THF (17mL) was slowly continuously added. The mixture was reacted at room temperature for 2 hours. Magnesium chips were greatly consumed to produce the target compound (crude), which was directly used in the next step.

### Step 2: Preparation of tert-butyl 3-(4-chloro-3-fluorophenyl)-3-hydroxyazetidine-1-carboxylate:

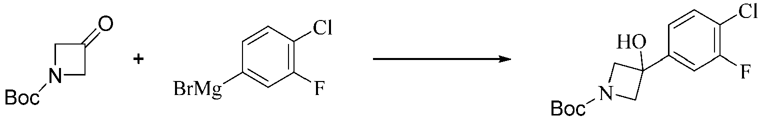

Under the protection of nitrogen gas, tert-butyl 3-oxoazetidine-1-carboxylate (3.00g, 1eq) was added to anhydrous THF(20mL). At 0°C, the reaction mixture (5.32g) obtained from the previous step was slowly added, and the resulting mixture was stirred at 0°C for 1 hour. TLC detection showed a new product was formed. A saturated ammonium chloride solution was added to the reaction mixture to quench the reaction. Then water (150mL) was added. The resulting mixture was extracted with MTBE (100mL×2). The organic phases were combined, washed with a saturated sodium chloride solution (100mL), dried over anhydrous sodium sulfate, and concentrated under a reduced pressure to produce a crude product. The crude product was purified by silica gel column chromatography (petroleum ether:ethyl acetate=5:1) to produce the target compound (4.70g). LCMS(ESI)[M+H-56]⁺=246.1.

### Step 3: Preparation of 3-(4-chloro-3-fluorophenyl)-3-hydroxyazetidine trifluoroacetate:

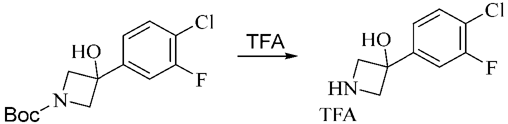

Tert-butyl 3-(4-chloro-3-fluorophenyl)-3-hydroxyazetidine-1-carboxylate (200mg, 1eq) was dissolved in dichloromethane (1mL). Trifluoroacetic acid (1mL) was added. The mixture was stirred at room temperature for 30 minutes. LCMS detection showed the raw material disappeared. The reaction mixture was concentrated under a reduced pressure to produce the target compound (240mg, crude, directly used in the next step). LCMS(ESI)[M+H]⁺=202.1.

### Step 4: Preparation of (R/S)-2-(3-(4-chloro-3-fluorophenyl)-3-hydroxyazetidin-1-yl)-4-((1-(hydroxymethyl)cyclobutyl)amino)-6,7-dihydrothieno[3,2-d]pyrimidine 5-oxide:

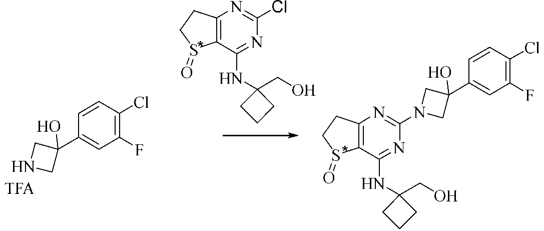

(R/S)-2-chloro-4-((1-(hydroxymethyl)cyclobutyl)amino)-6,7-dihydrothieno[3,2-d]pyrimidine 5-oxide (100mg, 1eq), 3-(4-chloro-3-fluorophenyl)-3-hydroxyazetidine trifluoroacetate (160mg, crude, obtained from the previous step), and N,N-diisopropylethylamine (4eq) were dissolved in a mixed solution of tetrahydrofuran (2.5mL) and water (0.5mL). The resulting mixture was heated to 70°C and stirred for 2 hours. LCMS detection showed that the starting material was completely consumed. The reaction mixture was added to water (20mL). The resulting mixture was filtered, and separated and purified by Prep-HPLC (C18, 10mmol/L aqueous ammonium bicarbonate solution, acetonitrile) to produce the target compound (100mg). LCMS(ESI)[M+H]⁺=453.2; ¹H NMR(400 MHz, DMSO-*d*₆)δ 7.61(t, *J*=8.1Hz, 1H), 7.55-7.47(m, 2H), 7.38(dd, *J*=8.5, 2.1Hz, 1H), 6.64(s, 1H), 4.87(t, *J*=5.7Hz, 1H), 4.18(s, 4H), 3.70(dd, *J*=5.9, 1.5Hz, 2H), 3.42(d, *J*=8.7Hz, 1H), 3.27-3.17(m, 1H), 3.01-2.82(m, 2H), 2.42-2.24(m, 2H), 2.20-2.05(m, 2H), 1.87-1.64(m, 2H).

### Example 47 (Compound 81)

### Synthesis of (R/S)-2-(3-(4-chloro-3-methoxyphenyl)-3-hydroxyazetidin-1-yl)-4-((tetrahydro-2H-pyran-4-yl)amino)-6,7-dihydrothieno[3,2-d]pyrimidine 5-oxide:

### Step 1: Preparation of 2-chloro-N-(tetrahydro-2H-pyran-4-yl)-6,7-dihydrothieno[3,2-d]pyrimidin-4-amine:

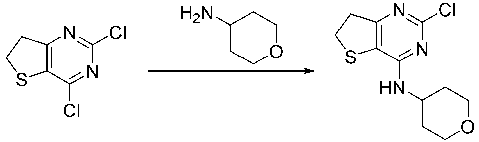

2,4-Dichloro-6,7-dihydrothieno[3,2-d]pyrimidine (5.00g, 1eq), 4-aminotetrahydropyran (1.1eq), and N,N-diisopropylethylamine (3eq) were dissolved in acetonitrile (50mL), and the mixture was stirred at 85°C for 4 hours. LCMS detection showed that the starting material was completely consumed. The reaction mixture was concentrated under a reduced pressure to produce a solid. The solid was slurrized with MTBE (50mL). The resulting mixture was filtered. The filter cake was washed with MTBE (20mL) and then oven-dried to produce the target compound (5.43g). LCMS(ESI)[M+H]⁺=272.1.

### Step 2: Preparation of (R/S)-2-chloro-4-((tetrahydro-2H-pyran-4-yl)amino)-6,7-dihydrothieno[3,2-d]pyrimidine 5-oxide:

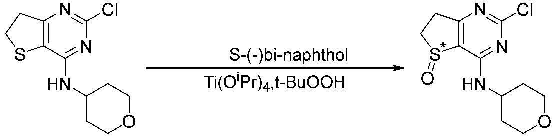

2-Chloro-N-(tetrahydro-2H-pyran-4-yl)-6,7-dihydrothieno[3,2-d]pyrimidin-4-amine (0.50g, 1eq), S-(-)bi-naphthol (0.1eq), and Ti(OⁱPr)₄ (0.05eq) were added to DCM (5mL) and water (0.5mL). The atmosphere was replaced with nitrogen gas three times. The mixture was stirred at room temperature for 1 hour, and 70% tert-butyl hydroperoxide (1.1eq) was added under an ice bath. The mixture was stirred at room temperature for 3 hours. LCMS detection showed a small amount of the raw material remained. Water (20mL) was added under an ice bath to quench the reaction. The mixture was extracted with DCM (20mL×2). The organic phases were combined, then successively washed with water (10mL) and a saturated saline solution (10mL), dried over anhydrous sodium sulfate, and then concentrated under a reduced pressure to produce a crude product. The crude product was purified with silica gel column chromatography (petroleum ether:ethyl acetate=3:1) to produce the target compound (187mg). LCMS(ESI)[M+H]⁺=288.1;

### Step 3: Preparation of (R/S)-2-(3-(4-chloro-3-methoxyphenyl)-3-hydroxyazetidin-1-yl)-4-((tetrahydro-2H-pyran-4-yl)amino)-6,7-dihydrothieno[3,2-d]pyrimidine 5-oxide:

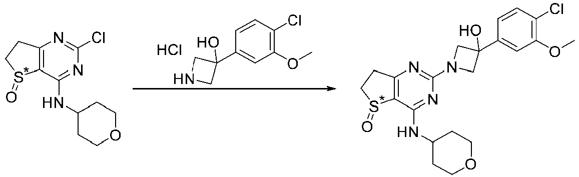

(R/S)-2-chloro-4-((tetrahydro-2H-pyran-4-yl)amino)-6,7-dihydrothieno[3,2-d]pyrimidine 5-oxide (50mg, 1eq), 3-(4-chloro-3-methoxyphenyl)-3-hydroxyazetidine hydrochloride (1.3eq), and N,N-diisopropylethylamine (5eq) were dissolved in a mixed solution of THF(1.5mL) and water (0.3mL). The mixture was heated to 70°C and stirred for 1 hour. LCMS detection showed that the starting material was completely consumed. The reaction mixture was added to water (20mL). The mixture was extracted with ethyl acetate (10mLx3). The organic phases were combined, then washed with water (10mL) and a saturated saline solution (10mL×2), dried over anhydrous sodium sulfate, and then concentrated under a reduced pressure to produce a crude product. The crude product was separated and purified by Prep-HPLC (C18, 10mmol/L aqueous ammonium bicarbonate solution, acetonitrile) to produce the target compound (55mg). LCMS(ESI) [M+H]⁺=465.2; ¹H NMR(400 MHz, DMSO-*d*₆)δ 7.68(d, *J*=7.5Hz, 1H), 7.43(d, *J*=8.3Hz, 1H), 7.25(d, *J*=2.0Hz, 1H), 7.09(d, *J*=8.2Hz, 1H), 6.50(s, 1H), 4.22(t, *J*=13.5Hz, 5H), 3.87(s, 4H), 3.43(dd, *J*=17.1, 8.5Hz, 1H), 3.30-3.11(m, 4H), 2.94(ddd, *J*=26.1, 15.5, 7.6Hz, 2H), 1.69(d, *J*=60.2Hz, 4H).

### Example 48 (Compound 82)

### Synthesis of (R/S)-2-(3-(4-chloro-3-fluorophenyl)-3-methoxyazetidin-1-yl)-4-((1-(hydroxymethyl)cyclobutyl)amino)-6,7-dihydrothieno[3,2-d]pyrimidine 5-oxide:

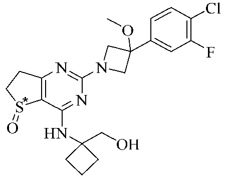

### Step 1: Preparation of tert-butyl 3-(4-chloro-3-fluorophenyl)-3-methoxyazetidine-1-carboxylate:

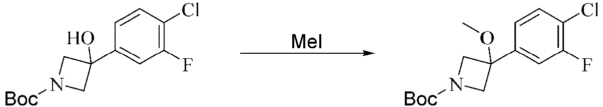

Tert-butyl 3-(4-chloro-3-fluorophenyl)-3-hydroxyazetidine-1-carboxylate (300mg, 1eq) was dissolved in DMF (3mL). The atmosphere was replaced with nitrogen gas three times. 60wt% NaH (2eq) was added under an ice bath. The mixture was stirred under the ice bath for 5 minutes. Iodomethane (2eq) was added. The resulting mixture was continuously stirred for 4 hours. LCMS detection showed that the starting material was completely consumed. The reaction mixture was quenched with a saturated aqueous ammonium chloride solution under an ice bath. Then the mixture was added to water (30mL), and extracted with MTBE (20mL×2). The organic phases were combined, then washed with water (10mL) and a saturated saline solution (10mL×2), dried over anhydrous sodium sulfate, and then concentrated under a reduced pressure to produce a crude product. The crude product was purified by silica gel column chromatography (petroleum ether:ethyl acetate=10:1) to produce the target compound (260mg).

### Step 2: Preparation of 3-(4-chloro-3-fluorophenyl)-3-methoxyazetidine trifluoroacetate:

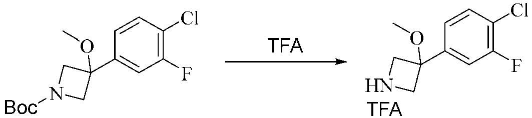

Tert-butyl 3-(4-chloro-3-fluorophenyl)-3-methoxyazetidine-1-carboxylate (260mg, 1eq) was added to a mixed solution of TFA (2mL) and DCM (3mL). The mixture was stirred at room temperature for 0.5 hours. LCMS detection showed that the starting material was completely consumed. The reaction mixture was concentrated under a reduced pressure to produce 3-(4-chloro-3-fluorophenyl)-3-methoxyazetidine trifluoroacetate (350mg, crude, directly used in the next step). LCMS(ESI)[M+H]⁺=216.1.

### Step 4: Preparation of (R/S)-2-(3-(4-chloro-3-fluorophenyl)-3-methoxyazetidin-1-yl)-4-((1-(hydroxymethyl)cyclobutyl)amino)-6,7-dihydrothieno[3,2-d]pyrimidine 5-oxide:

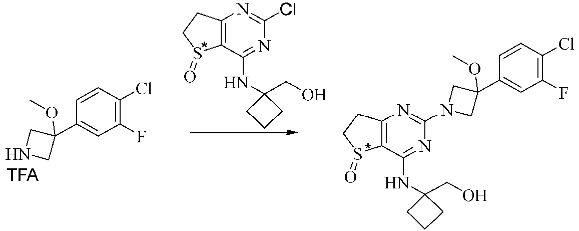

(R/S)-2-chloro-4-((1-(hydroxymethyl)cyclobutyl)amino)-6,7-dihydrothieno[3,2-d]pyrimidine 5-oxide (200mg, 1eq), 3-(4-chloro-3-fluorophenyl)-3-methoxyazetidine trifluoroacetate (343mg, crude, obtained from the previous step), and N,N-diisopropylethylamine (5eq) were added to a mixed solution of water (1mL) and THF(5mL). The reaction mixture was stirred at 70°C for 2 hours. LCMS detection showed that the starting material was completely consumed. The reaction mixture was added to water (20mL), filtered, and separated and purified by Prep-HPLC (C18, 0.1% aqueous formic acid solution, acetonitrile) to produce the target compound (209mg, yield 64%). LCMS(ESI)[M+H]⁺=467.3; ¹H NMR(400 MHz, DMSO-*d*₆)δ 7.66(t, *J*=8.0Hz, 1H), 7.58-7.48(m, 2H), 7.35(dd, *J*=8.3, 2.1Hz, 1H), 4.84(t, *J*=5.6Hz, 1H), 4.20(d, *J*=8.9Hz, 4H), 3.76-3.64(m, 2H), 3.43(dd, *J*=17.0, 8.3Hz, 1H), 3.22(dt, *J*=13.5, 8.3Hz, 1H), 3.06(s, 3H), 2.98-2.84(m, 2H), 2.30(dt, *J*=21.0, 10.3Hz, 2H), 2.19-2.10(m, 2H), 1.85-1.65(m, 2H).

### Example 49 (Compound 83)

### Synthesis of (R/S)-2-(3-(4-chlorophenyl)-3-methoxyazetidin-1-yl)-4-((1-(hydroxymethyl)cyclobutyl)amino)-6,7-dihydrothieno[3,2-d]pyrimidine 5-oxide:

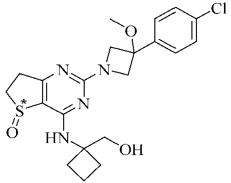

### Step 1: Preparation of (4-chlorophenyl)lithium:

Under the protection of nitrogen gas, 1-chloro-4-iodobenzene (4.18g, 1eq) was dissolved in anhydrous THF (50mL). The mixture was stirred until the solution became clear. At -78°C, a 2.5M solution of n-butyl lithium in hexane (1eq) was added dropwise. The reaction mixture was continuously stirred at -78°C for 0.5 hours to produce the target compound (crude), which was directly used in the next step.

### Step 2: Preparation of tert-butyl 3-(4-chlorophenyl)-3-hydroxyazetidine-1-carboxylate:

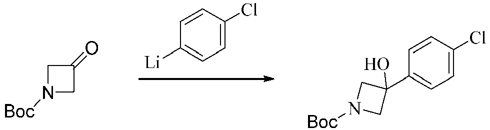

Under the protection of nitrogen gas, tert-butyl 3-oxoazetidine-1-carboxylate (2g, 1eq) was dissolved in anhydrous THF(10mL), and slowly added to the reaction mixture obtained from the previous step at -78°C. The resulting mixture was stirred at -78°C for 1 hour. TLC detection showed the reaction was completed. At 0°C, the reaction mixture was quenched with a saturated ammonium chloride solution (10mL) and purified to produce a crude product. The crude product was added to petroleum ether (30mL), stirred at room temperature for 1 hour, and filtered. The filter cake was washed with petroleum ether (20mL) to produce the target compound (2.56g). LCMS(ESI)[M+H-56]⁺=228.1.

### Step 3: Preparation of tert-butyl 3-(4-chlorophenyl)-3-methoxyazetidine-1-carboxylate:

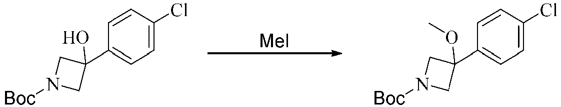

Under the protection of nitrogen gas, tert-butyl 3-(4-chlorophenyl)-3-hydroxyazetidine-1-carboxylate (500mg, 1eq) was dissolved in anhydrous DMF(5mL). 60wt% NaH (2.05eq) was added under an ice-water bath. The mixture was stirred for 10 minutes. Iodomethane (5eq) was added, and then the resulting mixture was continuously stirred for 30 minutes. LCMS detection showed that the starting material was completely consumed. The reaction mixture was quenched with a saturated NH₄Cl aqueous solution. Then the mixture was added to water (30mL), and extracted with methyl tert-butyl ether (20mL×2). The organic phases were combined, then successively washed with water (20mL) and a saturated saline solution (20mL×2), dried over anhydrous sodium sulfate, and concentrated under a reduced pressure to produce the target compound (585mg, crude, directly used in the next step).

### Step 4: Preparation of 3-(4-chlorophenyl)-3-methoxyazetidine hydrochloride:

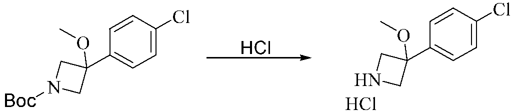

Tert-butyl 3-(4-chlorophenyl)-3-methoxyazetidine-1-carboxylate (585mg, crude, obtained from the previous step) was added to a 4M solution of hydrogen chloride in ethyl acetate (5mL). The mixture was stirred at room temperature for 0.5 hours. LCMS detection showed that the starting material was completely consumed. The reaction mixture was concentrated under a reduced pressure, and the residue was diluted with methyl tert-butyl ether (20mL). The resulting mixture was stirred at room temperature for 30 minutes, and filtered. The filter cake was washed with methyl tert-butyl ether (10mL) to produce the target compound (380mg). LCMS(ESI)[M+H]⁺=198.1.

### Step 5: Preparation of (R/S)-2-(3-(4-chlorophenyl)-3-methoxyazetidin-1-yl)-4-((1-(hydroxymethyl)cyclobutyl)amino)-6,7-dihydrothieno[3,2-d]pyrimidine 5-oxide:

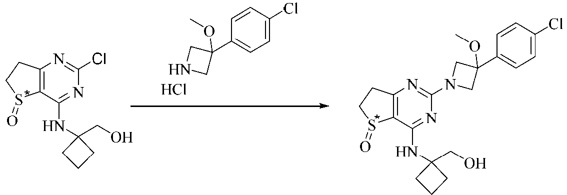

(R/S)-2-chloro-4-((1-(hydroxymethyl)cyclobutyl)amino)-6,7-dihydrothieno[3,2-d]pyrimidine 5-oxide (100mg, 1eq), 3-(4-chlorophenyl)-3-methoxyazetidine hydrochloride (1.22eq), and N,N-diisopropylethylamine (3.98eq) were added to a mixed solution of water (0.5mL) and THF(2.5mL). The reaction mixture was stirred at 70°C for 2 hours. LCMS detection showed that the starting material was completely consumed. The reaction mixture was added to water (20mL), filtered, and separated and purified by Prep-HPLC (C18, 10mmol/L aqueous ammonium bicarbonate solution, acetonitrile) to produce the target compound (82mg, yield 52%). LCMS(ESI)[M+H]⁺=449.3; ¹H NMR(400 MHz, DMSO-d₆)δ 7.49(s, 5H), 4.84(t, *J*=5.7Hz, 1H), 4.22(s, 3H), 3.77-3.65(m, 2H), 3.41(dt, *J*=16.6, 8.0Hz, 2H), 3.27-3.15(m, 2H), 2.99-2.82(m, 3H), 2.34(d, *J*=10.5Hz, 2H), 2.26(d, *J*=10.0Hz, 1H), 2.14(t, *J*=10.2Hz, 2H), 1.74(dq, *J*=19.6, 10.3Hz, 2H).

### Example 50 (Compound 84)

### Synthesis of (R/S)-2-(5-(2-hydroxypropan-2-yl)isoindolin-2-yl)-4-((tetrahydro-2H-pyran-4-yl)amino)-6,7-dihydrothieno[3,2-d]pyrimidine 5-oxide:

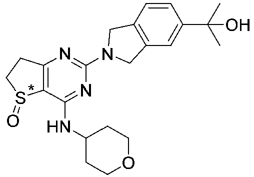

### Step 1: Preparation of methyl (R/S)-2-(5-oxido-4-((tetrahydro-2H-pyran-4-yl)amino)-6,7-dihydrothieno[3,2-d]pyrimidin-2-yl)isoindoline-5-carboxylate:

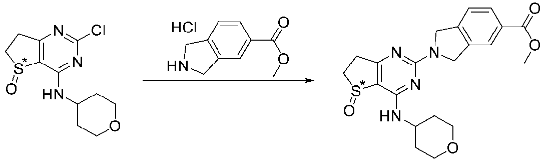

(R/S)-2-chloro-4-((tetrahydro-2H-pyran-4-yl)amino)-6,7-dihydrothieno[3,2-d]pyrimidine 5-oxide (130mg, 1eq), methyl isoindoline-5-carboxylate hydrochloride (1.2eq), and N,N-diisopropylethylamine (5eq) were dissolved in water (0.3mL) and THF(1.5mL). The mixture was heated to 70°C and stirred for 1 hour. LCMS detection showed that the starting material was completely consumed. The reaction mixture was added to water (20mL), and filtered. The filter cake was washed with water (20mL), collected, diluted with ethyl acetate (30mL), and concentrated under a reduced pressure to produce the target compound (170mg). LCMS(ESI)[M+H]⁺=429.3.

### Step 2: Preparation of (R/S)-2-(5-(2-hydroxypropan-2-yl)isoindolin-2-yl)-4-((tetrahydro-2H-pyran-4-yl)amino)-6,7-dihydrothieno[3,2-d]pyrimidine 5-oxide:

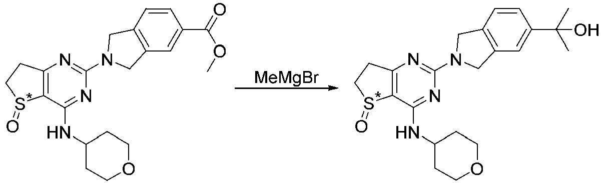

Under the protection of nitrogen gas, methyl (R/S)-2-(5-oxido-4-((tetrahydro-2H-pyran-4-yl)amino)-6,7-dihydrothieno[3,2-d]pyrimidin-2-yl)isoindoline-5-carboxylate (120mg, 1eq) was dissolved in THF(2mL). The mixture was stirred under an ice bath for 10 minutes. A 3M solution of methyl magnesium bromide/2-methyltetrahydrofuran (10.7eq) was added dropwise. The resulting mixture was continuously stirred for 1 hour. LCMS detection showed the reaction was completed. The reaction mixture was quenched with a saturated aqueous ammonium chloride solution under an ice bath. The mixture was added to water (30mL), and extracted with MTBE (20mL×2). The organic phases were combined, washed with a saturated saline solution (20mL), dried over anhydrous sodium sulfate, and then concentrated under a reduced pressure to produce a crude product. The crude product was separated and purified by Pre-HPLC (C18, 10mmol/L aqueous ammonium bicarbonate solution, acetonitrile) to produce the target compound (27mg). LCMS(ESI)[M+H]⁺=429.3; ¹H NMR(400 MHz, DMSO-*d*₆*)*δ 7.61(t, *J*=6.3Hz, 1H), 7.50(s, 1H), 7.40(t, *J*=8.3Hz, 1H), 7.31(dd, *J*=12.9, 8.0Hz, 1H), 5.04(d, *J*=5.1Hz, 1H), 4.81(d, *J*=8.2Hz, 3H), 4.30(s, 1H), 3.92(s, 2H), 3.44(q, *J*=14.4Hz, 3H), 3.23(dt, *J*=13.4, 8.5Hz, 2H), 3.03(d, *J*=7.2Hz, 1H), 2.90(dd, *J*=13.6, 7.1Hz, 1H), 1.84(d, *J*=12.9Hz, 2H), 1.64(dd, *J*=17.6, 9.3Hz, 2H), 1.44(s, 6H).

### Example 51 (Compound 85)

### Synthesis of (R/S)-2-(5-chloroisoindolin-2-yl)-4-((tetrahydro-2H-pyran-4-yl)amino)-6,7-dihydrothieno[3,2-d]pyrimidine 5-oxide:

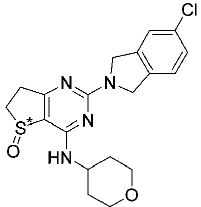

### Step 1: Preparation of (R/S)-2-chloro-4-((tetrahydro-2H-pyran-4-yl)amino)-6,7-dihydrothieno[3,2-d]pyrimidine 5-oxide:

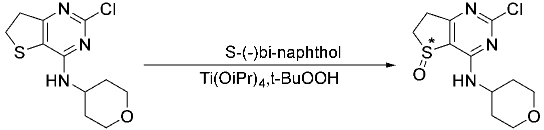

S-(-)bi-naphthol (0.2eq) was dissolved in DCM (10mL). Ti(OⁱPr)₄(0.2eq) was added. The mixture was reacted at 25°C under the protection of nitrogen gas for 1 hour. Water (0.14mL) was added. The mixture was continuously reacted for 0.5 hours. Then 2-chloro-N-(tetrahydro-2H-pyran-4-yl)-6,7-dihydrothieno[3,2-d]pyrimidin-4-amine (2.00g, 1eq) was added, and the resulting mixture was reacted for 1 hour. 70wt% tert-butyl hydroperoxide (1.1eq) was added. LCMS detection showed the reaction was completed. The reaction mixture was added to water (30mL), and extracted with DCM:MeOH=10:1 (20mL×2). The organic phases were combined, washed with water (20mL) and a saturated saline solution (20mL×2), dried over anhydrous sodium sulfate, and concentrated under a reduced pressure to produce the target compound (1.50g). LCMS(ESI)[M+H]⁺=288.1.

### Step 2: Preparation of (R/S)-2-(5-chloroisoindolin-2-yl)-4-((tetrahydro-2H-pyran-4-yl)amino)-6,7-dihydrothieno[3,2-d]pyrimidine 5-oxide:

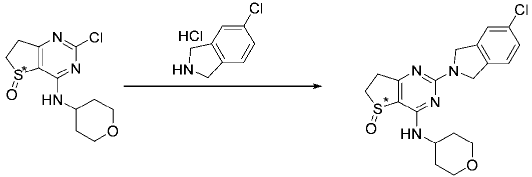

(R/S)-2-chloro-4-((tetrahydro-2H-pyran-4-yl)amino)-6,7-dihydrothieno[3,2-d]pyrimidine 5-oxide (100mg, 1eq) and 5-chloroisoindoline hydrochloride (0.91eq) were added to a mixed solution of THF(2mL) and H₂O (0.4mL). N,N-diisopropylethylamine (4.95eq) was added. The mixture was stirred at 70°C for 1 hour. LCMS detection showed a product was formed. The reaction mixture was added to water (30mL), and extracted with DCM:MeOH=10:1 (20mL×2). The organic phases were combined, then washed with water (20mL) and a saturated saline solution (20mL), dried over anhydrous sodium sulfate, and concentrated under a reduced pressure to produce a crude product. The crude product was separated and purified by Prep-HPLC (C18, 10mmol/L aqueous ammonium bicarbonate solution, acetonitrile) to produce the target compound (60mg). LCMS(ESI)[M+H]⁺=405.3; ¹H NMR(400 MHz, DMSO-*d*₆)δ 7.64(d, *J*=7.6Hz, 1H), 7.55-7.31(m, 3H), 4.81(d, *J*=7.9Hz, 4H), 4.29(s, 1H), 3.91(s, 2H), 3.53-3.35(m, 3H), 3.24(dt, *J*=13.4, 8.5Hz, 1H), 3.00(dd, *J*=17.0, 8.1Hz, 1H), 2.91(dd, *J*=13.4, 7.1Hz, 1H), 1.83(d, *J*=12.6Hz, 2H), 1.74-1.57(m, 2H).

### Example 52 (Compound 86)

### Synthesis of (R/S)-2-(3-(4-chloro-3-fluorophenyl)-3-methoxyazetidin-1-yl)-4-((tetrahydro-2H-pyran-4-yl)amino)-6,7-dihydrothieno[3,2-d]pyrimidine 5-oxide:

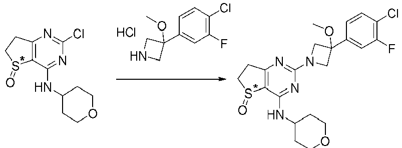

(R/S)-2-chloro-4-((tetrahydro-2H-pyran-4-yl)amino)-6,7-dihydrothieno[3,2-d]pyrimidine 5-oxide (150mg) and 3-(4-chloro-3-fluorophenyl)-3-methoxyazetidine hydrochloride (150mg) were added to a mixed solution of tetrahydrofuran (2.5mL) and H₂O (0.5mL). N,N-diisopropylethylamine (336mg) was added. The mixture was stirred at 70°C for 1 hour. The reaction mixture was added to water (20mL), and extracted with DCM:MeOH=10:1(10mL). The organic phases were combined, dried over anhydrous sodium sulfate, and then concentrated under a reduced pressure to produce a crude product. The crude product was separated and purified by Prep-HPLC (C18, 10mmol/L NH₄HCO₃/water, MeCN) to produce the target compound (70mg). LCMS(ESI)[M+H]⁺=467.3; ¹H NMR(400 MHz, DMSO-*d*₆)δ 7.71-7.62(m, 2H), 7.55(dd, *J*=10.4, 2.1Hz, 1H), 7.35(dd, *J*=8.4, 2.1Hz, 1H), 4.26(q, *J*=10.0Hz, 5H), 3.89(d, *J*=8.8Hz, 2H), 3.42(dq, *J*=15.1, 7.3Hz, 2H), 3.22(dt, *J*=13.7, 8.4Hz, 2H), 3.07(s, 3H), 2.93(ddd, *J*=21.7, 15.4, 7.7Hz, 2H), 1.77(d, *J*=12.6Hz, 2H), 1.61(qt, *J*=11.6, 5.8Hz, 2H).

### Example 53 (Compound 87)

### Synthesis of (R/S)-2-(5-(difluoromethoxy)isoindolin-2-yl)-4-((1-(hydroxymethyl)cyclobutyl)amino)-6,7-dihydrothieno[3,2-d]pyrimidine 5-oxide:

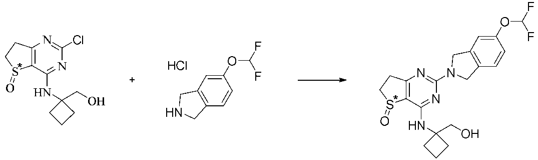

(R/S)-2-chloro-4-((1-(hydroxymethyl)cyclobutyl)amino)-6,7-dihydrothieno[3,2-d]pyrimidine 5-oxide (100mg), 5-(difluoromethoxy)isoindoline hydrochloride (100mg), and N,N-diisopropylethylamine (180mg) were added to a mixed solution of H₂O (0.5mL) and tetrahydrofuran (2.5mL). The reaction mixture was stirred at 70°C for 2 hours. The reaction mixture was concentrated under a reduced pressure to produce a crude product. The crude product was separated and purified by Prep-HPLC (C18, 1 0mmol/L NH₄HCO₃/water, MeCN) to produce the target compound (111mg). LCMS(ESI)[M+H]⁺=437.2; ¹H NMR(400 MHz, DMSO-*d*₆)δ 7.52-7.38(m, 2H), 7.33-6.99(m, 3H), 4.91-4.71(m, 5H), 3.79(d, *J*=5.8Hz, 2H), 3.46(dt, *J*=16.4, 7.9Hz, 1H), 3.24(dt, *J*=13.3, 8.2Hz, 1H), 2.94(ddd, *J*=35.6, 15.3, 7.6Hz, 2H), 2.38(dt, *J*=20.2, 10.0Hz, 2H), 2.28-2.17(m, 2H), 1.80(dd, *J*=19.0, 10.5Hz, 2H).

### Example 54 (Compound 88)

### Synthesis of (R/S)-2-(3-(4-chlorophenyl)-3-methoxyazetidin-1-yl)-4-((tetrahydro-2H-pyran-4-yl)amino)-6,7-dihydrothieno[3,2-d]pyrimidine 5-oxide:

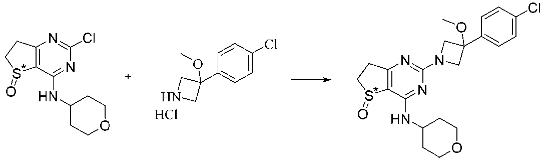

(R/S)-2-chloro-4-((tetrahydro-2H-pyran-4-yl)amino)-6,7-dihydrothieno[3,2-d]pyrimidine 5-oxide (200mg) and 3-(4-chlorophenyl)-3-methoxyazetidine hydrochloride (200mg) were added to a mixed solution of tetrahydrofuran (4mL) and H₂O (0.8mL). N,N-diisopropylethylamine (450mg) was added. The reaction mixture was stirred at 70°C for 1 hour. The reaction mixture was concentrated under a reduced pressure to produce a crude product. The crude product was separated and purified by Prep-HPLC (C18, 10mmol/L NH₄HCO₃/water, MeCN) to produce the target compound (270mg). LCMS(ESI) [M+H]⁺=449.2; ¹H NMR(400 MHz, DMSO-*d*₆)δ 7.67(d, *J*=7.6Hz, 1H), 7.50(s, 4H), 4.26(s, 5H), 3.88(s, 2H), 3.41 (dd, *J*=17.2, 8.7Hz, 2H), 3.22(dt, *J*=13.7, 8.4Hz, 2H), 3.05(s, 3H), 3.00-2.84(m, 2H), 1.75(s, 2H), 1.62(dt, *J*=13.6, 6.9Hz, 2H).

### Example 55 (Compound 89)

### Synthesis of (R/S)-2-(5-fluoroisoindolin-2-yl)-4-((1-(hydroxymethyl)cyclobutyl)amino)-6,7-dihydrothieno[3,2-d]pyrimidine 5-oxide:

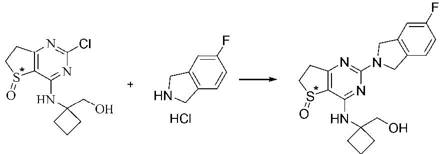

(R/S)-2-chloro-4-((1-(hydroxymethyl)cyclobutyl)amino)-6,7-dihydrothieno[3,2-d]pyrimidine 5-oxide (100mg), 5-fluoro-2,3-dihydro-1H-isoindoline hydrochloride (79mg), and N,N-diisopropylethylamine (180mg) were added to a mixed solution of H₂O (0.5mL) and tetrahydrofuran (2.5mL). The reaction mixture was stirred at 70°C for 2 hours. The reaction mixture was concentrated under a reduced pressure to produce a crude product. The crude product was separated and purified by Prep-HPLC (C18, 1 0mmol/L NH₄HCO₃/water, MeCN) to produce the target compound (75mg). LCMS(ESI)[M+H]⁺=389.0; ¹H NMR(400 MHz, DMSO-*d*₆)δ 7.49-7.37(m, 2H), 7.27(dd, *J*=23.1, 9.0Hz, 1H), 7.18-7.10(m, 1H), 4.92-4.70(m, 5H), 3.79(d, *J*=5.7Hz, 2H), 3.46(dt, *J*=16.4, 7.9Hz, 1H), 3.24(dt, *J*=13.4, 8.3Hz, 1H), 3.04-2.84(m, 2H), 2.38(dt, *J*=20.6, 10.4Hz, 2H), 2.23(t, *J*=9.8Hz, 2H), 1.80(dq, *J*=19.1, 9.8Hz, 2H).

### Example 56 (Compound 90)

### Synthesis of (R/S)-2-(5,6-difluoroisoindolin-2-yl)-4-((1-(hydroxymethyl)cyclobutyl)amino)-6,7-dihydrothieno[3,2-d]pyrimidine 5-oxide:

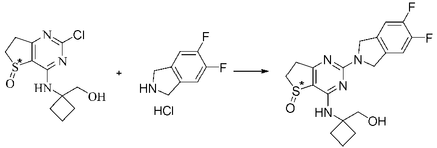

(R/S)-2-chloro-4-((1-(hydroxymethyl)cyclobutyl)amino)-6,7-dihydrothieno[3,2-d]pyrimidine 5-oxide (100mg), 5,6-difluoro-2,3-dihydro-1H-isoindoline hydrochloride (87mg), and N,N-diisopropylethylamine (180mg) were added to a mixed solution of H₂O (0.5mL) and tetrahydrofuran (2.5mL). The reaction mixture was stirred at 70°C for 2 hours. The reaction mixture was concentrated under a reduced pressure to produce a crude product. The crude product was separated and purified by Prep-HPLC (C18, 1 0mmol/L NH₄HCO₃/water, MeCN) to produce the target compound (119mg). LCMS(ESI)[M+H]⁺=407.0; ¹H NMR(400 MHz, DMSO-*d*₆)δ 7.61-7.42(m, 3H), 4.90-4.67(m, 5H), 3.79(d, *J*=6.0Hz, 2H), 3.45(dd, *J*=16.8, 8.2Hz, 1H), 3.23(dt, *J*=13.4, 8.3Hz, 1H), 3.02-2.85(m, 2H), 2.38(dd, *J*=22.1, 11.8Hz, 2H), 2.28-2.14(m, 2H), 1.79(dq, *J*=19.3, 9.9Hz, 2H).

### Example 57 (Compound 91)

### Synthesis of (R/S)-2-(5-(difluoromethyl)isoindolin-2-yl)-4-((1-(hydroxymethyl)cyclobutyl)amino)-6,7-dihydrothieno[3,2-d]pyrimidine 5-oxide:

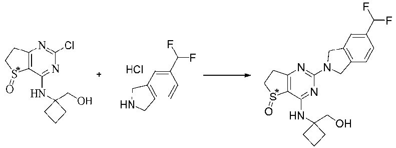

(R/S)-2-chloro-4-((1-(hydroxymethyl)cyclobutyl)amino)-6,7-dihydrothieno[3,2-d]pyrimidine 5-oxide (100mg), 5-(difluoromethyl)isoindoline hydrochloride (110mg), and N,N-diisopropylethylamine (180mg) were added to a mixed solution of H₂O (0.5mL) and tetrahydrofuran (2.5mL). The reaction mixture was stirred at 70°C for 2 hours. The reaction mixture was concentrated under a reduced pressure to produce a crude product. The crude product was separated and purified by Prep-HPLC (C18, 10mmol/L NH₄HCO₃/water, MeCN) to produce the target compound (93mg). LCMS(ESI)[M+H]⁺=421.3; ¹H NMR(400 MHz, DMSO-*d*₆)δ 7.69-7.39(m, 4H), 7.06(td, *J*=55.9, 5.3Hz, 1H), 4.86(dd, *J*=13.2, 6.0Hz, 5H), 3.80(d, *J*=5.8Hz, 2H), 3.47(dt, *J*=16.4, 7.8Hz, 1H), 3.24(dt, *J*=13.5, 8.3Hz, 1H), 2.99(dd, *J*=17.1, 8.1Hz, 1H), 2.89(dd, *J*=13.5, 7.2Hz, 1H), 2.39(dt, *J*=21.6, 10.6Hz, 2H), 2.24(t, *J*=9.9Hz, 2H), 1.80(dd, *J*=19.0, 9.7Hz, 2H).

### Example 58 (Compound 92)

### Synthesis of (R/S)-2-(3-(4-chlorophenyl)-3-(methoxy-d₃)azetidin-1-yl)-4-((1-(hydroxymethyl)cyclobutyl)amino)-6,7-dihydrothieno[3,2-d]pyrimidine 5-oxide:

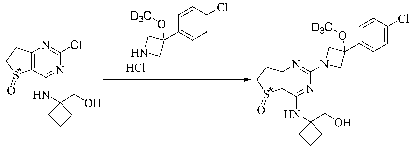

(R/S)-2-chloro-4-((1-(hydroxymethyl)cyclobutyl)amino)-6,7-dihydrothieno[3,2-d]pyrimidine 5-oxide (100mg), 3-(4-chlorophenyl)-3-(methoxy-d3)azetidine hydrochloride (123mg), and N,N-diisopropylethylamine (180mg) were added to a mixed solution of H₂O (0.5mL) and tetrahydrofuran (2.5mL). The reaction mixture was stirred at 70°C for 2 hours. The reaction mixture was concentrated under a reduced pressure to produce a crude product. The crude product was separated and purified by Prep-HPLC (C18, 10mmol/L NH₄HCO₃/water, MeCN) to produce the target compound (90mg). LCMS(ESI)[M+H]⁺=452.3; ¹H NMR(400 MHz, DMSO-*d*₆)δ 7.49(s, 4H), 4.84(t, *J*=5.6Hz, 1H), 4.22(s, 4H), 3.71(s, 2H), 3.46-3.37(m, 2H), 3.22(dt, *J*=15.4, 8.5Hz, 1H), 3.01-2.83(m, 2H), 2.39-2.23(m, 2H), 2.16(d, *J*=10.2Hz, 2H), 1.74(dq, *J*=18.9, 9.3Hz, 2H).

### Example 59 (Compound 94)

### Synthesis of (R/S)-2-(3-(4-chlorophenyl)-3-(methoxy-d₃)azetidin-1-yl)-4-((tetrahydro-2H-pyran-4-yl)amino)-6,7-dihydrothieno[3,2-d]pyrimidine 5-oxide:

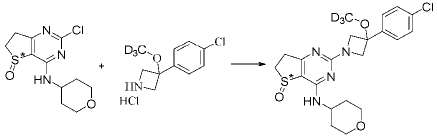

(R/S)-2-chloro-4-((tetrahydro-2H-pyran-4-yl)amino)-6,7-dihydrothieno[3,2-d]pyrimidine 5-oxide (100mg), 3-(4-chlorophenyl)-3-(methoxy-d3)azetidine hydrochloride (123mg), and N,N-diisopropylethylamine (180mg) were added to a mixed solution of H₂O (0.5mL) and tetrahydrofuran (2.5mL). The reaction mixture was stirred at 70°C for 2 hours. The reaction mixture was concentrated under a reduced pressure to produce a crude product. The crude product was separated and purified by Prep-HPLC (C18, 1 0mmol/L NH₄HCO₃/water, MeCN) to produce the target compound (51mg). LCMS(ESI)[M+H]⁺=452.3; ¹H NMR(400 MHz, DMSO-*d*₆)δ 7.66(d, *J*=7.6Hz, 1H), 7.50(s, 4H), 4.25(s, 5H), 3.88(s, 2H), 3.43(dt, *J*=17.0, 8.1Hz, 3H), 3.22(dt, *J*=13.6, 8.5Hz, 1H), 3.02-2.85(m, 2H), 1.76(s, 2H), 1.69-1.53(m, 2H).

### Example 60&61 (Compound 95&96)

### Synthesis of (R/S)-2-(3-(2-fluorophenyl)-3-(methoxy-d3)azetidin-1-yl)-4-((1-(hydroxymethyl)cyclobutyl)amino)-6,7-dihydrothieno[3,2-d]pyrimidine 5-oxide & (R/S)-2-(3-(4-fluorophenyl)-3-(methoxy-d3)azetidin-1-yl)-4-((1-(hydroxymethyl)cyclobutyl)amino)-6,7-dihydrothieno[3,2-d]pyrimidine 5-oxide:

### Step 1: Preparation of tert-butyl 3-(4-fluorophenyl)-3-hydroxyazetidine-1-carboxylate:

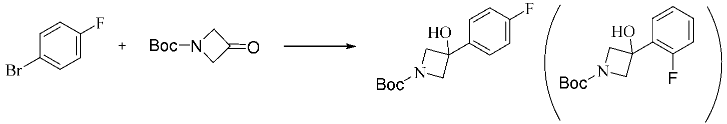

Under the protection of nitrogen gas, 1-bromo-4-fluorobenzene (5.00g) was dissolved in anhydrous tetrahydrofuran (50mL). A 2.5M solution of n-butyl lithium/hexane (13.7mL) was added dropwise at -78°C. The mixture was continuously reacted at -78°C for 0.5 hours. Tert-butyl 3-oxoazetidine-1-carboxylate (4.50g) was dissolved in anhydrous tetrahydrofuran (20mL), and this solution was slowly added to the reaction mixture at - 78°C. The resulting mixture was continuously stirred at -78°C for 1 hour. The reaction mixture was warmed up to 0°C. A saturated ammonium chloride solution (10mL) was added to quench the reaction. The resulting mixture was diluted with water (100mL), and extracted with methyl tert-butyl ether (100mL) twice. The organic phases were combined, washed with a saturated sodium chloride solution (100mL) once, dried over anhydrous sodium sulfate, filtered, and dried by rotary evaporation to produce a crude product. The crude product was purified by silica gel column chromatography (PE:EA=5:1) to produce the target compound (6.37g). LCMS(ESI)[M+H-56]⁺=212.1.

### Step 2: Preparation of tert-butyl 3-(4-fluorophenyl)-3-(methoxy-d3)azetidine-1-carboxylate:

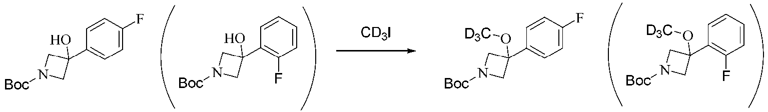

A mixture of compounds obtained from the reaction of step 1 (2g) was dissolved in DMF(5mL). The atmosphere was replaced with nitrogen gas three times. NaH (0.57g) was added under an ice bath. The resulting mixture was stirred under the ice bath for 10 minutes. Iodomethane-d3 (2.17g) was added, and the resulting mixture was continuously stirred for 30 minutes under the ice bath. LCMS detection showed that the starting material was completely consumed. The reaction mixture was quenched with a saturated NH₄Cl aqueous solution. The mixture was added to water (100mL), and extracted with methyl tert-butyl ether (80mL) twice. The organic phases were combined, then washed with water (50mL) once and with a saturated saline solution (50mL) twice, dried over anhydrous sodium sulfate, filtered and dried by rotary evaporation to produce the target compound (2.13g, crude). The crude product was directly used in the next step.

### Step 3: Preparation of 3-(4-fluorophenyl)-3-(methoxy-d3)azetidine hydrochloride:

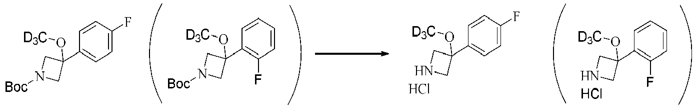

A mixture of compounds obtained from the reaction of step 2 (2.10g) was added to a 4M solution of hydrogen chloride/ethyl acetate (15mL). The mixture was stirred at room temperature for 0.5 hours. LCMS detection showed that the starting material was completely consumed. The reaction mixture was directly concentrated under a reduced pressure to produce the target compound (1.94g, crude). The crude product was directly used in the next step. LCMS(ESI)[M+H]⁺=185.1.

### Step 4: Preparation of (R/S)-2-(3-(2-fluorophenyl)-3-(methoxy-d3)azetidin-1-yl)-4-((1-(hydroxymethyl)cyclobutyl)amino)-6,7-dihydrothieno[3,2-d]pyrimidine 5-oxide & (R/S)-2-(3-(4-fluorophenyl)-3-(methoxy-d3)azetidin-1-yl)-4-((1-(hydroxymethyl)cyclobutyl)amino)-6,7-dihydrothieno[3,2-d]pyrimidine 5-oxide:

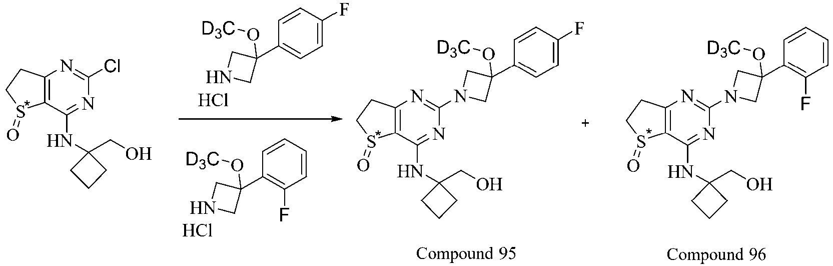

(R/S)-2-chloro-4-((1-(hydroxymethyl)cyclobutyl)amino)-6,7-dihydrothieno[3,2-d]pyrimidine 5-oxide (100mg), a mixture of compounds obtained from the reaction of step 3 (115mg), and N,N-diisopropylethylamine (180.49mg) were added to a mixed solution of H₂O (0.5mL) and tetrahydrofuran (2.5mL). The reaction mixture was stirred at 70°C for 2 hours. The reaction mixture was concentrated under a reduced pressure to produce a crude product. The crude product was separated and purified by Prep-HPLC (C18, 10mmol/L NH₄HCO₃/water, MeCN) to produce (R/S)-2-(3-(2-fluorophenyl)-3-(methoxy-d3)azetidin-1-yl)-4-((1-(hydroxymethyl)cyclobutyl)amino)-6,7-dihydrothieno[3,2-d]pyrimidine 5-oxide (7.4mg, P1) and (R/S)-2-(3-(4-fluorophenyl)-3-(methoxy-d3)azetidin-1-yl)-4-((1-(hydroxymethyl)cyclobutyl)amino)-6,7-dihydrothieno[3,2-d]pyrimidine 5-oxide (61mg, P2).

P1: LCMS(ESI)[M+H]⁺=436.2; ¹H NMR(400 MHz, DMSO-*d*₆)δ 7.59-7.40(m, 3H), 7.31-7.21(m, 2H), 4.85(t, *J*=5.7Hz, 1H), 4.37(s, 2H), 4.26(d, *J*=10.1Hz, 2H), 3.72(dd, *J*=5.8, 3.1Hz, 2H), 3.46-3.36(m, 1H), 3.21(dt, *J*=16.4, 8.3Hz, 1H), 2.99-2.82(m, 2H), 2.30(dq, *J*=21.0, 9.2Hz, 2H), 2.19-2.11(m, 2H), 1.76(dq, *J*=19.4, 9.9Hz, 2H).

P2: LCMS(ESI)[M+H]⁺=436.2; ¹H NMR(400 MHz, DMSO-*d*₆)δ 7.61-7.43(m, 3H), 7.26(t, *J*=8.9Hz, 2H), 4.84(t, *J*=5.6Hz, 1H), 4.22(s, 4H), 3.71(dd, *J*=5.7, 2.8Hz, 2H), 3.41(dt, *J*=16.3, 7.8Hz, 1H), 3.26-3.16(m, 1H), 2.90(ddd, *J*=21.8, 15.3, 7.7Hz, 2H), 2.31(dq, *J*=21.0, 10.3Hz, 2H), 2.15(d, *J*=7.9Hz, 2H), 1.75(dq, *J*=18.8, 9.8Hz, 2H).

### Example 62&63 (Compound 97&98)

### Synthesis of (R/S)-2-(3-(2-fluorophenyl)-3-(methoxy-d3)azetidin-1-yl)-4-((tetrahydro-2H-pyran-4-yl)amino)-6,7-dihydrothieno[3,2-d]pyrimidine 5-oxide & (R/S)-2-(3-(4-fluorophenyl)-3-(methoxy-d3)azetidin-1-yl)-4-((tetrahydro-2H-pyran-4-yl)amino)-6,7-dihydrothieno[3,2-d]pyrimidine 5-oxide:

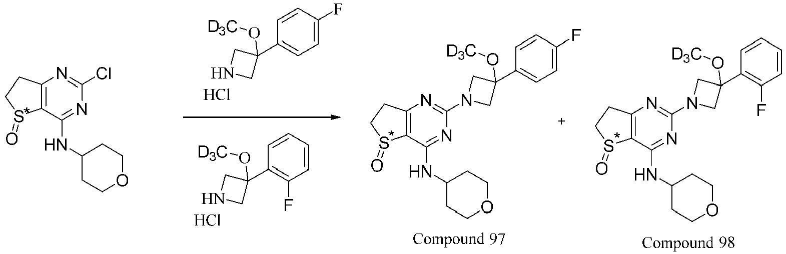

(R/S)-2-chloro-4-((tetrahydro-2H-pyran-4-yl)amino)-6,7-dihydrothieno[3,2-d]pyrimidine 5-oxide (150mg), a mixture of compounds obtained from the reaction of step 3 in Example 60&61 (172mg), and N,N-diisopropylethylamine (267mg) were added to a mixed solution of H₂O (0.5mL) and tetrahydrofuran (2.5mL). The reaction mixture was stirred at 70°C for 2 hours. The reaction mixture was concentrated under a reduced pressure to produce a crude product. The crude product was separated and purified by Prep-HPLC (C18, 10mmol/L NH₄HCO₃/water, MeCN) to produce (R/S)-2-(3-(2-fluorophenyl)-3-(methoxy-d3)azetidin-1-yl)-4-((tetrahydro-2H-pyran-4-yl)amino)-6,7-dihydrothieno[3,2-d]pyrimidine 5-oxide (8.4mg, P1) and (R/S)-2-(3-(4-fluorophenyl)-3-(methoxy-d3)azetidin-1-yl)-4-((tetrahydro-2H-pyran-4-yl)amino)-6,7-dihydrothieno[3,2-d]pyrimidine 5-oxide (66mg, P2).

P1: LCMS(ESI)[M+H]⁺=436.2; ¹H NMR(400 MHz, DMSO-*d*₆)δ 7.75(s, 1H), 7.49(dt, *J*=33.9, 7.0Hz, 2H), 7.31-7.22(m, 2H), 4.43(d, *J*=10.2Hz, 2H), 4.32(d, *J*=10.3Hz, 2H), 4.22(s, 1H), 3.89(s, 2H), 3.23(d, *J*=13.2Hz, 4H), 3.03-2.86(m, 2H), 1.83-1.55(m, 4H).

P2: LCMS(ESI)[M+H]⁺=436.2; ¹H NMR(400 MHz, DMSO-*d*₆)δ 7.67(d, *J*=7.6Hz, 1H), 7.57-7.44(m, 2H), 7.33-7.19(m, 2H), 4.24(h, *J*=10.8Hz, 5H), 3.87(d, *J*=7.1Hz, 2H), 3.48-3.37(m, 2H), 3.22(dt, *J*=13.6, 8.3Hz, 2H), 2.93(ddd, *J*=22.0, 15.4, 7.6Hz, 2H), 1.84-1.53(m, 4H).

### Example 64 (Compound 99)

### Synthesis of (R/S)-4-((1-(aminomethyl)cyclobutyl)amino)-2-(4-(5-chloropyrimidin-2-yl)piperidin-1-yl)-6,7-dihydrothieno[3,2-d]pyrimidine 5-oxide:

### Step 1: Preparation of tert-butyl ((1-((2-chloro-6,7-dihydrothieno[3,2-d]pyrimidin-4-yl)amino)cyclobutyl)methyl)carbamate:

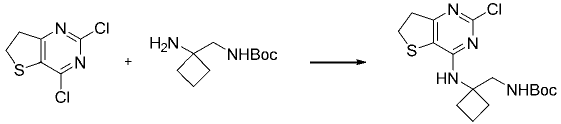

2,4-dichloro-6,7-dihydrothieno[3,2-d]pyrimidine (1.00g), tert-butyl ((1-aminocyclobutyl)methyl)carbamate (1.00g), and N,N-diisopropylethylamine (1.87g) were added to acetonitrile (50mL). The mixture was stirred at 85°C for 24 hours, concentrated under a reduced pressure, dissolved in methyl tert-butyl ether (50mL), washed with water (30mL) twice and with a saturated NaCl solution (30mL) once, dried over anhydrous sodium sulfate, filtered, and dried by rotary evaporation to produce a crude product. The crude product was purified by silica gel column chromatography (PE:EA=3: 1) to produce the target compound (0.85g). LCMS(ESI)[M+H]⁺=371.3.

### Step 2: Preparation of tert-butyl (R/S)-((1-((2-chloro-5-oxido-6,7-dihydrothieno[3,2-d]pyrimidin-4-yl)amino)cyclobutyl)methyl)carbamate:

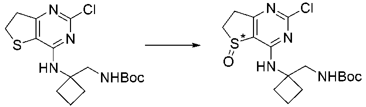

S-(-)bi-naphthol (78mg) was dissolved in DCM (10mL). Tetraisopropyl titanate (77mg) was added. The mixture was reacted at 25°C under the protection of nitrogen gas for 1 hour. Water (0.14mL) was added. The resulting mixture was continuously reacted for 0.5 hours. Then tert-butyl ((1-((2-chloro-6,7-dihydrothieno[3,2-d]pyrimidin-4-yl)amino)cyclobutyl)methyl)carbamate (500mg) was added, and the resulting mixture was reacted for 1 hour. Tert-butyl peroxide (200mg) was added. The reaction mixture was added to water (30mL), and extracted with DCM:MeOH=10:1 (20mL) twice. The organic phases were combined, then washed with water (20mL) once and with a saturated saline solution (20mL) twice, dried over anhydrous sodium sulfate, filtered, and dried by rotary evaporation to produce a crude product. The crude product was purified by silica gel column chromatography (PE:EA=0.1:1) to produce the target compound (340mg). LCMS(ESI)[M+H]⁺=387.1.

### Step 3: Preparation of tert-butyl (R/S)-((1-((2-(4-(5-chloropyrimidin-2-yl)piperidin-1-yl)-5-oxido-6,7-dihydrothieno[3,2-d]pyrimidin-4-yl)amino)cyclobutyl)methyl)carbamate:

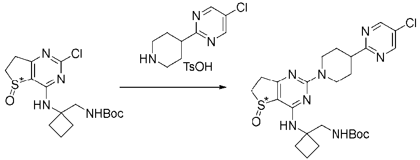

Tert-butyl (R/S)-((1-((2-chloro-5-oxido-6,7-dihydrothieno[3,2-d]pyrimidin-4-yl)amino)cyclobutyl)methyl)carbamate (200mg), 5-chloro-2-(piperidin-4-yl)pyrimidine p-toluenesulfonate (572mg), and N,N-diisopropylethylamine (267mg) were added to a mixed solution of H₂O (1mL) and tetrahydrofuran (5mL). The reaction mixture was stirred at 70°C for 2 hours, diluted with water (20mL), and extracted with DCM:MeOH=10:1 (20mL) twice. The organic phases were combined, then washed with a saturated NaCl solution (20mL) washed twice, dried over anhydrous sodium sulfate, filtered, and concentrated under a reduced pressure to produce the target compound (380mg). LCMS(ESI)[M+H]⁺=548.2.

### Step 4: Preparation of (R/S)-4-((1-(aminomethyl)cyclobutyl)amino)-2-(4-(5-chloropyrimidin-2-yl)piperidin-1-yl)-6,7-dihydrothieno[3,2-d]pyrimidine 5-oxide:

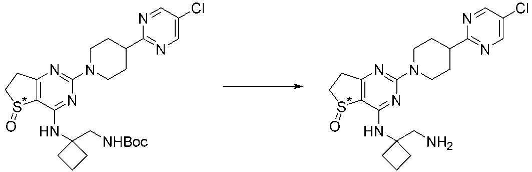

Tert-butyl (R/S)-((1-((2-(4-(5-chloropyrimidin-2-yl)piperidin-1-yl)-5-oxido-6,7-dihydrothieno[3,2-d]pyrimidin-4-yl)amino)cyclobutyl)methyl)carbamate (370mg) was added to a mixed solution of TFA (3mL) and DCM (3mL). The mixture was stirred at room temperature for 1 hour. The reaction mixture was concentrated under a reduced pressure, and separated and purified by Pre-HPLC (C18, 10mmol/L NH₄HCO₃/water, MeCN) to produce the target compound (107mg). LCMS(ESI)[M+H]⁺=448.2; ¹H NMR(400 MHz, DMSO-*d*₆)δ 8.86(d, *J*=0.8Hz, 2H), 7.49(d, *J*=44.5Hz, 1H), 4.72(t, *J*=14.1Hz, 2H), 3.44-3.38(m, 2H), 3.29-3.12(m, 4H), 3.11-2.82(m, 5H), 2.32(p, *J*=9.9Hz, 2H), 2.11(t, *J*=10.4Hz, 2H), 1.97(d, *J*=13.1Hz, 2H), 1.70(dd, *J*=49.0, 11.9Hz, 4H).

### Example 65 (Compound 100)

### Synthesis of (R/S)-4-((1-(hydroxymethyl)cyclobutyl)amino)-2-(3-methoxy-3-(4-methoxyphenyl)azetidin-1-yl)-6,7-dihydrothieno[3,2-d]pyrimidine 5-oxide:

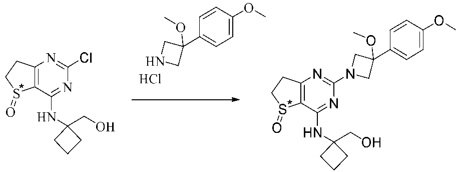

(R/S)-2-chloro-4-((1-(hydroxymethyl)cyclobutyl)amino)-6,7-dihydrothieno[3,2-d]pyrimidine 5-oxide (200mg), 3-methoxy-3-(4-methoxyphenyl)azetidine hydrochloride (200mg), and N,N-diisopropylethylamine (360mg) were added to a mixed solution of H₂O (1mL) and tetrahydrofuran (5mL). The reaction mixture was stirred at 70°C for 2 hours. The reaction mixture was concentrated under a reduced pressure to produce a crude product. The crude product was separated and purified by Prep-HPLC (C18, 1 0mmol/L NH₄HCO₃/water, MeCN) to produce the target compound (101mg). LCMS(ESI)[M+H]⁺=445.2; ¹H NMR(400 MHz, DMSO-*d*₆)δ 7.48(s, 1H), 7.41-7.30(m, 2H), 7.03-6.92(m, 2H), 4.85(t, *J*=5.6Hz, 1H), 4.19(s, 4H), 3.81-3.66(m, 4H), 3.46-3.33(m, 2H), 3.21(dt, *J*=16.5, 8.3Hz, 1H), 2.98(s, 5H), 2.31(dq, *J*=20.9, 10.3Hz, 2H), 2.14(t, *J*=10.2Hz, 2H), 1.86-1.64(m, 2H).

### Example 66 (Compound 101)

### Synthesis of (R/S)-(1-((2-(3-(4-chlorophenyl)-3-methoxyazetidin-1-yl)-5-oxido-6,7-dihydrothieno[3,2-d]pyrimidin-4-yl)amino)cyclobutyl)methyl (2-(dimethylamino)ethyl) carbonate:

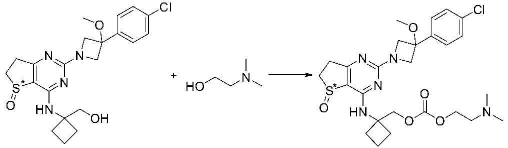

(R/S)-2-(3-(4-chlorophenyl)-3-methoxyazetidin-1-yl)-4-((1-(hydroxymethyl)cyclobutyl)amino)-6,7-dihydrothieno[3,2-d]pyrimidine 5-oxide (120mg) was added to DCM (3mL). N,N'-carbonyldiimidazole (48mg) was added, and then the mixture was reacted at room temperature for 1 hour. LCMS detection showed that the starting material was completely consumed. Caesium carbonate (175mg) and 2-(dimethylamino)ethan-1-ol (65mg) were added to the reaction mixture. The resulting mixture was reacted at room temperature for 2 hours. The reaction mixture was purified by silica gel column chromatography (DCM:MeOH=20:1) to produce a crude product. The crude product was separated and purified by Prep-HPLC (C18, 0.1% TFA/water, MeCN) to produce the target compound (4.1mg). LCMS(ESI)[M+H]⁺=564.2; ¹H NMR(400 MHz, DMSO-*d*₆)δ 9.52(s, 1H), 7.98(s, 1H), 7.50(s, 3H), 4.72(s, 2H), 4.51(d, *J*=11.1Hz, 1H), 4.37(d, *J*=5.6Hz, 2H), 4.22(s, 2H), 3.38(d, *J*=4.9Hz, 3H), 3.23(d, *J*=13.6Hz, 2H), 3.03(s, 2H), 2.91(dd, *J*=14.1, 7.2Hz, 2H), 2.79(d, *J*=4.6Hz, 4H), 2.67(d, *J*=2.1Hz, 1H), 2.39-2.31(m, 2H), 2.25(s, 2H), 1.84(s, 2H), 1.25(d, *J*=7.2Hz, 2H).

### Example 67 (Compound 102)

### Synthesis of (R/S)-(1-((2-(3-(4-chloro-3-fluorophenyl)-3-methoxyazetidin-1-yl)-5-oxido-6,7-dihydrothieno[3,2-d]pyrimidin-4-yl)amino)cyclobutyl)methyl (2-(dimethylamino)ethyl) carbonate:

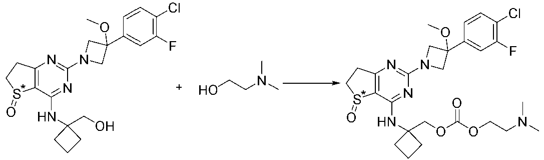

(R/S)-2-(3-(4-chloro-3-fluorophenyl)-3-methoxyazetidin-1-yl)-4-((1-(hydroxymethyl)cyclobutyl)amino)-6,7-dihydrothieno[3,2-d]pyrimidine 5-oxide (500mg) was added to DCM (5mL). N,N'-carbonyldiimidazole (262mg) was added, and the resulting mixture was reacted at room temperature for 1 hour. Caesium carbonate (712mg) and 2-(dimethylamino)ethan-1-ol (200mg) were added to the reaction mixture. The resulting mixture was reacted at room temperature for 2 hours. The reaction mixture was purified by silica gel column chromatography (DCM:MeOH=20:1) to produce a crude product. The crude product was separated and purified by Prep-HPLC (C18, 0.1% TFA/water, MeCN) to produce the target compound (21mg). LCMS(ESI)[M+H]⁺=582.2; ¹H NMR(400 MHz, DMSO-*d*₆)δ 9.51(s, 1H), 8.01(s, 1H), 7.67(t, *J*=8.0Hz, 1H), 7.55(d, *J*=10.4Hz, 1H), 7.35(d, *J*=8.1Hz, 1H), 4.71(s, 2H), 4.51(d, *J*=10.8Hz, 1H), 4.36(t, *J*=5.2Hz, 2H), 4.22(s, 2H), 3.38(d, *J*=5.1Hz, 2H), 3.27-3.19(m, 2H), 3.08-2.87(m, 6H), 2.80(d, *J*=4.7Hz, 4H), 2.69-2.65(m, 1H), 2.33(d, *J*=2.6Hz, 2H), 2.22(d, *J*=19.1Hz, 2H), 1.85(d, *J*=8.5Hz, 2H).

### Example 68 (Compound 103)

### Synthesis of (R/S)-4-((1-(hydroxymethyl)cyclobutyl)amino)-2-(3-methoxy-3-(3-methoxyphenyl)azetidin-1-yl)-6,7-dihydrothieno[3,2-d]pyrimidine 5-oxide:

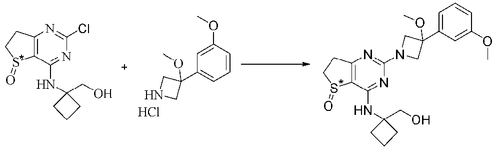

(R/S)-2-chloro-4-((1-(hydroxymethyl)cyclobutyl)amino)-6,7-dihydrothieno[3,2-d]pyrimidine 5-oxide (200mg), 3-methoxy-3-(3-methoxyphenyl)azetidine hydrochloride (320mg), and N,N-diisopropylethylamine (360mg) were added to a mixed solution of H₂O (1mL) and tetrahydrofuran (5mL). The reaction mixture was stirred at 70°C for 2 hours. LCMS detection showed that the starting material was completely consumed. The reaction mixture was concentrated under a reduced pressure to produce a crude product. The crude product was separated and purified by Prep-HPLC (C18, 10mmol/L NH₄HCO₃/water, MeCN) to produce the target compound (132mg). LCMS(ESI)[M+H]⁺=445.3; ¹H NMR(400 MHz, DMSO-d₆)δ 7.49(s, 1H), 7.36(t, *J*=7.9Hz, 1H), 7.08-6.88(m, 3H), 4.85(t, *J*=5.7Hz, 1H), 4.19(d, *J*=7.9Hz, 4H), 3.82-3.64(m, 5H), 3.41(dt, *J*=16.4, 7.8Hz, 1H), 3.21(dt, *J*=13.4, 8.3Hz, 1H), 3.03(s, 3H), 2.99-2.83(m, 2H), 2.31(dq, *J*=21.1, 10.2Hz, 2H), 2.20-2.07(m, 2H), 1.76(dt, *J*=18.8, 10.0Hz, 2H).

### Example 69 (Compound 104)

### Synthesis of (R/S)-4-((1-(aminomethyl-d2)cyclobutyl)amino)-2-(4-(5-chloropyrimidin-2-yl)piperidin-1-yl)-6,7-dihydrothieno[3,2-d]pyrimidine 5-oxide:

### Step 1: Preparation of 1-((2-chloro-6,7-dihydrothieno[3,2-d]pyrimidin-4-yl)amino)cyclobutane-1-carboxamide:

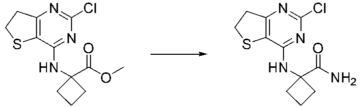

Methyl 1-((2-chloro-6,7-dihydrothieno[3,2-d]pyrimidin-4-yl)amino)cyclobutane-1-carboxylate (1.00g) was added to a 7M solution of ammonia/methanol (10mL). The mixture was stirred at 60°C in a closed condition for 48 hours. The reaction solution was concentrated under a reduced pressure to produce a crude product. The crude product was purified by silica gel column chromatography (PE:EA=0.1:1) to produce the target compound (477mg). LCMS(ESI)[M+H]⁺=285.1.

### Step 2: Preparation of N-(1-(aminomethyl-d2)cyclobutyl)-2-chloro-6,7-dihydrothieno[3,2-d]pyrimidin-4-amine:

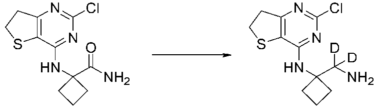

1-((2-Chloro-6,7-dihydrothieno[3,2-d]pyrimidin-4-yl)amino)cyclobutane-1-carboxamide(927mg) was added to tetrahydrofuran (30mL). The atmosphere was replaced with nitrogen gas three times. The mixture was cooled in an ice bath for 5 minutes. Lithium aluminum deuteride (440mg) was added in batches under nitrogen, and the mixture was stirred at 70°C for 2 hours. Under an ice bath, a 15% NaOH aqueous solution (0.5mL) was added dropwise to the reaction mixture. The resulting mixture was dried over anhydrous sodium sulfate, and filtered. The filter cake was washed with DCM:MeOH=10:1 (50mL). The filtrate was collected, and concentrated under a reduced pressure to produce the target compound (1.10g). LCMS(ESI)[M+H]⁺=273.1.

### Step 3: Preparation of tert-butyl ((1-((2-chloro-6,7-dihydrothieno[3,2-d]pyrimidin-4-yl)amino)cyclobutyl)methyl-d2)carbamate:

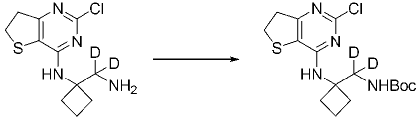

N-(1-(aminomethyl-d2)cyclobutyl)-2-chloro-6,7-dihydrothieno[3,2-d]pyrimidin-4-amine (1.10g) and di-tert-butyl dicarbonate (0.90g) were added to MeOH(SmL). The mixture was stirred at room temperature for 2 hours. LCMS detection showed a product was formed. The reaction mixture was diluted with methyl tert-butyl ether (50mL), washed with water (20mL) twice and with a saturated saline solution (20mL) twice, dried over anhydrous sodium sulfate, filtered and concentrated under a reduced pressure to produce a crude product. The crude product was purified by silica gel column chromatography (PE:EA=3:1) to produce the target compound (257mg). LCMS(ESI)[M+H]⁺=373.3.

### Step 4: Preparation of tert-butyl (R/S)-((1-((2-chloro-5-oxido-6,7-dihydrothieno[3,2-d]pyrimidin-4-yl)amino)cyclobutyl)methyl-d2)carbamate:

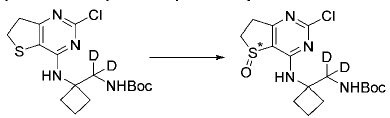

S-(-)bi-naphthol (40mg) was dissolved in DCM (10mL). Tetraisopropyl titanate (40mg) was added. The mixture was reacted at 25°C under the protection of nitrogen gas for 1 hour. Water (0.02mL) was added, and the reaction continued for 0.5 hours. Then tert-butyl ((1-((2-chloro-6,7-dihydrothieno[3,2-d]pyrimidin-4-yl)amino)cyclobutyl)methyl-d2)carbamate (257mg) was added, and the mixture was reacted for 1 hour. Then tert-butyl peroxide (98mg) was added. The reaction mixture was added to water (30mL), and extracted with DCM:MeOH=10:1 (20mL) twice. The organic phases were combined, then washed with water (20mL) once and with a saturated saline solution (20mL) once, dried over anhydrous sodium sulfate, filtered and concentrated under a reduced pressure to produce a crude product. The crude product was purified by silica gel column chromatography (DCM:MeOH=20:1) to produce the target compound (135mg). LCMS(ESI)[M+H]⁺=389.1.

### Step 5: Preparation of tert-butyl (R/S)-((1-((2-(4-(5-chloropyrimidin-2-yl)piperidin-1-yl)-5-oxido-6,7-dihydrothieno[3,2-d]pyrimidin-4-yl)amino)cyclobutyl)methyl-d2)carbamate:

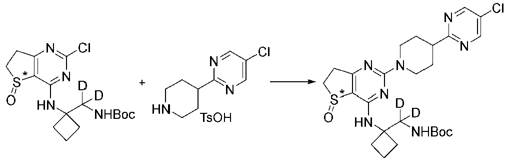

Tert-butyl (R/S)-((1-((2-chloro-5-oxido-6,7-dihydrothieno[3,2-d]pyrimidin-4-yl)amino)cyclobutyl)methyl-d2)carbamate (135mg), 5-chloro-2-(piperidin-4-yl)pyrimidine p-toluenesulfonate (256mg) and N,N-diisopropylethylamine (181mg) were added to a mixed solution of tetrahydrofuran (2mL) and H₂O (0.4mL). The mixture was stirred at 70°C for 1 hour. Water (20mL) was added to the reaction mixture. The mixture was extracted with methyl tert-butyl ether (10mL) twice. The organic phases were combined, then washed with a saturated NaCl solution (10mL) once, dried over anhydrous sodium sulfate, filtered, and concentrated under a reduced pressure to produce the target compound (330mg). LCMS(ESI)[M+H]⁺=550.2.

### Step 6: Preparation of (R/S)-4-((1-(aminomethyl-d2)cyclobutyl)amino)-2-(4-(5-chloropyrimidin-2-yl)piperidin-1-yl)-6,7-dihydrothieno[3,2-d]pyrimidine 5-oxide:

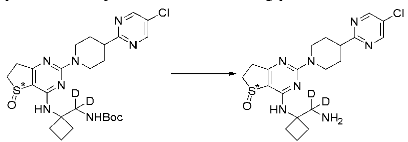

Tert-butyl (R/S)-((1-((2-(4-(5-chloropyrimidin-2-yl)piperidin-1-yl)-5-oxido-6,7-dihydrothieno[3,2-d]pyrimidin-4-yl)amino)cyclobutyl)methyl-d2)carbamate (330mg) was added to a mixed solution of DCM (1mL) and TFA (1mL). The mixture was stirred at room temperature for 1 hour. The reaction mixture was concentrated under a reduced pressure, and separated and purified by Prep-HPLC (C18, 10mmol/L NH₄HCO₃/water, MeCN) to produce the target compound (49mg). LCMS(ESI)[M+H]⁺=450.1; ¹H NMR(400 MHz, DMSO-*d*₆)δ 8.86(s, 2H), 7.43(s, 1H), 4.70(d, *J*=13.4Hz, 2H), 3.48-3.38(m, 2H), 3.26-3.14(m, 3H), 3.06(t, *J*=12.4Hz, 2H), 2.98-2.82(m, 2H), 2.39-2.25(m, 2H), 2.10(t, *J*=9.8Hz, 2H), 1.97(d, *J*=12.9Hz, 2H), 1.83-1.54(m, 4H).

### Example 70 (Compound 105)

### Synthesis of (R/S)-2-(3-(4-(difluoromethoxy)phenyl)-3-methoxyazetidin-1-yl)-4-((1-(hydroxymethyl)cyclobutyl)amino)-6,7-dihydrothieno[3,2-d]pyrimidine 5-oxide:

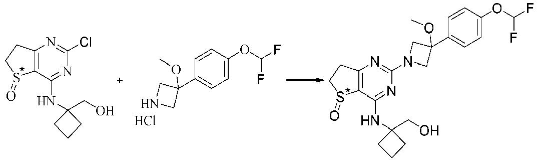

(R/S)-2-chloro-4-((1-(hydroxymethyl)cyclobutyl)amino)-6,7-dihydrothieno[3,2-d]pyrimidine 5-oxide (200mg), 3-(4-(difluoromethoxy)phenyl)-3-methoxyazetidine hydrochloride (370mg), and N,N-diisopropylethylamine (360mg) were added to a mixed solution of H₂O (0.5mL) and tetrahydrofuran (2.5mL). The reaction mixture was stirred at 70°C for 2 hours. The reaction mixture was concentrated under a reduced pressure to produce a crude product. The crude product was separated and purified by Prep-HPLC (C18, 10mmol/L NH₄HCO₃/water, MeCN) to produce the target compound (128mg). LCMS(ESI)[M+H]⁺=481.1; ¹H NMR(400 MHz, DMSO-*d*₆)δ 7.58-7.44(m, 3H), 7.31-7.07(m, 3H), 4.84(t, *J*=5.7Hz, 1H), 4.22(d, *J*=8.5Hz, 1H), 3.72(dd, *J*=5.7, 2.7Hz, 2H), 3.41(dd, *J*=16.9, 8.3Hz, 1H), 3.31(s, 1H), 3.22(dt, *J*=13.6, 8.3Hz, 1H), 3.03(s, 3H), 2.99-2.84(m, 2H), 2.40-2.25(m, 2H), 2.20-2.09(m, 2H), 1.75(dq, *J*=18.9, 10.2Hz, 2H), 1.24(s, 2H).

### Example 71 (Compound 106)

### Synthesis of (R/S)-2-(3-(4-(cyclopropylmethoxy)phenyl)-3-methoxyazetidin-1-yl)-4-((1-(hydroxymethyl)cyclobutyl)amino)-6,7-dihydrothieno[3,2-d]pyrimidine 5-oxide:

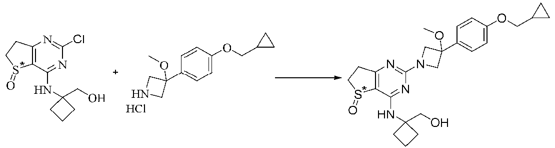

(R/S)-2-chloro-4-((1-(hydroxymethyl)cyclobutyl)amino)-6,7-dihydrothieno[3,2-d]pyrimidine 5-oxide (150mg), 3-(4-(cyclopropylmethoxy)phenyl)-3-methoxyazetidine hydrochloride (240mg), and N,N-diisopropylethylamine (272mg) were added to a mixed solution of H₂O (0.5mL) and tetrahydrofuran (2.5mL). The reaction mixture was stirred at 70°C for 2 hours. The reaction mixture was concentrated under a reduced pressure to produce a crude product. The crude product was separated and purified by Prep-HPLC (C18, 10mmol/L NH₄HCO₃/water, MeCN) to produce the target compound (36mg). LCMS(ESI)[M+H]⁺=485.2; ¹H NMR(400 MHz, DMSO-*d*₆)δ 7.45(s, 1H), 7.38-7.30(m, 2H), 7.00-6.92(m, 2H), 4.85(t, *J*=5.6Hz, 1H), 4.19(s, 2H), 3.83(d, *J*=7.0Hz, 2H), 3.72(dd, *J*=5.5, 2.8Hz, 2H), 3.47-3.38(m, 1H), 3.27-3.16(m, 1H), 2.99(s, 5H), 2.39-2.24(m, 2H), 2.20-2.10(m, 2H), 1.75(dq, *J*=19.3, 10.0Hz, 2H), 1.24(s, 3H), 0.61-0.50(m, 2H), 0.38-0.27(m, 2H).

### Example 72 (Compound 107)

### Synthesis of (R/S)-4-((1-(hydroxymethyl)cyclobutyl)amino)-2-(3-methoxy-3-(4-(pyrrolidin-1-yl)phenyl)azetidin-1-yl)-6,7-dihydrothieno[3,2-d]pyrimidine 5-oxide:

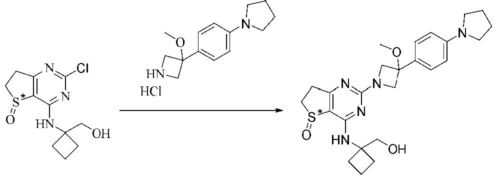

(R/S)-2-chloro-4-((1-(hydroxymethyl)cyclobutyl)amino)-6,7-dihydrothieno[3,2-d]pyrimidine 5-oxide (71mg), 1-(4-(3-methoxyazetidin-3-yl)phenyl)pyrrolidine hydrochloride (80mg), and N,N-diisopropylethylamine (129mg) were added to a mixed solution of water (0.5mL) and tetrahydrofuran (2mL). The reaction mixture was stirred at 70°C for 2 hours. The reaction mixture was added to water (10mL). Then the system was extracted with ethyl acetate (10mL) and separated into phases. The aqueous phase was then extracted with ethyl acetate (5mL) twice. The organic phases were combined, washed with a saturated saline solution (20mL), dried over anhydrous sodium sulfate, filtered, and concentrated to dryness under a reduced pressure to produce a crude product. The crude product was separated and purified by Prep-HPLC (C18, 1 0mmol/L NH₄HCO₃/water, MeCN) to produce the target compound (27mg). LCMS(ESI)[M+H]⁺=484.4; ¹H NMR(400 MHz, DMSO-*d*₆)δ 7.42(s, 1H), 7.29-7.15(m, 2H), 6.56(d, *J*=8.6Hz, 2H), 4.84(t, *J*=5.6Hz, 1H), 4.15(d, *J*=8.9Hz, 4H), 3.80-3.64(m, 2H), 3.44-3.36(m, 1H), 3.25-3.16(m, 5H), 2.95(s, 3H), 2.93-2.81(m, 2H), 2.28(d, *J*=11.2Hz, 2H), 2.15(s, 2H), 2.00-1.89(m, 4H), 1.75(dq, *J*=19.6, 10.4, 9.5Hz, 2H).

### Example 73 (Compound 108)

### Synthesis of (R/S)-2-(3-methoxy-3-(4-methoxyphenyl)azetidin-1-yl)-4-((tetrahydro-2H-pyran-4-yl)amino)-6,7-dihydrothieno[3,2-d]pyrimidine 5-oxide:

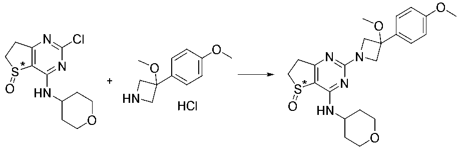

(R/S)-2-chloro-4-((tetrahydro-2H-pyran-4-yl)amino)-6,7-dihydrothieno[3,2-d]pyrimidine 5-oxide (100mg), 3-methoxy-3-(4-methoxyphenyl)azetidine hydrochloride (100mg), and N,N-diisopropylethylamine (180mg) were added to a mixed solution of H₂O (0.5mL) and tetrahydrofuran (2.5mL). The reaction mixture was stirred at 70°C for 2 hours. LCMS detection showed that the starting material was completely consumed. The reaction mixture was concentrated under a reduced pressure to produce a crude product. The crude product was separated and purified by Prep-HPLC (C18, 10mmol/L NH₄HCO₃/water, MeCN) to produce the target compound (20mg). LCMS(ESI)[M+H]⁺=445.2; ¹H NMR(400 MHz, DMSO-*d*₆)δ 7.62(d, *J*=7.6Hz, 1H), 7.43-7.31(m, 2H), 7.04-6.91(m, 2H), 4.23(t, *J*=10.0Hz, 4H), 3.93-3.83(m, 2H), 3.77(s, 3H), 3.47-3.36(m, 2H), 3.31-3.16(m, 3H), 3.00(s, 3H), 2.97-2.85(m, 2H), 1.77(d, *J*=12.4Hz, 2H), 1.61(td, *J*=11.7, 4.4Hz, 2H).

### Example 74 (Compound 109)

### Synthesis of (R/S)-2-(3-(4-(dimethylamino)phenyl)-3-methoxyazetidin-1-yl)-4-((1-(hydroxymethyl)cyclobutyl)amino)-6,7-dihydrothieno[3,2-d]pyrimidine 5-oxide:

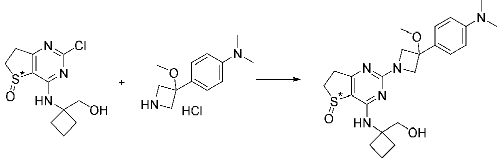

(R/S)-2-chloro-4-((1-(hydroxymethyl)cyclobutyl)amino)-6,7-dihydrothieno[3,2-d]pyrimidine 5-oxide (100mg), 4-(3-methoxyazetidin-3-yl)-N,N-dimethylaniline hydrochloride (130mg), and N,N-diisopropylethylamine (0.18g) were added to a mixed solution of H₂O (0.4mL) and tetrahydrofuran (2mL). The reaction mixture was stirred at 70°C for 1 hour. LCMS detection showed that the starting material was completely consumed. The reaction mixture was concentrated under a reduced pressure to produce a crude product. The crude product was separated and purified by Prep-HPLC (C18, 10mmol/L NH₄HCO₃/water, MeCN) to produce the target compound (82mg). LCMS(ESI)[M+H]⁺=458.2; ¹H NMR(400 MHz, DMSO-*d*₆)δ 7.43(s, 1H), 7.31-7.15(m, 2H), 6.81-6.66(m, 2H), 4.85(t, *J*=5.6Hz, 1H), 4.16(d, *J*=8.7Hz, 4H), 3.71(dd, *J*=5.8, 2.5Hz, 2H), 3.40(dt, *J*=16.2, 7.8Hz, 1H), 3.21(dt, *J*=13.5, 8.2Hz, 1H), 2.93(d, *J*=22.5Hz, 11H), 2.31(dq, *J*=21.1, 10.3Hz, 2H), 2.20-2.09(m, 2H), 1.75(dq, *J*=19.9, 10.0Hz, 2H).

### Example 75 (Compound 110)

### Synthesis of (R/S)-2-(3-(3-(dimethylamino)phenyl)-3-methoxyazetidin-1-yl)-4-((1-(hydroxymethyl)cyclobutyl)amino)-6,7-dihydrothieno[3,2-d]pyrimidine 5-oxide:

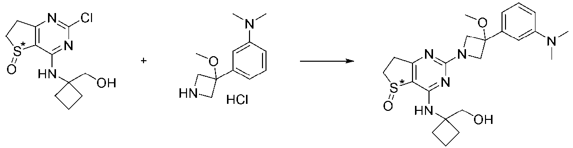

(R/S)-2-chloro-4-((1-(hydroxymethyl)cyclobutyl)amino)-6,7-dihydrothieno[3,2-d]pyrimidine 5-oxide (100mg), 3-(3-methoxyazetidin-3-yl)-N,N-dimethylaniline hydrochloride (211mg), and N,N-diisopropylethylamine (0.18g) were added to a mixed solution of H₂O (0.4mL) and tetrahydrofuran (2mL). The reaction mixture was stirred at 70°C for 2 hours. The reaction mixture was concentrated under a reduced pressure to produce a crude product. The crude product was separated and purified by Prep-HPLC (C18, 10mmol/L NH₄HCO₃/water, MeCN) to produce the target compound (87mg). LCMS(ESI)[M+H]⁺=458.2; ¹H NMR(400 MHz, DMSO-*d*₆)δ 7.45(s, 1H), 7.23(dd, *J*=8.9, 7.4Hz, 1H), 6.71(qd, *J*=4.2, 1.6Hz, 3H), 4.84(t, *J*=5.6Hz, 1H), 4.18(d, *J*=8.7Hz, 4H), 3.71(dd, *J*=5.7, 2.4Hz, 2H), 3.41(dt, *J*=16.4, 7.8Hz, 1H), 3.21(dt, *J*=13.4, 8.3Hz, 1H), 3.02(s, 3H), 2.91(s, 8H), 2.31(dq, *J*=21.6, 10.0Hz, 2H), 2.20-2.11(m, 2H), 1.75(dq, *J*=19.9, 10.4Hz, 2H).

### Example 76 (Compound 111)

### Synthesis of (R/S)-2-(3-(benzo[d][1,3]dioxol-5-yl)-3-methoxyazetidin-1-yl)-4-((1-(hydroxymethyl)cyclobutyl)amino)-6,7-dihydrothieno[3,2-d]pyrimidine 5-oxide:

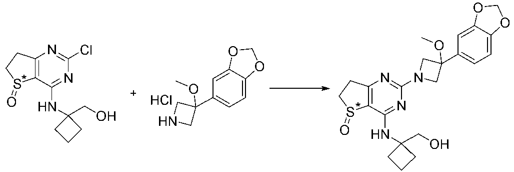

(R/S)-2-chloro-4-((1-(hydroxymethyl)cyclobutyl)amino)-6,7-dihydrothieno[3,2-d]pyrimidine 5-oxide (100mg), 3-(benzo[d][1,3]dioxol-5-yl)-3-methoxyazetidine hydrochloride (130mg), and N,N-diisopropylethylamine (0.18g) were added to a mixed solution of H₂O (0.5mL) and tetrahydrofuran (2.5mL). The reaction mixture was stirred at 70°C for 2 hours. The reaction mixture was concentrated under a reduced pressure to produce a crude product. The crude product was separated and purified by Prep-HPLC (C18, 10mmol/L NH₄HCO₃/water, MeCN) to produce the target compound (40mg). LCMS(ESI)[M+H]⁺=459.1; ¹H NMR(400 MHz, DMSO-*d*₆)δ 7.45(s, 1H), 6.97(dd, *J*=21.6, 1.1Hz, 3H), 6.04(s, 2H), 4.83(t, *J*=5.6Hz, 1H), 4.15(d, *J*=9.4Hz, 4H), 3.71(dd, *J*=5.7, 2.5Hz, 2H), 3.41(dt, *J*=16.5, 7.9Hz, 1H), 3.21(dt, *J*=13.4, 8.3Hz, 1H), 3.03-2.83(m, 5H), 2.30(dt, *J*=20.9, 10.4Hz, 2H), 2.20-2.09(m, 2H), 1.75(dq, *J*=29.0, 10.2Hz, 2H).

### Example 77 (Compound 112)

### Synthesis of (R/S)-4-((1-(hydroxymethyl)cyclobutyl)amino)-2-(3-methoxy-3-(4-(methylamino)phenyl)azetidin-1-yl)-6,7-dihydrothieno[3,2-d]pyrimidine 5-oxide:

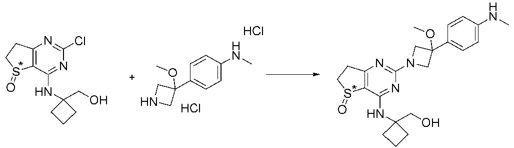

(R/S)-2-chloro-4-((1-(hydroxymethyl)cyclobutyl)amino)-6,7-dihydrothieno[3,2-d]pyrimidine 5-oxide (40mg), 4-(3-methoxyazetidin-3-yl)-N-methylaniline dihydrochloride (80mg), and N,N-diisopropylethylamine (0.07g) were added to a mixed solution of H₂O (0.2mL) and tetrahydrofuran (1mL). The reaction mixture was stirred at 70°C for 2 hours. LCMS detection showed that the starting material was completely consumed. The reaction mixture was concentrated under a reduced pressure to produce a crude product. The crude product was separated and purified by Prep-HPLC (C18, 10mmol/L NH₄HCO₃/water, MeCN) to produce the target compound (35mg). LCMS(ESI)[M+H]⁺=444.2; ¹H NMR(400 MHz, DMSO-*d*₆)δ 7.44(s, 1H), 7.24-7.07(m, 2H), 6.55(d, *J*=8.5Hz, 2H), 5.77(d, *J*=5.1Hz, 1H), 4.85(d, *J*=5.6Hz, 1H), 4.13(d, *J*=9.3Hz, 4H), 3.71(dd, *J*=5.8, 2.7Hz, 2H), 3.40(dt, *J*=17.8, 8.5Hz, 1H), 3.21(dt, *J*=16.2, 8.4Hz, 1H), 2.94(s, 5H), 2.67(d, *J*=5.0Hz, 3H), 2.30(dt, *J*=21.1, 10.3Hz, 2H), 2.14(t, *J*=10.2Hz, 2H), 1.91-1.64(m, 2H).

The following compounds were synthesized with reference to the above relevant examples:

| Compound | Structure | Synthesis Reference Example | LCMS (ESI) [M+H]⁺ | Compound | Structure | Synthesis Reference Example | LCMS (ESI) [M+H]⁺ |
|---|---|---|---|---|---|---|---|
| 6 | 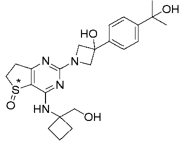 | 1 | 459.2 | 49 | 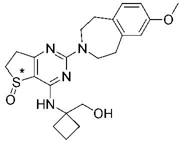 | 12 | 428.9 |
| 9 | 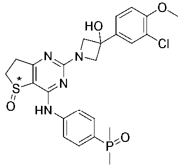 | 1 | 533.3 | 50 | 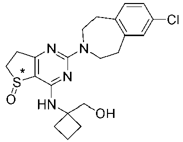 | 12 | 433.1 |
| 32 | 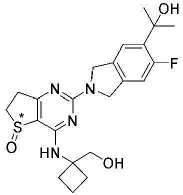 | 19 | 447.2 | 51 | 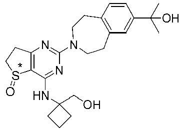 | 12 | 457.0 |
| 33 | 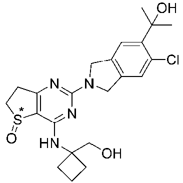 | 19 | 463.3 | 52 | 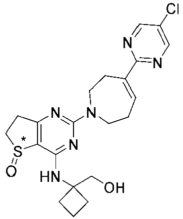 | 9 | 461.1 |
| 34 | 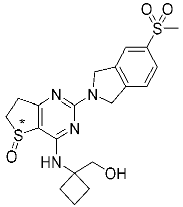 | 12 | 449.1 | 53 | 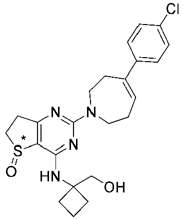 | 9 | 459.2 |
| 36 | 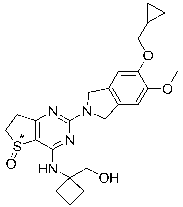 | 12 | 471.4 | 54 | 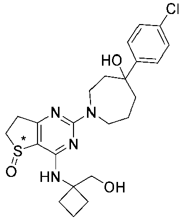 | 1 | 476.8 |
| 37 | 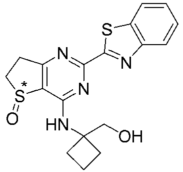 | 20 | 387.1 | 55 | 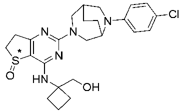 | 12 | 488.2 |
| 38 | 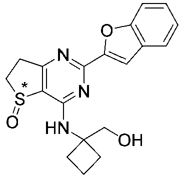 | 20 | 370.0 | 56 | 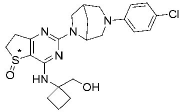 | 12 | 474.0 |
| 39 | 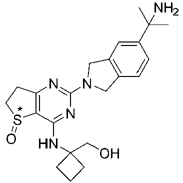 | 12 | 428.2 | 57 | 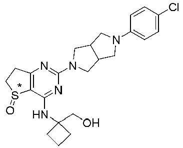 | 12 | 474.0 |
| 41 | 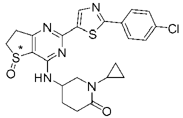 | 20 | 500.1 | 58 | 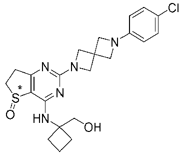 | 12 | 460.1 |
| 42 | 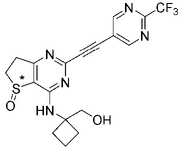 | 20 | 424.2 | 59 | 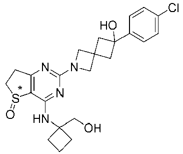 | 1 | 475.3 |
| 43 | 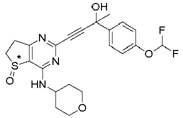 | 20 | 464.2 | 60 | 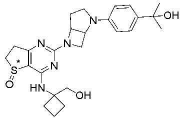 | 12 | 484.0 |
| 44 | 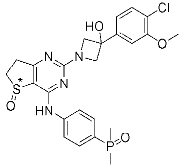 | 1 | 533.2 | 61 | 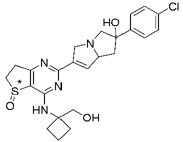 | 10 | 487.0 |
| 45 | 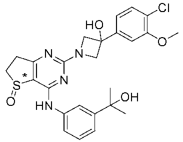 | 1 | 515.1 | 62 | 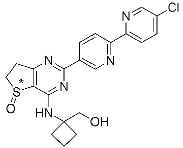 | 20 | 441.8 |
| 46 | 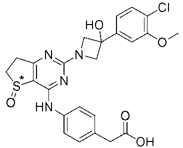 | 1 | 515.2 | 63 | 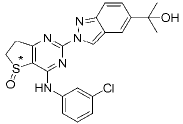 | 20 | 454.0 |
| 47 | 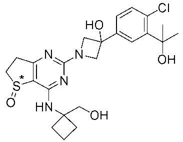 | 1 | 493.0 | 64 | 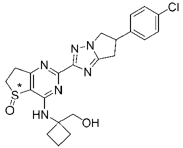 | 20 | 470.9 |
| 48 | 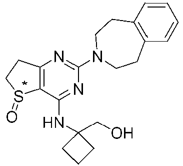 | 12 | 399.4 | 93 | 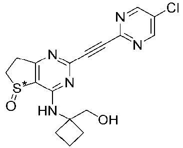 | 20 | 390.2 |

### Biological assay and evaluation

Assay 1: In vitro evaluation of inhibitory activities of the compounds against PDE4B2 and PDE4D2 enzymes
1. Assay materials and instruments:

| Reagents and materials | Suppliers |
|---|---|
| PDE4B2/PDE4D2 | BPS bioscience |
| Cyclic-3',5'-AMP | Sigma |
| Roflumilast | MCE |
| AMP-Glo^{™} detection kit | Promega |
| Tris | amresco |
| Bovine serum albumin | Perkin Elmer |
| MgCl₂ | Sigma |
| Tween-20 | Solarbio |
| DTT | Invitrogen |
| DMSO | Sigma |

| Instruments and consumables | Suppliers |
|---|---|
| 96-well polypropylene plate | Nunc |
| 384-well plate | Greiner |
| Plate shaker | QILINBEIER |
| Centrifuge | Eppendorf |
| Envision multi-label Reader | PerkinElmer |
| Vortex mixer | IKA |
| Echo | Labcyte |

2. Assay procedure:
2.1 Compound preparation and treatment
2.1.1 Preparing stock solutions of compounds in DMSO: Compound powders were dissolved in 100% DMSO to produce 10 mM stock solutions, and oscillated in a shaker until the compounds were completely dissolved.
2.1.2 Storing stock solutions of compounds in DMSO: Stock solutions of compounds in DMSO were stored in a desiccator at room temperature.
2.1.3 Preparing working stock solutions: a) A solution of Roflumilast (reference compound) in DMSO was diluted to 60 µM, and then serially diluted 4-fold into 10 concentration gradients. B) The solutions of the test compounds in DMSO were diluted to 200 µM, and then serially diluted 3-fold. C) A 200X positive control (60 µM Roflumilast) and a 200X vehicle control (100% DMSO) were prepared. D) The assay plate was centrifuged at 1000 rpm for 1 minute.
2.2 Compound testing
   (1) 20 nL of compound dilution solutions were transferred with Echo 550 into each well of the assay plate.
   (2) The assay plate was sealed and centrifuged at 1000 rpm for 1 minute.
   (3) 2X PDE4B2/PDE4D2 was prepared in an ice-cold PDE assay buffer.
   (4) 2 µL of 2X PDE4B2/PDE4D2 was added to each well of the assay plate prepared in step 2.
   (5) The assay plate was sealed and allowed to equilibrate at room temperature for 10 minutes.
   (6) 2X Cyclic-3',5'-AMP was prepared in a PDE assay buffer.
   7) 2µL of 2X Cyclic-3',S'-AMP (prepared in step 6) was added to each well of the assay plate (prepared in step 5) to initiate the reaction. The plate was incubated at room temperature for 60 minutes.
   8) 4µL AMP-Glo Reagent I was added, and the plate was incubated at room temperature for 60 minutes.
   9) 8µL AMP detection reagent was added. The plate was incubated at room temperature for 60 minutes.
   10) The RLU signal was read on an Envision 2105 plate reader.

Assay result:

**Table 1: Assay results of PDE4B2 enzyme inhibitory activity**

| Compound No. | PDE4B2 IC₅₀ (nM) | PDE4D2/PDE4B2 selectivity | Compound No. | PDE4B2 IC₅₀ (nM) | PDE4D2/PDE4B2 selectivity |
|---|---|---|---|---|---|
| Control 1 | D | C | Compound 83 | A | A |
| Compound 1 | B | | Compound 84 | A | |
| Compound 2 | B | | Compound 85 | A | |
| Compound 3 | A | B | Compound 86 | A | B |
| Compound 4 | B | | Compound 87 | B | B |
| Compound 7 | B | | Compound 88 | A | B |
| Compound 8 | B | | Compound 91 | B | |
| Compound 14 | B | B | Compound 92 | A | A |
| Compound 16 | B | | Compound 94 | A | B |
| Compound 25 | B | | Compound 95 | B | |
| Compound 26 | B | | Compound 96 | B | |
| Compound 27 | B | | Compound 97 | A | |
| Compound 29 | A | | Compound 98 | B | |
| Compound 31 | B | | Compound 100 | A | |
| Compound 35 | B | | Compound 105 | A | B |
| Compound 65 | B | | Compound 106 | A | B |
| Compound 79 | A | | Compound 107 | A | B |
| Compound 80 | B | | Compound 108 | A | B |
| Compound 81 | B | | Compound 109 | A | |
| Compound 82 | A | A | Compound 112 | A | |

IC₅₀ value of PDE4B2 enzyme inhibition: A≤2nM; 2nM<B≤5nM; 5nM<C≤20nM; 20nM<D≤50nM, wherein IC₅₀ values of PDE4B2 enzyme inhibition for compounds 79, 82, 83, 86, 88, 92, 94, 100, 105, 107, 108 and 109 were less than 1nm.

PDE4D2/PDE4B2 selectivity (=Ratio of IC₅₀ value of PDE4D2 enzyme inhibition/IC₅₀ value of PDE4B2 enzyme inhibition): 5<A≤10; 2<B≤5; C≤2.

Control 1 was the compound 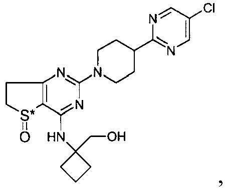 which was disclosed in CN103889970B.

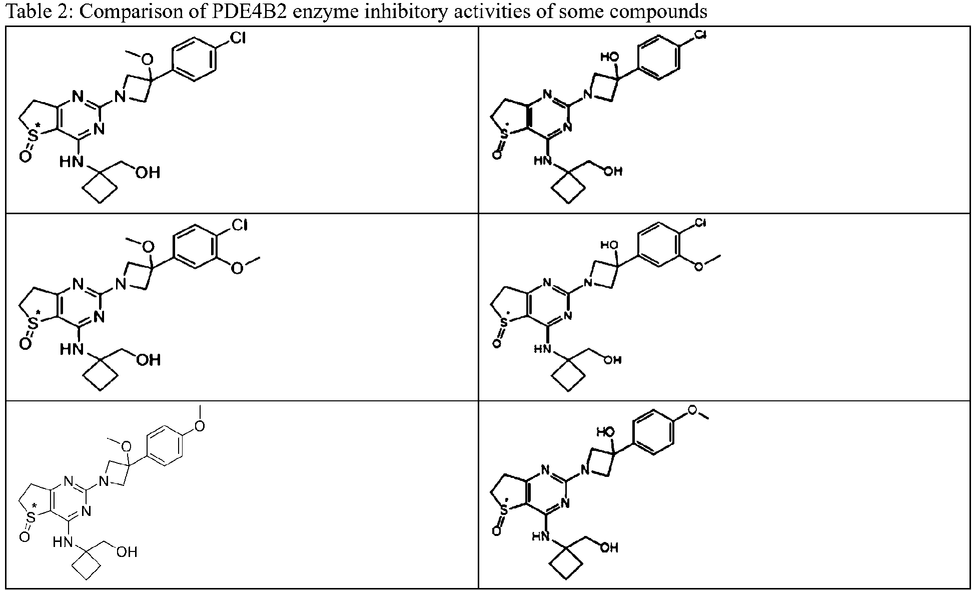

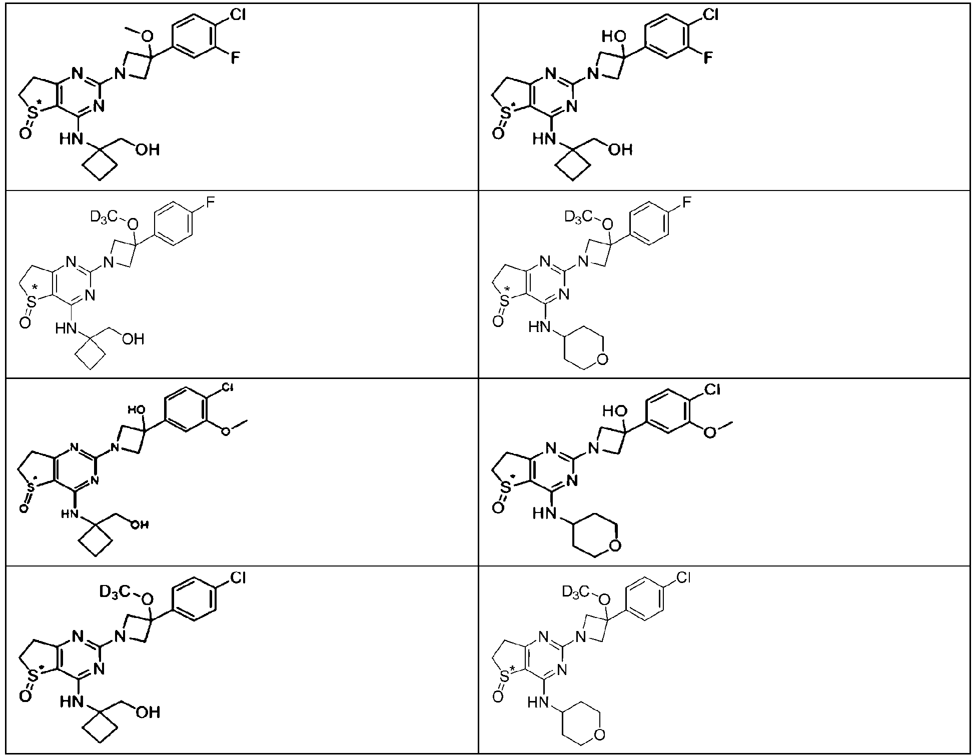

In the above table, each ratio of the PDE4B2 enzyme inhibitory activity of a compound in the left column to that of the corresponding compound in the right column was less than 1.

Assay 2: Pharmacokinetic assay by using LC-MS/MS to determine the compound concentration in mice Assay principle: LC-MS/MS was used to determine the drug concentration of the target drug in plasma at different times, and to plot the pharmacokinetic curve of the target compound in vivo.

### Assay method:

An accurately weighed amount of the test compound was dissolved in 5% DMSO (INNOCHEM, INBZZHCAE) by vortex mixing. Subsequently, 10% Solutol (BASF, 75389809T0) was added and homogenized by vortexing. Finally, 85% saline (Fengyuan, 220505102) was incorporated with vortex mixing to achieve dosing formulations at 0.4 mg/mL and 1.0 mg/mL.

The mice were sourced from CD-1 male mice (JH Laboratory Animal Co. LTD), with three mice for each group. The mice were administered with 2mg/kg intravenously (IV) or 10mg/kg by oral gavage (PO). The blood samples were collected at 5min (IV group only), 15min, 30min, 1h, 2h, 4h, 8h, and 24h (PO group only) after administration. Approximately 30 µL blood samples were collected at each time point and placed into anticoagulant tubes containing EDTA-K2 anticoagulant. Plasma was obtained by centrifugation within 30 minutes. Whole blood samples before centrifugation were placed on wet ice. For each test compound, 42 plasma samples were collected. All collected plasma samples were stored on dry ice or in a freezer until analysis. The drug concentration was determined using a Triple-quadrupole MS system (SCIEX), including standard curve and quality control preparation, and sample preparation.

Standard curve and quality control preparation: a working solution was prepared by ACN:H2O (1:1) dilution, and 3µL of the above standard curve and quality control working solution was added to 57µL of blank plasma. Sample preparation: 7µL of plasma sample was added to 70µL of internal standard solution (Propranolol, Tolbutamide, Glipizide, Osalmid, each 200 ng/mL in ACN). The mixture was mixed for 5 minutes and centrifuged at 4000rpm for 10 minutes. 50µL of supernatant was taken and transferred to a new plate containing 200µL ultrapure water for sample analysis.

Chromatographic conditions were optimized for each sample, regarding mobile phase composition, elution gradient conditions, flow rate, retention time, etc. The chromatographic column was Waters Acquity UPLC HSS T3 1.8 µm, 2.1×50 mm. The injection volume was 2 µL.

An electrospray ionization source was used in mass spectrometry. In the positive ion detection mode, the multichannel reaction monitoring (MRM) mode was selected for secondary mass spectrometry analysis.

Based on the drug concentration-time data, pharmacokinetic parameters were calculated using WinNonlin 8.2 software according to a non-compartmental model, including peak concentration Cₘₐₓ, peak time Tₘₐₓ, area under the drug-time curve AUC, and elimination half-life t_{1/2}. The AUC was calculated using the linear trapezoidal method (linear up log down).

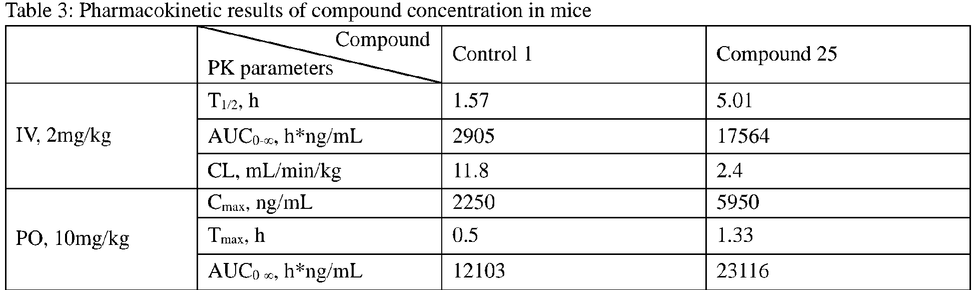

The assay results showed that the compound of the present invention had good in vivo pharmacokinetics and had the potential to become drugs.

Assay 3: Test of the inhibition of compounds on LPS-induced TNF-α secretion from human PBMC

Frozen hPBMC cell stock solution was rapidly thawed in a 37°C water bath. The cells were transferred to complete medium (RPMI 1640+10% FBS+1% P/S). The suspension was centrifuged at 1000 rpm for 5 minutes. The supernatant was discarded, and the cells were resuspended in 1 mL complete medium (RPMI 1640 + 10% FBS + 1% P/S). After gentle pipetting to mix, the cells were counted using trypan blue staining. Cells were seeded into a 96-well cell culture plate at 5×10⁴ cells/well, with 100 µL/well of complete medium. Test compounds were prepared at 4×the final concentration and added to the cells at 50 µL/well, followed by a 30-minute pre-incubation. Control wells without compound were simultaneously set up. LPS was prepared as a 4×solution to achieve a final stimulation concentration of 10 ng/mL and added to the cells at 50µL/well. Control wells without LPS were simultaneously set up. The incubation of cells was continued and 10% of supernatant was collected for detection after 24 hours. The collected supernatant was detected according to the Human TNF-α kit of R&D Company (VAL105G). The inhibitory activities of the compounds of the present invention on LPS-induced TNF-α secretion from human PBMC were determined according to the above method.

**Table 4: Test results for inhibitory activities of the test compounds on LPS-induced TNF-α secretion from human PBMC**

| Compound No. | TNF-α secretion IC₅₀ (nM) |
|---|---|
| Control 1 | 146 |
| Compound 83 | 1.355 |

The experimental results showed that the compound of the present invention had excellent inhibitory activity on human PBMC secretion of TNFα, could better inhibit the secretion of inflammatory factor TNFα from human PBMC, and had good anti-inflammatory effect.

## Claims

1. A compound represented by formula (I), a stereoisomer or tautomer of said compound, a mixture thereof, or a pharmaceutically acceptable salt of said compound: 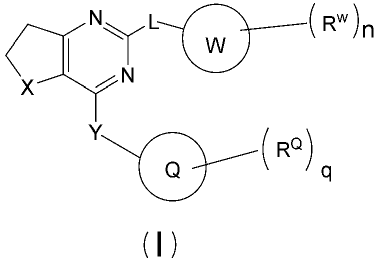 wherein, X is selected from S, SO, and SO₂;
Y is NR^{Y};
R^{Y} is hydrogen or deuterium;
ring Q is selected from C₃₋₈cycloalkyl, C₄₋₈cycloalkenyl, 3-10 membered heterocyclyl, 5-6 membered heteroaryl, and C₆₋₁₂ aryl;
each R^{Q} is selected from halogen, oxo, formyl, acetyl, methylsulfonyl, ethylsulfonyl, methylsulfonamido, ethylsulfonamido, phosphate, carboxylate, -CN, -OH, -SH, -NO₂, -NH₂, -CONH₂, 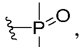 -L^{Y}-OH, -L^{Y}-SH, - L^{Y}-C(O)OH, -L^{Y}-NH-C(O)H, -L^{Y}-C(O)NH₂, -L^{Y}-NHC(O)OH, -L^{Y}-C(O)H, 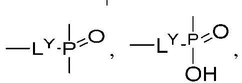 C₁₋₆alkyl, C₂₋₆alkenyl, C₂₋₆alkynyl, C₁₋₆alkoxyl, C₁₋₆alkylthio, C₃₋₆cycloalkyl, C₄₋₆cycloalkenyl, 3-6 membered heterocyclyl, haloC₁₋₆alkyl (e.g. haloC₁₋₃alkyl), -L^{Y}-OC(O)R^{d}, and -L^{Y}-OC(O)OR^{d}; preferably, each R^{Q} is selected from halogen, oxo, formyl, acetyl, methylsulfonyl, ethylsulfonyl, methylsulfonamido, ethylsulfonamido, phosphate, carboxylate, -CN, -OH, -SH, -NO₂, -NH₂, -CONH₂, 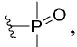 -L^{Y}-OH, -L^{Y}-SH, -L^{Y}-C(O)OH, -L^{Y}-NH-C(O)H, -L^{Y}-C(O)NH₂, -L^{Y}-NHC(O)OH, -L^{Y}-C(O)H, 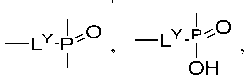 C₁₋₆alkyl, C₂₋₆alkenyl, C₂₋₆alkynyl, C₁₋₆alkoxyl, C₁₋₆alkylthio, C₃₋₆cycloalkyl, C₄₋₆cycloalkenyl, 3-6 membered heterocyclyl, haloC₁₋₆alkyl (e.g. haloC₁₋₃alkyl), and - L^{Y}-OC(O)R^{d}; more preferably, each R^{Q} is selected from halogen, oxo, formyl, acetyl, methylsulfonyl, ethylsulfonyl, methylsulfonamido, ethylsulfonamido, phosphate, carboxylate, -CN, -OH, -SH, -NO₂, -NH₂, - CONH₂, 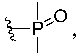 -L^{Y}-OH, -L^{Y}-SH, -L^{Y}-C(O)OH, -L^{Y}-NH-C(O)H, -L^{Y}-C(O)NH₂, -L^{Y}-NHC(O)OH, -L^{Y}-C(O)H, 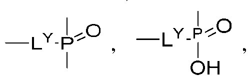 C₁₋₆alkyl, C₂₋₆alkenyl, C₂₋₆alkynyl, C₁₋₆alkoxyl, C₁₋₆alkylthio, C₃₋₆cycloalkyl, C₄₋₆cycloalkenyl, 3-6 membered heterocyclyl, and haloC₁₋₆alkyl (e.g. haloC₁₋₃alkyl);
L^{Y} is optionally substituted and selected from C₁₋₄alkylene, C₂₋₆alkenylene, C₂₋₆alkynylene, and C₁₋₄alkyleneoxy, wherein "optionally substituted" refers to being optionally substituted by one or more substituents selected from halogen, oxo, -CN, -OH, -SH, -NO₂, -NH₂, C₁₋₃alkyl, and haloC₁₋₃alkyl;
L is a bond or an optionally substituted group selected from C₁₋₄alkylene, C₂₋₄alkenylene, C₂₋₄alkynylene, and C₁₋₄alkyleneoxy, wherein "optionally substituted" refers to being optionally substituted by one or more substituents selected from deuterium, halogen, oxo, -CN, -OH, -SH, -NO₂, -NH₂, C₁₋₆alkyl, C₂₋₆alkenyl, C₂₋₆alkynyl, C₁₋₆alkoxyl, C₁₋₆alkylthio, C₃₋₈cycloalkyl, and haloC₁₋₆alkyl;
ring W is C₄₋₁₆cycloalkyl, C₄₋₁₆cycloalkenyl, saturated or partially unsaturated 4-18 membered heterocyclyl, 5-16 membered heteroaryl, or C₆₋₁₈ aryl;
each R^{W} is independently selected from halogen, oxo, formyl, acetyl, methylsulfonyl, ethylsulfonyl, methylsulfonamido, ethylsulfonamido, acetylamino, -CN, -OH, -SH, -NO₂, -NH₂, -CONH₂, -Z-(R^{Z})ₘ, -NR^{b}R^{c}, - C(O)OR^{d}, and an optionally substituted group selected from C₁₋₆alkyl, C₂₋₆alkenyl, C₂₋₆alkynyl, C₁₋₆alkoxyl, and C₁₋₆alkylthio, wherein "optionally substituted" refers to being optionally substituted by one or more substituents selected from deuterium, halogen, oxo, oxime, -CN, -OH, -SH, -NO₂, -NH₂, -C(O)OR^{d}, C₁₋₆alkyl, C₂₋₆alkenyl, C₂₋₆alkynyl, C₁₋₆alkoxyl, C₁₋₆alkylthio, C₃₋₈cycloalkyl, C₃₋₈cycloalkenyl, 3-8 membered heterocyclyl, 5-6 membered heteroaryl, C₆₋₁₂ aryl, haloC₁₋₆alkyl, haloC₁₋₆alkoxyl, halophenyl, and -O-C₃₋₆cycloalkyl; R^{b} and R^{c} are each independently selected from H, C₁₋₆alkyl, C₂₋₆alkenyl, C₁₋₆hydroxyalkyl, and haloC₁₋₆alkyl; R^{d} is selected from H, C₁₋₆alkyl, C₁₋₆hydroxyalkyl, haloC₁₋₆alkyl, and C₁₋₆alkylamino; preferably, each R^{W} is independently selected from halogen, oxo, formyl, acetyl, methylsulfonyl, ethylsulfonyl, methylsulfonamido, ethylsulfonamido, acetylamino, -CN, -OH, -SH, -NO₂, -NH₂, -CONH₂, -Z-(R^{Z})ₘ, -NR^{b}R^{c}, -C(O)OR^{d}, and an optionally substituted group selected from C₁₋₆alkyl, C₂₋₆alkenyl, C₂₋₆alkynyl, C₁₋₆alkoxyl, and C₁₋₆alkylthio, wherein "optionally substituted" refers to being optionally substituted by one or more substituents selected from deuterium, halogen, oxo, oxime, -CN, -OH, -SH, -NO₂, -NH₂, -C(O)OR^{d}, C₁₋₆alkyl, C₂₋₆alkenyl, C₂₋₆alkynyl, C₁₋₆alkoxyl, C₁₋₆alkylthio, C₃₋₈cycloalkyl, C₃₋₈cycloalkenyl, 3-8 membered heterocyclyl, 5-6 membered heteroaryl, C₆₋₁₂ aryl, haloC₁₋₆alkyl, haloC₁₋₆alkoxyl, halophenyl, and -O-C₃₋₆cycloalkyl; R^{b} and R^{c} are each independently selected from H, C₁₋₆alkyl, C₂₋₆alkenyl, C₁₋₆hydroxyalkyl, and haloC₁₋₆alkyl; R^{d} is selected from H, C₁₋₆alkyl, C₁₋₆hydroxyalkyl, and haloC₁₋₆alkyl; more preferably, each R^{W} is independently selected from halogen, oxo, formyl, acetyl, methylsulfonyl, ethylsulfonyl, methylsulfonamido, ethylsulfonamido, -CN, -OH, -SH, -NO₂, -NH₂, -CONH₂, -Z-(R^{Z})ₘ, and an optionally substituted group selected from C₁₋₆alkyl, C₂₋₆alkenyl, C₂₋₆alkynyl, C₁₋₆alkoxyl, and C₁₋₆alkylthio, wherein "optionally substituted" refers to being optionally substituted by one or more substituents selected from halogen, oxo, oxime, -CN, -OH, -SH, -NO₂, -NH₂, C₁₋₆alkyl, C₂₋₆alkenyl, C₂₋₆alkynyl, C₁₋₆alkoxyl, C₁₋₆alkylthio, C₃₋₈cycloalkyl, C₃₋₈cycloalkenyl, 3-8 membered heterocyclyl, 5-6 membered heteroaryl, C₆₋₁₂ aryl, haloC₁₋₆alkyl, haloC₁₋₆alkoxyl, halophenyl, and -O-C₃₋₆cycloalkyl;
Z is C₄₋₁₆cycloalkyl, C₄₋₁₆cycloalkenyl, saturated or partially unsaturated 4-18 membered heterocyclyl, 5-16 membered heteroaryl, or C₆₋₁₂ aryl;
R^{Z} is each independently selected from halogen, -CN, -OH, -SH, -NO₂, -NH₂, oxo, C₁₋₆alkyl, C₂₋₆alkenyl, C₂₋₆alkynyl, C₁₋₆alkoxyl, C₁₋₆hydroxyalkyl, haloC₁₋₆alkyl, haloC₁₋₆alkoxyl, saturated or partially unsaturated 4-6 membered heterocyclyl, -OR^{Z1}, and -NR^{Z2}R^{Z3}; R^{Z1} is selected from C₁₋₆alkyl and haloC₁₋₆alkyl; R^{Z2} and R^{Z3} are each independently selected from H and C₁₋₆alkyl; preferably, R^{Z} is each independently selected from halogen, - CN, -OH, -SH, -NO₂, -NH₂, oxo, C₁₋₆alkyl, C₂₋₆alkenyl, C₂₋₆alkynyl, C₁₋₆alkoxyl, C₁₋₆hydroxyalkyl, haloC₁₋₆alkyl, and haloC₁₋₆alkoxyl; more preferably, R^{Z} is each independently selected from halogen, -CN, -OH, -SH, -NO₂, - NH₂, C₁₋₆alkyl, C₂₋₆alkenyl, C₂₋₆alkynyl, C₁₋₆alkoxyl, C₁₋₆hydroxyalkyl, haloC₁₋₆alkyl, and haloC₁₋₆alkoxyl; n is 0, 1, 2, 3, 4 or 5;
q is 0, 1, 2, 3, 4 or 5;
m is 0, 1, 2, 3, 4 or 5;
provided that:
(1) when L is a bond, 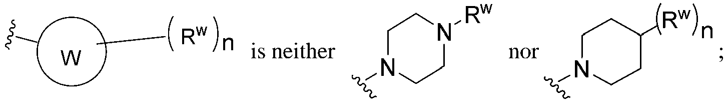
(2) when L is a bond, and 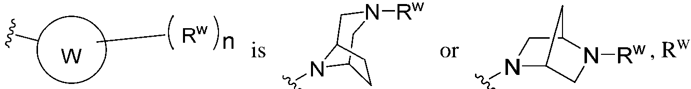 is neither a substituted phenyl nor 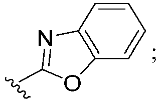
(3) when X is SO, and L is a bond, 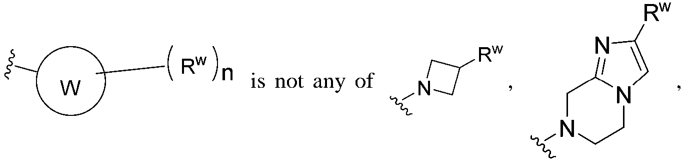 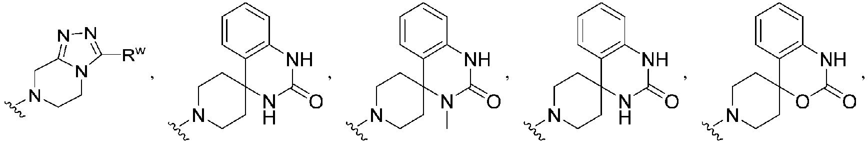 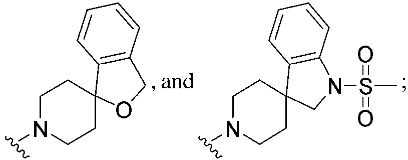 preferably, when X is SO, and L is a bond, 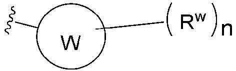 is not any of 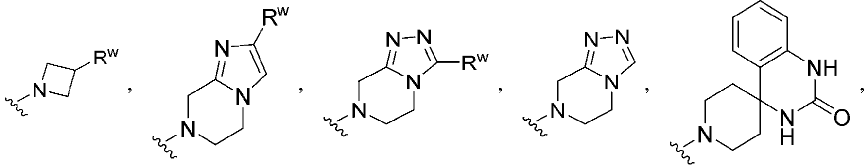 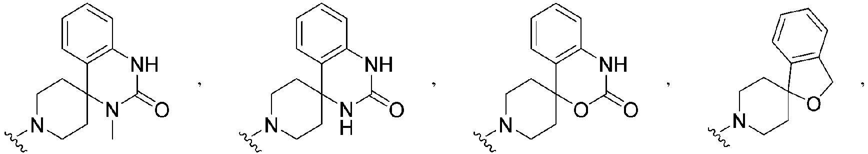 and 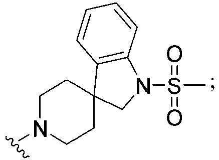
(4) when X is S or SO₂, and L is a bond, ring Q and ring W are not a substituted phenyl at the same time; or when X is S, L is a bond, and ring Q is phenyl, ring W is not 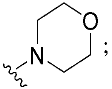
(5) when X is S or SO₂, and L is a bond, ring Q is not 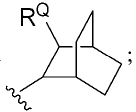 The heteroatoms in the heterocyclyl and heteroaryl are independently selected from O, N and S, and the number of heteroatoms is preferably 1, 2 or 3.

2. The compound of claim 1, a stereoisomer or tautomer of said compound, a mixture thereof, or a pharmaceutically acceptable salt of said compound, wherein the compound is a compound represented by any of formulae (I-A), (I-B) and (I-C): 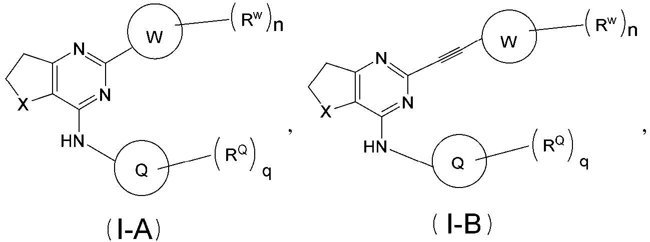 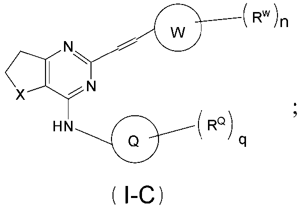 wherein, X, q, R^{Q}, ring Q, n, R^{W}, and ring W are as defined in claim 1.

3. The compound of claim 1, a stereoisomer or tautomer of said compound, a mixture thereof, or a pharmaceutically acceptable salt of said compound, wherein the compound is a compound represented by any of formulae (II-A) to (II-E): 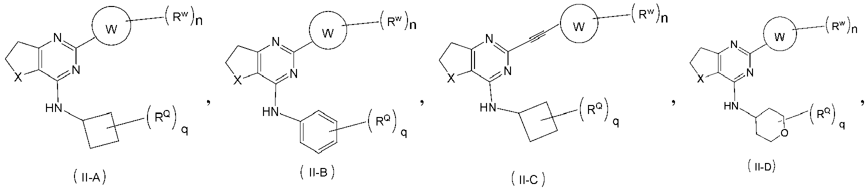 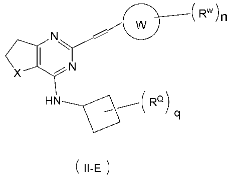 wherein X, n, R^{W}, ring W, R^{Q}, and q are as defined in claim 1.

4. The compound of claim 1, a stereoisomer or tautomer of said compound, a mixture thereof, or a pharmaceutically acceptable salt of said compound, wherein the compound is a compound represented by any of formulae (III-A) to (III-H): 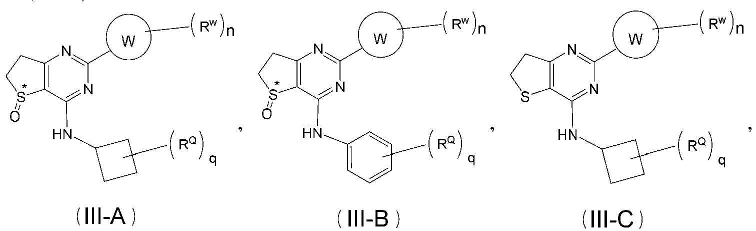 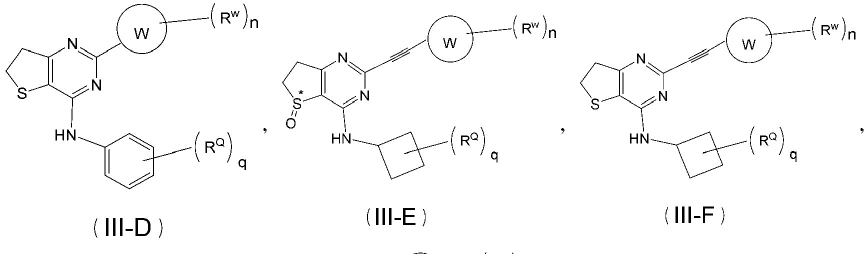 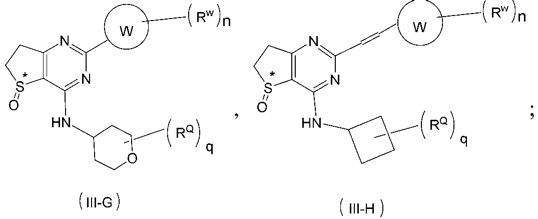 wherein, n, R^{W}, ring W, R^{Q}, and q are as defined in claim 1, S* is a chiral sulfur atom, preferably in R configuration.

5. The compound of claim 1, a stereoisomer or tautomer of said compound, a mixture thereof, or a pharmaceutically acceptable salt of said compound, wherein the compound is a compound represented by any of formulae (IV-A) to (IV-E): 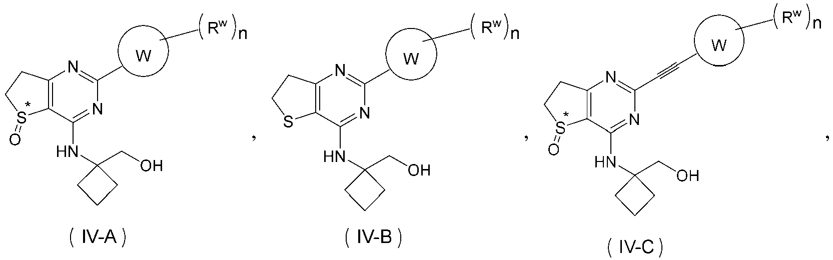 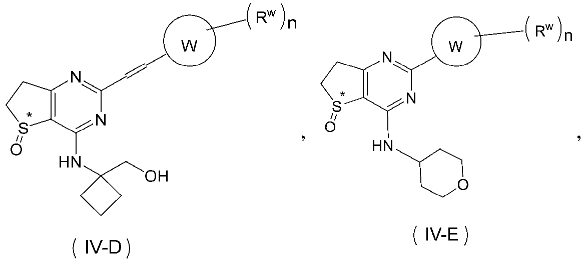 wherein, n, R^{W}, and ring W are as defined in claim 1, S* is a chiral sulfur atom, preferably in R configuration.

6. The compound according to any one of claims 1-5, a stereoisomer or tautomer of said compound, a mixture thereof, or a pharmaceutically acceptable salt of said compound, wherein
R^{Y} is deuterium; or R^{Y} is hydrogen; and/or
ring Q is selected from C₄₋₆cycloalkyl, C₄₋₆cycloalkenyl, 3-8 membered heterocyclyl, 5-6 membered heteroaryl, and phenyl;
preferably, ring Q is selected from C₄₋₆cycloalkyl, 3-6 membered heterocyclyl, and phenyl;
more preferably, ring Q is selected from cyclobutyl, cyclohexenyl, tetrahydrofuranyl, tetrahydropyrrolyl, piperidinyl, piperazinyl, phenyl, and tetrahydropyranyl;
most preferably, ring Q is phenyl or tetrahydropyranyl; and/or
each R^{Q} is independently selected from halogen, oxo, formyl, acetyl, methylsulfonyl, ethylsulfonyl, methylsulfonamido, ethylsulfonamido, phosphate, carboxylate, -CN, -OH, -SH, -NO₂, -NH₂, -CONH₂, 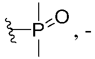 L^{Y}-OH, -L^{Y}-C(O)OH, -L^{Y}-C(O)H, 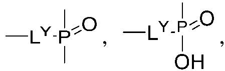 C₁₋₃alkyl, C₂₋₄alkenyl, C₂₋₄alkynyl, C₁₋₃alkoxyl, C₁₋₃alkylthio, C₃₋₆cycloalkyl, C₃₋₆cycloalkenyl, 3-6 membered heterocyclyl, haloC₁₋₃alkyl, and -L^{Y}-OC(O)R^{d};
preferably, each R^{Q} is independently selected from halogen, oxo, acetyl, 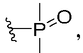 -L^{Y}-OH, -L^{Y}-C(O)OH, C₁₋₃alkyl, C₃₋₆cycloalkyl, 3-6 membered heterocyclyl, and -L^{Y}-OC(O)R^{d};
more preferably, each R^{Q} is independently selected from halogen, oxo, acetyl, 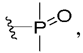 -L^{Y}-OH, -L^{Y}-C(O)OH, Cₗ₃alkyl, C₃₋₆cycloalkyl, and -L^{Y}-OC(O)R^{d};
most preferably, each R^{Q} is independently selected from hydroxymethyl, carboxymethyl, acetyl, 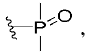 hydroxyisopropyl, Cl, oxo, cyclopropyl, and 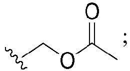 and/or
L^{Y} is optionally substituted and selected from C₁₋₄alkylene, wherein "optionally substituted" refers to being optionally substituted by one or more substituents selected from halogen, oxo, -CN, -OH, -SH, -NO₂, -NH₂, C₁₋₃alkyl, and haloC₁₋₃alkyl;
preferably, L^{Y} is optionally substituted and selected from C₁₋₂alkylene, wherein "optionally substituted" refers to being optionally substituted by one or more substituents selected from halogen, -OH, -SH, -NH₂, C₁₋₃alkyl, and haloC₁₋₃alkyl;
more preferably, L^{Y} is optionally substituted and selected from methylene and ethylene, wherein "optionally substituted" refers to being optionally substituted by one or more substituents selected from halogen, -OH, -SH, methyl, and ethyl; and/or
L is a bond or an optionally substituted group selected from: C₁₋₃alkylene, C₂₋₄alkenylene, and C₂₋₄alkynylene, wherein "optionally substituted" refers to being optionally substituted by one or more substituents selected from deuterium, halogen, oxo, -CN, -OH, -SH, -NH₂, C₁₋₃alkyl, C₂₋₄alkenyl, C₂₋₄alkynyl, C₁₋₃alkoxyl, C₁₋₃alkylthio, C₃₋₈cycloalkyl, and haloC₁₋₃alkyl;
preferably, L is a bond or an optionally substituted group selected from: C₂₋₄alkenylene and C₂₋₄alkynylene, wherein "optionally substituted" refers to being optionally substituted by one or more substituents selected from deuterium, -CN, -OH, -SH, -NH₂, and C₁₋₃alkyl;
more preferably, L is a bond or an optionally substituted group selected from: ethenyl, propenyl, ethynyl, and propynyl, wherein "optionally substituted" refers to being optionally substituted by one or more substituents selected from deuterium, -CN, -OH, -SH, -NH₂, and C₁₋₃alkyl;
most preferably, L is a bond; or L is ethynyl; or L is an optionally substituted propynyl, wherein "optionally substituted" refers to being optionally substituted by one or more substituents selected from deuterium, -CN, -OH, -SH, -NH₂, and C₁₋₃alkyl; and/or
ring W is C₄₋₁₂cycloalkyl, C₄₋₁₂cycloalkenyl, saturated or partially unsaturated 4-12 membered heterocyclyl, 5-16 membered heteroaryl, or C₆₋₁₂ aryl;
preferably, ring W is C₄₋₅monocyclic cycloalkyl, C₅₋₁₀bicyclic cycloalkyl, C₄₋₈ monocyclic cycloalkenyl, C₇₋₁₀bicyclic cycloalkenyl, saturated or partially unsaturated 4-8 membered monocyclic heterocyclyl, saturated or partially unsaturated 7-12 membered bicyclic heterocyclyl, 5-6 membered monocyclic heteroaryl, 9-16 membered bicyclic heteroaryl, phenyl, or naphthyl;
more preferably, ring W is 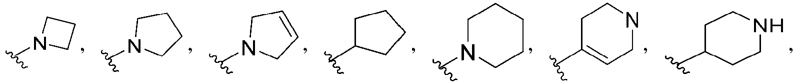 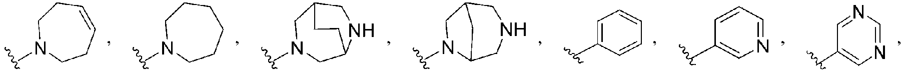 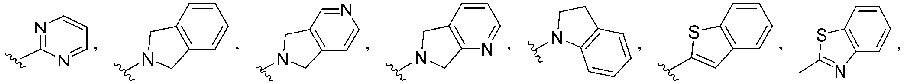 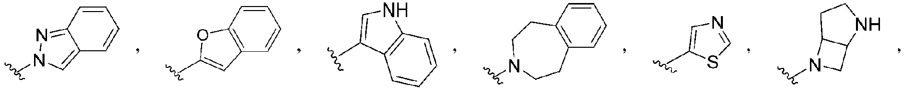 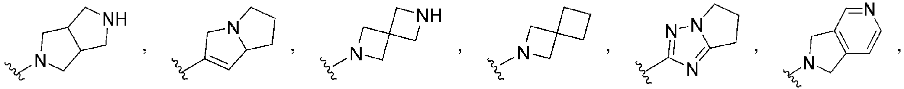 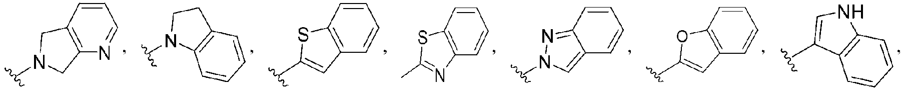 or

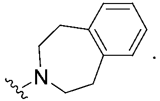

7. The compound according to any one of claims 1-6, a stereoisomer or tautomer of said compound, a mixture thereof, or a pharmaceutically acceptable salt of said compound, wherein 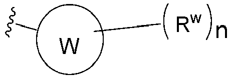 is 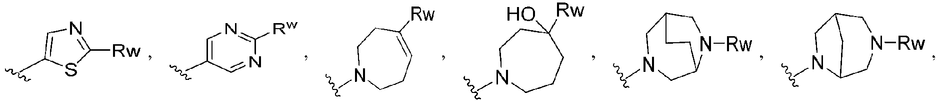 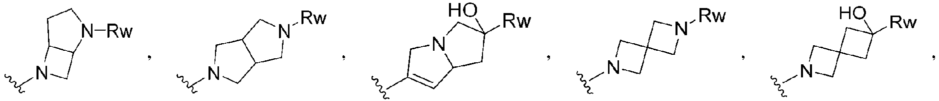 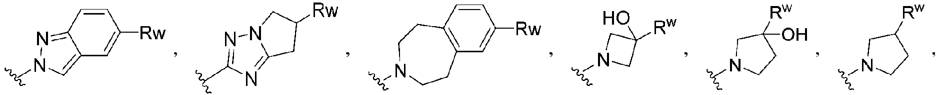 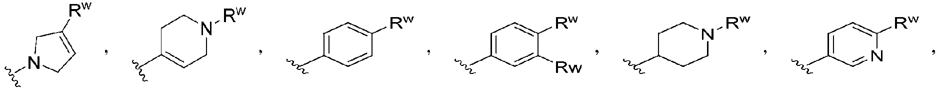 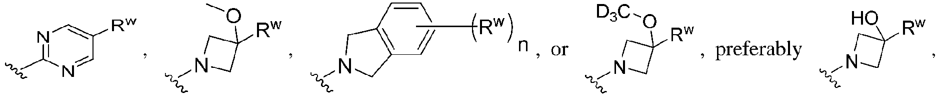 or 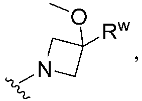 more preferably

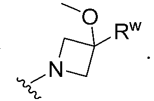

8. The compound according to any one of claims 1-7, a stereoisomer or tautomer of said compound, a mixture thereof, or a pharmaceutically acceptable salt of said compound, wherein
each R^{W} is independently selected from halogen, oxo, formyl, acetyl, methylsulfonyl, ethylsulfonyl, methylsulfonamido, ethylsulfonamido, -CN, -OH, -SH, -NO₂, -NH₂, -CONH₂, -Z-(R^{Z})ₘ, an optionally substituted C₁₋₆alkyl, and an optionally substituted C₁₋₆alkoxyl, wherein "optionally substituted" refers to being optionally substituted by one or more substituents selected from halogen, oxo, oxime, -CN, -OH, -SH, -NO₂, -NH₂, C₁₋₆alkyl, C₂₋₆alkenyl, C₂₋₆alkynyl, C₁₋₆alkoxyl, C₁₋₆alkylthio, C₃₋₈cycloalkyl, C₃₋₈cycloalkenyl, 3-8 membered heterocyclyl, 5-6 membered heteroaryl, C₆₋₁₂ aryl, haloC₁₋₆alkyl, haloC₁₋₆alkoxyl, halophenyl, and -O-C₃₋₆cycloalkyl;
Z is C₄₋₁₀cycloalkyl, C₄₋₁₀cycloalkenyl, saturated or partially unsaturated 4-12 membered heterocyclyl, 5-12 membered heteroaryl, or C₆₋₁₂ aryl;
R^{Z} is each independently selected from halogen, -CN, -OH, -SH, -NO₂, -NH₂, C₁₋₃alkyl, C₂₋₆alkenyl, C₂₋₆alkynyl, C₁₋₃alkoxyl, C₁₋₃hydroxyalkyl, haloC₁₋₃alkyl, and haloC₁₋₃alkoxyl;
preferably, each R^{W} is independently selected from halogen, formyl, acetyl, methylsulfonyl, ethylsulfonyl, methylsulfonamido, ethylsulfonamido, -CN, -OH, -SH, -NH₂, -Z-(R^{Z})ₘ, an optionally substituted C₁₋₄alkyl, and an optionally substituted C₁₋₄alkoxyl, wherein "optionally substituted" refers to being optionally substituted by one or more substituents selected from halogen, -CN, -OH, -SH, -NO₂, -NH₂, C₁₋₃alkyl, C₁₋₃alkoxyl, C₃₋₆cycloalkyl, 3-8 membered heterocyclyl, 5-6 membered heteroaryl, C₆₋₁₂ aryl, haloC₁₋₃alkyl, haloC₁₋₃alkoxyl, and halophenyl;
Z is pyridinyl, pyridazinyl, pyrimidinyl, pyrazinyl, 1,3,5-triazinyl, or phenyl;
R^{Z} is each independently selected from halogen, -CN, -OH, -SH, -NO₂, -NH₂, C₁₋₃alkyl, C₁₋₃alkoxyl, C₁₋₃hydroxyalkyl, haloC₁₋₃alkyl, and haloC₁₋₃alkoxyl.

9. The compound of claim 1, a stereoisomer or tautomer of said compound, a mixture thereof, or a pharmaceutically acceptable salt of said compound, wherein the compound is a compound represented by formula (IV-A) or (IV-E), preferably the compound is a compound represented by formula (IV-A): 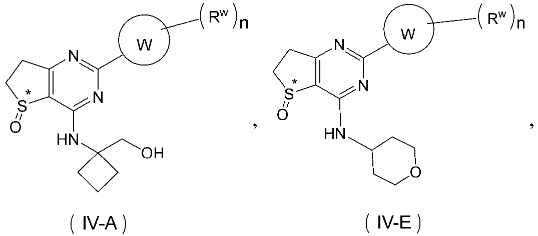 wherein 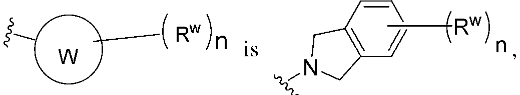 each R^{W} is independently selected from methylsulfonyl, methylsulfonamido, ethylsulfonamido, F, Cl, -CN, -OH, -SH, -NH₂, 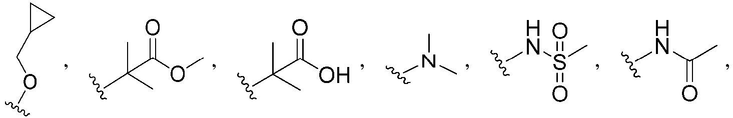 and an optionally substituted group selected from: methyl, ethyl, iso-propyl, methoxy, ethoxy, methylthio, and ethylthio, wherein "optionally substituted" refers to being optionally substituted by one or more substituents selected from halogen, -CN, -OH, -NH₂, C₁₋₃alkyl, C₁₋₃alkoxyl, C₁₋₃haloalkyl, and C₁₋₃haloalkoxyl, preferably each R^{W} is independently selected from methylsulfonyl, methylsulfonamido, ethylsulfonamido, F, Cl, -CN, -OH, -SH, -NH₂,

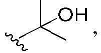

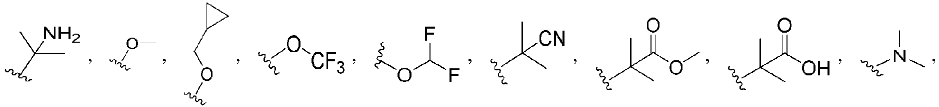

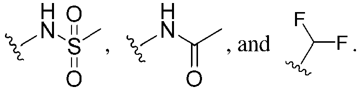

10. The compound of claim 1, a stereoisomer or tautomer of said compound, a mixture thereof, or a pharmaceutically acceptable salt of said compound, wherein the compound is a compound represented by formula (IV-A) or (IV-E), preferably the compound is a compound represented by formula (IV-A): 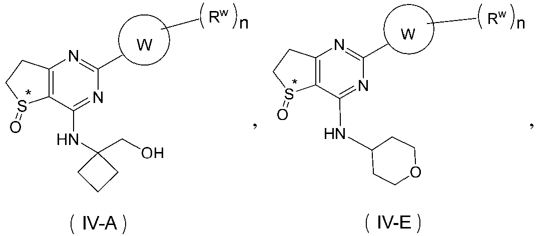 wherein 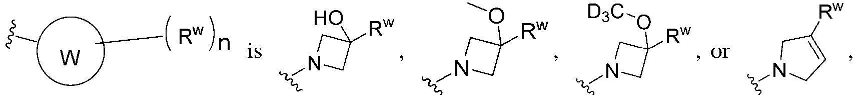 preferably 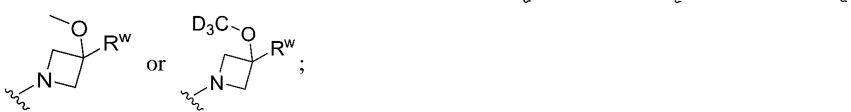 each R^{W} is independently selected from 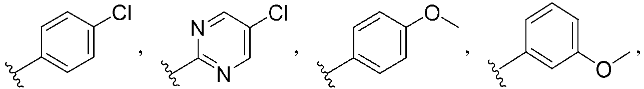 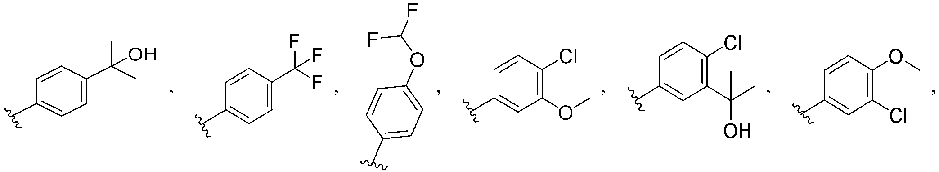 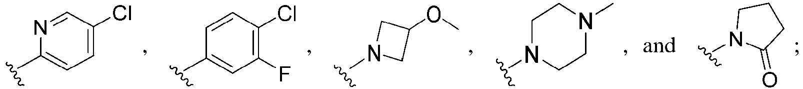 preferably, each R^{W} is independently selected from

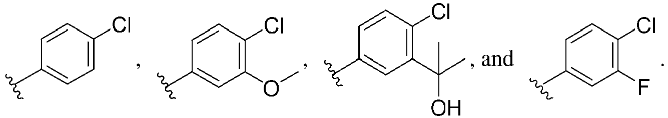

11. The compound according to any one of claims 1-9, a stereoisomer or tautomer of said compound, a mixture thereof, or a pharmaceutically acceptable salt of said compound, wherein
m is 0, 1, 2 or 3;
preferably, m is 1, 2 or 3;
more preferably, m is 1 or 2; and/or
n is 0, 1, 2 or 3;
preferably, n is 0, 1 or 2;
more preferably, n is 1 or 2; and/or
q is 0, 1, 2 or 3;
preferably, q is 0, 1 or 2;
more preferably, q is 1 or 2.

12. A compound, a stereoisomer or tautomer of said compound, a mixture thereof, or a pharmaceutically acceptable salt of said compound, wherein said compound is selected from:
| 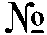 | Structure | 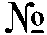 | Structure | 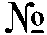 | Structure | 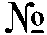 | Structure |
|---|---|---|---|---|---|---|---|
| 1 | 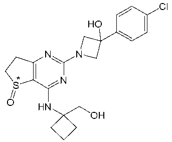 | 2 | 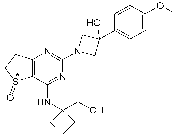 | 3 | 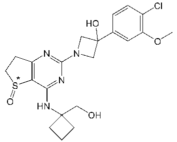 | 4 | 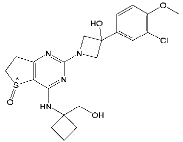 |
| 5 | 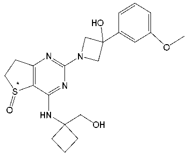 | 6 | 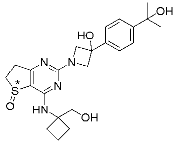 | 7 | 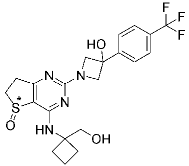 | 8 | 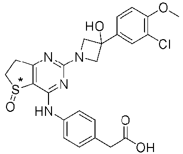 |
| 9 | 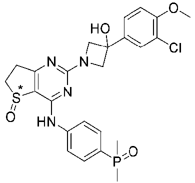 | 10 | 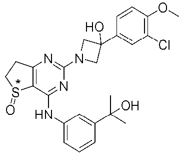 | 11 | 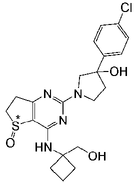 | 12 | 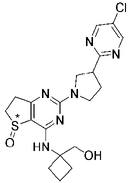 |
| 13 | 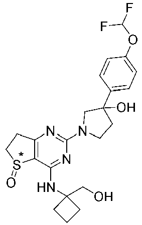 | 14 | 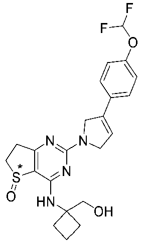 | 15 | 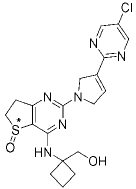 | 16 | 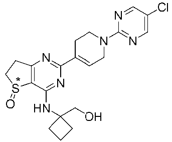 |
| 17 | 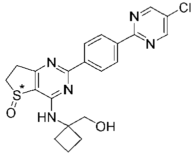 | 18 | 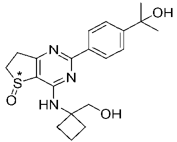 | 19 | 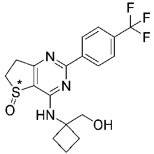 | 20 | 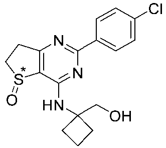 |
| 21 | 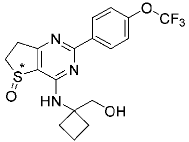 | 22 | 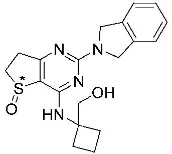 | 23 | 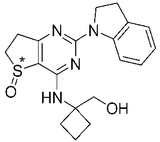 | 24 | 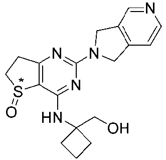 |
| 25 | 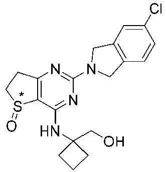 | 26 | 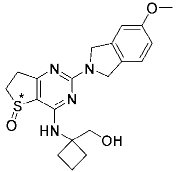 | 27 | 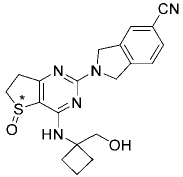 | 28 | 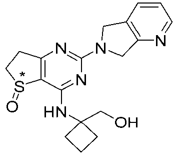 |
| 29 | 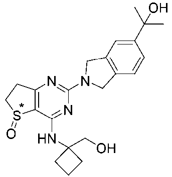 | 30 | 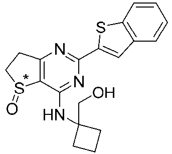 | 31 | 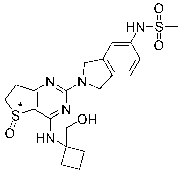 | 32 | 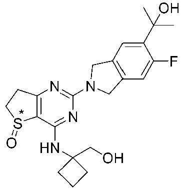 |
| 33 | 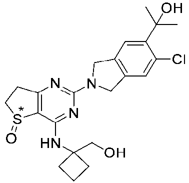 | 34 | 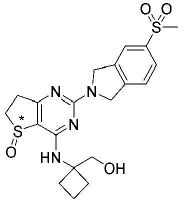 | 35 | 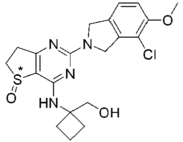 | 36 | 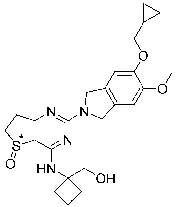 |
| 37 | 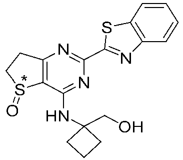 | 38 | 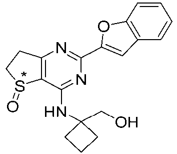 | 39 | 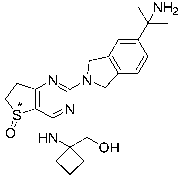 | 40 | 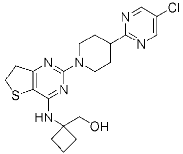 |
| 41 | 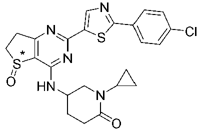 | 42 | 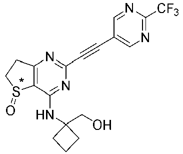 | 43 | 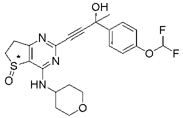 | 44 | 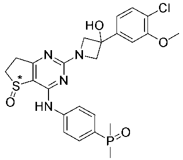 |
| 45 | 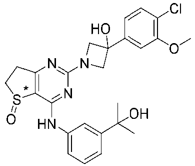 | 46 | 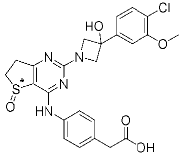 | 47 | 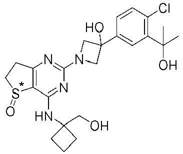 | 48 | 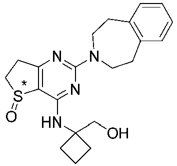 |
| 49 | 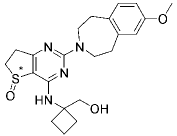 | 50 | 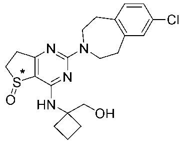 | 51 | 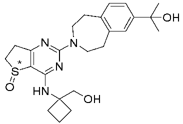 | 52 | 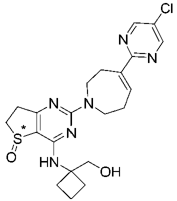 |
| 53 | 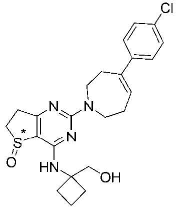 | 54 | 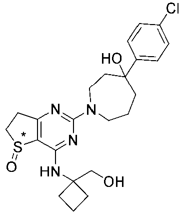 | 55 | 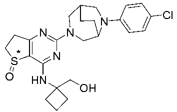 | 56 | 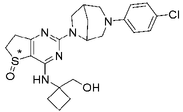 |
| 57 | 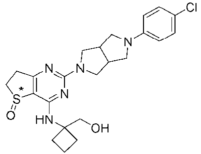 | 58 | 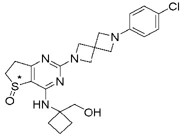 | 59 | 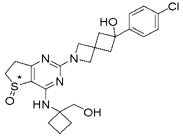 | 60 | 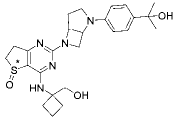 |
| 61 | 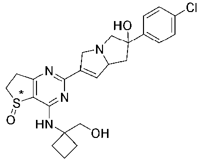 | 62 | 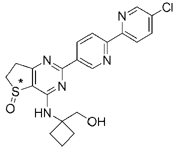 | 63 | 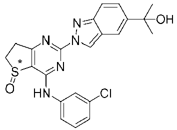 | 64 | 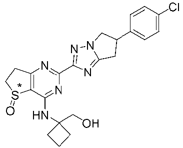 |
| 65 | 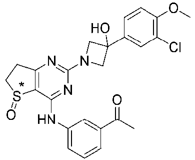 | 66 | 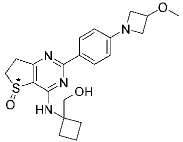 | 67 | 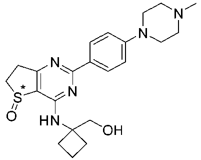 | 68 | 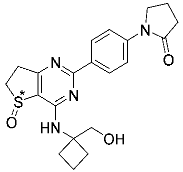 |
| 69 | 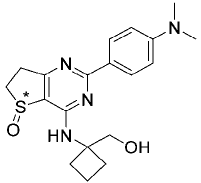 | 70 | 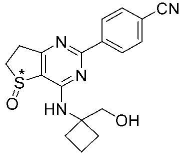 | 71 | 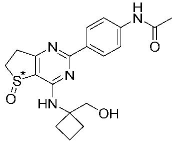 | 72 | 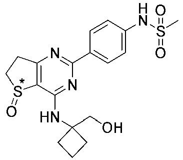 |
| 73 | 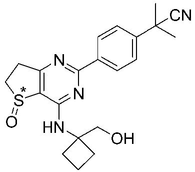 | 74 | 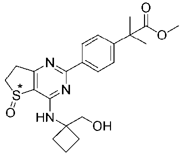 | 75 | 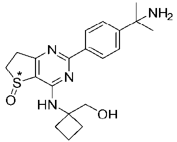 | 76 | 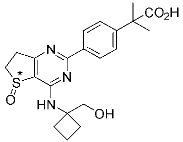 |
| 77 | 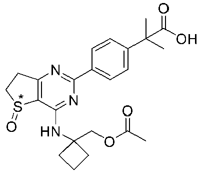 | 78 | 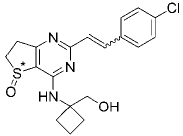 | 79 | 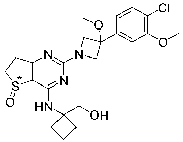 | 80 | 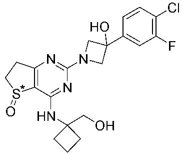 |
| 81 | 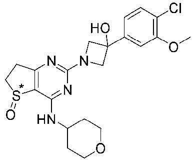 | 82 | 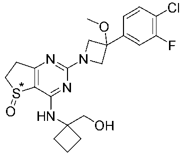 | 83 | 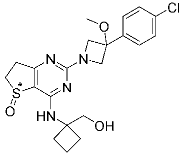 | 84 | 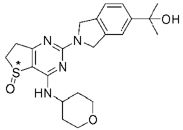 |
| 85 | 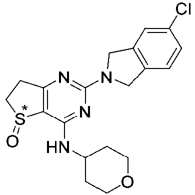 | 86 | 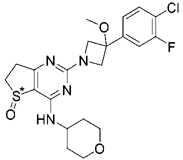 | 87 | 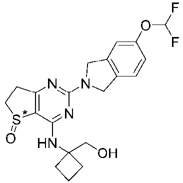 | 88 | 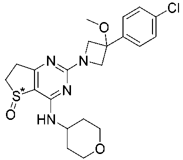 |
| 89 | 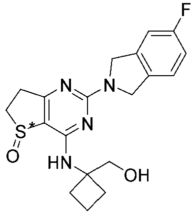 | 90 | 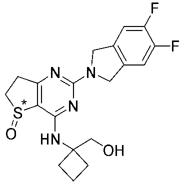 | 91 | 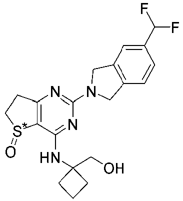 | 92 | 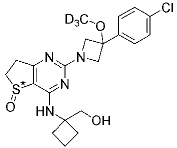 |
| 93 | 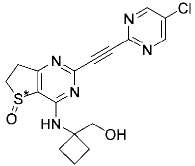 | 94 | 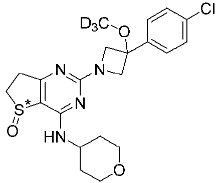 | 95 | 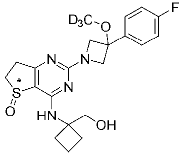 | 96 | 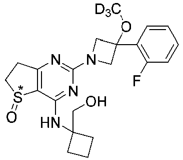 |
| 97 | 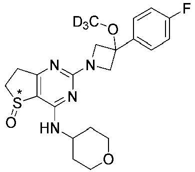 | 98 | 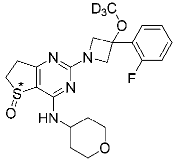 | 99 | 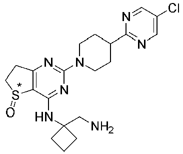 | 100 | 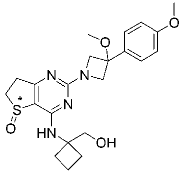 |
| 101 | 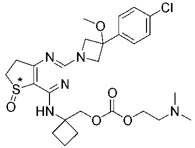 | 102 | 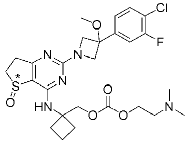 | 103 | 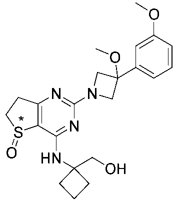 | 104 | 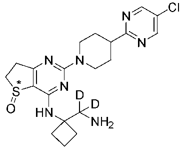 |
| 105 | 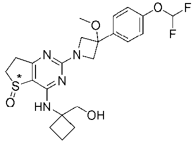 | 106 | 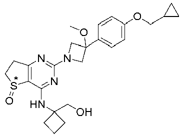 | 107 | 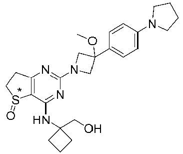 | 108 | 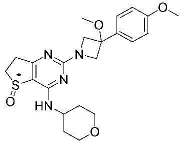 |
| 109 | 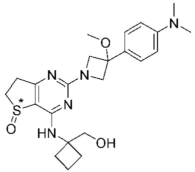 | 110 | 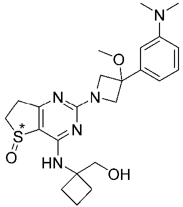 | 111 | 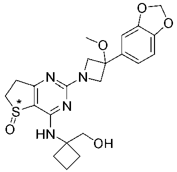 | 112 | 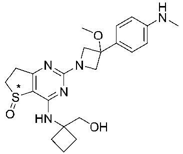 |

13. A pharmaceutical composition, which contains a therapeutically effective amount of the compound according to any one of claims 1-12, a stereoisomer or tautomer of said compound, a mixture thereof, or a pharmaceutically acceptable salt of said compound.

14. Use of the compound according to any one of claims 1-12, a stereoisomer or tautomer of said compound, a mixture thereof, or a pharmaceutically acceptable salt of said compound, or the pharmaceutical composition according to claim 13, in the manufacture of a medicament for treating and/or preventing a PDE4B-mediated disease, preferably said disease includes respiratory disease, gastrointestinal disease, inflammatory disease, allergic disease, autoimmune disease or cancer, for example:
said respiratory disease is selected from respiratory or pulmonary diseases accompanied by increased mucus production, airway inflammation and/or obstructive diseases; further preferably, said respiratory disease is selected from idiopathic pulmonary fibrosis, progressive pulmonary fibrosis, interstitial pneumonia, chronic obstructive pulmonary disease (COPD), alpha-1 antitrypsin deficiency, chronic rhinosinusitis, asthma or chronic bronchitis;
said gastrointestinal disease is selected from regional ileitis, ulcerative colitis or Crohn's disease;
said inflammatory disease is selected from dry eye syndrome or glaucoma;
said autoimmune disease is selected from atopic dermatitis, seborrheic dermatitis, psoriasis, urticaria, multiple sclerosis and diffuse connective tissue diseases such as systemic lupus erythematosus, rheumatoid arthritis, dermatomyositis, polymyositis, vasculitis, and Sjogren's syndrome.

15. The compound according to any one of claims 1-12, a stereoisomer or tautomer of said compound, a mixture thereof, or a pharmaceutically acceptable salt of said compound for use in treating and/or preventing a PDE4B-mediated disease, preferably said disease includes respiratory disease, gastrointestinal disease, inflammatory disease, allergic disease, autoimmune disease or cancer, for example:
said respiratory disease is selected from respiratory or pulmonary diseases accompanied by increased mucus production, airway inflammation and/or obstructive diseases; further preferably, said respiratory disease is selected from idiopathic pulmonary fibrosis, progressive pulmonary fibrosis, interstitial pneumonia, chronic obstructive pulmonary disease (COPD), alpha-1 antitrypsin deficiency, chronic rhinosinusitis, asthma or chronic bronchitis;
said gastrointestinal disease is selected from regional ileitis, ulcerative colitis or Crohn's disease;
said inflammatory disease is selected from dry eye syndrome or glaucoma;
said autoimmune disease is selected from atopic dermatitis, seborrheic dermatitis, psoriasis, urticaria, multiple sclerosis and diffuse connective tissue diseases such as systemic lupus erythematosus, rheumatoid arthritis, dermatomyositis, polymyositis, vasculitis, and Sjogren's syndrome.

16. A method for treating and/or preventing a PDE4B-mediated disease, which method comprises administering to a subject in need thereof a therapeutically effective amount of the compound according to any one of claims 1-12, a stereoisomer or tautomer of said compound, a mixture thereof, or a pharmaceutically acceptable salt of said compound, or the pharmaceutical composition according to claim 13, preferably said disease includes respiratory disease, gastrointestinal disease, inflammatory disease, allergic disease, autoimmune disease or cancer, for example:
said respiratory disease is selected from respiratory or pulmonary diseases accompanied by increased mucus production, airway inflammation and/or obstructive diseases; further preferably, said respiratory disease is selected from idiopathic pulmonary fibrosis, progressive pulmonary fibrosis, interstitial pneumonia, chronic obstructive pulmonary disease (COPD), alpha-1 antitrypsin deficiency, chronic rhinosinusitis, asthma or chronic bronchitis;
said gastrointestinal disease is selected from regional ileitis, ulcerative colitis or Crohn's disease;
said inflammatory disease is selected from dry eye syndrome or glaucoma;
said autoimmune disease is selected from atopic dermatitis, seborrheic dermatitis, psoriasis, urticaria, multiple sclerosis and diffuse connective tissue diseases such as systemic lupus erythematosus, rheumatoid arthritis, dermatomyositis, polymyositis, vasculitis, and Sjogren's syndrome.
